(19) Europäisches Patentamt
European Patent Office
Office européen des brevets

(11) EP 2 244 731 B1

(12) **EUROPEAN PATENT SPECIFICATION**

(45) Date of publication and mention of the grant of the patent:
**10.05.2017 Bulletin 2017/19**

(21) Application number: **09706780.5**

(22) Date of filing: **27.01.2009**

(51) Int Cl.:
*A61K 39/145* (2006.01) *A61K 39/00* (2006.01)
*A61P 37/00* (2006.01)

(86) International application number:
**PCT/US2009/032154**

(87) International publication number:
**WO 2009/097291 (06.08.2009 Gazette 2009/32)**

(54) **CANINE INFLUENZA VACCINES**

IMPFSTOFF GEGEN HUNDEGRIPPE

VACCINS CONTRE LA GRIPPE CANINE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **28.01.2008 US 20656**

(43) Date of publication of application:
**03.11.2010 Bulletin 2010/44**

(73) Proprietor: **Merial, Inc.**
**Duluth, GA 30096 (US)**

(72) Inventors:
- **MINKE, Jules, Maarten**
  **F-69960 Corbas (FR)**
- **KARACA, Kemal**
  **Overpark, KS 66221 (US)**
- **YAO, Jiansheng**
  **North York, Ontario M2H 3H1 (CA)**

(74) Representative: **D Young & Co LLP**
**120 Holborn**
**London EC1N 2DY (GB)**

(56) References cited:
**WO-A-2007/024947 US-A1- 2008 241 184**

- **KARACA KEMAL ET AL: "EVALUATION OF THE ABILITY EQUINE INFLUENZA VIRUS OF CANARYPOX-VECTORED VACCINES TO INDUCE HUMORAL IMMUNE RESPONSES AGAINST CANINE INFLUENZA VIRUSES IN DOGS" AMERICAN JOURNAL OF VETERINARY RESEARCH, AMERICAN VETERINARY MEDICINE ASSOCIATION, US, vol. 68, no. 2, 1 February 2007 (2007-02-01), pages 208-212, XP009081173 ISSN: 0002-9645**
- **CRAWFORD P C ET AL: "Transmission of Equine influenza virus to dogs" SCIENCE, AMERICAN ASSOCIATION FOR THE ADVANCEMENT OF SCIENCE, US, WASHINGTON, DC, vol. 310, no. 5747, 21 October 2005 (2005-10-21), pages 482-485, XP003003531 ISSN: 0036-8075**
- **PFEIFFER JENNIFER ET AL: "Efficacy of Commercial Vaccines in Protecting Chickens and Ducks Against H5N1 Highly Pathogenic Avian Influenza Viruses from Vietnam", AVIAN DISEASES, vol. 54, no. 1, Suppl. S, March 2010 (2010-03), pages 262-271,**

Remarks:
The file contains technical information submitted after the application was filed and not included in this specification

EP 2 244 731 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## FIELD

The present description encompasses influenza vaccines, in particular canine influenza vaccines. The vaccine may be a recombinant poxvirus vaccine or an inactivated vaccine.

## BACKGROUND OF THE INVENTION

Respiratory diseases resembling influenza have infected thousands of dogs in the U.S. Recurrent outbreaks of severe respiratory disease characterized by coughing and fever have occurred in greyhounds at racing kennels. Pathological findings included severe pulmonary and plural hemorrhage, accurate to subacute erosive to hyperplastic tracheitis, bronchitis and bronchiolitis and bronchopneumonia. In 2004, eight greyhounds in Jacksonville, Florida were killed by an equine influenza virus that jumped the species barrier from horses to dogs.

Equine influenza is a disease of horses, and the virus is in the same group of viruses that cause flu in people. The disease is present in horse populations throughout Europe, North America and parts of Asia, with horses typically developing a fever and a dry hacking cough. In the early stages of the disease, horses are reluctant to eat or drink for several days, but usually recover in two to three weeks.

H3N8 subtypes of equine influenza were previously isolated from lungs of two dogs from Florida and one dog fromTexas who had died from the infection. Genetic sequence analyses and phylogenetic comparisons determined that all three canine isolates were closely related and evolved from contemporary strains of equine influenza H3N8. Immunohistochemistry demonstrated influenza antigen in bronchial gland epithelial cells, bronchial and bronchialar epithelial cells and in alveolar macrophages. Seroconversion to canine influenza virus was demonstrated by hemagglutination inhibition and microneutralization assays.

Molecular and antigenic analyses of three influenza viruses isolated from outbreaks of severe respiratory disease in racing greyhounds revealed that they are closely related to H3N8 equine influenza virus (see, e.g., Crawford et al., Science. 2005 Oct 21;310(5747):482-5. Epub 2005 Sep 26). Phylogenetic analysis indicated that the canine influenza virus genomes form a monophyletic group, consistent with a single interspecies virus transfer. Molecular changes in the hemagglutinin suggested adaptive evolution in the new host. The etiologic role of this virus in respiratory disease was supported by the temporal association of rising antibody titers with disease and by experimental inoculation studies. The geographic expansion of the infection and its persistence for several years indicate efficient transmission of canine influenza virus among greyhounds. Evidence of infection in pet dogs suggests that this infection may also become enzootic in this population.

In 2005, a mutated form of the Bird Flu (H3N8 Virus) was reported to have killed greyhounds in Massachusetts. The document WO2007024947 describes a method of eliciting an immune response against influenza in a canine, comprising administering a formulation comprising an avipox expression vector comprising a polynucleotide encoding an influenza antigen and a pharmaceutically or veterinarily acceptable carrier, excipient or vehicle in an effective amount for eliciting an immune response.

The document by Karaca K et al (2007), American Journal of Veterinary Research, vol.68, pp.208-212 describes an evaluation of the ability of canarypox-vectored equine influenza virus vaccines to induce humoral immune responses against canine influenza viruses in dogs.

Accordingly, there is a need for an effective vaccine against influenza in canines.

## SUMMARY OF THE INVENTION

The invention in its broadest sense is as defined in the independent claims.

The present invention relates to a *Canidae* vaccine for use in reducing the duration of influenza viral shedding in the nasal cavity in a canine, wherein said vaccine comprises an effective amount of an expression vector that contains and expresses *in vivo* in a canine a polynucleotide encoding equine influenza antigen comprising equine influenza H3 operatively linked to a promoter and optionally to an enhancer, and a pharmaceutically or veterinarily acceptable carrier, excipient, or vehicle.

The present description encompasses canine influenza vaccines, which may be a recombinant canine influenza vaccine.

In an embodiment wherein the canine influenza vaccine is a recombinant vaccine, advantageously, the vector is an avipox expression vector.

In an advantageous embodiment, the equine influenza H3 antigen, epitope or immunogen, is derived from a canine infected with influenza. For example, but not by limitation, influenza virus may be isolated from the broncho alveolar lavage and/or lung tissues of an affected dog. Isolation and characterization of the nucleotide sequence of the

influenza virus inflecting the dog may be done by routine experimentation by a person of ordinary skill in the art.

The equine influenza H3 antigen, epitope or immunogen may be isolated from an equine influenza. The equine influenza may be an Ohio equine influenza, Kentucky equine influenza or a Newmarket equine influenza.

The avipox expression vector may be an attenuated avipox expression vector. In one embodiment, the avipox expression vector may be a canarypox vector, advantageously ALVAC. The canarypox vector may be CP 2242, CP1529 or CP1533 or expressing the H3 of a canine influenza isolate vCP2328)

The present description also encompasses an inactivated influenza vaccine. The inactivated influenza vaccine may be an inactivated canine influenza. In another embodiment, the inactivated influenza vaccine may be an equine influenza, advantageously an Ohio equine influenza, Kentucky equine influenza or a Newmarket equine influenza. The vaccine may be inactivated with formalin or beta-propiolactone.

The description also relates to method of eliciting an immune response against influenza in a canine, which may comprise administering a formulation comprising any one of the above recombinant influenza vaccine or inactivated vaccine and a pharmaceutically or veterinarily acceptable carrier, excipient or vehicle in an effective amount for eliciting an immune response. In an advantageous embodiment, an adjuvant may be added. The adjuvant may be aluminum hydroxide, alumimum phosphate, a carbomer or an oil-water-emulsion and optionally may comprise CpG. Advantageously, the administration may be subcutaneous or intramuscular.

The description further relates to method of inducing an immune response against influenza in a canine, which may comprise administering a formulation comprising any one of the above recombinant influenza vaccine or inactivated vaccine and a pharmaceutically or veterinarily acceptable carrier, excipient or vehicle in an effective amount for inducing an immune response. In an advantageous embodiment, an adjuvant may be added. The adjuvant may be aluminum hydroxide, alumimum phosphate, a carbomer or an oil-water-emulsion, immunostimulating complex (ISCOM), and optionally may comprise CpG. Advantageously, the administration may be subcutaneous or intramuscular.

The description further encompasses a kit for performing a method of eliciting or inducing an immune response which may comprise any one of the recombinant influenza vaccines or inactivated vaccines and instructions for performing the method.

It is noted that in this disclosure and particularly in the claims and/or paragraphs, terms such as "comprises", "comprised", "comprising" can mean "includes", "included", "including".

These and other embodiments are disclosed by the following Detailed Description.

## BRIEF DESCRIPTION OF THE DRAWINGS

The following detailed description, given by way of example, but not intended to limit the invention solely to the specific embodiments described, may best be understood in conjunction with the accompanying drawings, in which:

FIG. 1 illustrates the sequence of the insert in PJT004 (SEQ ID NO: 1);

FIG. 2 illustrates the sequence of the insert in PJT005 (SEQ ID NO: 2);

FIG. 3 illustrates a comparison of the amino acid sequence of EIV Ohio 03 strain HA to that of New Market strain H3 HA (SEQ ID NOS: 3 and 4);

FIG. 4A illustrates a construction of an ALVAC donor plasmid for generation of an ALVAC recombinant expressing codon optimized EIV H3 HA (Ohio 03);

FIG. 4B illustrates pALVAC C5 H6p-synthetic EIV H3 HA, pJY1571.1;

FIG. 5A illustrates a predicted amino acid sequence of product(s): EIV H3 HA;

FIG. 5B illustrates a nucleotide sequence of arms and insert with translation

FIG. 6 illustrates a vCP2242 Western blot analysis. A 1/1,000 dilution of pooled anti-EIV antibody was used for the analysis. Lane 1: 5 μl Fermentas Prestain protein marker, lane 2: 15 μl ALVAC cell pellet, lane 3: 15 μl vCP2242. cell pellet, lane 4: 15 μl vCP2242. cell pellet, lane 5: 15 μl vCP1533 cell pellet, lane 6: space, lane 7: 40 μl ALVAC supernatant, lane 8: 40 μl vCP2242. supernatant, lane 9: 40 μl vCP2242. supernatant and lane 10: 40 μl vCP1533 supernatant.

FIG. 7 illustrates the HI antibody responses to canine influenza virus (CIV) in dogs vaccinated with canarypox-vectored equine influenza virus (EIV) vaccines expressing either the hemagglutinin gene of A/equine/Kentucky/94 (vCP1529) or the hemagglutin gene of A2/equine/Ohio/03 (vCP2242). vCP2242 was administered by the subcutaneous (SC in FIG. 7) or the transdermal route (TD in FIG. 7) whereas vCP1529 was administered subcutaneously (SC in FIG. 7).

FIG. 8 illustrates the mean viral titers found in nasal swabs in control animals and vaccinated animals following intranasal challenge with influenza A/canine/NY/110659. Data =log10 virus isolation titers on MDCK cells.

FIG. 9 illustrates the mean body temperatures of control dogs and of dogs vaccinated subcutaneously twice at a 21-day interval with the hemagglutin gene of A2/equine/Ohio/03 (vCP2242) followed by challenge at day 43 with CIV-NY05 isolate (influenza A/canine/NY/110659).

FIG. 10 illustrates the mean body temperatures of control dogs (CON) and of dogs vaccinated subcutaneously twice at a 21-day interval with the hemagglutin gene of A2/equine/Ohio/03 (vCP2242) (VAC) followed by challenge at day 43 with CIV-NY05 isolate on days 42, 44, and 45. This figure is a box plot that has the mean (symbolized as a +) and the median (horizontal line), the 25$^{th}$ and 75$^{th}$ percentile (bottom and top of the box), minimum and maximum observations (whiskers), and outliers (small squares).

FIG. 11 illustrates the HI antibody responses to CIV HA antigen of A2/equine/Ohio/03 (vCP2242) followed by challenge at day 43 with CIV-NY05 isolate at three weeks post second vaccination and two weeks post challenge (Vac57), and in control dogs at two weeks post challenge (Cont57).

FIG. 12 illustrates the protective immune response of vCP2242 as characterized by the decreased time of virus shedding in the nasal cavity of dogs vaccinated subcutaneously twice at a 21-day interval with the hemagglutin gene of A2/equine/Ohio/03 (vCP2242) followed by challenge at day 43 with CIV-NY05 isolate as compared to control dogs.

FIG. 13 illustrates the the protective immune response of vCP2242 as characterized by the decreased duration of virus shedding in the nasal cavity of dogs vaccinated subcutaneously twice at a 21-day interval with the hemagglutin gene of A2/equine/Ohio/03 (vCP2242) followed by challenge at day 43 with CIV-NY05 isolate as compared to control dogs.

## DETAILED DESCRIPTION

The invention in its broadest sense is as defined in the independent claims.

The present description is based, in part, on Applicants' studies demonstrating a recombinant canarypox expressing equine influenza HA is immunogenic in dogs.

The present description encompasses any influenza antigen, epitope or immunogen that elicits an immunogenic response in an animal, advantageously a vertebrate, more advantageously a dog. The influenza antigen, epitope or immunogen may be any influenza antigen, epitope or immunogen, such as, but not limited to, a protein, peptide or fragment thereof, that elicits, induces or stimulates a response in an animal, advantageously a vertebrate, more advantageously a dog.

In an advantageous embodiment, the canine influenza antigen, epitope or immunogen is derived from a canine infected with influenza. For example, but not by limitation, influenza virus may be isolated from the broncho alveolar lavage and/or lung tissues of an affected dog. Isolation and characterization of the nucleotide sequence of the influenza infecting the dog may be done by routine experimentation by a person of ordinary skill in the art.

In another advantageous embodiment, the canine influenza, antigen, epitope or immunogen may be derived from an equine infected with influenza or an equine influenza strain. Advantageously, the equine influenza strain is an Ohio equine influenza, Kentucky equine influenza strain or a Newmarket equine influenza strain. The canine influenza antigen, epitope or immunogen may be determined by one of ordinary skill of the art from the nucleotide sequences of Examples 4 and 5. Advantageously, the canine influenza antigen, epitope or immunogen is a hemagglutinin (HA) (e.g., HA precursor, H1, H2, protein, matrix protein (e.g., matrix protein M1 or M2), neuraminidase, nonstructural (NS) protein (e.g., NS1 or NS2), nucleoprotein (NP) and polymerase (e.g., PA polymerase, PB 1 polymerase 1 or PB2 polymerase 2).

Examples of Kentucky equine influenza strains that may be used in methods of the present description include, but are not limited to, equine influenza strains A/eq/Kentucky/98 (see, e.g., Crouch et al., Vaccine. 2004 Dec 2;23(3):418-25), A/Equi 2 (Kentucky 81) (see, e.g., Short et al., J Vet Pharmacol Ther. 1986 Dec;9(4):426-32, Horner & Ledgard, N Z Vet J. 1988 Dec;36(4):205-6), A/equine/Kentucky/1/81 (Eq/Ky) (see, e.g., Breathnach et al., Vet Immunol Immunopathol. 2004 Apr;98(3-4):127-36), A/Equine/Kentucky/1/81 (H3N8) (see, e.g., Olsen et al., Vaccine. 1997 Jul;15(10):1149-56, Morley et al. Vet Microbiol. 1995 Jun;45(1):81-92, Ozaki et al., Vet Microbiol. 2001 Sep 20;82(2): 111-9, Sugiura et al., J Virol Methods. 2001 Oct;98(1):1-8, see, e.g., Sugiura et al., J Virol Methods. 2001 Oct;98(1):1-8, Goto et al., J Vet Med Sci. 1993 Feb;55(1):33-7, Goto et al., J Vet Med Sci. 1993 Feb;55(1):33-7), A/Equine/Kentaclcy/l/91 (H3N8) (see, e.g., Youngner et al., Am J Vet Res. 2001 Aug;62(8):1290-4), A/Equine/Kentucky/1277/90 (Eq/Kentucky) (see, e.g., Webster & Thomas, Vaccine. 1993;11(10):987-93), A/Equine/Kentucky/2/91 (H3N8) (see, e.g., Donofrio et al., J Vet Diagn Invest. 1994 Jan;6(1):39-43), A/Equine/Kentucky/79 (H3N8) (see, e.g., Donofrio et al., J Vet Diagn Invest. 1994 Jan;6(1):39-43), A/equine/Kentucky/81 (see, e.g., Sugiura et al., J Virol Methods. 2001 Oct;98(1):1-8), A/equine/Kentucky/91 (H3N8) (see, e.g., Gross et al., Equine Vet J. 1998 Nov;30(6):489-97), A/equine-2/Kentucky/95 (H3N8) (see, e.g., Heldens et al., Vet J. 2004 Mar;167(2):150-7) and A/equine-2/Kentucky/98 (see, e.g., Chambers et al., Equine Vet J. 2001 Nov;33(7):630-6).

Examples of Newmarket equine influenza strains that may be used in methods of the present description include, but are not limited to, equine influenza strains A/eq/Newmarket/1/77 (see, e.g., Lindstrom et al., Arch Virol. 1998;143(8):1585-98), A/eq/Newmarket/5/03 (see, e.g., Edlund Toulemonde et al., Vet Rec. 2005 Mar 19;156(12):367-71), A/Equi 2 (H3N8), Newmarket 1/93 (see, e.g., Mohler et al., Biotechnol Bioeng. 2005 Apr 5;90(1):46-58, Nayak et al., J Chromatogr B Analyt Technol Biomed Life Sci. 2005 Jul 8), A/equi-2/Newmarket-1/93

(see, e.g., Heldens et al., J Immunol Methods. 2002 Jun 1;264(1-2):11-7), A/equine/Newmarket/2/93 (see, e.g., Wattrang et al., Viral Immunol. 2003;16(1):57-67), A/equine/Newmarket/79 (H3N8) (see, e.g., Duhaut & Dimmock. Virology. 2000 Sep 30;275(2):278-85, Noble & Dimmock, J Gen Virol. 1994 Dec;75 (Pt 12):3485-91, Duhaut & Dimmock, Virology. 1998 Sep 1;248(2):241-53, Hannant & Mumford, Vet Immunol Immunopathol. 1989 Jul;21(3-4):327-37, Hannant et al., Vet Microbiol. 1989 Apr;19(4):293-303, Hannant et al., Vet Rec. 1988 Feb 6;122(6):125-8, Richards et al., Vet Immunol Immunopathol. 1992 Jun;33(1-2):129-43, Heldens et al., Vet J. 2004 Mar;167(2):150-7), A/equine/Newmarket/1/77 (H7N7) (see, e.g., Goto et al., J Vet Med Sci. 1993 Feb;55(1):33-7, Sugiura et al., J Virol Methods. 2001 Oct;98(1):1-8, Sugiura et al., J Virol Methods. 2001 Oct;98(1):1-8) and A/equine-2/Newmarket-2/93 (see, e.g., Heldens et al., Vet J. 2004 Mar;167(2):150-7).

The present description also encompasses other equine influenza viruses, such as, but not limited to, equine influenza virus A/eq/Miami/63 (H3N8) (see, e.g., van Maanen et al., Vet Microbiol. 2003 Jun 10;93(4):291-306), A/equi 1 (Prague strain) (see, e.g., Homer & Ledgard, N Z Vet J. 1988 Dec;36(4):205-6, Short et al., J Vet Pharmacol Ther. 1986 Dec;9(4):426-32), A/Equi 2 (Miami) (see, e.g., Short et al., J Vet Pharmacol Ther. 1986 Dec;9(4):426-32), A/equi-1/Prague/56 (Pr/56) (see, e.g., Heldens et al., J Immunol Methods. 2002 Jun 1;264(1-2):11-7), A/equi-2/Suffolk/89 (Suf/89) (see, e.g., Heldens et al., J Immunol Methods. 2002 Jun 1;264(1-2):11-7), A/Equine 2/Sussex/89 (H3N8) (see, e.g., Mumford et al., Vet Rec. 1994 Feb 12;134(7):158-62), A/equine/Sussex/89 (see, e.g., Wattrang et al., Viral Immunol. 2003;16(1):57-67), A/equine-2/Saskatoon/90 (see, e.g., Chambers et al., Equine Vet J. 2001 Nov;33(7):630-6), A/Equine/Prague/1/56 (H7N7) (see, e.g., Donofrio et al., J Vet Diagn Invest. 1994 Jan;6(1):39-43, Morley et al, Vet Microbiol. 1995 Jun;45(1):81-92),, A/equine/Miami/1/63 (H3N8) (see, e.g., Morley et al. Vet Microbiol. 1995 Jun;45(1):81-92, Ozaki et al., Vet Microbiol. 2001 Sep 20;82(2):111-9, Thomson et al., Vet Rec. 1977 May 28;100(22):465-8, Mumford et al., Epidemiol Infect. 1988 Jun;100(3):501-10, Donofrio et al., J Vet Diagn Invest. 1994 Jan;6(1):39-43, Mumford et al., J Hyg (Lond). 1983 Jun;90(3):385-95), A/Aichi/2/68 (H3N2) (see, e.g., Ozaki et al., Vet Microbiol. 2001 Sep 20;82(2): 111-9), A/equine/Tokyo/2/71 (H3N8) (see, e.g., Goto et al., J Vet Med Sci. 1993 Feb;55(1):33-7), A/eq/LaPlata/1/88 (see, e.g., Lindstrom et al., Arch Virol. 1998;143(8):1585-98), A/Equine/Jilin/1/89 (Eq/Jilin) (see, e.g., Webster & Thomas, Vaccine. 1993;11(10):987-93), A/Equine/Alaska/1/91 (H3N8) (see, e.g., Webster & Thomas, Vaccine. 1993;11(10):987-93), A/equine/Saskatoon/1/91 (H3N8) (see, e.g., Morley et al. Vet Microbiol. 1995 Jun;45(1):81-92), A/equine/Rome/5/91 (H3N8) (see, e.g., Sugiura et al., J Virol Methods. 2001 Oct;98(1):1-8), A/equine/La Plata/1/93 (H3N8) (see, e.g., Ozaki et al., Vet Microbiol. 2001 Sep 20;82(2):111-9), A/equine/La Plata/1/93 (LP/93) (see, e.g., Sugiura et al., J Virol Methods. 2001 Oct;98(1):1-8), A/eq/Holland/1/95 (H3N8) (see, e.g., van Maanen et al., Vet Microbiol. 2003 Jun 10;93(4):291-306) and A/eq/Holland/2/95 (H3N8) (see, e.g., van Maanen et al., Vet Microbiol. 2003 Jun 10;93(4):291-306).

In another advantageous embodiment, the canine influenza, antigen, epitope or immunogen may be derived from an equine infected with influenza or an equine influenza strain derived from a recent isolate.

The influenza antigen, epitope or immunogen may also be isolated from any influenza strain such as, but not limited to, avian H5N1 influenza virus, A/Hong Kong/156/97 (A/HK/156/97) (see, e.g., Leneva et al., Antimicrob Agents Chemother. 2001 Apr;45(4):1216-24), avian H7N1 influenza strain (see, e.g., Foni et al., New Microbiol. 2005 Jan;28(1):31-5), avian H9N2 influenza virus (see, e.g., Leneva et al., Antimicrob Agents Chemother. 2001 Apr;45(4):1216-24), avian influenza virus A/Chicken/HK/G9/97 (H9N2) (see, e.g., Leneva et al., Antimicrob Agents Chemother. 2001 Apr;45(4):1216-24), avian influenza virus A/Quail/HK/G1/97 (H9N2) (see, e.g., Leneva et al., Antimicrob Agents Chemother. 2001 Apr;45(4):1216-24), avian influenza virus A/Teal/HK/W312/97 (H6N1) (see, e.g., Leneva et al., Antimicrob Agents Chemother. 2001 Apr;45(4):1216-24), avian pandemic influenza A viruses of avian origin (see, e.g., Audsley & Tannock, Expert Opin Biol Ther. 2004 May;4(5):709-17), cold-adapted (ca) and temperature sensitive (ts) master donor strain, A/Leningrad/134/17/57 (H2N2) (see, e.g., Youil et al., Virus Res. 2004 Jun 15;102(2):165-76), equine influenza virus (A/Equi 2 (H3N8), Newmarket 1/93) (see, e.g., Mohler et al., Biotechnol Bioeng. 2005 Apr 5;90(1):46-58; Nayak et al., J Chromatogr B Analyt Technol Biomed Life Sci. 2005 Jul 8), equine-2 influenza virus (EIV; subtype H3N8) (see, e.g., Virology. 2001 Aug 15;287(1):202-13), equine-2 influenza virus, A/Equine/Kentucky/1/91 (H3N8) (see, e.g., Youngner et al., Am J Vet Res. 2001 Aug;62(8):1290-4), human influenza virus A(H3N2) isolates (see, e.g., Abed et al., J Infect Dis. 2002 Oct 15;186(8):1074-80), human influenza virus A/Memphis/1/71 (H3N2) (see, e.g., Suzuki et al., Biochem J. 1996 Sep 1;318 (Pt 2):389-93), human influenza virus A/Nanchang/933/95 (H3N2) virus (see, e.g., Scholtissek et al., J Virol. 2002 Feb;76(4):1781-6), human influenza virus A/PR/8/34 (H1N1) virus (see, e.g., Scholtissek et al., J Virol. 2002 Feb;76(4):1781-6), human influenza virus A/Singapore/57 (H2N2) virus (see, e.g., Scholtissek et al., J Virol. 2002 Feb;76(4):1781-6), influenza virus A (see, e.g., Chare et al., J Gen Virol. 2003 Oct;84(Pt 10):2691-703), influenza virus A PR/8/34 (PR8) virus (H1N1 subtype) (see, e.g., Mantani et al., Planta Med. 2001 Apr;67(3):240-3), influenza virus A/Aichi/2/68(H3N2) (see, e.g., Miyamoto et al., Antiviral Res. 1998 Aug;39(2):89-100), influenza virus A/Ann Arbor/6/60 cold-adapted virus (see, e.g., Treanor et al., J Virol. 1994 Dec;68(12):7684-8), influenza virus A/Beijing 32/92 (H3N2) (see, e.g., Zakay-Rones et al., J Altern Complement Med. 1995 Winter;1(4):361-9), influenza virus A/Charlottesville/31/95 (H1N1) (see, e.g., Gubareva et al., J Gen Virol. 2002 Nov;83(Pt 11):2683-92), influenza virus A/Kawasaki/86 (H1N1) virus (see, e.g., Staschke et al., Virology. 1998 Sep 1;248(2):264-74), influenza virus

A/Korea/82 (H3N2) (see, e.g., Treanor et al., J Virol. 1994 Dec;68(12):7684-8), influenza virus A/Leningrad/134/57 (see, e.g., Egorov et al., J Virol. 1998 Aug;72(8):6437-41), influenza virus A/NWS/33 (H1N1) (see, e.g., Sidwell et al., Antiviral Res. 1998 Feb;37(2):107-20), influenza virus A/PR/8/34(H1N1) (see, e.g., Miyamoto et al., Antiviral Res. 1998 Aug;39(2):89-100), influenza virus A/PR8/34 (see, e.g., Nunes-Correia et al., Biochemistry. 1999 Jan 19;38(3):1095-101, Tree et al., Vaccine. 2001 May 14;19(25-26):3444-50), influenza virus A/Puerto Rico (PR)/8/34 (see, e.g., Egorov et al., J Virol. 1998 Aug;72(8):6437-41), influenza virus A/Puerto Rico/8-Mount Sinai (see, e.g., Mazanec et al., J Virol. 1995 Feb;69(2): 1339-43), influenza virus A/Shangdong 9/93 (H3N2) (see, e.g., Zakay-Rones et al., J Altern Complement Med. 1995 Winter; 1(4):361-9, Sidwell et al., Antiviral Res. 1998 Feb;37(2):107-20), influenza virus A/Shingapol/1/57(H2N2) (see, e.g., Miyamoto et al., Antiviral Res. 1998 Aug;39(2):89-100), influenza virus A/Singapore 6/86 (H1N1) (see, e.g., Zakay-Rones et al., J Altern Complement Med. 1995 Winter;1(4):361-9), influenza virus A/Singapore/1/57 (H2N2) (see, e.g., Bantia et al., Antimicrob Agents Chemother. 1998 Apr;42(4):801-7), influenza virus A/Texas 36/91 (H1N1) (see, e.g., Zakay-Rones et al., J Altern Complement Med. 1995 Winter; 1(4):361-9), influenza virus A/Texas/36/91 (H1N1) virus (see, e.g., Gubareva et al., J Infect Dis. 2001 Feb 15;183(4):523-31, Halperin et al., Vaccine. 1998 Aug;16(13):1331-5), influenza virus A/Texas/36/91(H1N1) (see, e.g., Hayden et al., Antiviral Res. 1994 Oct;25(2):123-31), influenza virus A/Udorn/72 virus infection (see, e.g., Shimizu et al., Virology. 1999 Feb 15;254(2):213-9), influenza virus A/Victoria/3/75 (H3N2) (see, e.g., Sidwell et al., Antiviral Res. 1998 Feb;37(2):107-20), influenza virus A/Virginia/88(H3N2) (see, e.g., Hayden et al., Antiviral Res. 1994 Oct;25(2):123-31), influenza virus A/WSN/33 (H1N1) (see, e.g., Lu et al., Arch Virol. 2002;147(2):273-84), influenza virus A/WSN/33 (see, e.g., Gujuluva et al., Virology. 1994 Nov 1;204(2):491-505), influenza virus B (see, e.g., Chare et al., J Gen Virol. 2003 Oct;84(Pt 10):2691-703), influenza virus B/Ann Arbor 1/86 (see, e.g., Zakay-Rones et al., J Altern Complement Med. 1995 Winter;1(4):361-9), influenza virus B/Harbin/7/94 (see, e.g., Halperin et al., Vaccine. 1998 Aug;16(13):1331-5, influenza virus B/Hong Kong/5/72 (see, e.g., Sidwell et al., Antiviral Res. 1998 Feb;37(2):107-20), influenza virus B/Lee/40 (see, e.g., Miyamoto et al., Antiviral Res. 1998 Aug;39(2):89-100), influenza virus B/Victoria group (see, e.g., Nakagawa et al., J Virol Methods. 1999 Apr;79(1): 13-20), influenza virus B/Yamagata 16/88 (see, e.g., Zakay-Rones et al., J Altern Complement Med. 1995 Winter;1(4):361-9), influenza virus B/Yamagata group (see, e.g., Nakagawa et al., J Virol Methods. 1999 Apr;79(1):113-20), influenza virus B/Yamanashi/166/98 (see, e.g., Hoffmann et al., Proc Natl Acad Sci USA. 2002 Aug 20;99(17):11411-6), influenza virus C (see, e.g., Chare et al., J Gen Virol. 2003 Oct;84(Pt 10):2691-703), influenza virus strain A/Equi/2/Kildare/89 (see, e.g., Quinlivan et al., J Clin Microbiol. 2004 Feb;42(2):759-63), influenza virus type B/Panama 45/90 (see, e.g., Zakay-Rones et al., J Altern Complement Med. 1995 Winter;1(4):361-9), live, cold-adapted, temperature-sensitive (ca/ts) Russian influenza A vaccines (see, e.g., Palker et al., Virus Res. 2004 Oct;105(2):183-94), Madin Darby Canine Kidney (MDCK)-derived cell line (see, e.g., Halperin et al., Vaccine. 2002 Jan 15;20(7-8):1240-7), mouse-adapted influenza virus A/Guizhou/54/89 (H3N2 subtype) (see, e.g., Nagai et al., Biol Pharm Bull. 1995 Feb;18(2):295-9), mouse-adapted influenza virus A/PR/8/34 (A/PR8) (see, e.g., Nagai et al., Antiviral Res. 1995 Jan;26(1):11-25), mouse-adapted influenza virus B/Ibaraki/2/85 (see, e.g., Nagai et al., Biol Pharm Bull. 1995 Feb;18(2):295-9), Russian live attenuated influenza vaccine donor strains A/Leningrad/134/17/57, A/Leningrad/134/47/57 and B/USSR/60/69 (see, e.g., Audsley & Tannock, J Virol Methods. 2005 Feb;123(2):187-93), swine H1 and H3 influenza viruses (see, e.g., Gambaryan et al., Virus Res. 2005 Jul1), swine influenza A viruses (see, e.g., Landolt et al., Am J Vet Res. 2005 Jan;66(1):119-24), swine influenza virus (SIV) (see, e.g., Clavijo et al., Can J Vet Res. 2002 Apr;66(2):117-21), swine influenza virus A/Sw/Ger 2/81 (see, e.g., Zakay-Rones et al., J Altern Complement Med. 1995 Winter;1(4):361-9), swine influenza virus A/Sw/Ger 8533/91 (see, e.g., Zakay-Rones et al., J Altern Complement Med. 1995 Winter; 1(4):361-9), turkey influenza virus A/Tur/Ger 3/91 (see, e.g., Zakay-Rones et al., J Altern Complement Med. 1995 Winter;1(4):361-9) and turkey influenza virus A/turkey/Minnesota/833/80 (H4N2) (see, e.g., Gubareva et al., J Virol. 1997 May;71(5):3385- 90).

As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a protein, a polypeptide, a peptide, an epitope, a hapten, or any combination thereof. Alternately, the immunogen or antigen may comprise a toxin or antitoxin.

The term "immunogenic protein or peptide" as used herein also refers includes peptides and polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the description comprises or consists essentially of or consists of at least one epitope or antigenic determinant. The term epitope relates to a protein site able to induce an immune reaction of the humoral type (B cells) and/or cellular type (T cells).

The term "immunogenic protein or peptide" further contemplates deletions, additions and substitutions to the sequence, so long as the polypeptide functions to produce an immunological response as defined herein. In this regard, particularly preferred substitutions will generally be conservative in nature, *i.e.,* those substitutions that take place within a family of amino acids. For example, amino acids are generally divided into four families: (1) acidic-aspartate and

glutamate; (2) basic--lysine, arginine, histidine; (3) non-polar-alanine, valine, leucine, isoleucine, proline, phenylalanine, methionine, tryptophan; and (4) uncharged polar-glycine, asparagine, glutamine, cystine, serine threonine, tyrosine. Phenylalanine, tryptophan, and tyrosine are sometimes classified as aromatic amino acids. It is reasonably predictable that an isolated replacement of leucine with isoleucine or valine, or vice versa; an aspartate with a glutamate or vice versa; a threonine with a serine or vice versa; or a similar conservative replacement of an amino acid with a structurally related amino acid, will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The terms "immunogenic" protein or polypeptide as used herein also refers to an amino acid sequence which elicits an immunological response as described above. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, *e.g.,* Epitope Mapping Protocols in Methods in Molecular Biology, Vol. 66 (Glenn E. Morris, Ed., 1996) Humana Press, Totowa, N.J. For example, linear epitopes may be determined by *e.g.*, concurrently synthesizing large numbers of peptides on solid supports, the peptides corresponding to portions of the protein molecule, and reacting the peptides with antibodies while the peptides are still attached to the supports. Such techniques are known in the art and described in, *e.g.*, U.S. Pat. No. 4,708,871; Geysen et al. (1984) Proc. Natl. Acad. Sci. USA 81:3998-4002; Geysen et al. (1986) Molec. Immunol. 23:709- 715.

Similarly, conformational epitopes are readily identified by determining spatial conformation of amino acids such as by, *e.g.*, x-ray crystallography and 2-dimensional nuclear magnetic resonance. See, *e.g.*, Epitope Mapping Protocols, *supra.* Methods especially applicable to the proteins of *T. parva* are fully described in the PCT Application Serial No. PCT/US2004/022605.

Synthetic antigens are also included within the definition, for example, polyepitopes, flanking epitopes, and other recombinant or synthetically derived antigens. See, *e.g.*, Bergmann et al. (1993) Eur. J. Immunol. 23:2777-2781; Bergmann et al. (1996) J. Immunol. 157:3242-3249; Suhrbier, A. (1997) Immunol. and Cell Biol. 75:402-408; Gardner et al. (1998) 12th World AIDS Conference, Geneva, Switzerland, Jun. 28-Jul, 3, 1998. Immunogenic fragments, for purposes of the present description, will usually include at least about 3 amino acids, preferably at least about 5 amino acids, more preferably at least about 10-15 amino acids, and most preferably 25 or more amino acids, of the molecule. There is no critical upper limit to the length of the fragment, which could comprise nearly the full-length of the protein sequence, or even a fusion protein comprising at least one epitope of the protein.

Accordingly, a minimum structure of a polynucleotide expressing an epitope is that it comprises or consists essentially of or consists of nucleotides to encode an epitope or antigenic determinant of an influenza protein or polyprotein. A polynucleotide encoding a fragment of the total protein or polyprotein, more advantageously, comprises or consists essentially of or consists of a minimum of 21 nucleotides, advantageously at least 42 nucleotides, and preferably at least 57, 87 or 150 consecutive or contiguous nucleotides of the sequence encoding the total protein or polyprotein. Epitope determination procedures, such as, generating overlapping peptide libraries (Hemmer B. et al., Immunology Today, 1998,19 (4), 163-168), Pepscan (Geysen et al., (1984) Proc. Nat. Acad. Sci. USA, 81, 3998-4002; Geysen et al., (1985) Proc. Nat. Acad. Sci. USA, 82,178-182; Van der Zee R. et al., (1989) Eur. J. Immunol., 19,43-47; Geysen H.M., (1990) Southeast Asian J. Trop. Med. Public Health, 21, 523-533; Multipin.RTM. Peptide Synthesis Kits de Chiron) and algorithms (De Groot A. et al., (1999) Nature Biotechnology, 17, 533-561), and in PCT Application Serial No. PCT/US2004/022605 can be used in the practice of the description, without undue experimentation. Other documents cited herein may also be consulted for methods for determining epitopes of an immunogen or antigen and thus nucleic acid molecules that encode such epitopes.

A "polynucleotide" is a polymeric form of nucleotides of any length, which contain deoxyribonucleotides, ribonucleotides, and analogs in any combination. Polynucleotides may have three-dimensional structure, and may perform

any function, known or unknown. The term "polynucleotide" includes double-, single-stranded, and triple-helical molecules. Unless otherwise specified or required, any embodiment of the description described herein that is a polynucleotide encompasses both the double stranded form and each of two complementary forms known or predicted to make up the double stranded form of either the DNA, RNA or hybrid molecule.

The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A polynucleotide may comprise modified nucleotides, such as methylated nucleotides and nucleotide analogs, uracyl, other sugars and linking groups such as fluororibose and thiolate, and nucleotide branches. The sequence of nucleotides may be further modified after polymerization, such as by conjugation, with a labeling component. Other types of modifications included in this definition are caps, substitution of one or more of the naturally occurring nucleotides with an analog, and introduction of means for attaching the polynucleotide to proteins, metal ions, labeling components, other polynucleotides or solid support. The polynucleotides can be obtained by chemical synthesis or derived from a microorganism.

The description further comprises a complementary strand to a polynucleotides encoding an influenza antigen, epitope or immunogen. The complementary strand can be polymeric and of any length, and can contain deoxyribonucleotides, ribonucleotides, and analogs in any combination.

The terms "protein", "peptide", "polypeptide" and "polypeptide fragment" are used interchangeably herein to refer to polymers of amino acid residues of any length. The polymer can be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

An "isolated" polynucleotide or polypeptide is one that is substantially free of the materials with which it is associated in its native environment. By substantially free, is meant at least 50%, advantageously at least 70%, more advantageously at least 80%, and even more advantageously at least 90% free of these materials.

Hybridization reactions can be performed under conditions of different "stringency." Conditions that increase stringency of a hybridization reaction are well known. See for example, "Molecular Cloning: A Laboratory Manual", second edition (Sambrook et al. 1989). Examples of relevant conditions include (in order of increasing stringency): incubation temperatures of 25°C, 37°C, 50°C, and 68°C; buffer concentrations of 10 x SSC, 6 x SSC, 1 x SSC, 0.1 x SSC (where SSC is 0.15 M NaCl and 15 mM citrate buffer) and their equivalent using other buffer systems; formamide concentrations of 0%, 25%, 50%, and 75%; incubation times from 5 minutes to 24 hours; 1, 2 or more washing steps; wash incubation times of 1, 2, or 15 minutes; and wash solutions of 6 x SSC, 1 x SSC, 0.1 x SSC, or deionized water.

The description further encompasses polynucleotides encoding functionally equivalent variants and derivatives of the influenza polypeptides and functionally equivalent fragments thereof which may enhance, decrease or not significantly affect properties of the polypeptides encoded thereby. These functionally equivalent variants, derivatives, and fragments display the ability to retain influenza activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. Conservative amino acid substitutions are glycine/alanine; valine/isoleucine/leucine; asparagine/glutamine; aspartic acid/glutamic acid; serine/threonine/methionine; lysine/arginine; and phenylalanine/tyrosine/tryptophan. In one embodiment, the variants have at least 50%, at least 55%, at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% homology or identity to the influenza polynucleotide or polypeptide of interest.

For the purposes of the present description, sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990; 87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson & Lipman, Proc. Natl. Acad. Sci. USA 1988; 85: 2444-2448.

Advantageous for use according to the present description is the WU-BLAST (Washington University BLAST) version 2.0 software. WU-BLAST version 2.0 executable programs for several UNIX platforms can be downloaded from ftp ://blast.wustl.edu/blast/executables. This program is based on WU-BLAST version 1.4, which in turn is based on the

public domain NCBI-BLAST version 1.4 (Altschul & Gish, 1996, Local alignment statistics, Doolittle ed., Methods in Enzymology 266: 460-480; Altschul et al., Journal of Molecular Biology 1990; 215: 403-410; Gish & States, 1993;Nature Genetics 3: 266-272; Karlin & Altschul, 1993;Proc. Natl. Acad. Sci. USA 90: 5873- 5877).

In general, comparison of amino acid sequences is accomplished by aligning an amino acid sequence of a polypeptide of a known structure with the amino acid sequence of a the polypeptide of unknown structure. Amino acids in the sequences are then compared and groups of amino acids that are homologous are grouped together. This method detects conserved regions of the polypeptides and accounts for amino acid insertions and deletions. Homology between amino acid sequences can be determined by using commercially available algorithms (see also the description of homology above). In addition to those otherwise mentioned herein, mention is made too of the programs BLAST, gapped BLAST, BLASTN, BLASTP, and PSI-BLAST, provided by the National Center for Biotechnology Information. These programs are widely used in the art for this purpose and can align homologous regions of two amino acid sequences.

In all search programs in the suite the gapped alignment routines are integral to the database search itself Gapping can be turned off if desired. The default penalty (Q) for a gap of length one is Q=9 for proteins and BLASTP, and Q=10 for BLASTN, but may be changed to any integer. The default per-residue penalty for extending a gap (R) is R=2 for proteins and BLASTP, and R=10 for BLASTN, but may be changed to any integer. Any combination of values for Q and R can be used in order to align sequences so as to maximize overlap and identity while minimizing sequence gaps. The default amino acid comparison matrix is BLOSUM62, but other amino acid comparison matrices such as PAM can be utilized.

Alternatively or additionally, the term "homology " or "identity", for instance, with respect to a nucleotide or amino acid sequence, can indicate a quantitative measure of homology between two sequences. The percent sequence homology can be calculated as

$$(N_{ref} - N_{dif})*100/N_{ref},$$

wherein $N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{ref}$ = 8; $N_{dif}$=2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur & Lipman, Proc Natl Acad Sci USA 1983; 80: 726), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics ™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the description and can be, derived from DNA sequences, by thymidine (T) in the DNA sequence being considered equal to uracil (U) in RNA sequences.

And, without undue experimentation, the skilled artisan can consult with many other programs or references for determining percent homology.

The description further encompasses the influenza polynucleotides contained in a vector molecule or an expression vector and operably linked to a promoter element and optionally to an enhancer.

The vector is advantageously a poxvirus, particularly a vaccinia virus or an avipox virus, such as fowlpox virus and canarypox virus. Advantageously, the virus is a canarypox virus. Advantageous canarypox strains may be an attenuated strain. The vector can express at least one epitope from Kentucky strains, Newmarket strains and/or Ohio strains. Advantageous canarypox constructs include, but are not limited to, vCP 1529, vCP 1533 and vCP 2242. Recombinant avipox viruses (see, e.g., U.S. Patent Nos. 5,505,941 and 5,756,103), such as an attenuated recombinant canarypox virus, for instance ALVAC, or an attenuated fowlpox virus, for instance TROVAC, are especially advantageous. In one advantageous embodiment, the recombinant ALVAC vaccine described by Edlund Toulemonde et al., Vet Rec. 2005 Mar 19;156(12):367-71 may be used as a canine influenza vaccine. Other viruses that may be used in methods of the description include, but are not limited to, vaccinia viruses, such as an attenuated vaccinia virus, for instance NYVAC, adenoviruses, such as canine adenoviruses (CAV), and herpesviruses, such as canine herpesvirus (CHV) or a feline herpesvirus (FHV).

A "vector" refers to a recombinant DNA or RNA plasmid or virus that comprises a heterologous polynucleotide to be delivered to a target cell, either in vitro or in vivo. The heterologous polynucleotide may comprise a sequence of interest for purposes of therapy; and may optionally be in the form of an expression cassette. As used herein, a vector needs not be capable of replication in the ultimate target cell or subject. The term includes cloning vectors also included

are viral vectors.

The term "recombinant" means a polynucleotide semisynthetic, or synthetic origin which either does not occur in nature or is linked to another polynucleotide in an arrangement not found in nature.

"Heterologous" means derived from a genetically distinct entity from the rest of the entity to which it is being compared. For example, a polynucleotide, may be placed by genetic engineering techniques into a plasmid or vector derived from a different source, and is a heterologous polynucleotide. A promoter removed from its native coding sequence and operatively linked to a coding sequence other than the native sequence is a heterologous promoter.

The polynucleotides of the description may comprise additional sequences, such as additional encoding sequences within the same transcription unit, controlling elements such as promoters, ribosome binding sites, polyadenylation sites, additional transcription units under control of the same or a different promoter, sequences that permit cloning, expression, homologous recombination, and transformation of a host cell, and any such construct as may be desirable to provide embodiments of this description.

Elements for the expression of an influenza antigen, epitope or immunogen are advantageously present in a vector. In minimum manner, this comprises, consists essentially of, or consists of an initiation codon (ATG), a stop codon and a promoter, and optionally also a polyadenylation sequence for certain vectors such as plasmid and certain viral vectors, e.g., viral vectors other than poxviruses. When the polynucleotide encodes a polyprotein fragment, e.g. an influenza peptide, advantageously, in the vector, an ATG is placed at 5' of the reading frame and a stop codon is placed at 3'. Other elements for controlling expression may be present, such as enhancer sequences, stabilizing sequences, such as intron and signal sequences permitting the secretion of the protein.

Methods for making and/or administering a vector or recombinants or plasmid for expression of gene products of genes of the description either *in vivo* or *in vitro* can be any desired method, e.g., a method which is by or analogous to the methods disclosed in, or disclosed in documents cited in: U.S. Patent Nos. 4,603,112; 4,769,330; 4,394,448; 4,722,848; 4,745,051; 4,769,331; 4,945,050; 5,494,807; 5,514,375; 5,744,140; 5,744,141; 5,756,103; 5,762,938; 5,766,599; 5,990,091; 5,174,993; 5,505,941; 5,338,683; 5,494,807; 5,591,639; 5,589,466; 5,677,178; 5,591,439; 5,552,143; 5,580,859; 6,130,066; 6,004,777; 6,130,066; 6,497,883; 6,464,984; 6,451,770; 6,391,314; 6,387,376; 6,376,473; 6,368,603; 6,348,196; 6,306,400; 6,228,846; 6,221,362; 6,217,883; 6,207,166; 6,207,165; 6,159,477; 6,153,199; 6,090,393; 6,074,649; 6,045,803; 6,033,670; 6,485,729; 6,103,526; 6,224,882; 6,312,682; 6,348,450 and 6; 312,683; U.S. patent application Serial No. 920,197, filed October 16,1986; WO 90/01543; WO91/11525; WO 94/16716; WO 96/39491; WO 98/33510; EP 265785; EP 0 370 573; Andreansky et al., Proc. Natl. Acad. Sci. USA 1996;93:11313-11318; Ballay et al., EMBO J. 1993;4:3861-65; Felgner et al., J. Biol. Chem. 1994;269:2550-2561; Frolov et al., Proc. Natl. Acad. Sci. USA 1996;93:11371-11377; Graham, Tibtech 1990;8:85-87; Grunhaus et al., Sem. Virol. 1992;3:237-52; Ju et al., Diabetologia 1998;41:736-739; Kitson et al., J. Virol. 1991;65:3068-3075; McClements et al., Proc. Natl. Acad. Sci. USA 1996;93:11414-11420; Moss, Proc. Natl. Acad. Sci. USA 1996;93:11341-11348; Paoletti, Proc. Natl. Acad. Sci. USA 1996;93:11349-11353; Pennock et al., Mol. Cell. Biol. 1984;4:399-406; Richardson (Ed), Methods in Molecular Biology 1995;39, "Baculovirus Expression Protocols," Humana Press Inc.; Smith et al. (1983) Mol. Cell. Biol. 1983;3:2156-2165; Robertson et al., Proc. Natl. Acad. Sci. USA 1996;93:11334-11340; Robinson et al., Sem. Immunol. 1997;9:271; and Roizman, Proc. Natl. Acad. Sci. USA 1996;93:11307-11312. Thus, the vector can be any suitable recombinant virus or virus vector, such as a poxvirus (e.g., vaccinia virus, avipox virus, canarypox virus, fowlpox virus, raccoonpox virus, swinepox virus, etc.), adenovirus (e.g., human adenovirus, canine adenovirus), herpesvirus (e.g. canine herpesvirus), baculovirus, retrovirus, etc.; or the vector can be a plasmid. The herein cited documents, in addition to providing examples of vectors useful in the practice of the description can also provide sources for non-influenza peptides or fragments thereof to be expressed by vector or vectors in, or included in, the compositions of the description.

The present description also relates to preparations comprising vectors, such as expression vectors, e.g., therapeutic compositions. The preparations can comprise, consist essentially of, or consist of one or more vectors, e.g., expression vectors, such as *in vivo* expression vectors, comprising, consisting essentially or consisting of (and advantageously expressing) one or more of influenza antigens, epitopes or immunogens. Advantageously, the vector contains and expresses a polynucleotide that includes, consists essentially of, or consists of a polynucleotide coding for (and advantageously expressing) an influenza antigen, epitope or immunogen, in a pharmaceutically or veterinarily acceptable carrier, excipient or vehicle. Thus, according to an embodiment of the description, the other vector or vectors in the preparation comprises, consists essentially of or consists of a polynucleotide that encodes, and under appropriate circumstances the vector expresses one or more other proteins of an influenza antigen, epitope or immunogen (e.g., hemagglutinin, neuraminidase, nucleoprotein) or a fragment thereof.

According to another embodiment, the vector or vectors in the preparation comprise, or consist essentially of, or consist of polynucleotide(s) encoding one or more proteins or fragment(s) thereof of an influenza antigen, epitope or immunogen, the vector or vectors expressing the polynucleotide(s). The preparation advantageously comprises, consists essentially of, or consists of, at least two vectors comprising, consisting essentially of, or consisting of, and advantageously also expressing, advantageously *in vivo* under appropriate conditions or suitable conditions or in a suitable host cell,

polynucleotides from different canine influenza isolates encoding the same proteins and/or for different proteins, but advantageously the same proteins. Preparations containing one or more vectors containing, consisting essentially of or consisting of polynucleotides encoding, and advantageously expressing, advantageously *in vivo,* an influenza antigen, fusion protein or an epitope thereof. The description is also directed at mixtures of vectors that contain, consist essentially of, or consist of coding for, and express, different influenza antigens, epitopes or immunogens, e.g., an influenza antigen, epitope or immunogen from different species such as, but not limited to, humans,, pigs, , in addition to avian species including chicken, ducks and geese.

According to one embodiments, the expression vector is a viral vector, in particular an *in vivo* expression vector. In an advantageous embodiment, the expression vector is an adenovirus vector. Advantageously, the adenovirus is a human Ad5 vector, an El-deleted and/ or an E3-deleted adenovirus.

In one particular embodiment the viral vector is a poxvirus, e.g. a vaccinia virus or an attenuated vaccinia virus, (for instance, MVA, a modified Ankara strain obtained after more than 570 passages of the Ankara vaccine strain on chicken embryo fibroblasts; *see* Stickl & Hochstein-Mintzel, Munch. Med. Wschr., 1971, 113, 1149-1153; Sutter et al., Proc. Natl. Acad. Sci. U.S.A., 1992, 89, 10847-10851; available as ATCC VR-1508; or NYVAC, *see* U.S. Patent No. 5,494,807, for instance, Examples 1 to 6 and *et seq* of U.S. Patent No. 5,494,807 which discuss the construction ofNYVAC, as well as variations of NYVAC with additional ORFs deleted from the Copenhagen strain vaccinia virus genome, as well as the insertion of heterologous coding nucleic acid molecules into sites of this recombinant, and also, the use of matched promoters; see also WO96/40241), an avipox virus or an attenuated avipox virus (e.g., canarypox, fowlpox, dovepox, pigeonpox, quailpox, ALVAC or TROVAC; see, e.g., U.S. Patent No. 5,505,941, 5,494,807), swinepox, raccoonpox, camelpox, or myxomatosis virus.

According to another embodiment, the poxvirus vector is a canarypox virus or a fowlpox virus vector, advantageously an attenuated canarypox virus or fowlpox virus. In this regard, is made to the canarypox available from the ATCC under access number VR-111. Attenuated canarypox viruses are described in U.S. Patent No. 5,756,103 (ALVAC) and WO01/05934. Numerous fowlpox virus vaccination strains are also available, e.g. the DIFTOSEC CT strain marketed by MERIAL and the NOBILIS VARIOLE vaccine marketed by INTERVET; and, reference is also made to U.S. Patent No. 5,766,599 which pertains to the attenuated fowlpox strain TROVAC.

For information on the method to generate recombinants thereof and how to administer recombinants thereof, the skilled artisan can refer documents cited herein and to WO90/12882, e.g., as to vaccinia virus mention is made of U.S. Patents Nos. 4,769,330, 4,722,848, 4,603,112, 5,110,587, 5,494,807, and 5,762,938 *inter alia*; as to fowlpox, mention is made of U.S. Patents Nos. 5,174,993, 5,505,941 and US-5,766,599 *inter alia*; as to canarypox mentionis made of U.S. Patent No. 5,756,103 *inter alia*; as to swinepox mention is made of U.S. Patent No. 5,382,425 *inter alia*; and, as to raccoonpox, mention is made of WO00/03030 *inter alia.*

When the expression vector is a vaccinia virus, insertion site or sites for the polynucleotide or polynucleotides to be expressed are advantageously at the thymidine kinase (TK) gene or insertion site, the hemagglutinin (HA) gene or insertion site, the region encoding the inclusion body of the A type (ATI); see also documents cited herein, especially those pertaining to vaccinia virus. In the case of canarypox, advantageously the insertion site or sites are ORF(s) C3, C5 and/or C6; see also documents cited herein, especially those pertaining to canarypox virus. In the case of fowlpox, advantageously the insertion site or sites are ORFs F7 and/or F8; see also documents cited herein, especially those pertaining to fowlpox virus. The insertion site or sites for MVA virus area advantageously as in various publications, including Carroll M. W. et al., Vaccine, 1997, 15 (4), 387-394; Stittelaar K. J. et al., J. Virol., 2000, 74 (9), 4236-4243; Sutter G. et al., 1994, Vaccine, 12 (11), 1032-1040; and, in this regard it is also noted that the complete MVA genome is described in Antoine G., Virology, 1998, 244, 365-396, which enables the skilled artisan to use other insertion sites or other promoters.

Advantageously, the polynucleotide to be expressed is inserted under the control of a specific poxvirus promoter, e.g., the vaccinia promoter 7.5 kDa (Cochran et al., J. Virology, 1985, 54, 30-35), the vaccinia promoter I3L (Riviere et al., J. Virology, 1992, 66, 3424-3434), the vaccinia promoter HA (Shida, Virology, 1986, 150, 451-457), the cowpox promoter ATI (Funahashi et al., J. Gen. Virol., 1988, 69, 35-47), the vaccinia promoter H6 (Taylor J. et al., Vaccine, 1988, 6, 504-508; Guo P. et al. J. Virol., 1989, 63, 4189-4198; Perkus M. et al., J. Virol., 1989, 63, 3829-3836), *inter alia.*

In a particular embodiment the viral vector is an adenovirus, such as a human adenovirus (HAV) or a canine adenovirus (CAV).

In one embodiment the viral vector is a human adenovirus, in particular a serotype 5 adenovirus, rendered incompetent for replication by a deletion in the E1 region of the viral genome, in particular from about nucleotide 459 to about nucleotide 3510 by reference to the sequence of the hAd5 disclosed in Genbank under the accession number M73260 and in the referenced publication J. Chroboczek et al Virol. 1992, 186, 280-285. The deleted adenovirus is propagated in E1-expressing 293 (F. Graham et al J. Gen. Virol. 1977, 36, 59-72) or PER cells, in particular PER.C6 (F. Falloux et al Human Gene Therapy 1998, 9, 1909-1917). The human adenovirus can be deleted in the E3 region, in particular from about nucleotide 28592 to about nucleotide 30470. The deletion in the E1 region can be done in combination with a deletion in the E3 region (*see, e.g.* J. Shriver et al. Nature, 2002, 415, 331-335, F. Graham et al

Methods in Molecular Biology Vol .7: Gene Transfer and Expression Protocols Edited by E. Murray, The Human Press Inc, 1991, p 109-128; Y. Ilan et al Proc. Natl. Acad. Sci. 1997, 94, 2587-2592; US6,133,028; US6,692,956; S. Tripathy et al Proc. Natl. Acad. Sci. 1994, 91, 11557-11561; B. Tapnell Adv. Drug Deliv. Rev.1993, 12, 185-199;X. Danthinne et al Gene Thrapy 2000, 7, 1707-1714; K. Berkner Bio Techniques 1988, 6, 616-629; K. Berkner et al Nucl. Acid Res. 1983, 11, 6003-6020; C. Chavier et al J. Virol. 1996, 70, 4805-4810). The insertion sites can be the E1 and/or E3 loci (region) eventually after a partial or complete deletion of the E1 and/or E3 regions. Advantageously, when the expression vector is an adenovirus, the polynucleotide to be expressed is inserted under the control of a promoter functional in eukaryotic cells, such as a strong promoter, preferably a cytomegalovirus immediate-early gene promoter (CMV-IE promoter), in particular the enhancer / promoter region from about nucleotide -734 to about nucleotide +7 in M. Boshart et al Cell 1985, 41, 521-530 or the enhancer / promoter region from the pCI vector from Promega Corp. The CMV-IE promoter is advantageously of murine or human origin. The promoter of the elongation factor 1α can also be used. A muscle specific promoter can also be used (X. Li et al Nat. Biotechnol. 1999, 17, 241-245). Strong promoters are also discussed herein in relation to plasmid vectors. In one embodiment, a splicing sequence can be located downstream of the enhancer / promoter region. For example, the intron 1 isolated from the CMV-IE gene (R. Stenberg et al J. Virol. 1984, 49, 190), the intron isolated from the rabbit or human β-globin gene, in particular the intron 2 from the b-globin gene, the intron isolated from the immunoglobulin gene, a splicing sequence from the SV40 early gene or the chimeric intron sequence isolated from the pCI vector from Promege Corp. comprising the human β-globin gene donor sequence fused to the mouse immunoglobulin acceptor sequence (from about nucleotide 890 to about nucleotide 1022 in Genbank under the accession number CVU47120). A poly(A) sequence and terminator sequence can be inserted downstream the polynucleotide to be expressed, e.g. a bovine growth hormone gene, in particular from about nucleotide 2339 to about nucleotide 2550 in Genbank under the accession number BOVGHRH, a rabbit β-globin gene or a SV40 late gene polyadenylation signal.

In another embodiment the viral vector is a canine adenovirus, in particular a CAV-2 (see, e.g. L. Fischer et al. Vaccine, 2002, 20, 3485-3497; U.S. Patent No. 5,529,780; U.S. Patent No. 5,688,920; PCT Application No. WO95/14102). For CAV, the insertion sites can be in the E3 region and /or in the region located between the E4 region and the right ITR region (see U.S. Patent No. 6,090,393; U.S. Patent No. 6,156,567). In one embodiment the insert is under the control of a promoter, such as a cytomegalovirus immediate-early gene promoter (CMV-IE promoter) or a promoter already described for a human adenovirus vector. A poly(A) sequence and terminator sequence can be inserted downstream the polynucleotide to be expressed, e.g. a bovine growth hormone gene or a rabbit β-globin gene polyadenylation signal.

In another particular embodiment the viral vector is a herpesvirus such as a canine herpesvirus (CHV) or a feline herpesvirus (FHV). For CHV, the insertion sites may be in particular in the thymidine kinase gene, in the ORF3, or in the UL43 ORF (see U.S. Patent No. 6,159,477). In one embodiment the polynucleotide to be expressed is inserted under the control of a promoter functional in eukaryotic cells, advantageously a CMV-IE promoter (murine or human).. A poly(A) sequence and terminator sequence can be inserted downstream the polynucleotide to be expressed, e.g. bovine growth hormone or a rabbit β-globin gene polyadenylation signal.

According to a yet further embodiment, the expression vector is a plasmid vector or a DNA plasmid vector, in particular an *in vivo* expression vector. In a specific, non-limiting example, the pVR1020 or 1012 plasmid (VICAL Inc.; Luke C. et al., Journal of Infectious Diseases, 1997,175, 91-97; Hartikka J. et al., Human Gene Therapy, 1996, 7,1205-1217, see, e.g., U.S. Patent Nos. 5,846,946 and 6,451,769) can be utilized as a vector for the insertion of a polynucleotides sequence. The pVR1020 plasmid is derived from pVR1012 and contains the human tPA signal sequence. In one embodiment the human tPA signal comprises from amino acid M(1) to amino acid S(23) in Genbank under the accession number HUMTPA14. In another specific, non-limiting example, the plasmid utilized as a vector for the insertion of a polynucleotide sequence can contain the signal peptide sequence of equine IGF1 from amino acid M(24) to amino acid A(48) in Genbank under the accession number U28070. Additional information on DNA plasmids which may be consulted or employed in the practice are found, for example, in U.S. Patent Nos. 6,852,705; 6,818,628; 6,586,412; 6,576,243; 6,558,674; 6,464,984; 6,451,770; 6,376,473 and 6,221,362.

The term plasmid covers any DNA transcription unit comprising a polynucleotide according to the description and the elements necessary for its *in vivo* expression in a cell or cells of the desired host or target; and, in this regard, it is noted that a supercoiled or non-supercoiled, circular plasmid, as well as a linear form, are intended to be within the scope of the description..

Each plasmid comprises or contains or consists essentially of, in addition to the polynucleotide encoding an influenza antigen, epitope or immunogen, optionally fused with a heterologous peptide sequence, variant, analog or fragment, operably linked to a promoter or under the control of a promoter or dependent upon a promoter. In general, it is advantageous to employ a strong promoter functional in eukaryotic cells. The preferred strong promoter is the immediate early cytomegalovirus promoter (CMV-IE) of human or murine origin, or optionally having another origin such as the rat or guinea pig. The CMV-IE promoter can comprise the actual promoter part, which may or may not be associated with the enhancer part. Reference can be made to EP-A-260 148, EP-A-323 597, U.S. Patents Nos. 5,168,062, 5,385,839,

and 4,968,615, as well as to PCT Application No WO87/03905. The CMV-IE promoter is advantageously a human CMV-IE (Boshart M. et al., Cell., 1985, 41, 521-530) or murine CMV-IE.

In more general terms, the promoter has either a viral or a cellular origin. A strong viral promoter other than CMV-IE that may be usefully employed in the practice of the invention is the early/late promoter of the SV40 virus or the LTR promoter of the Rous sarcoma virus. A strong cellular promoter that may be usefully employed in the practice of the invention is the promoter of a gene of the cytoskeleton, such as e.g. the desmin promoter (Kwissa M. et al., Vaccine, 2000, 18,2337-2344), or the actin promoter (Miyazaki J. et al., Gene, 1989, 79, 269-277).

Functional sub fragments of these promoters, i.e., portions of these promoters that maintain an adequate promoting activity, are included within the present description, e.g. truncated CMV-IE promoters according to PCT Application No. WO98/00166 or U.S. Patent No. 6,156,567 can be used in the practice of the invention. A promoter in the practice of the invention consequently includes derivatives and sub fragments of a full-length promoter that maintain an adequate promoting activity and hence function as a promoter, preferably promoting activity substantially similar to that of the actual or full-length promoter from which the derivative or sub fragment is derived, e.g., akin to the activity of the truncated CMV-IE promoters of U.S. Patent No. 6,156,567 to the activity of full-length CMV-IE promoters. Thus, a CMV-IE promoter in the practice of the invention can comprise or consist essentially of or consist of the promoter portion of the full-length promoter and/or the enhancer portion of the full-length promoter, as well as derivatives and sub fragments.

Preferably, the plasmids comprise or consist essentially of other expression control elements. It is particularly advantageous to incorporate stabilizing sequence(s), e.g., intron sequence(s), preferably the first intron of the hCMV-IE (PCT Application No. WO89/01036), the intron II of the rabbit β-globin gene (van Ooyen et al., Science, 1979, 206, 337-344).

As to the polyadenylation signal (polyA) for the plasmids and viral vectors other than poxviruses, use can more be made of the poly(A) signal of the bovine growth hormone (bGH) gene (*see* U.S. Patent No. 5,122,458), or the poly(A) signal of the rabbit β-globin gene or the poly(A) signal of the SV40 virus.

According to another embodiment, the expression vectors are expression vectors used for the *in vitro* expression of proteins in an appropriate cell system. The expressed proteins can be harvested in or from the culture supernatant after, or not after secretion (if there is no secretion a cell lysis typically occurs or is performed), optionally concentrated by concentration methods such as ultrafiltration and/or purified by purification means, such as affinity, ion exchange or gel filtration-type chromatography methods.

A "host cell" denotes a prokaryotic or eukaryotic cell that has been genetically altered, or is capable of being genetically altered by administration of an exogenous polynucleotide, such as a recombinant plasmid or vector. When referring to genetically altered cells, the term refers both to the originally altered cell and to the progeny thereof. Advantageous host cells include, but are not limited to, baby hamster kidney (BHK) cells, colon carcinoma (Caco-2) cells, COS7. cells, MCF-7 cells, MCF-10A cells, Madin-Darby canine kidney (MDCK) lines, mink lung (Mv1Lu) cells, MRC-5 cells, U937 cells and VERO cells. Polynucleotides comprising a desired sequence can be inserted into a suitable cloning or expression vector, and the vector in turn can be introduced into a suitable host cell for replication and amplification. Polynucleotides can be introduced into host cells by any means known in the art. The vectors containing the polynucleotides of interest can be introduced into the host cell by any of a number of appropriate means, including direct uptake, endocytosis, transfection, f-mating, electroporation, transfection employing calcium chloride, rubidium chloride, calcium phosphate, DEAE-dextran, or other substances; microprojectile bombardment; lipofection; and infection (where the vector is infectious, for instance, a retroviral vector). The choice of introducing vectors or polynucleotides will often depend on features of the host cell.

In an advantageous embodiment, the description provides for the administration of a therapeutically effective amount of a formulation for the delivery and expression of an influenza antigen, epitope or immunogen in a target cell. Determination of the therapeutically effective amount is routine experimentation for one of ordinary skill in the art. In one embodiment, the formulation comprises an expression vector comprising a polynucleotide that expresses an influenza antigen, epitope or immunogen and a pharmaceutically or veterinarily acceptable carrier, vehicle or excipient. In an advantageous embodiment, the pharmaceutically or veterinarily acceptable carrier, vehicle or excipient facilitates transfection and/or improves preservation of the vector or protein.

The pharmaceutically or veterinarily acceptable carriers or vehicles or excipients are well known to the one skilled in the art. For example, a pharmaceutically or veterinarily acceptable carrier or vehicle or excipient can be a 0.9% NaCl (e.g., saline) solution or a phosphate buffer. Other pharmaceutically or veterinarily acceptable carrier or vehicle or excipients that can be used for methods of this description include, but are not limited to, poly-(L-glutamate) or polyvinylpyrrolidone. The pharmaceutically or veterinarily acceptable carrier or vehicle or excipients may be any compound or combination of compounds facilitating the administration of the vector (or protein expressed from a vector in *vitro*); advantageously, the carrier, vehicle or excipient may facilitate transfection and/or improve preservation of the vector (or protein). Doses and dose volumes are herein discussed in the general description and can also be determined by the skilled artisan from this disclosure read in conjunction with the knowledge in the art, without any undue experimentation.

The cationic lipids containing a quaternary ammonium salt which are advantageously but not exclusively suitable

for plasmids, are advantageously those having the following formula:

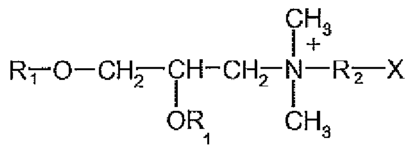

in which $R_1$ is a saturated or unsaturated straight-chain aliphatic radical having 12 to 18 carbon atoms, $R_2$ is another aliphatic radical containing 2 or 3 carbon atoms and X is an amine or hydroxyl group, e.g. the DMRIE. In another embodiment the cationic lipid can be associated with a neutral lipid, e.g. the DOPE.

Among these cationic lipids, preference is given to DMRIE (N-(2-hydroxyethyl)-N,N-dimethyl-2,3-bis(tetradecyloxy)-1-propane ammonium; WO96/34109), advantageously associated with a neutral lipid, advantageously DOPE (dioleoyl-phosphatidyl-ethanol amine; Behr J. P., 1994, Bioconjugate Chemistry, 5, 382-389), to form DMRIE-DOPE.

Advantageously, the plasmid mixture with the adjuvant is formed extemporaneously and advantageously contemporaneously with administration of the preparation or shortly before administration of the preparation; for instance, shortly before or prior to administration, the plasmid-adjuvant mixture is formed, advantageously so as to give enough time prior to administration for the mixture to form a complex, e.g. between about 10 and about 60 minutes prior to administration, such as approximately 30 minutes prior to administration.

When DOPE is present, the DMRIE:DOPE molar ratio is advantageously about 95: about 5 to about 5:about 95, more advantageously about 1: about 1, e.g., 1:1.

The DMRIE or DMRIE-DOPE adjuvant:plasmid weight ratio can be between about 50: about 1 and about 1: about 10, such as about 10: about 1 and about 1 :about 5, and advantageously about 1: about 1 and about 1: about 2, e.g., 1:1 and 1:2.

The description also provides for inactivated canine influenza vaccines. As used herein, the term "inactivated vaccine" means a vaccine composition containing an infectious organism or pathogen that is no longer capable of replication or growth. Inactivation may be accomplished by a variety of methods sufficient to prevent replication or growth of the organism while maintaining its immunogenicity.

The inactivated vaccine may be an inactivated form of an isolate of an influenza virus from an affected dog. The virus may be isolated from the alveoli or lung of an affected dog. In another embodiment, the inactivated vaccine may be an inactivated equine influenza. In an advantageous embodiment, the equine influenza is a Kentucky or Newmarket equine influenza. The inactivated vaccine may be an inactivated version of any one of the influenza strains described above.

An inactivated vaccine may be prepared as well from the harvested culture fluid. The virus may be produced either by inoculation of 10-11-day embryonated eggs (J. Violay et al US6,048,537) or by inoculation of BHK-21 cell culture (C. Ross et al Archiv. Für die gesamte Virusforschung 1970, 30, 82-88; T. Tolstova et al Acta Virol. 1966, 10, 315-321; Ho. Merten et al Adv. Exp. Med. Biol. 1996, 397, 141-151), of MDCK cell culture (J. Tree et al Vaccine 2001, 19, 3444-3450; Y. Ghendon et al Vaccine 2005,23,4678-4684; R. Brands et al Dev. Biol. Stand. 1999, 98, 93-100; R. Youil et al J Virol. Methods 2004, 120,23-31), of Vero cell culture (O. Kistner et al Vaccine 1998, 16, 960-968; E. Govorkova et al J. Virol. 1996, 70, 5519-5524). The allantoic fluid or the cell culture supernatant can be clarified by low centrifugation and/or filtration. The virus can be concentrated by ultrafiltration and can be purified by zonal centrifugation on sucrose gradient (J. Violay et al US6,048,537; O. Kistner et al idem), by gel filtration (D. Nayak et al J. Chromatogr. B Analyt. Technol, Biomed. Life Sci. 2005, 823, 75-81; S. Tomita et al Kitasato Arch. Exp. Med. 1971,44, 185-196).

Inactivation may be achieved by treating the viruses by any of the methods commonly employed to make inactivated vaccines. These methods include but are not limited to formaldehyde treatment (O. Kistner et al idem; A. Garcia et al Avian Diseases 1998, 42, 248-256), betapropriolactone treatment (B. Bdowsky et al Vaccine 1991, 9,398-402 and Vaccine 1993, 11, 343-348; N. Keverin et al Arch. Virol. 2000, 145, 1059-1066), ethylene-imine treatment (D. Swayne et al Avian Diseases 2001, 45, 355-365), treatment with organic solvents, treatment with detergents, treatment with Tween-ether or treatment with Triton X-100 (J. Vilay et al idem) for allantoic fluid. For the inactivation the concentration can be about 0.01-0.2 % w/v for the formaldehyde; about 0.03-0.2 % w/v for the betapropiolactone; about 0.5-20 mM for ethyleneimine. The methods recited herein serve as art-known examples for inactivating virus. Inactivated virus vaccines are usually administered mixed with an adjuvant. The inactivated vaccine can be administered to the animal by any of a plurality of methods which include but are not limited to inoculation intramuscularly, intradermally, or subcutaneously, spraying, ocularly, nasally, orally, or in ovo.

The immunogenic compositions and vaccines according to the description may comprise or consist essentially of one or more adjuvants. Suitable adjuvants for use in the practice of the present invention are (1) polymers of acrylic or methacrylic acid, maleic anhydride and alkenyl derivative polymers, (2) immunostimulating sequences (ISS), such as oligodeoxyribonucleotide sequences having one ore more non-methylated CpG units (Klinman et al., Proc. Natl.

Acad. Sci., USA, 1996, 93,2879-2883; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, e.g., DDA (5) cytokines, (6) aluminum hydroxide or aluminum phosphate, (7) saponin or (8) other adjuvants discussed in any document cited in the instant application, or (9) any combinations or mixtures thereof, or (10) immunostimulating complexes (ISCOMs) that are open cage-like complexes built up by cholesterols, lipids, immunogen, and saponins from the bark of the tree Quillaia saponaria Molina (Sjolander et al., J. Leukocyte Biol., 1998, 64 (6), 713-723).

The oil in water emulsion (3), which is especially appropriate for viral vectors, can be based on: light liquid paraffin oil (European pharmacopoeia type), isoprenoid oil such as squalane, squalene, oil resulting from the oligomelization of alkenes, e.g. isobutene or decene, esters of acids or alcohols having a straight-chain alkyl group, such as vegetable oils, ethyl oleate, propylene glycol, di(caprylatelcaprate), glycerol tri(caprylate/caprate) and propylene glycol dioleate, or esters of branched, fatty alcohols or acids, especially isostearic acid esters.
The oil is used in combination with emulsifiers to form an emulsion. The emulsifiers may be nonionic surfactants, such as: esters of on the one hand sorbitan, mannide (e.g. anhydromannitol oleate), glycerol, polyglycerol or propylene glycol and on the other hand oleic, isostearic, ricinoleic or hydroxystearic acids, said esters being optionally ethoxylated, orpolyoxypropylene-polyoxyethylene copolymer blocks, such as Pluronic, e.g., L121.

The type (1) immunostimulating complex matrix type (ISCOM™ matrix) adjuvants are advantageous choices, especially since it has been shown that this class of adjuvants are capable of up-regulating MHC Class II expression by APCs. An ISCOM™. matrix consists of (optionally fractionated) saponins (triterpenoids) from Quillaja saponaria, cholesterol, and phospholipid. When admixed with the immunogenic protein, the resulting particulate formulation is what is known as an ISCOM particle where the saponin constitutes 60-70% w/w, the cholesterol and phospholipid 10-15% w/w, and the protein 10-15% w/w. Details relating to composition and use of immunostimulating complexes can e.g. be found in Morein B et al., 1995, Clin. Immunother. 3: 461-475 as well as Barr IG and Mitchell GF, 1996, Immunol. and Cell Biol. 74: 8-25 provide useful instructions for the preparation of complete immunostimulating complexes.

Among the type (1) adjuvant polymers, preference is given to polymers of crosslinked acrylic or methacrylic acid, especially crosslinked by polyalkenyl ethers of sugars or polyalcohols. These compounds are known under the name carbomer (Pharmeuropa, vol. 8, no. 2, June 1996). One skilled in the art can also refer to U.S. Patent No. 2,909,462, which provides such acrylic polymers crosslinked by a polyhydroxyl compound having at least three hydroxyl groups, preferably no more than eight such groups, the hydrogen atoms of at least three hydroxyl groups being replaced by unsaturated, aliphatic radicals having at least two carbon atoms. The preferred radicals are those containing 2 to 4 carbon atoms, e.g. vinyls, allyls and other ethylenically unsaturated groups. The unsaturated radicals can also contain other substituents, such as methyl. Products sold under the name Carbopol (BF Goodrich, Ohio, USA) are especially suitable. They are crosslinked by allyl saccharose or by allyl pentaerythritol. Among them, reference is made to Carbopol 974P, 934P and 971P.

As to the maleic anhydride-alkenyl derivative copolymers, preference is given to EMA (Monsanto), which are straight-chain or crosslinked ethylene-maleic anhydride copolymers and they are, for example, crosslinked by divinyl ether. Reference is also made to J. Fields et al., Nature 186: 778-780, June 4,1960.

With regard to structure, the acrylic or methacrylic acid polymers and EMA are preferably formed by basic units having the following formula:

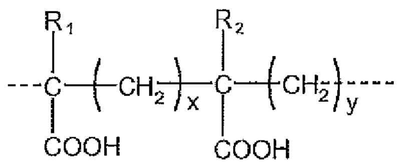

in which:

- $R_1$ and $R_2$, which can be the same or different, represent H or $CH_3$
- x = 0 or 1, preferably x = 1
- y = 1 or 2, with x + y = 2.

For EMA, x = 0 and y = 2 and for carbomers x = y = 1.

These polymers are soluble in water or physiological salt solution (20 g/l NaCl) and the pH can be adjusted to 7.3 to 7.4, e.g., by soda (NaOH), to provide the adjuvant solution in which the expression vector(s) can be incorporated. The polymer concentration in the final vaccine composition can range between 0.01 and 1.5% w/v, advantageously 0.05 to 1% w/v and preferably 0.1 to 0.4% w/v.

The cytokine or cytokines (5) can be in protein form in the immunogenic or vaccine composition, or can be co-expressed in the host with the immunogen or immunogens or epitope(s) thereof. Preference is given to the co-expression of the cytokine or cytokines, either by the same vector as that expressing the immunogen or immunogens or epitope(s) thereof, or by a separate vector therefor.

The description comprehends preparing such combination compositions; for instance by admixing the active components, advantageously together and with an adjuvant, carrier, cytokine, and/or diluent.

Cytokines that may be used include, but are not limited to, granulocyte colony stimulating factor (G-CSF), granulocyte/macrophage colony stimulating factor (GM-CSF), interferon $\alpha$ (IFN $\alpha$), interferon $\beta$ (IFN $\beta$), interferon y, (IFN $\gamma$), interleukin-1$\alpha$ (IL-1 $\alpha$), interleukin-1 $\beta$ (IL-1 $\beta$), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (1L-11), interleukin-12 (IL-12), tumor necrosis factor $\alpha$ (TNF $\alpha$), tumor necrosis factor $\beta$ (TNF $\beta$), and transforming growth factor $\beta$ (TGF $\beta$). It is understood that cytokines can be co-administered and/or sequentially administered with the immunogenic or vaccine composition of the present description. Thus, for instance, the vaccine of the instant description can also contain an exogenous nucleic acid molecule that expresses in vivo a suitable cytokine, e.g., a cytokine matched to this host to be vaccinated or in which an immunological response is to be elicited (for instance, a canine cytokine for preparations to be administered to dogs).

Advantageously, the pharmaceutical and/or therapeutic compositions and/or formulations according to the description comprise or consist essentially of or consist of an effective quantity to elicit a therapeutic response of one or more expression vectors and/or polypeptides as discussed herein; and, an effective quantity can be determined from this disclosure, including the documents cited herein, and the knowledge in the art, without undue experimentation.

In the case of therapeutic and/or pharmaceutical compositions based on a plasmid vector, a dose can comprise, consist essentially of or consist of, in general terms, about in 1 $\mu$g to about 2000 $\mu$g, advantageously about 50 $\mu$g to about 1000 $\mu$g and more advantageously from about 100 $\mu$g to about 800 $\mu$g of plasmid expressing the influenza antigen, epitope or immunogen. When the therapeutic and/or pharmaceutical compositions based on a plasmid vector is administered with electroporation the dose of plasmid is generally between about 0.1 $\mu$g and 1mg, advantageously between about 1 $\mu$g and 100 $\mu$g, advantageously between about 2 $\mu$g and 50 $\mu$g. The dose volumes can be between about 0.1 and about 2 ml, advantageously between about 0.2 and about 1 ml. These doses and dose volumes are suitable for the treatment of canines and other mammalian target species such as equines and felines.

The therapeutic and/or pharmaceutical composition contains per dose from about $10^4$ to about $10^{11}$, advantageously from about $10^5$ to about $10^{10}$ and more advantageously from about $10^6$ to about $10^9$ viral particles of recombinant adenovirus expressing an influenza antigen, epitope or immunogen. In the case of therapeutic and/or pharmaceutical compositions based on a poxvirus, a dose can be between about $10^2$ pfu and about $10^9$ pfu. The pharmaceutical composition contains per dose from about $10^5$ to $10^9$, advantageously from about $10^6$ to $10^8$ pfu of poxvirus or herpesvirus recombinant expressing the influenza antigen, epitope or immunogen.

The dose volume of compositions for target species that are mammals, e.g., the dose volume of canine compositions, based on viral vectors, e.g., non-poxrirus-viral-vector-based compositions, is generally between about 0.1 to about 2.0 ml, preferably between about 0.1 to about 1.0 ml, and more preferably between about 0.5 ml to about 1.0 ml.

With inactivated compositions of the virus or organism or pathogen produced on the new cell culture, the animal may be administered approximately $10^4$-$10^9$ equivalent $CCID_{50}$ (titer before inactivation), advantageously approximately $10^5$-$10^8$ equivalent $CCID_{50}$ in a single dosage unit. Alternately, embryonated chicken eggs may provide a substrate for pathogen propagation, in which case the unit of measure is median egg infectious dose ($EID_{50}$). The volume of one single dosage unit can be between 0.2 ml and 5.0 ml and advantageously between 0.5 ml and 2.0 ml and more advantageously about 2.0 ml. One or more administrations can be done; e.g. with two injections at 2-4 weeks interval, and advantageously with a boost about 3 weeks after the first injection.

In an advantageous embodiment, an animal, advantageously a dog, is vaccinated with two doses of inactivated vaccine at about 3 to 4 week intervals via the subcutaneous route, although an intramuscular route is also contemplated. Blood samples may be collected on the day of the first and/or second vaccination and about 2 to 4 weeks after the second vaccination to determine the levels of anti-influenza virus specific antibodies by methods known to one of skill in the art, for example, virus neutralization, hemagglutination inhibition, ELISA or single radial heamolysis (SRH) tests.

The efficacy of the inactivated vaccines may be tested about 2 to 4 weeks after the second immunization by challenging animals, advantageously dogs, with a virulent strain of influenza, advantageously the influenza H3N8 strain. The animal may be challenged by spray, intra-nasally, intra-tracheally and/or orally. The challenge viral may be about $10^{5-8}$ EID50 in a volume depending upon the route of administration. For example, if the administration is by spray, a virus suspension is aerosolized to generate about 1 to 100 $\mu$m droplets, if the administration is intra-nasal, intra-tracheal or oral, the volume of the challenge virus is about 0.5 ml, 1-2 ml and 5-10 ml, respectively. Animals may be observed daily for 14 days following challenge for clinical signs, for example, fever, cough, nasal, ocular discharge, respiratory distress, anorexia and lethargy. In addition, groups of animals may be euthanized and evaluated for pathological findings of pulmonary and pleural hemorrhage, tracheitis, bronchitis, broncholilitis, and bronchopneumonia. Tracheal swabs may

be collected from all animals post challenge days 1-14 for virus isolation. The presence or absence of viral antigens in respiratory tissues may be evaluated by immunohistochemistry, for example, on days 3, 7 and 10 post-challenge. Blood samples may be collected post-challenge (e.g., on days 7 and 14 post-challenge) and may be analyzed for the presence of anti-influenza H3N8 virus specific antibody.

It should be understood by one of skill in the art that the disclosure herein is provided by way of example and the present invention is not limited thereto. From the disclosure herein and the knowledge in the art, the skilled artisan can determine the number of administrations, the administration route, and the doses to be used for each injection protocol, without any undue experimentation.

The present description contemplates at least one administration to an animal of an efficient amount of the therapeutic composition made according to the description. The animal may be male, female, pregnant female and newborn. This administration may be via various routes including, but not limited to, intramuscular (IM), intradermal (ID) or subcutaneous (SC) injection or via intranasal or oral administration. The therapeutic composition according to the description can also be administered by a needleless apparatus (as, for example with a Pigjet, Biojector or Vitajet apparatus (Bioject, Oregon, USA)). Another approach to administer plasmid compositions is to use electroporation (see, e.g. S. Tollefsen et al. Vaccine, 2002, 20, 3370-3378; S. Tollefsen et al. Scand. J. Immunol., 2003, 57, 229-238; S. Babiuk et al., Vaccine, 2002, 20, 3399-3408; PCT Application No. WO99/01158). In another embodiment, the therapeutic composition is delivered to the animal by gene gun or gold particle bombardment. In an advantageous embodiment, the animal is a vertebrate. In a more advantageous embodiment, the vertebrate is a dog.

One embodiment of the description is a method of eliciting an immune response against influenza in an animal, comprising administering a formulation for delivery and expression of a recombinant poxvirus influenza vaccine or inactivated influenza vaccine in an effective amount for eliciting an immune response. Still another embodiment of the description is a method of inducing an immunological or protective response against influenza in an animal, comprising administering to the animal an effective amount of a formulation for delivery and expression of an influenza antigen, epitope or immunogen wherein the formulation comprises recombinant poxvirus influenza vaccine or inactivated influenza vaccine and a pharmaceutically or veterinarily acceptable carrier, vehicle or excipient.

The description relates to a method to elicit, induce or stimulate the immune response of an animal, advantageously a vertebrate. In one embodiment, the vertebrate is a dog but may also be a cat.

Another embodiment of the description is a kit for performing a method of inducing an immunological or protective response against influenza in an animal comprising a recombinant influenza poxvirus vaccine or an inactivated influenza vaccine and instructions for performing the method of delivery in an effective amount for eliciting an immune response in the animal.

The invention will now be further described by way of the following non-limiting examples.

EXAMPLE 1: Vaccination Of Dogs With Canarypox Expressing H3 Genes

A study was conducted in which dogs were vaccinated dogs on days 0 and 21 with a canarypox expressing H3 genes from Kentucky (CP1529) and Newmarket (CP1533) equine influenza. The construction of CP1529 and CP1533 is described in Examples 1 (c5 locus), 4 and 5 of International Patent Publication WO99/44633.

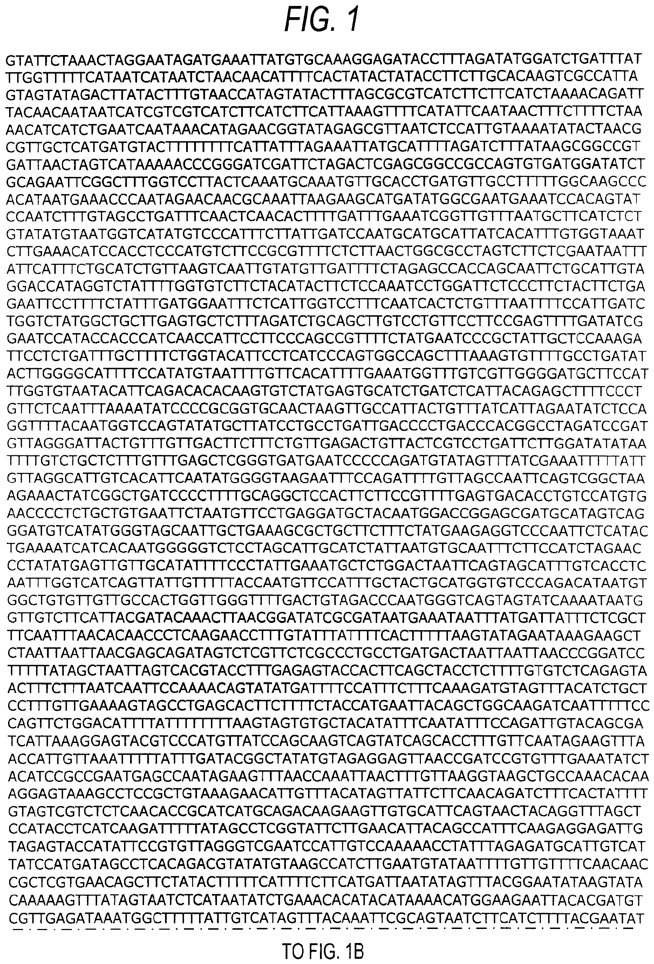

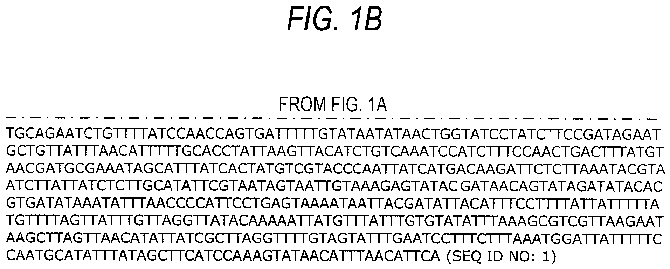

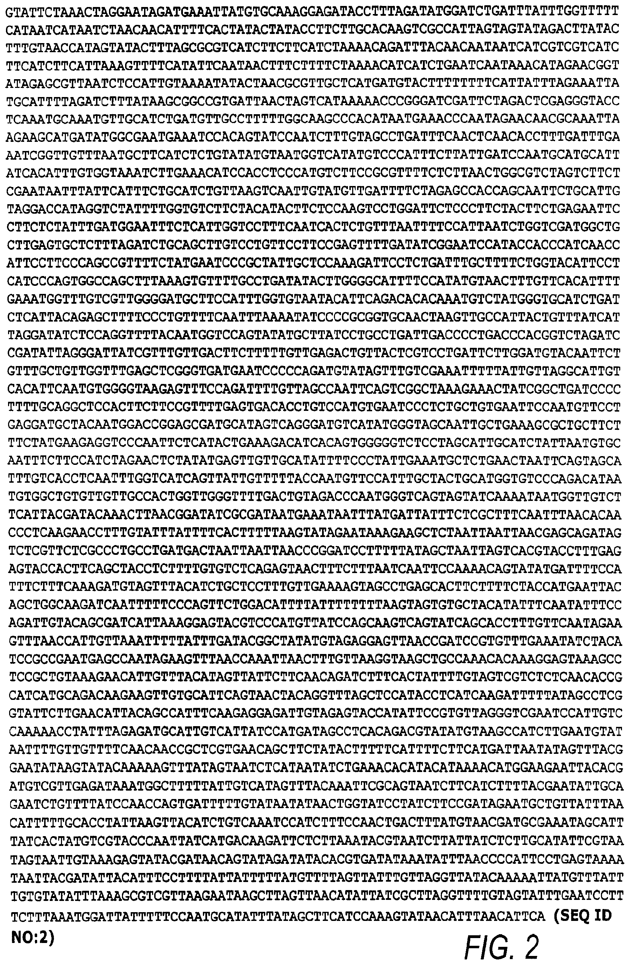

The nucleotide sequence of the donor plasmids pJT004 and pJT005 are presented in FIGS. 1 and 2.

Sera was collected and tested against H3N8 influenza viruses and the other viruses. As shown in Table 1, canarypox expressing hemagglutinin H3 genes induced a substantial amount of antibodies which specifically reacted with H3N8 strains but not with H1N1 or H7N7. Importantly antibodies were detectable within two weeks after the first immunization.

Accordingly, a canarypox expressing an influenza HA gene is immunogenic in dogs.

Table 1: Vaccination of dogs with canarypox expressing H3 hemaglutinin genes

| Antigen | Bleed | VACCINATED GROUP | | | | | CONTROL GROUP | | | | |
|---|---|---|---|---|---|---|---|---|---|---|---|
| N/1/93 (H3N8) | D0 | 0 | 0 | 0 | 0 | 0 | 0 | 0 | 0 | 0 | 0 |
| | D14 | 0 | 63.8 | 69.9 | 31.4 | 96.7 | n.s. | n.s. | n.s. | n.s. | n.s. |
| | D21 | 104.1 | 91.4 | 121.5 | 74.6 | 121.5 | n.s. | n.s. | n.s. | n.s. | n.s. |
| | D36 | 106 | 96.7 | 111.7 | 69.9 | 123.6 | n.s. | n.s. | n.s. | n.s. | n.s. |
| | D51 | 76.2 | 55.1 | 74.6 | 38.3 | 69.9 | n.s. | n.s. | n.s. | n.s. | n.s. |
| N/2/93 (H3N8) | D0 | 0 | 0 | 0 | 0 | 0 | 0 | 0 | 0 | 0 | 0 |
| | D14 | 0 | 25.1 | 59.4 | 29.2 | 60.8 | n.s. | n.s. | n.s. | n.s. | n.s. |
| | D21 | 86.2 | 71.4 | 102.2 | 65.3 | 406 | n.s. | n.s. | n.s. | n.s. | n.s. |

(continued)

| Antigen | Bleed | VACCINATED GROUP | | | | | CONTROL GROUP | | | | |
|---|---|---|---|---|---|---|---|---|---|---|---|
| | D36 | 82.8 | 74.6 | 96.7 | 62.3 | 100.4 | n.s. | n.s. | n.s. | n.s. | n.s. |
| | D51 | 52.3 | 37.1 | 66.8 | 26.1 | 57.9 | n.s. | n.s. | n.s. | n.s. | n.s. |
| Pr/56 (H7N7) | D0 | 0 | 0 | 0 | 0 | 0 | 0 | 0 | 0 | 0 | 0 |
| | D14 | 0 | 19.3 | 0 | 24.1 | 0 | n.s. | n.s. | n.s. | n.s. | n.s. |
| | D21 | 0 | 0 | 0 | 0 | 0 | n.s. | n.s. | n.s. | n.s. | n.s. |
| | D36 | 0 | 0 | 0 | 0 | 0 | n.s. | n.s. | n.s. | n.s. | n.s. |
| | D51 | 0 | 0 | 0 | 0 | 0 | n.s. | n.s. | n.s. | n.s. | n.s. |
| PR8 (H1N1) | D0 | 0 | 0 | 0 | 0 | 0 | 0 | 0 | 0 | 0 | 0 |
| | D14 | 0 | 16.7 | 0 | 19.3 | 0 | n.s. | n.s. | n.s. | n.s. | n.s. |
| | D21 | 0 | 0 | 0 | 0 | 0 | n.s. | n.s. | n.s. | n.s. | n.s. |
| | D36 | 0 | 0 | 0 | 0 | 0 | n.s. | n.s. | n.s. | n.s. | n.s. |
| | D51 | 0 | 0 | 0 | 0 | 0 | 0 | 0 | 0 | 0 | 0 |
| n.s. = no sample | | | | | | | | | | | |

EXAMPLE 2: Contruction of the donor plasmid pALVAC C5 H6p-synthetic EIV H3 HA, pJY1571.1)

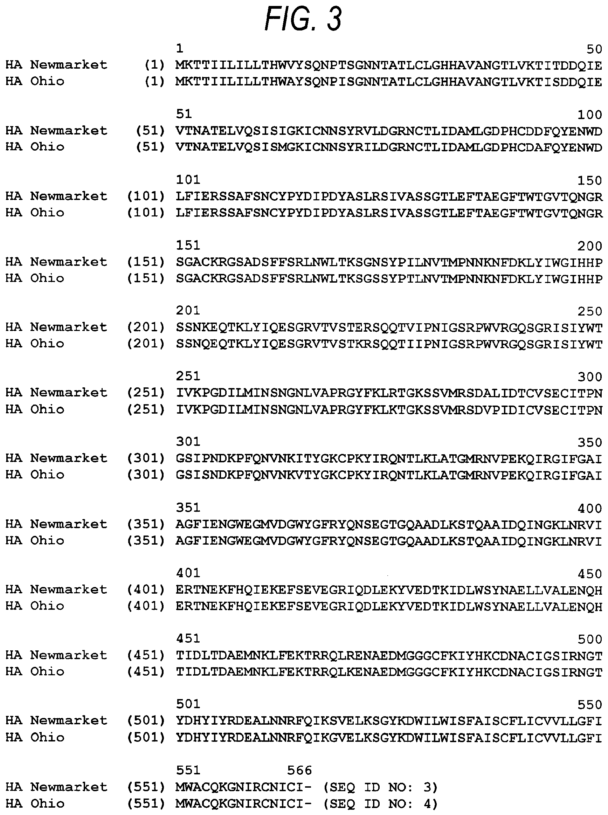

The HA gene was derived from equine influenza virus (EIV) H3N8 Ohio 03 strain isolated from a horse in 2003. The HA gene was synthetic with codon optimization for expression in mammlian cells. The amino acid sequence of EIV Ohio 03 strain HA was compared to that of New Market strain H3 HA and is presented in FIG. 3.

The purpose was the construction of an ALVAC donor plasmid for generation of an ALVAC recombinant expressing codon optimized EIV H3 HA (Ohio 03). The plasmid name was pALVAC C5 H6p-synthetic EIV H3 HA, pJY1571.1. The plasmid backbone is pALVAC C5 H6p. The promoter was a H6 promoter.

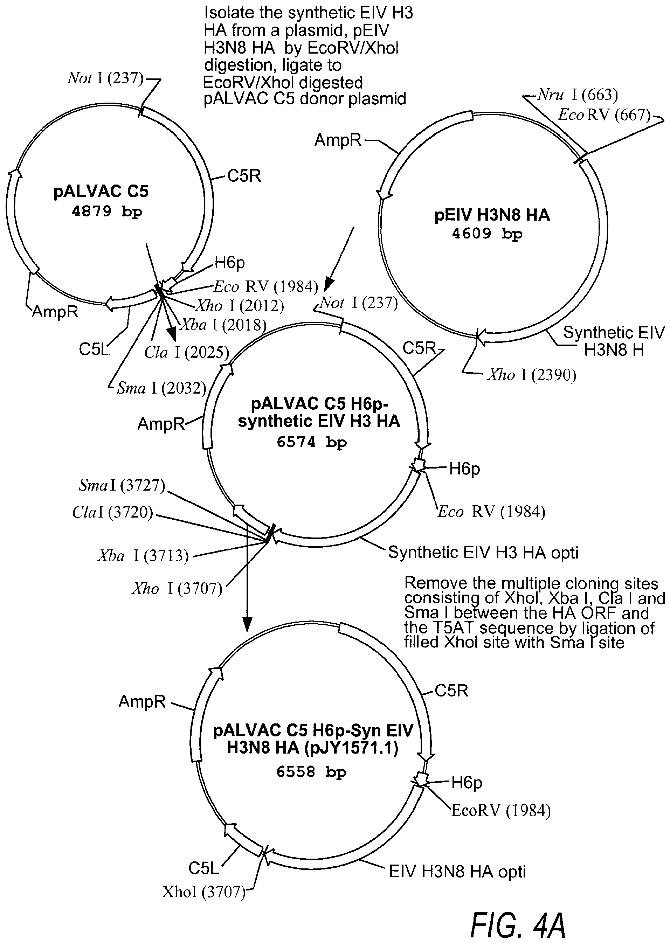

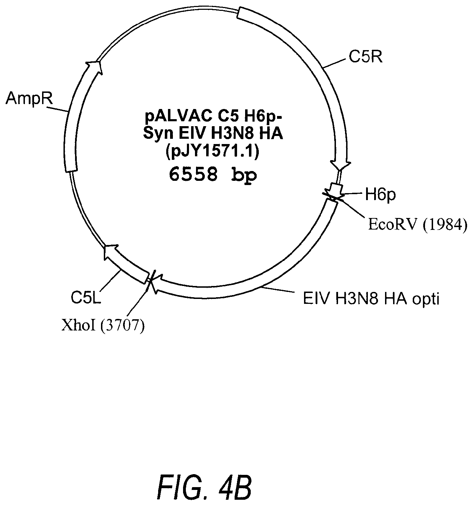

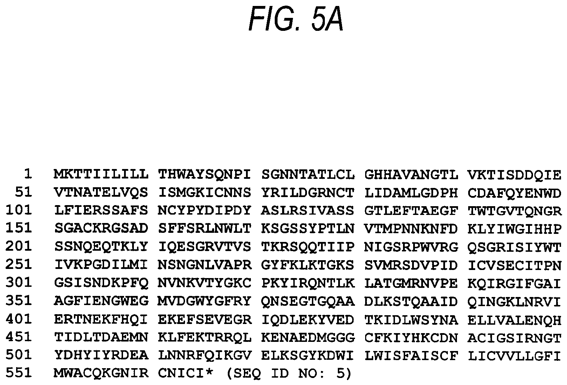

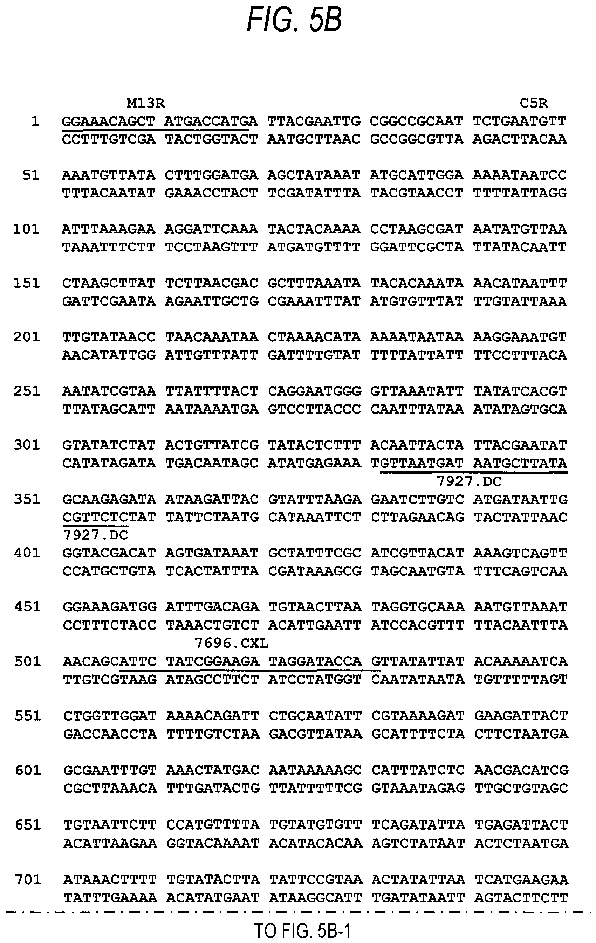

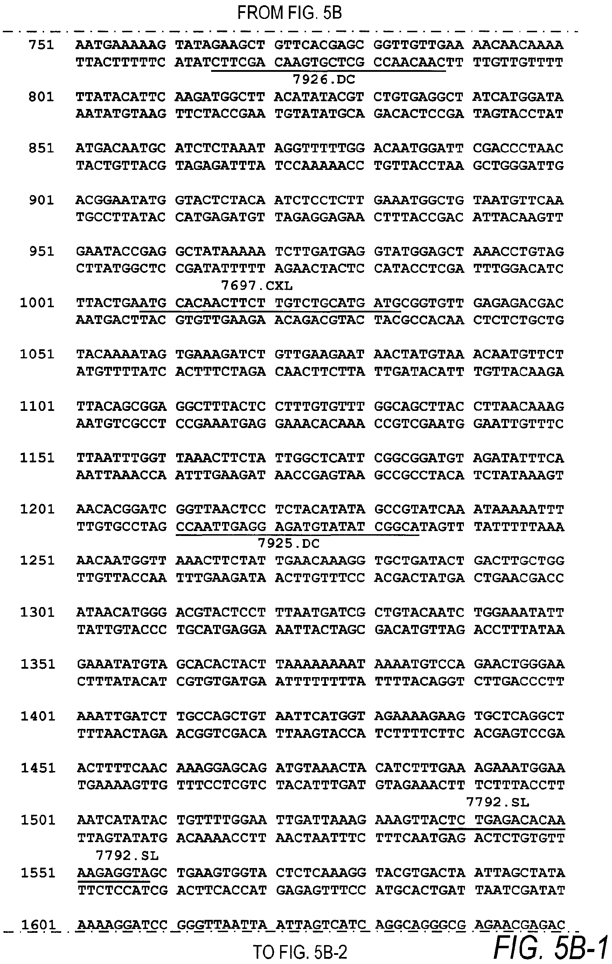

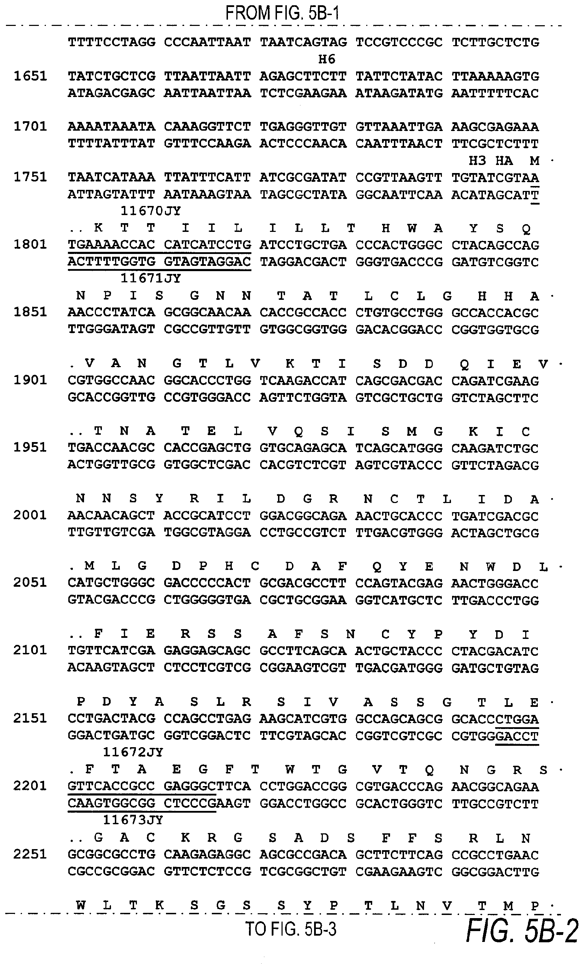

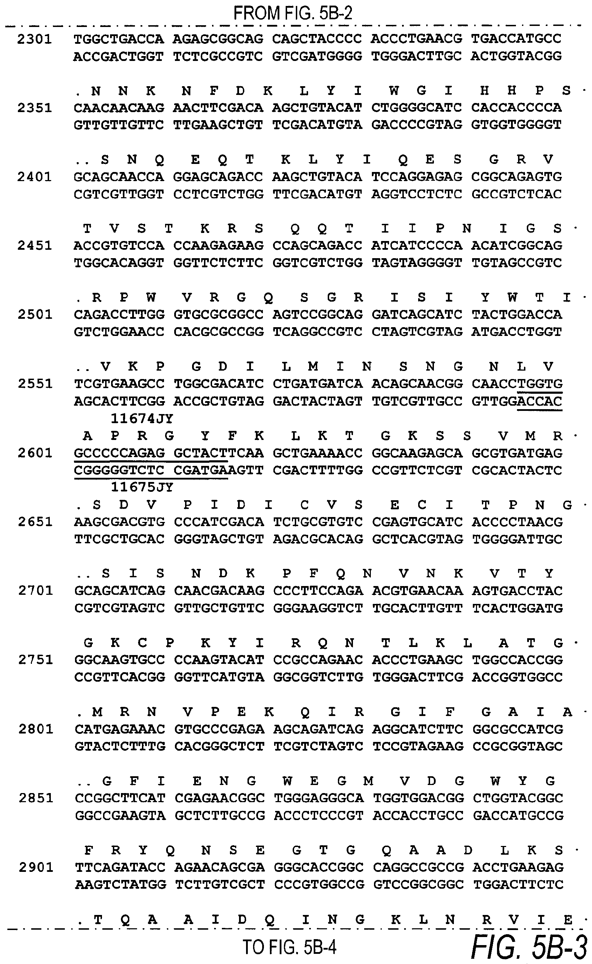

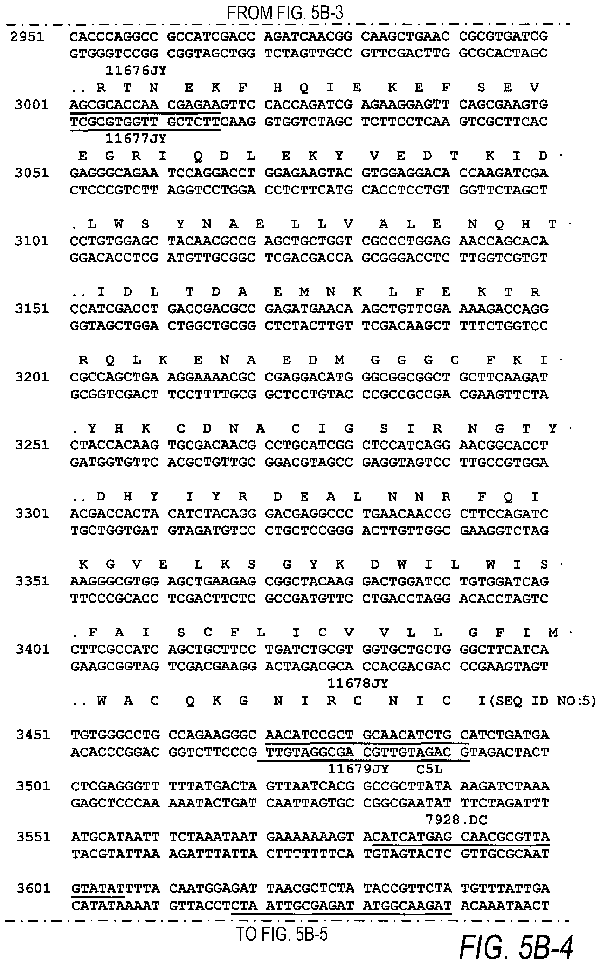

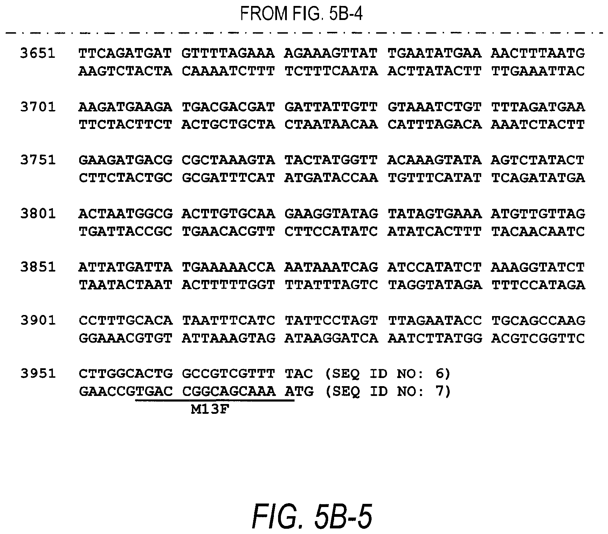

The description of plasmid construction was as follows and presented schematically in FIG. 4A. The synthetic EIV H3 HA (Ohio 03) was isolated from a plasmid pEIV H3N8 HA by EcoRV/XhoI digestion, and ligated to EcoRV/XhoI digested donor plasmid pALVAC C5 H6p to create pALVAC C5 H6p-Synthetic EIV H3 HA. In the resulting plasmid, there are multiple cloning sites consisting of XhoI, Xba I, Cla I and Sma I between the HA ORF and the T5AT sequence which serves as the transcription termination signal. To bring the HA ORF and the T5AT sequence close together, those cloning sites were then subsequently removed by ligation of re-filled XhoI site with Sma 1 site. The resulting plasmid pJY1571.1 was then sequenced and confirmed to contain the correct sequence. A diagram of the resulting plasmid is presented in FIG. 4B. The predicted amino acid sequence of EIV H3 HA is shown in FIG. 5A and the nucleotide sequence of arms and insert with translation is shown in FIG. 5B.

EXAMPLE 3: Construction of the recombinant canarypox vCP2242

The purpose of this example was the generation and characterization of ALVAC recombinant containing EIV H3N8 codon optimized HA inserted at C5 loci of ALVAC (vCP2242). The parental virus was ALVAC, the donor plasmid was pJY1571.1, the insertion site was a C5 Locus, the promoter was a H6 promoter and cells for in vitro recombination were primary chicken embryo fibroblast cells (1°CEF).

The in vitro recombination (IVR) was performed by transfection of 1°CEF cells with 15 $\mu$g of Not I-linearized donor plasmid pJY1571.1. The transfected cells were subsequently infected with ALVAC as rescue virus at MOI of 10. After 24 hr, the transfected-infected cells were harvested, sonicated and used for recombinant virus screening.

Recombinant plaques were screened based on the plaque lift hybridization method using a 821 bp EIV syn HA specific probe labeled with horse radish peroxidase (HRP) according to the manufacturer's protocol. After four sequential rounds of plaque purification, the recombinant designated as vCP2242 was generated and confirmed by hybridization as 100% positive for the EIV syn HA insert and 100% negative for the C5 ORF.

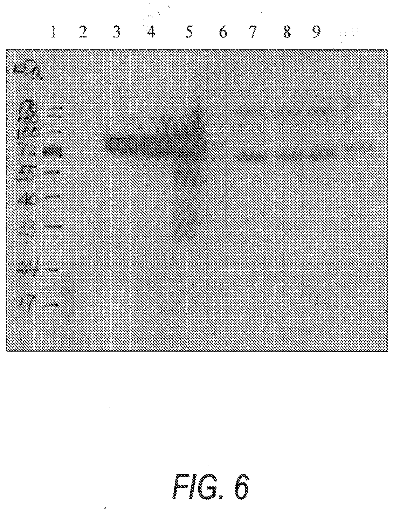

Expression analysis and sequence analysis were performed. Expression analysis was performed by Western blot. Primary CEF cells were infected with vCP2242 stock at MOI of 10 and incubated at 37 C for 26.5 hrs. The cells and culture supernatant were then harvested. Sample proteins were separated on a 10% SDS-PAGE gel, transferred to Immobilon nylon membrane, and probed with a pool of monoclonal mouse anti-EIV HA antibodies (anti Eq/AK/91: 124-1D9-1, 124-3E3-3, 124-4F3-2 and H3N8 A Eq/miami/63 pool at 1/1,000 dilution). Peroxidase-conjugated goat anti-mouse antiserum was used as a secondary antibody and the bands were visualized using luminol reagents. vCP2242 showed a protein expression profile with a 80 kDa protein expressed in cell pellet, but not in the culture medium as

presented in FIG. 6.

Results of the sequence analysis demonstrated that the sequences of the EIV syn HA and C5L and C5R of ALVAC were correct.

EXAMPLE 4: Kentucky Equine Influenza Nucleotide Sequences

DEFINITION Influenza A virus (A/equine/Kentucky/5/02(H3N8)) nonstructural protein gene, complete cds.

ACCESSION AY855345; SEQ ID NO: 8

```
  1 agcaaaagca gggtgacaaa aacataatgg attccaacac tgtgtcaagc tttcaggtag
 61 actgttttct ttggcatgtc cgcaaacgat tcgcagacca agaactgggt gatgccccat
121 tccttgaccg gcttcgccga gaccagaagt ccctaagggg aagaggtatc actcttggtc
181 tggacatcga aacagccact catgcaggaa agcagatagt ggagcagatt ctggaaaagg
241 aatcagatga ggcacttaaa atgaccattg cctctgttcc tacttcacgc tacttaactg
301 acatgactct tgatgagatg tcaagagact ggttcatgct catgcccaag caaaaagtaa
361 caggctccct atgtataaga atggaccagg caatcatgga taagaacatc atacttaaag
421 caaactttag tgtgattttc gaaaggctgg aaacactaat actacttaga gccttcaccg
481 aagaaggagc agtcgttggc gaaatttcac cattaccttc tcttccagga catactaatg
541 aggatgtcaa aaatgcaatt ggggtcctca tcggaggact taaatggaat gataatacgg
601 ttagaatctc tgaaactcta cagagattcg cttggagaag cagtcatgag aatgggagac
661 cttcattccc ttcaaagcag aaatgaaaaa tggagagaac aattaagcca gaaatttgaa
721 gaaataagat ggttgattga agaagtgcga catagattga aaaatacaga aaatagtttt
781 gaacaaataa catttatgca agccttacaa ctattgcttg aagtagaaca agagataaga
841 actttctcgt ttcagcttat ttaatgataa aaaacaccct tgtttctact
```

DEFINITION Influenza A virus (A/equine/Kentucky/5/02(H3N8)) matrix protein gene, complete cds.

ACCESSION AY855344; SEQ ID NO: 9

```
  1 agcaaaagca ggtagatatt taaagatgag tcttctgacc gaggtcgaaa cgtacgttct
 61 ctctatcgta ccatcaggcc ccctcaaagc cgagatcgcg cagagacttg aagatgtctt
121 tgcagggaag aacaccgatc ttgaggcact catggaatgg ctaaagacaa gaccaatcct
181 gtcacctttg actaaaggga ttttaggatt tgtattcacg ctcaccgtgc ccagtgagcg
241 aggactgcag cgtagacgct ttgtccaaaa tgcccttagt ggaaacggag atccaaacaa
301 catggacaga gcagtaaaac tgtacaggaa gcttaaaaga gaaataacat tccatggggc
361 aaaagaggtg gcactaagct attccactgg tgcactagcc agctgcatgg gactcatata

 421 caacagaatg ggaactgtga caaccgaagt ggcatttggc ctggtatgcg ccacatgtga
 481 acagattgct gattcccagc atcgatctca caggcagatg gtgacaacaa ccaacccatt
 541 aatcagacat gaaaacagaa tggtattagc cagtaccacg gctaaagcca tggaacagat
 601 ggcaggatca agtgagcagg cagcagaggc catggaggtt gctagtaagg ctaggcagat
 661 ggtacaggca atgagaacca ttgggaccca ccctagctcc agtgccggtt tgaaagatga
 721 tctccttgaa aatttacagg cctaccagaa acggatggga gtgcaaatgc agcgattcaa
 781 gtgatcctct cgttattgca gcaagtatca ttgggatctt gcacttgata ttgtggattc
 841 ttgatcgtct tttcttcaaa ttcatttatc gtcgccttaa atacgggttg aaaagagggc
 901 cttctacgga aggagtacct gagtctatga gggaagaata tcggcaggaa cagcagaatg
 961 ctgtggatgt tgacgatggt cattttgtca acatagagct ggagtaaaaa actaccttgt
1021 ttctact
```

DEFINITION Influenza A virus (A/equine/Kentucky/5/02(H3N8)) neuraminidase gene, complete cds.

ACCESSION AY855343; SEQ ID NO: 10

```
   1 agcaaaagca ggagtttaaa atgaatccaa atcaaaagat aatagcaatt ggatttgcat
  61 cattggggat attaatcatt aacgtcattc tccatgtagt cagcattata gtaacagtac
 121 tggtcctcaa taacaatgga acaggtctga actgcaaagg gacgatcata agagagtaca
 181 atgaaacagt aagagtagaa aaaattactc aatggtataa taccagtgca attaagtaca
 241 tagagagacc tccaaatgaa tactacatga acaacaccga accactttgt gaggcccaag
 301 gctttgcacc attttccaaa gataatggaa tacgaattgg gtcgagaggc catgtttttg
 361 tgataagaga accttttgta tcatgttcgc cctcagaatg tagaaccttt ttcctcacac
 421 agggctcatt actcaatgac aaacattcta acggcacagt aaaggaccga agtccatata
 481 ggactttgat gagtgtcaaa atagggcaat cacctaatgt gtatcaagct aggtttgaat
 541 cggtggcatg gtcagcaaca gcatgccatg atggaaaaaa atggatgaca gttggagtca
 601 cagggcccga caatcaagca attgcagtag tgaactatgg aggtgttccg gttgatatta
 661 ttaattcatg ggcaggggat atcttaagaa cccaagaatc atcatgcacc tgcattaaag
 721 gagactgtta ttgggtaatg actgatggac cggcaaatag gcaagctaaa tataggatat
 781 tcaaagcaaa agatggaaga gtaattggac agactgatat aagtttcaat gggggacaca
 841 tagaggagtg ttcttgttac cccaatgaag ggaaggtgga atgcatatgc agggacaatt
 901 ggactggaac aaatagacca attctggtaa tatcttctga tctatcgtac acagttggat
 961 atttgtgtgc tggcattccc actgacactc ctaggggaga ggatagtcaa ttcacaggct
1021 catgtacaag acctttggga aataaaggat acggtgtaaa aggtttcggg tttcgacaag
1081 gaactgacgt atgggccgga aggacaatta gtaggacttc aagatcagga ttcgaaataa
1141 taaaaatcag gaatggttgg acacagaaca gtaaagacca aatcaggagg caagtgatta
1201 tcgatgaccc aaattggtca ggatatagcg gttctttcac attgccggtt gaactaacaa
1261 aaaagggatg tttggtcccc tgtttctggg ttgaaatgat tagaggtaaa cctgaagaaa
1321 caacaatatg gacctctagc agctccattg tgatgtgtgg agtagatcat aaaattgcca
1381 gttggtcatg gcacgatgga gctattcttc cctttgacat cgataagatg taatttacga
1441 aaaaactcct tgtttctact
```

DEFINITION Influenza A virus (A/equine/Kentucky/5/02(H3N8)) nucleoprotein gene, complete cds.

ACCESSION AY855342; SEQ ID NO: 11

```
   1 agcaaaagca gggtagataa tcactcactg agtgacatca aagtcatggc gtctcaaggc
  61 accaaacgat cctatgaaca gatggaaact gatggggaac gccagaatgc aactgaaatc
 121 agagcatctg tcggaaggat ggtgggagga atcggccggt tttatgttca gatgtgtact
 181 gagcttaaac taaacgacca tgaagggcgg ctgattcaga acagcataac aatagaaagg
 241 atggtacttt cggcattcga cgaaagaaga aacaagtatc tcgaggagca tcccagtgct
 301 ggaaaagacc ctaagaaaac gggaggcccg atatacagaa ggaaagatgg gaaatggatg
 361 agggaactca tcctccatga taaagaagaa atcatgagaa tctggcgtca ggccaacaat
 421 ggtgaagacg ctactgctgg tcttactcat atgatgatct ggcactccaa tctcaatgac
 481 accacatacc aaagaacaag ggctcttgtt cggactggga tggatcccag aatgtgctct
 541 ctgatgcaag gctcaaccct cccacggaga tctggagccg ctggtgctgc agtaaaaggt
 601 gttggaacaa tggtaatgga actcatcaga atgatcaaac gcggaataaa tgatcggaat
 661 ttctggagag gtgaaaatgg tcgaaggacc agaattgctt atgaaagaat gtgcaatatc
 721 ctcaaaggga aatttcagac agcagcacaa cgggctatga tggaccaggt gagggaaggc
 781 cgcaatcctg gaaacgctga gattgaggat ctcattttct tagcacgatc agcacttatt

 841 ttgagaggat cagtagccca taaatcatgc ctacctgcct gtgtttatgg ccttgcagta
 901 accagtgggt atgactttga gaaggaagga tactctctgg ttggaattga tcctttcaaa
 961 ctactccaga acagtcaaat tttcagtcta atcagaccaa aagaaaaccc agcacacaag
1021 agccagttgg tgtggatggc atgccattct gcagcatttg aggacctgag agttttgaat
1081 ttcattagag gaaccaaagt aatcccaaga ggacagttaa caaccagagg agttcaaatt
1141 gcttcaaatg aaaacatgga gacaatagat tctagcacac ttgaactgag aagcaaatat
1201 tgggcaataa ggaccagaag tggaggaaac accagtcaac agagagcatc tgcaggacag
1261 ataagtgtgc aacctacttt ctcagtacag agaaatcttc cctttgagag agcaaccatt
1321 atggctgcat tcactggtaa cactgaaggg aggacttccg acatgagaac ggaaatcata
1381 aggatgatgg aaagtgccaa atcagaagat gtgtctttcc aggggcgggg agtcttcgag
1441 ctctcggacg aaaaggcaac gaacccgatc gtgccttcct ttgacatgag caatgaaggg
1501 tcttatttct tcggagacaa tgctgaggaa tttgacagtt aaagaaaaat acccttgttt
1561 ctact
```

DEFINITION Influenza A virus (A/equine/Kentucky/5/02(H3N8)) hemagglutinin precursor, gene, complete cds.

ACCESSION AY855341; SEQ ID NO: 12

```
   1 agcaaaagca ggggatattt ctgtcaatca tgaagacaac cattattttg atactactga
  61 cccattgggc ctacagtcaa aacccaatca gtggcaacaa cacagccaca ttgtgtctgg
 121 gacaccatgc agtagcaaat ggaacattgg taaaaacaat aagtgatgat caaattgagg
 181 tgacaaatgc tacagaatta gttcagagca tttcaatggg gaaaatatgc aacaactcat
 241 atagaattct agatggaaga aattgcacat taatagatgc aatgctagga gaccccccact
 301 gtgacgtctt tcagtatgag aattgggacc tctttataga aagaagcagc gctttcagca
 361 attgctaccc atatgacatc cctgactatg catcgctccg atccattgta gcatcctcag
 421 gaacattgga attcacagca gagggattca catggacagg tgtcactcaa aacggaagaa
 481 gtggagcctg caaaagggga tcagccgata gtttctttag ccgactgaat tggctaacaa
 541 aatctggaaa ctcttacccc acattgaatg tgacaatgcc taacaataaa aatttcgaca
 601 agctatacat ctgggggatt catcacccga gctcaaatca agagcagaca aaattgtaca
 661 tccaagaatc aggacgagta acagtctcaa caaaaagaag tcaacaaaca ataatcccta
 721 acatcggatc tagaccgtgg gtcagaggtc aatcaggcag gataagcata tactggacca
 781 ttgtaaaacc tggagatatc ctaatgataa acagtaatgg caacttagtt gcaccgcggg
 841 gatattttaa attgaaaaca gggaaaagct ctgtaatgag atcagatgca cccatagaca
 901 tttgtgtgtc tgaatgtatt acaccaaatg gaagcatctc caacgacaag ccattccaaa
 961 atgtgaacaa agttacatat ggaaaatgcc ccaagtatat caggcaaaac actttaaagt
1021 tggccactgg gatgaggaat gtaccagaaa agcaaatcag aggaatcttt ggagcaatag
1081 cgggattcat cgaaaacggc tgggaaggaa tggttgatgg gtggtatggg ttccgatatc
1141 aaaactctga aggaacaggg caagctgcag atctaaagag cactcaagca gccatcgacc
1201 agattaatgg aaagttaaac agagtgattg aaagaaccaa tgagaaattc catcaaatag
1261 agaaggaatt ctcagaagta gaaggaagaa ttcaggactt ggagaaatat gtagaagaca
1321 ccaaaataga cctatggtcc tacaatgcag aattgctggt ggctctagaa aatcaacata
1381 caattgactt aacagatgca gaaatgaata aattattcga gaagactaga cgccagttaa
1441 gagaaaacgc agaagacatg ggaggtggat gtttcaagat ttaccacaaa tgtgataatg
1501 catgcattgg atcaataaga aatgggacat atgaccatta catatacaga gatgaagcat
1561 taaacaaccg atttcaaatc aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct
```

DEFINITION Influenza A virus (A/equine/Kentucky/5/02(H3N8)) PA polymerase gene, complete cds.

ACCESSION AY855340; SEQ ID NO: 13

```
   1 agcgaaagca ggtactgatc caaaatggaa gactttgtgc gacagtgctt caatccaatg
  61 atcgtcgagc ttgcggaaaa ggcaatgaaa gaatatggag aggacccgaa aatcgaaaca
 121 aacaaatttg cagcaatatg cactcacttg gaagtctgct tcatgtactc ggatttccac
 181 tttattaatg aactgggtga gtcagtggtc atagagtctg gtgacccaaa tgctcttttg
 241 aaacacagat ttgaaatcat tgaggggaga gatcgaacaa tggcatggac agtggtaaac
```

```
 301 agcatctgca acaccacaag agctgaaaaa cctaaatttc ttccagattt atacgactat
 361 aaggagaaca gatttgttga aattggtgtg acaaggagag aagttcacat atactacctg
 421 gagaaggcca acaaaataaa gtctgagaaa acacatatcc acattttctc atttacagga
 481 gaggaaatgg ctacaaaagc ggactatact cttgatgaag agagtagagc caggatcaag
 541 accagactat tcactataag acaagaaatg gccagtagag gcctctggga ttcctttcgt
 601 cagtccgaga gaggcgaaga gacaattgaa gaaagatttg aaatcacagg gacgatgcgc
 661 aagcttgcca attacagtct cccaccgaac ttctccagcc ttgaaaattt tagagtctat
 721 gtggatggat tcgaaccgaa cggctgcatt gagagtaagc tttctcaaat gtccaaagaa
 781 gtaaatgcca gaatcgaacc attttcaaag acaacacccc gaccactcaa aatgccaggt
 841 ggtccaccct gccatcagcg atctaaattc ttgctaatgg atgctctgaa actgagcatt
 901 gaggacccaa gtcacgaggg agagggaata ccactatatg atgcaatcaa atgcatgaaa
 961 actttctttg gatggaaaga gcccagtatt gttaaaccac atgaaaaggg tataaacccg
1021 aactatctcc aaacttggaa gcaagtatta gaagaaatac aagaccttga gaacgaagaa
1081 aggacccccca agaccaagaa tatgaaaaaa acaagccaat tgaaatgggc actaggtgaa
1141 aatatggcac cagagaaagt ggattttgag gattgtaaag acatcagtga tttaaaacag
1201 tatgacagcg atgagccaga aacaaggtct cttgcaagtt ggattcaaag tgagttcaac
1261 aaagcttgtg agctgacaga ttcaagctgg atagagctcg atgaaattgg ggaggatgtc
1321 gccccaatag aatacattgc gagcatgagg agaaattatt ttactgctga gatttcccat
1381 tgtagagcaa cagaatatat aatgaaagga gtgtacatca acactgctct actcaatgca
1441 tcctgtgctg cgatggatga atttcaatta attccgatga taagtaaatg caggaccaaa
1501 gaagggagaa ggaaaacaaa tttatatgga ttcataataa agggaaggtc ccatttaaga
1561 aatgatactg acgtggtgaa ctttgtaagt atggaatttt ctctcactga tccaagattt
1621 gagccacaca aatgggaaaa atactgcgtt ctagaaattg gagacatgct tctaaggact
1681 gctgtaggtc aagtgtcaag acccatgttt ttgtatgtaa ggacaaatgg aacctctaaa
1741 attaaaatga aatggggaat ggaaatgagg cgctgcctcc ttcagtctct gcaacagatt
1801 gaaagcatga tcgaagctga gtcctcagtc aaagaaaagg acatgaccaa agaatttttt
1861 gagaacaaat cagagacatg gcctatagga gagtccccca aaggagtgga agagggctca
1921 atcgggaagg tttgcaggac cttattagca aaatctgtgt ttaacagttt atatgcatct
1981 ccacaactgg aagggttttc agctgaatct aggaaattac ttctcattgt tcaggctctt
2041 agggataacc tggaacctgg aacctttgat attgggggt tatatgaatc aattgaggag
2101 tgcctgatta atgatccctg ggttttgctt aatgcatctt ggttcaactc cttccttaca
2161 catgcactga agtagttgtg gcaatgctac tatttgctat ccatactgtc caaaaaagta
2221 ccttgtttct act
```

DEFINITION Influenza A virus (A/equine/Kentucky/5/02(H3N8)) PB1 polymerase 1 gene, complete cds.

ACCESSION AY855339; SEQ ID NO: 14

```
   1 agcgaaagca ggcaaaccat ttgaatggat gtcaatccga ctctactttt cttaaaggtg
  61 ccagcgcaaa atgctataag cacaacattc ccttatactg gagatcctcc ctacagtcat
 121 ggaacaggga caggatacac catggatact gtcaacagaa cacaccaata ttcagaaaag
 181 gggaaatgga caacaaacac tgagattgga gcaccacaac ttaatccaat cgatggacca
 241 cttcctgaag acaatgaacc aagtgggtac gcccaaacag attgtgtatt ggaagcaatg
 301 gctttccttg aagaatccca tcccggaatc tttgaaaatt cgtgtcttga aacgatggag
 361 gtgattcagc agacaagagt ggacaaacta acacaaggcc gacaaactta tgattggacc
 421 ttgaatagga atcaacctgc cgcaacagca cttgctaata caattgaagt gttcagatca
 481 aatggtctga cttccaatga atcagggagg ttgatggact tcctcaaaga tgtcatggag
 541 tccatgaaca aggaagaaat ggaaataaca acacacttcc aacgaaagag aagagtaaga
 601 gacaacatga caaagagaat ggtaacacag agaaccatag ggaagaaaaa acaacgatta
 661 aacagaaaga gttatctaat cagaacatta accctaaaca caatgaccaa ggacgctgag
 721 agagggaaat tgaaacgacg agcaatcgca accccaggga tgcagataag aggatttgta
 781 tattttgttg aaacactagc ccgaagaata tgtgaaaagc ttgaacaatc aggattgcca
 841 gttggcggta atgagaaaaa ggccaaactg gctaatgtcg tcagaaaaat gatgactaat
 901 tcccaagaca ctgaactctc cttcaccatc actggggaca ataccaaatg gaatgaaaat
 961 cagaacccac gcatattcct ggcaatgatc acatacataa ctagaaacca gccagaatgg
1021 ttcagaaatg ttctaagcat tgcaccgatt atgttctcaa ataaaatggc aagactgggg
1081 aaaggatata tgtttgaaag caaaagtatg aaactgagag ctcaaatacc agcagaaatg
1141 ctagcaagca ttgacctgaa atatttcaat gattcaacaa aaaagaaaat taaaaagata
1201 cgaccacttc tggttgacgg gactgcttca ctgagtcctg gcatgatgat gggaatgttc
1261 aacatgttga gcactgtgct gggtgtatcc atattaaacc tgggccagag gaaatacaca

1321 aagaccacat actggtggga tggtctgcaa tcatccgatg actttgcttt gatagtgaat
1381 gcgcctaatc atgaaggaat acaagctgga gtagacagat tctatagaac ttgcaaactg
1441 gtcgggatca acatgagcaa aaagaagtcc tacataaata gaaccggaac attcgaattc
1501 acaagctttt tctaccggta tggttttgta gccaatttca gcatggaact acccagtttt
1561 ggggtttccg gaataaatga atctgcagac atgagcattg gagtgacagt catcaaaaac
1621 aacatgataa ataatgatct cggtcctgcc acggcacaaa tggcactcca actcttcatt
1681 aaggattacc ggtacacata ccggtgccat agaggtgata cccagataca aaccagaaga
1741 tcttttgagt tgaagaaact gtgggaacag actcgatcaa agactggtct actggtatca
1801 gatgggggtc caaacctata taacatcaga aacctacaca tcccggaagt ttgtttaaaa
1861 tgggagctaa tggatgaaga ttataagggg aggctatgca atccattaaa tcctttcgtt
1921 agtcacaaag aaattgaatc agtcaacagt gcagtagtaa tgcctgcgca tggccctgcc
1981 aaaagcatgg agtatgatgc tgttgcaaca acacattctt ggatccccaa gaggaaccgg
2041 tccatattga acacaagcca aaggggaata ctcgaagatg agcagatgta tcagaaatgc
2101 tgcaacctgt ttgaaaaatt cttccccagc agctcataca gaagaccagt cggaatttct
2161 agtatggttg aggccatggt gtccagggcc cgcattgatg cacgaattga cttcgaatct
2221 ggacggataa agaaggatga gttcgctgag atcatgaaga tctgttccac cattgaagag
2281 ctcagacggc aaaaatagtg aatttagctt gatcttcatg aaaaaatgcc ttgtttctac
2341 t
```

DEFINITION Influenza A virus (A/equine/Kentucky/5/02(H3N8)) PB2 polymerase 2 gene, complete cds.

ACCESSION AY855338; SEQ ID NO: 15

```
   1 agcgaaagca ggtcaaatat attcaatatg gagagaataa aagaactgag agatctgatg
  61 ttacaatccc gcacccgcga gatactaaca aaaactactg tggaccacat ggccataatc
 121 aagaaataca catcaggaag acaagagaag aaccctgcac ttaggatgaa atggatgatg
 181 gcaatgaaat acccaattac agcagataag aggataatgg agatgattcc tgagagaaat
 241 gaacagggac aaaccctttg gagcaaaacg aacgatgctg gctcagaccg cgtaatggta
 301 tcacctctgg cagtgacatg gtggaatagg aatggaccaa caacgagcac aattcattat
 361 ccaaaagtct acaaaactta ttttgaaaag gttgaaagat tgaaacacgg aacctttggc
 421 cccgttcatt ttaggaatca agtcaagata agacgaagag ttgatgtaaa ccctggtcac
 481 gcggacctca gtgccaaaga agcacaagat gtgatcatgg aagttgtttt cccaaatgaa
 541 gtgggagcca gaattctaac atcggaatca caactaacaa taaccaaaga gaaaaaggaa
 601 gaacttcagg actgcaaaat tgctcccttg atggtagcat acatgctaga aagagagttg
 661 gtccgaaaaa caaggttcct cccagtggca ggcggaacaa gcagtgtata cattgaagtg
 721 ttgcatctga ctcagggaac atgctgggag caaatgtaca ccccaggagg agaagttaga
 781 aacgatgata ttgatcaaag tttaattatt gcagcccgga acatagtgag aagagcgaca
 841 gtatcagcag atccactagc atccctactg gaaatgtgcc acagtacaca gattggtgga
 901 ataaggatgg tagacatcct taagcagaat ccaacagagg aacaagctgt ggatatatgc
 961 aaagcagcaa tgggattgag aattagctca tcattcagct ttggtggatt caccttcaaa
1021 agaacaagtg gatcatcagt caagagagaa gaagaaatgc ttacgggcaa ccttcaaaca
1081 ttgaaaataa gagtgcatga gggctatgaa gaattcacaa tggtcggaag aagagcaaca
1141 gccattctca gaaaggcaac cagaagattg attcaattga tagtaagtgg gagagatgaa
1201 caatcaattg ctgaagcaat aattgtagcc atggtgtttt cgcaagaaga ttgcatgata
1261 aaagcagttc gaggcgattt gaactttgtt aatagagcaa atcagcgttt gaaccccatg
1321 catcaactct tgaggcattt ccaaaaagat gcaaaagtgc ttttccaaaa ttggggaatt
1381 gaacccatcg acaatgtaat gggaatgatt ggaatattgc ctgacatgac cccaagcacc
1441 gagatgtcat tgagaggagt gagagtcagc aaaatgggag tggatgagta ctccagcact
1501 gagagagtgg tggtgagcat tgaccgtttt ttaagagttc gggatcaaag gggaaacata
1561 ctactgtccc ctgaagaagt cagtgaaaca caaggaacgg aaaagctgac aataatttat
1621 tcgtcatcaa tgatgtggga gattaatggt cccgaatcag tgttggtcaa tacttatcaa
1681 tggatcatca ggaactggga aattgtaaaa attcagtggt cacaggaccc cacaatgtta
1741 tacaataaga tagaatttga gccattccag tccctggtcc ctagggccac cagaagccaa
1801 tacagcggtt tcgtaagaac cctgtttcag caaatgcgag atgtacttgg aacatttgat
1861 actgctcaaa taataaaact cctccctttt gccgctgctc ctccggaaca gagtaggatg
1921 cagttctctt ctttgactgt taatgtaaga ggatcgggaa tgaggatact tgtaagaggc
1981 aattccccag tgttcaacta caataaagcc actaaaaggc tcacagtcct cggaaaggat
2041 gcaggtgcgc ttactgagga cccagatgaa ggtacggctg gagtagaatc tgctgttcta
2101 agagggtttc tcattttagg taaagaaaac aagagatatg gcccagcact aagcatcaat
2161 gaactaagca aacttgcaaa aggggagaaa gccaatgtac taattgggca aggggacgta

2221 gtgttggtaa tgaaacggaa acgtgactct agcatactta ctgacagcca gacagcgacc
2281 aaaaggattc ggatggccat caattagtgt tgaattgttt aaaaacgacc ttgtttctac
2341 t
```

DEFINITION Influenza A virus (A/equine/Kentucky/1/91(H3N8)) hemagglutinin precursor (HA) gene, complete cds.

ACCESSION L39918; SEQ ID NO: 16

```
   1 agcaaaagca ggggatattt ctgtcaatca tgaagacaac cattattttg atactactga
  61 cccattgggt ctacagtcaa aacccaacca gtggcaacaa cacagccaca ttatgtctgg
 121 gacaccatgc agtagcaaat ggaacattgg taaaaacaat aactgatgac caaattgagg
 181 tgacaaatgc tactgaatta gttcagagca tttcaatagg gaaaatatgc aacaactcat
 241 atagagttct agatggaaga aattgcacat taatagatgc aatgctagga gacccccact
 301 gtgatgtctt tcagtatgag aattgggacc tcttcataga aagaagcagc gctttcagca
 361 gttgctaccc atatgacatc cctgactatg catcgctccg gtccattgta gcatcctcag
 421 gaacattgga attcacagca gagggattca catggacagg tgtcactcaa aacggaagaa
 481 gtggatcctg caaaagggga tcagccgata gtttctttag ccgactgaat tggctaacaa
 541 aatctggaaa ctcttacccc acattgaatg tgacaatgcc taacaataaa aatttcgaca
 601 aactatacat ctggggggatt catcacccga gctcaaacga agagcagaca aaattgtaca
 661 tccaagaatc aggacgagta acagtctcaa caaaaagaag tcaacaaaca ataatcccta
 721 acatcggatc tagaccgtgg gtcaggggtc aatcaggcag gataagcata tactggacca
 781 ttgtaaaacc tggagatatc ctaatgataa acagtaatgg caacttagtt gcaccgcggg
 841 gatattttaa attgaaaaca gggaaaagct ctgtaatgag atcagatgca cccatagaca
 901 tttgtgtgtc tgaatgtatt acaccaaatg gaagcatccc caacgacaaa ccatttcaaa
 961 atgtgaacaa agttacatat ggaaaatgcc ccaagtatat caggcaaaac actttaaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctgcag atctaaagag cactcaagca gccatcgacc
1201 agatcaatgg aaaattaaac agagtgattg aaaggaccaa tgagaaattc catcaaatag
1261 agaaggaatt ctcagaagta gaagggagaa tccaggattt ggagaagtat gtagaagaca
1321 ccaaaataga cctatggtcc tacaatgcag aattgctggt ggctctagaa aatcaacata
1381 caattgactt aacagatgca gaaatgaata aattattcga gaagactaga cgccagttaa
1441 gagaaaacgc ggaagacatg ggaggtggat gtttcaagat ataccacaaa tgtgataatg
1501 catgcattgg atcaataaga aatgggacat atgaccatta catatacaga gatgaagcat
1561 taaacaaccg gtttcaaatc aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct
```

DEFINITION Influenza A virus (A/equine/Kentucky/1/92(H3N8)) hemagglutinin precursor (HA) gene, complete cds.

ACCESSION L39917; SEQ ID NO: 17

```
   1 agcaaaagca ggggatattt ctgtcaatca tgaagacaac cattattttg atactactga
  61 cccattgggt ctacagtcaa aacccaacca gtggcaacaa cacagccaca ttatgtctgg
 121 gacaccatgc agtagcaaat ggaacattgg taaaaacaat aactgatgac caaattgagg
 181 tgacaaatgc tactgaatta gttcagagca tttcaatagg gaaaatatgc aacaactcat
 241 atagggttct agatggaaga aattgcacat taatagatgc aatgctagga gacccccact
 301 gtgatgtctt tcagtatgag aattgggacc tcttcataga aagaagcagc gctttcagca
 361 attgctaccc atatgacatc cctgactatg catcgctccg gtccattgta gcatcctcag
 421 gaacattaga attcacagca gagggattca catggacagg tgtcactcaa aacggaagaa
 481 gtggagcctg caaaagggga tcagccgata gtttctttag ccgactgaat tggctaacaa
 541 aatctggaaa ctcttacccc acattgaatg tgacaatgcc taacaataaa aatttcgaca
 601 aactatacat ctggggggatt catcacccga gctcaaacaa tgagcagaca aaattgtata
 661 tccaagaaac aggacgagta acagtctcaa caaaaagaag tcaacaaaca ataatcccta
 721 acatcggatc tagaccgtgg gtcaggggtc aatcaggcag gataagcata tactggacca
 781 ttgtaaaacc tggagatatc ctaatgataa acagcaatgg caacttagtt gcaccgcggg
 841 gatattttaa attgagaaca gggagaagct ctgtaatgag atcagatgca cccatagaca
```

```
 901 tttgtgtgtc tgaatgtatt acaccaaatg gaagcatccc caacgacaaa ccatttcaaa
 961 atgtgaacaa agttacatat ggaaaatgcc ccaaatatat caggcaaaac actttaaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctgcag atctaaagag cactcaagca gccatcgacc
1201 agatcaatgg aaaattaaac agagtgattg aaaggaccaa tgagaaattc catcaaatag
1261 agaaggaatt ctcagaagta gaagggagaa tccaggattt ggagaagtat gtagaagaca
1321 ccaaaataga cctatggtcc tacaatgcag aattgctagt ggctctagaa aatcaacata
1381 caattgactt aacagacgca gaaatgaata aattattcga gaagactaga cgccagttaa
1441 gagaaaacgc ggaagacatg ggaggtggat gtttcaagat ttaccacaaa tgtgataatg
1501 catgcattgg atcaataaga aatgggacat atgaccatta catatacaga gatgaagcat
1561 taaacaaccg atttcaaatc aaaggtgttg aattaaaatc aggctacaaa gattggatac
1621 tgtggatttc attcgccata tcatgcttct taatttgtgt tgttctattg ggtttcatta
1681 tgtgggcttg ccaaaaaggc aacatcagat gcaacatttg catttgagta aactgatagt
1741 taaaaacacc cttgtttcta ct
```

DEFINITION Influenza A virus (A/equine/Kentucky/1/90(H3N8)) hemagglutinin precursor (HA) gene, complete cds.

ACCESSION L39915; SEQ ID NO: 18

```
   1 agcaaaagca ggggatattt ctgtcaatca tgaaaacaac cattattttg atactactga
  61 cccattgggt ctacagtcaa aacccaacca gtggcaacaa cacagccaca ttatgtctgg
 121 gacaccatgc agtagcaaat ggaacattgg taaaaacaat aactgatgac caaattgagg
 181 tgacaaatgc tactgaatta gttcagagca tttcaatagg aaaaatatgc aacaactcat
 241 atagggttct agatggaaga aattgcacat taatagatgc aatgctagga gaccctcact
 301 gtgatgtctt tcagtatgag aattgggacc tcttcataga aagaagcagc gctttcagca
 361 attgctaccc atatgacatc cctgactatg catcgctccg gtccattgta gcatcctcag
 421 gaacattaga attcacagca gagggattca catggacagg tgtcactcaa aacggaggaa
 481 gtggagcctg caaaagagga tcagccgata gtttctttag ccgactgaat tggctaacaa
 541 aatctggaaa ctcttacccc acattgaatg tgacaatgcc taacaataaa aatttcgaca
 601 aactatacat ctgggggatt catcacccga gctcaaacaa tgagcagaca aaattgtata
 661 tccaagaatc aggacgagta acagtctcaa caaaaagaag tcaacaaaca ataatcccta
 721 acatcggatc tagaccgtgg gtcaggggtc aatcaggcag gataagcata tactggacca
 781 ttgtaaaacc tggagatatc ctaatgataa acagtaatgg caacttagtt gcaccgcggg
 841 gatattttaa attgagaaca gggaaaagct ctgtaatgag atcagatgca cccatagaca
 901 cttgtgtgtc tgaatgtatt acaccaaatg gaagcatccc caacgacaaa ccatttcaaa
 961 atgtgaacaa agttacatat ggaaaatgcc ccaagtatat caggcaaaac actttaaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctgcag atctaaagag cactcaagca gccatcgacc
1201 agatcaatgg aaaattaaac agagtgattg aaaggaccaa tgagaaattc catcaaatag
1261 agaaggaatt ctcagaagta gaagggagaa tcaaggactt ggagaagtat gtagaagaca
1321 ccaaaataga cctatggtcc tacaatgcag aattgctggt ggctctagaa aatcaacata
1381 caattgactt aacagatgca gaaatgaata aattattcga gaagactaga cgccagttaa
1441 gagaaaacgc ggaagacatg ggaggtggat gtttcaagat ctaccacaaa tgtgataatg
1501 catgcattgg atcaataaga aatgggacat atgaccatta catatacaaa gatgaagcat
1561 taaacaaccg atttcaaatc aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct
```

DEFINITION Influenza A virus (A/equine/Kentucky/1/94(H3N8)) hemagglutinin precursor (HA) gene, complete cds.

ACCESSION L39914; SEQ ID NO: 19

```
   1 agcaaaagca ggggatattt ctgtcaatca tgaagacaac cattattttg atactactga
  61 cccattgggt ctacagtcaa aacccaacca gtggcaacaa cacagccaca ttatgtctgg
 121 gacaccatgc agtagcaaat ggaacattgg taaaaacaat aactgatgac caaattgagg
 181 tgacaaatgc tactgaatta gttcagagca tttcaatagg gaaaatatgc aacaactcat
 241 atagagttct agatggaaga aattgcacat taatagatgc aatgctagga gacccccact
 301 gtgatgtctt tcagtatgag aattgggacc tcttcataga aagaagcagc gctttcagca
 361 attgctaccc atatgacatc cctgactatg catcgctccg gtccattgta gcatcctcag
 421 gaacattgga attcacagca gagggattca catggacagg tgtcactcaa aacggaagaa
 481 gtggagcctg caaaagggga tcagccgata gtttctttag ccgactgaat tggctaacaa
 541 aatctggaaa ctcttacccc acattgaatg tgacaatgcc taacaataaa aatttcgaca
 601 aactatacat ctgggggatt catcacccga gctcaaacca acagcaaaca gaattgtaca
 661 tccaagaatc aggacgagta acagtctcaa caaaaagaag tcaacaaacg ataatcccta
 721 atatcggatc tagaccgtgg gtcaggggtc aatcaggcag gataagcata tactggacca
 781 ttgtaaaacc tggagatatc ctaatgataa acagtaatgg caacttagtt gcaccgcggg
 841 gatattttaa attgaaaaca gggaaaagct ctgtaatgag atcagatgca cccatagaca
 901 tttgtgtgtc tgaatgtatt acaccaaatg gaagcatccc caacgacaaa ccatttcaaa
 961 atgtgaacaa agttacatat ggaaaatgcc ccaagtatat caggcaaaac actttaaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctgcag atctaaagag cactcaagca gccatcgacc
1201 agattaatgg aaaattaaac agagtgattg aaaggaccaa tgagaaattc catcaaatag
1261 agaaggaatt ctcagaagta gaagggagaa tccaggactt ggagaagtat gtagaagaca
1321 ccaaaataga cctatggtcc tacaatgcag aattgctggt ggctctagaa aatcaacata
1381 caattgactt aacagatgca gaaatgaata aattattcga gaagactaga cgccagttaa
1441 gagaaaacgc ggaagacatg ggaggtggat gtttcaagat ttaccacaaa tgtgataatg
1501 catgcattgg atcaataaga aatgggacat atgaccatta catatacaga gatgaagcat
1561 taaacaaccg atttcaaatc aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct
```

DEFINITION Influenza A virus (A/equine/Kentucky/1/81(H3N8)) nucleoprotein (NP) gene, complete cds.

ACCESSION AY291288; SEQ ID NO: 20

```
   1 agcaaaagca gggtagataa tcactcactg agtgacatca aagtcatggc gtctcaaggc
  61 accaaacgat cttatgagca gatggaaact ggtggggaac gccagaatgc aactgaaatc
 121 agagcatctg ttggaaggat ggtgggagga atcggccggt tctatgttca aatgtgtact
 181 gagcttaaac tcaacgacca tgaagggcgg ctgattcaga acagcataac aatagaaagg
 241 atggtacttt cggcattcga cgaaagaaga aacaagtacc tcgaggagca tcccagtgct
 301 gggaaagacc ccaagaaaac gggaggcccg atatacagaa ggagagatgg gaaatggatg
 361 agagaactca tcctccatga taaagaagaa atcatgagga tctggcgtca ggccaacaat
 421 ggtgaagacg ctactgctgg tcttactcat atgatgatct ggcactccaa tctcaatgac
 481 accacctacc aaagaacaag ggctcttgtt cgggctggga tggatcccag aatgtgctct
 541 ctgatgcaag gatcaactct cccacggaga tctggagctg ccggtgctgc agtgaagggt
 601 gttggaacaa tggtaatgga actcatcagg atgatcaaac gcgggataaa tgatcgaaac
 661 ttctggagag gtgaaaatgg tcgaagaacc agaattgctt atgaaagaat gtgcaacatc
 721 ctcaagggga aattccaaac agcagcacaa cgagcaatga tggaccaagt gagggagggc
 781 cgcaatcctg gaaatgctga gattgaggat ctcattttct tggcacgatc agcactcatt
 841 ctgagaggat cagtagccca taaatcatgc ctacctgcct gtgtttatgg ccttgcagta
 901 gccagtgggt atgactttga gaaagaggga tactctctgg ttggaattga tcctttcaaa
 961 ctactccaga acagccaaat tttcagtcta atcagaccga aagaaaatcc agcacacaag
1021 agccagctgg tgtggatggc atgccattct gcagcatttg aggacctgag agtttcgaat
1081 ttcattagag gaaccaaagt aatcccaaga ggacagttag caaccagggg agtgcaaatt
1141 gcttcaaatg aaaacatgga gacaatagat tctagcacac tcgaactgag gagcagatat
1201 tgggcaataa ggaccaggag tgggggaaac accagtcaac agagagcatc tgcaggacag
1261 ataagtgtgc aacccacttt ctcagtgcag agaaatcttc cctttgaaag agcaaccatt
1321 atggctgcat tcactggaaa cactgagggg aggacttccg acatgagaac ggaaatcata
1381 aggatgatgg aaaatgccag atcagaagat gtgtctttcc aggggcgggg agtcttcgag
1441 ctctcggacg aaaaggcaac gaacccgatc gtgccttcct ttgacatgag caatgaaggg
1501 tcttatttct tcggagacaa tgctgaggag tttgacagtt aaagaaaaat acccttgttt
1561 ctact
```

DEFINITION Influenza A virus (A/Equine/Kentucky/1/92(H3N8)) gene for hemagglutinin precursor, partial cds.

ACCESSION D30683; SEQ ID NO: 21

```
   1 gtcaatcatg aagacaacca ttattttgat actactgacc cattgggtct acagtcaaaa
  61 cccaaccagt ggcaacaaca cagccacatt atgtctggga caccatgcag tagcaaatgg
 121 aacattggta aaaacaataa ctgatgacca aattgaggtg acaaatgcta ctgaattagt
 181 tcagagcatt tcaataggga aaatatgcaa caactcatat agggttctag atggaagaaa
 241 ttgcacatta atagatgcaa tgctaggaga cccccactgt gatgtctttc agtatgagaa
 301 ttgggacctc ttcatagaaa gaagcagcgc tttcagcaat tgctacccat atgacatccc
 361 tgactatgca tcgctccggt ccattgtagc atcctcagga acattagaat tcacagcaga
 421 gggattcaca tggacaggtg tcactcaaaa cggaggaagt ggagcctgca aaaggggatc
 481 agccgatagt ttctttagcc gactgaattg gctaacaaaa tctggaaact cttaccccac
 541 attgaatgtg acaatgccta acaataaaaa tttcgacaaa ctatacatct gggggattca
 601 tcacccgagc tcaaacaatg agcagacaaa attgtatatc caagaaacag gacgagtaac
 661 agtctcaaca aaaagaagtc aacaaacaat aatccctaac atcggatcta gaccgtgggt
 721 caggggtcaa tcaggcagga taagcatata ctggaccatt gtaaaacctg gagatatcct
 781 aatgataaac agcaatggca acttagttgc accgcgggga tattttaaat tgagaacagg
 841 gagaagctct gtaatgagat cagatgcacc catagacatt tgtgtgtctg aatgtattac
 901 accaaatgga agcatcccca acgacaaacc atttcaaaat gtgaacaaag ttacatatgg
 961 aaaatgcccc aaatatatca ggcaaaacac tttaaagctg gccactggga tgaggaatgt
1021 accagaaaag caaatcagag gaatctttgg agcaatagcg ggattcatag aaaacggctg
1081 ggaaggaatg gttgat
```

DEFINITION Influenza A virus (A/equine/Kentucky/1/97(H3N8)) hemagglutinin precursor (HA1) mRNA, partial cds.

ACCESSION AF197249; SEQ ID NO: 22

```
   1 agcaaaagca ggggatattt ctgtcaatca tgaagacaac cattattttg atactactga
  61 cccattgggc ctacagtcaa aacccaatca gtggcaacaa cacagccaca ttgtgtctgg
 121 gacaccatgc agtagcaaat ggaacattgg taaaaacaat aactgatgat caaattgagg
 181 tgacaaatgc tactgaatta gttcagagca tttcaatggg gaaaatatgc aacaactcat
 241 atagagttct agatggaaga aattgcacat taatagatgc aatgctagga gacccccact
 301 gtgatgtctt tcagtatgag aattgggacc tctttataga aagaagcagc gctttcagca
 361 attgctaccc atatgacatc cctgactatg catcgctccg gtccattgta gcatcctcag
 421 gaacattgga attcacagca gagggattca catggacagg tgtcactcaa aacggaagaa
 481 gtggagcctg caaaagggga tcagccgata gtttctttag ccgactgaat tggctaacaa
 541 aatctggaaa ctcttacccc acattgaatg tgacaatgcc taacaataaa aatttcgaca
 601 aactatacat ctgggggatt catcacccga gctcaaacca agagcagaca aaattgtaca
 661 tccaagaatc aggacgagta acagtctcaa caaaaagaag tcaacaaaca ataatcccta
 721 acatcggatc tagaccgtgg gtcaggggtc aatcaggcag gataagcata tactggacca
 781 ttgtaaaacc tggagatatc ctaatgataa acagtaatgg caacttagtt gcaccgcggg
 841 gatattttaa attgaaaaca gggaaaagct ctgtaatgag atcagatgca cccatagaca
 901 tttgtgtgtc tgaatgtatt acaccaaatg gaagcatctc caacgacaaa ccattccaaa
 961 atgtgaacaa agttacatat ggaaaatgcc ccaagtatat caggcaaaac actttaaagc
1021 tggccactgg gatgaggaat gaaccagaaa agcaaatcag a
```

DEFINITION Influenza A virus (A/equine/Kentucky/1/96(H3N8)) hemagglutinin precursor (HA1) mRNA, partial cds.

ACCESSION AF197248; SEQ ID NO: 23

```
   1 agcaaaagca ggggatattt ctgtcaatca tgaagacaac cattattttg atactactga
  61 cccattgggt ctacagtcaa aacccaacca gtggcaacaa cacagccaca ttatgtctgg
 121 gacaccatgc agtagcaaat ggaacattgg taaaaacaat aactgatgac caaattgagg
 181 tgacaaatgc tactgaatta gttcagagca tttcaatagg gaaaatatgc aacaactcat
 241 atagagttct agatggaaga aattgcacat taatagatgc aatgctagga gacccccact
 301 gtgatgtctt ccagtatgag aattgggacc tcttcataga aagaagcagc gctttcagca

 361 attgctaccc atatgacatc cctgactatg catcgctccg gtccattgta gcatcctcag
 421 gaacattaga attcacagca gagggattca catggacagg tgtcactcaa aacggaagaa
 481 gtggagcctg caaaagggga tcagccgata gtttctttag ccgactgaat tggctaacaa
 541 aatctggaaa ctcttacccc acattgaatg tgacaatgcc taacaataaa aatttcgaca
 601 aactatacat ctgggggatt catcacccga gctcaaacca aaagcagaca gaattgtaca
 661 tccaagaatc aggacgagta acagtctcaa caaaaagaag tcaacaaacg ataatcccta
 721 atatcggatc tagaccgtgg gttaggggtc aatcaggcag gataagcata tactggacca
 781 ttgtaaaacc tggagatatc ctaatgataa acagtaatgg caacttagtt gcaccgcggg
 841 gatattttaa attgaaaaca gggaaaagct ctgtaatgag atcagatgca cccatagaca
 901 tttgtgtgtc tgaatgtatt acaccaaatg gaagcatccc caacgacaaa ccatttcaaa
 961 atgtgaacaa agttacatat ggaaaatgcc ccaagtatat caggcaaaac actttaaagc
1021 tggccactgg gatgaggaat gtaccagaaa agcaaatcag a
```

DEFINITION Influenza A virus (A/equine/Kentucky/9/95(H3N8)) hemagglutinin precursor (HA1) mRNA, partial cds.

ACCESSION AF197247; SEQ ID NO: 24

```
   1 agcaaaagca ggggatattt ctgtcaatca tgaagacaac cattattttg atactactga
  61 cccattgggt ctacagtcaa aacccaacca gtggaaacaa cacagccaca ttatgtctgg
 121 gacaccatgc agtagcaaat ggaacattgg taaaaacaat aactgatgac caaattgagg
 181 tgacaaatgc tactgaatta gttcagagca tttcaatagg gaaaatatgc aacaactcat
 241 atagagttct agatggaaga aattgcacat taatagatgc aatgctagga gaccccccact
 301 gtgatgtctt ccagtatgag aattgggacc tcttcataga aagaagcagc gctttcagca
 361 attgctaccc atatgacatc cctgactatg catcgctccg gtccattgta gcatcctcag
 421 gaacattgga attcacagca gagggattca catggacagg tgtcactcaa aacggaagaa
 481 gtggagcctg caaaagggga tcagccgata gtttctttag ccgactgaat tggctaacaa
 541 aatctggaaa ctcttacccc acattgaatg tgacaatgcc taacaataaa aatttcgaca
 601 aactatacat ctgggggatt catcacccga gctcaaacca aaagcagaca gaattgtaca
 661 tccaagaatc aggacgagta acagtctcaa caaaaagaag tcaacaaacg ataatcccta
 721 atatcggatc tagaccgtgg gtcaggggtc aatcaggcag gataagcata tactggacca
 781 ttgtaaaacc tggagatatc ctaatgataa acagtaatgg caacttagtt gcaccgcggg
 841 gatattttaa attgaaaaca gggaaaagct ctgtaatgag atcagatgca cccatagaca
 901 tttgtgtgtc tgaatgtatt acaccaaatg gaagcatccc caacgacaaa ccatttcaaa
 961 atgtgaacaa agttacatat ggaaaatgcc ccaagtatat caggcaaaac actttaaagc
1021 tggccactgg gatgaggaat gtaccagaaa agcaaatcag a
```

DEFINITION Influenza A virus (A/equine/Kentucky/1/98(H3N8)) hemagglutinin precursor (HA1) mRNA, partial cds.

ACCESSION AF197241; SEQ ID NO: 25

```
   1 agcaaaagca ggggatattt ctgtcaatca tgaagacaac cattattttg atactactga
  61 cccattgggt ctacagtcaa aacccaacca gtggaaacaa cacagccaca ttatgtctgg
 121 gacaccatgc agtagcaaat ggaacattgg taaaaacaat aactgatgac caaattgagg
 181 tgacaaatgc tactgaatta gttcagagca tttcaatagg gaaaatatgc aacaactcat
 241 ataaagttct agatggaaga aattgcacat taatagatgc aatgctagga gaccccccact
 301 gtgatgtctt ccagtatgag aattgggacc tcttcataga aagaagcagc gctttcagca
 361 attgctaccc atatgacatc cctgactatg catcgctccg gtccattgta gcatcctcag
 421 gaacattaga attcacagca gagggattca catggacagg tgtcactcaa aacggaagaa
 481 gtggagcctg caaaagggga tcagccgata gtttctttag ccgactgaat tggctaacaa
 541 aatctggaaa ctcttacccc acattgaatg tgacaatgcc taacaataaa aatttcgaca
 601 aactatacat ctgggggatt catcacccga gctcaaacca acagcagaca gaattgtaca
 661 tccaagaatc aggacgagta acagtctcaa caaaaagaag tcaacaaacg atagtcccta
 721 atatcggatc tagaccgtgg gttaggggtc aatcaggcag gataagcata tactggacca
 781 ttgtaaaacc tggagatatc ctaatgataa acagtaatgg caacttagtt gcaccgcggg
 841 gatattttaa attgaaaaca gggaaaagct ctgtaatgag atcagatgca cccatagaca
 901 tttgtgtgtc tgaatgtatt acaccaaatg gaagcatccc caacgacaaa ccatttcaaa
 961 atgtgaacaa agttacatat ggaaaatgcc ccaagtatat caggcaaaac actttaaagc
1021 tggccactgg gatgaggaat ataccagaaa agcaaatcag a
```

DEFINITION Influenza A virus (A/eq/Kentucky/81(H3N8)) hemagglutinin mRNA, complete cds.

ACCESSION U58195; SEQ ID NO: 26

```
   1 agcaaaagca ggggatactt tctgtcaatc atgaagacaa ccattatttt gatactactg
  61 acccattggg tctacagtca aaacccaacc agtggcaaca acacagccac actatgtctg
 121 ggacaccatg cagtagcaaa tggaacattg gtaaaaacaa taactgatga ccaaattgag
 181 gtgacaaatg ctactgaatt agttcagagc acttcaatag ggaaaatatg caacaaccca
 241 tatagggttc tagatggaag aaactgcaca ttaatagatg caatgctagg agatccccac
 301 tgtgatgttt ttcagtatga gaattgggac ctcttcatag aaagaagcag cgctttcagc
 361 aattgctacc catatgacat ccctgactat gcatcgctcc ggtctattgt ggcatcttca
 421 ggaacattag aattcacagc agagggattc acatggacag gtgtcactca aaacggagga
 481 agtggagcct gcagaagggg gtcagccgat agtttcttta gccgactgaa ttggctaaca
 541 aaatctggaa attcttaccc cacattgaat gtaacaatgc ctaacaataa caatttcgat
 601 aaactataca tctgggggat ccatcacccg agcacaaaca atgagcagac aaaattgtat
 661 atccaagaat cagggcgagt aacagtctca acaaaaagaa gtcaacaaac aataatcccc
 721 aacatcggat ctagaccgtg ggtcaggggt caatcaggca ggataagcat atattggacc
 781 attgtgaaac ctggagatat cctaatgata aacagtaatg gcaacttagt tgcaccgcgg
 841 ggatatttta aaatgcgaac agggaaaagc tctgtaatga gatcagatgc acccatagac
 901 acttgtgtgt ccgagtgtat tacaccaaat ggaagcatcc ccaacgacaa accatttcaa
 961 aatgtgaaca aagttacata tggaaaatgc cccaagtata tcaagcagaa tactttgaag
1021 ctggccactg ggatgaggaa tgtaccagaa aagcaaatca gaggaatctt tggagcaata
1081 gcgggattca tagaaaacgg ctgggaagga atggttgatg ggtggtatgg attccgatat
1141 cagaattcgg aaggaacagg acaagctgca gatctaaaga gcactcaagc agccatcgac
1201 cagatcaatg gaaaattgaa cagagtgatt gaaaggacca atgagaaatt ccatcaaata
1261 gagaaggaat tctcagaagt agaagggaga atccaggact tggagaagta tgtagaagac
1321 accaaaatag acctatggtc ctacaatgca gagttactgg tggctctaga aaatcaacat
1381 acgattgact taacagatgc agaaatgaat aaattattcg agaagactag gcgccagtta
1441 agagaaaacg cggaagacat gggggggtgga tgtttcaaga tttatcacaa atgtgataat
1501 gcatgcattg gatcaataag aaatgggaca tatgaccatt acatatacag agatgaagca
1561 ttaaacaacc gatttcaaat taaaggtgtt gaattgaaat caggctacaa agattggata
1621 ctgtggattt cattcgccat atcatgcttc ttaatttgcg ttgttctatt gggtttcatc
1681 atgtgggctt gccaaaaagg caacatcaga tgcaacattt gcatttgagt aaactgataa
1741 ttaaaaacac ccttgtttct act
```

DEFINITION Influenza A virus (A/equine/Kentucky/2/86 (H3N8)) membrane protein M1 and membrane protein M2 genes, complete cds.

ACCESSION M63540; SEQ ID NO: 27

```
   1 agcaaaagca ggtagatatt taaagatgag tcttctaacc gaggtcgaaa cgtacgttct
  61 ctctattgta ccatcaggcc ccctcaaagc cgagatcgcg cagagacttg aagatgtctt
 121 tgcagggaag aacaccgatc ttgaggcact catggaatgg ctaaagacaa gaccaatcct
 181 gtcacctctg actaaaggga ttttaggatt tgtgttcacg ctcaccgtgc ccagtgagcg
 241 aggactgcaa cgtagacgct ttgtccaaaa tgcccttagt ggaaacggag atccaaataa
 301 catggacaga gcagtaaaac tgtacaagaa gcttaaaaga gaaataacat tccatggggc
 361 aaaagaggtg gcactcagct attccactgg tgcactagcc agctgcatgg gactcatata
 421 caacagaatg ggactgtga caaccgaagt ggcatttggc ctggtatgcg ccacatgtga
 481 acagattgct gattcccagc atcgatctca caggcagatg gtgacaacaa ccaacccact
 541 aatcagacat gaaaacagaa tggtactagc cagtaccaca gctaaaacca tggagcaggt
 601 ggcagggtcg agtgagcagg cagcagaggc catggaggtt gctagtaagg ccaggcagat
 661 ggtgcaggca atgaggacca ttgggaccca ccctagctcc agtgccggtt tgaaagatga
 721 tcttcttgaa aatttgcagg cctaccagaa acggatggga gtgcaaatgc agcggttcaa
 781 gtgatcctct cgttattgca gcaagtatca ttgggatctt gcacttgata ttgtggattc
 841 ttgatcgcct ttcttcaaa ttcatttatc gtctccttaa atacggtttg aaaagagggc
 901 cttctacgga aggagtacct gagtctatga gggaagaata tcggcaggaa cagcagaatg
 961 ctgtggatgt tgacgatggt cattttgtca acatagagct ggagtaaaaa actaccttgt
1021 ttctact
```

DEFINITION Influenza A virus isolate A/equine/Kentucky/76 nonstructural protein, complete cds.

ACCESSION M80971; SEQ ID NO: 28

```
  1 agcaaaagca gggtgacaaa aacataatgg attccaacac tgtgtcaagc tttcaggtag
 61 actgttttct ttggcatgtc cgcaaacgat ttgcagacca agaactgggt gatgccccat
121 tccttgaccg gcttcgccga gaccagaagt ccctaaaagg aagaggcagc actcttggtc
181 tggacatcga aacagccact cgtgcaggaa agcagatagt ggagcggatt ctggaagagg
241 agtcagatga ggcacttaaa atgaccattg cctctgttcc tgcttcacgc tacttaactg
301 acatgactct tgatgagatg tcaagagact ggttcatgct catgcccaag cagaaagtaa
361 caggctccct atgtataagg atggaccagg caatcatgga taagaacatc atactaaaag
421 caaactttag tgtgattttc gaaaggctgg agacactaat actacttaga gctttcaccg
481 aagaaggagc agtcgttggc gaaatttcac cattgccttc tcttccagga catactaatg
541 aggatgtcaa aaatgcaatt ggggtcctca tcggaggact taaatggaat gataacacag
601 ttagaatctc tgaaactcta cagagattcg cttggagaag cagtcatgag aatgggagac
661 cttcattccc tccaaagcag aaacgaaaaa tggcgagaac aattgagtca gaagtttgaa
721 gaaataaggt ggttgattga agaagtgcga catagattga aaaatacaga aaatagtttt
781 gaacaaataa catttatgca agccttacaa ctattgcttg aagtagaaca agagataaga
841 actttctcgt ttcagcttat ttaatgataa aaaacaccct tgtttctact
```

DEFINITION Influenza A virus (A/eq/Kentucky/92(H3N8)) matrix proteins M1 and M2 (M) gene, complete cds.

ACCESSION AF001683; SEQ ID NO: 29

```
  1 atgagtcttc tgaccgaggt cgaaacgtac gttctctcta tcgtaccatc aggccccctc
 61 aaagccgaga tcgcgcagag acttgaagat gtctttgcag ggaagaacac cgatcttgag
121 gcactcatgg aatggctaaa gacaagacca atcctgtcac ctctgactaa agggatttta
181 ggattcgtat tcacgctcac cgtgcccagt gagcgaggac tgcagcgtag acgctttgtc
241 caaaatgccc ttagtggaaa cggagatcca aacaacatgg acagagcagt aaaactgtac
301 aggaaactta aaagagaaat aacattccat ggggcaaaag aggtggcact cagctattcc
361 actggtgcac tagccagctg catgggactc atatacaaca gaatgggaac tgtgacaacc
421 gaagtggcat ttggcctagt atgcgccaca tgtgaacaga ttgctgattc ccagcatcga
481 tctcacaggc agatggtgac aacaaccaac ccattaatca gacatgaaaa cagaatggta
541 ttagccagta ccacggctaa agccatggag cagatggcag ggtcgagtga gcaggcagca
601 gaggccatgg aggttgctag taaggctagg cagatggtac aggcaatgag gaccattggg
661 acccacccta gctccagtgc cggtttgaaa aatgatctcc ttgaaaattt gcaggcctac
721 cagaaacgga tgggagtgca aatgcagcga ttcaagtgat cctctcgtta ttgcagcaag
781 tatcattggg atcttgcact tgatattgtg gattcttgat cgccttttct tcaaattcat
841 ttatcgtcgc cttaaatacg ggttgaaaag agggccttct acggaaggag tacctgagtc
901 tatgagggaa gaatatcggc aggaacagca gaatgctgtg gatgttgacg atggtcattt
961 tgtcaacata gagctggagt aa
```

DEFINITION Influenza A virus (A/eq/Kentucky/81(H3N8)) matrix proteins M1 and M2 (M) gene, complete cds.

ACCESSION AF001676; SEQ ID NO: 30

```
  1 atgagtcttc taaccgaggt cgaaacgtac gttctctcta tcgtaccatc aggccccctc
 61 aaagccgaga tcgcgcagag acttgaagat gtctttgcag ggaagaacac cgatcttgag
121 gcactcatgg aatggctaaa gacaagacca atcctgtcac ctctgactaa agggatttta
181 ggatttgtgt tcacgctcac cgtgcccagt gagcgaggac tgcagcgtag acgctttgtc
241 caaaatgccc ttagtggaaa cggagatcca aacaacatgg acagagcagt aaaactgtac
301 aggaagctta aaagagaaat aacattccat ggggcaaaag aggtggcact cagctattcc
361 actggtgcac tagccagctg catgggactc atatacaaca gaatggggac tgtgacaacc
421 gaagtggcat ttggcctggt atgcgccaca tgtgaacaga ttgctgattc ccagcatcga
481 tctcacaggc agatggtgac aacaaccaac ccactaatca gacatgaaaa cagaatggta
541 ctagccagta ccacagctaa agccatggaa cagatggcag ggtcgagtga gcaggcagca
```

```
601 gaggccatgg aggttgctag taaggccagg cagatggtac aggcaatgag gaccattggg
661 acccacccta gctccagtgc cggtttgaaa gatgatcttc ttgaaaattt gcaggcctac
721 cagaaacgga tgggagtgca aatgcagcga ttcaagtgac cctctcgtta ttgcagcaag
781 tatcattggg atcttgcact tgatattgtg gattcttgat cgccttttct tcaaattcat
841 ttatcgtcgc cttaaatacg gtttgaaaag agggccttct acggaaggag tacctgagtc
901 tatgagggaa gaatatcggc aggaacagca gaatgctgtg gatgttgacg atggtcattt
961 tgtcaacata gagctggagt aa
```

DEFINITION Influenza A virus (A/eq/Kentucky/92(H3N8)) nonstructural proteins NS1 and NS2 (NS) gene, complete cds.

ACCESSION AF001671; SEQ ID NO: 31

```
  1 atggattcca acactgtgtc aagctttcag gtagactgtt ttctttggca tgtccgcaaa
 61 cgattcgcag accaagaact gggtgatgcc ccattccttg accggcttcg ccgagaccag
121 aagtccctaa aaggaagagg tagcactctt ggtctggaca tcgaaacagc cactcgtgca
181 ggaaagcaga tagtggagca gattctggaa gaggaatcag atgaggcact taaaatgacc
241 attgcctctg ttcctgcttc acgctactta actgacatga ctcttgatga gatgtcaaga
301 gactggttca tgctcatgcc caagcagaaa gtaacaggct ccctatgtat aagaatggac
361 caggcaatca tggataagaa catcatactt aaagcaaact ttagtgtgat tttcgaaagg
421 ctggaaacac taatactact tagagccttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataat acggttagaa tctctgaaac tctacagaga
601 ttcgcttgga gaagcagtca tgagaatggg agaccttcat tccctccaaa gcagaaacga
661 aaaatggaga gaacaattga gccagaagtt tgaagaaata agatggttga ttgaagaagt
721 gcgacataga ttgaaaaata cagaaaatag ttttgaacaa ataacattta tgcaagcctt
781 acaactattg cttgaagtag aacaagagat aagaactttc tcgtttcagc ttatttaa
```

DEFINITION Influenza A virus (A/eq/Kentucky/1/88(H3N8)) nonstructural proteins NS1 and NS2 (NS) gene, complete cds.

ACCESSION AF001664; ; SEQ ID NO: 32

```
  1 atggattcca acactgtgtc aagctttcag gtagactgtt ttctttggca tgtccgcaaa
 61 cgatttgcag accaagaact gggtgatgcc ccattccttg accggcttcg ccgagaccag
121 aagtccctaa aaggaagagg cagcactctt ggtctggaca tcgaaacagc cactcgtgca
181 ggaaagcaga tagtggagca gattctggaa gaggaatcag atgaggcact taaaatgacc
241 attgcctctg ttcctgcttc acgctactta actgacatga ctcttgatga gatgtcaaga
301 gactggttca tgctcatgcc caagcagaaa gtaacaggct ccctatgtat aaggatggac
361 caggcaatca tggataagaa catcatacta aaagcaaact ttagtgtgat tttcgaaagg
421 ctggagacac taatactact tagagccttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataat acagttagag tctctgaaac tctacagaga
601 ttcgcttgga gaagcagtca tgagaatggg agaccttcat tccctccaaa gcagaaacga
661 aaaatggcga gaacaattga gccagaagtt tgaagaaata agatggttga ttgaagaagt
721 gcgacataga ttgaaaaata cagaaaatag ttttgaacaa ataacattta tgcaagcctt
781 acaactattg cttgaagtag aacaagagat aagaactttc tcgtttcagc ttatttaa
```

DEFINITION Influenza A/equine/Kentucky/2/86 (H3N8) nucleoprotein (seg 5) mRNA, complete cds.

ACCESSION M30751; SEQ ID NO: 33

```
   1 agcaaaagca gggtagataa tcactcactg agtgacatca aagtcatggc gtctcaaggc
  61 accaaacgat cttatgagca gatggaaact ggtggggaac gccagaatgc aactgaaatc
 121 agagcatctg tcggaaggat ggtgggagga atcggccggt tctatgttca gatgtgtact
 181 gagcttaaac tcaacgacca tgaagggcgg ctgattcaga acagcataac aatagaaagg
 241 atggtacttt cggcattcga cgaaagaaga aacaagtacc tcgaggagca tcccagtgct
 301 gggaaagacc ccaagaaaac gggaggcccg atatacagaa ggaaagatgg gaaatggatg
 361 agagaactca tcctccatga taaagaagaa atcatgagga tctggcgtca ggccaacaat

 421 ggtgaagacg ctactgctgg tcttactcat atgatgatct ggcactccaa tctcaatgac
 481 accacatacc aaagaacaag ggctcttgtt cgggctggga tggatcccag aatgtgctct
 541 ctgatgcaag gatcaaccct cccacggaga tctggagctg ccggtgctgc agtaaaaggt
 601 gttggaacaa tggtaatgga actcatcagg atgatcaaac gcgggataaa tgatcgaaat
 661 ttctggagag gtgaaaatgg tcgaagaacc agaattgctt atgaaagaat gtgcaatatc
 721 ctcaaaggga aattccaaac agcagcacaa cgggcaatga tggaccaagt gagggagggc
 781 cgcaatcctg gaaatgctga gattgaggat ctcattttct tggcacgatc agcactcatt
 841 ttgagaggat cagtagccca taaatcatgc ctacctgcct gtgtttatgg ccttgcagta
 901 gccagtgggt atgactttga gaaggaagga tactctctgg ttggaattga tcctttcaaa
 961 ctactccaga acagccaaat tttcagtcta atcagaccga aagaaaatcc agcacacaag
1021 agccagttgg tgtggatggc atgccattct gcagcatttg aggacctgag agttttgaat
1081 ttcattagag gaaccaaagt aatcccaaga ggacagttag caaccagagg agtgcaaatt
1141 gcttcaaatg aaaacatgga gacaatagat tctagcacac tcgaactgag gagcagatat
1201 tgggcaataa ggaccaggag tggagggaac accagtcaac agagagcatc tgcaggacag
1261 ataagtgtgc aacccacttt ctcagtgcag agaaatcttc cctttgaaag agcaaccatt
1321 atggctgcat tcactgggaa cactgagcgg aggacttccg acatgagaac ggaaatcata
1381 aggatgatgg aaaatgccag atcagaagat gtgtctttcc aggggcgggg agtcttcgag
1441 ctctcggacg aaaaggcaac gaacccgatc gtgccttcct ttgacatgag caatgaaggg
1501 tcttatttct tcggagacaa tgctgaggag tttgacagtt aaagaaaaat acccttgttt
1561 ctact
```

DEFINITION Influenza A/Equine/Kentucky/2/86 (H3N8), PB2 polymerase, complete cds.

ACCESSION M73526 M36049; SEQ ID NO: 34

```
   1 agcaaaagca ggtcaaatat attcaatatg gagagaataa aagaactgag agatctaatg
  61 tcacagtccc gcacccgcga gatactaaca aaaactactg tggaccatat ggccataatc
 121 aagaaataca catcaggaag acaagagaag aaccccgcac ttaggatgaa gtggatgatg
 181 gcaatgaaat acccaattac agcagataag aggataatgg aaatgattcc tgagagaaat
 241 gaacaggggc aaaccctttg gagcaaaacg aacgatgctg gctcagaccg cgtaatggta
 301 tcacctctgg cagtgacatg gtggaatagg aatggaccaa caacgagcac aattcattat
 361 ccaaaagtct acaaaactta ttttgaaaaa gttgaaaggt taaaacacgg aacctttggc
 421 cccgttcatt ttaggaatca agtcaagata agacggagag ttgacgtaaa ccctggtcac
 481 gcggacctca gtgccaaaga agcacaagat gtgatcatgg aagttgtttt cccaaatgaa
 541 gtgggagcca gaattctaac atcggaatca caactaacaa taaccaaaga gaaaaaagaa
 601 gaacttcagg actgcaaaat tgccccttg atggtagcat acatgctaga aagagagttg
 661 gtccgaaaaa caaggttcct cccagtggct ggcggaacaa gcagtgtata cattgaggtg
 721 ttgcatctga ctcagggaac gtgctgggaa caaatgtaca ccccaggagg agaagttaga
 781 aacgatgaca ttgatcaaag tttaattatt gctgcccgga acatagtgaa aagagcgaca
 841 gtatcagcag atccactagc atccctgctg gagatgtgcc acagtacaca gattggtgga
 901 ataaggatgg tagacatcct taagcagaat ccaacagagg aacaagctgt ggatatatgc
 961 aaagcagcaa tggggttaag aattagctca tcattcagct ttggtggatt cacctttaag
1021 agaacaagtg gatcatcagt caagagagaa gaagaaatgc ttacgggcaa ccttcaaaca
1081 ttgaaaataa gagtgcatga gggctatgaa gaattcacaa tggtcggaag aagagcaaca
1141 gccattctca gaaagacaac cagaagattg attcaattga tagtaagtgg gagagatgaa
1201 cagtcaattg ctgaagcaat aattgtagcc atggtgtttt cgcaagaaga ttgcatgata
1261 aaagcagttc gaggcgattt gaacttcgtt aatagagcaa atcagcgctt gaaccccatg
1321 catcaactct tgaggcattt ccaaaaggat gcaaaagtgc ttttccagaa ttggggatt
1381 gaacccatcg acaatgtgat gggaatgatc ggaatattgc ccgacatgac cccaagcacc
1441 gagatgtcat tgagaggagt gagagtcagc aaaatgggag tggatgagta ctccagcact
1501 gagagagtgg tggtgagcat tgaccgtttt ttaagagttc gggatcaaag gggaaacata
1561 ctactgtccc ctgaagaggt cagtgaaaca caaggaacgg aaaagctgac aataatttat
1621 tcatcatcaa tgatgtggga gattaatggt cccgagtcag tgttggtcaa tacttatcaa
1681 tggatcatca gaaactggga aattgtgaaa attcaatggt cacaggatcc cacaatgtta
1741 tacaataaga tagaatttga gccattccag tccctggtcc ctagggccac cagaagccaa
1801 tacagcggtt tcgtaaggac cctgtttcag caaatgcgag atgtacttgg aacatttgac
1861 actgctcaaa taataaaact cctccctttt gccgctgctc ctccggaaca gagtagaatg
1921 cagttctctt ctttgactgt taatgtaaga ggatcgggaa tgaggatact tgtaagaggc
1981 aattccccag tgttcaacta caacaaagcc actaagaggc tcacagtcct cggaaaggat
2041 gcaggtgcgc ttactgaaga cccagatgaa ggtacggctg gagtagaatc tgctgttctg
2101 agagggtttc tcatcttagg taaagaaaac aagagatatg gcccagcact aagcatcaat

2161 gaactgagca aacttacaaa aggggagaaa gctaatgtgc taattgggca aggggacgtg
2221 gtgttggtaa tgaaacggaa acgtgactct agcatactta ctgacagtca gacagcgacc
2281 aaaaggattc ggatggccat caattagtgt tgaattgttt aaaaacgacc ttgtttctac
2341 t
```

DEFINITION Influenza A/equine/Kentucky/1/87 (H3N8) hemagglutinin (HA) RNA (seg. 4), complete cds.

ACCESSION M24728 J04336; SEQ ID NO: 35

```
   1 agcaaaagca ggggatattt ctgtcaatca tgaagacaac cattgttttg atactactga
  61 cccattgggt ctacagtcaa aacccaacca gtggcaacaa cacagccaca ttatgtctgg
 121 gacaccatgc agtagcaaat ggaacactgg taaaaacaat aactgatgac cagattgagg
 181 tgacaaatgc tactgaatta gttcagagca tttcaatagg gaaaatatgc aacaactcat
 241 atagggttct agatggaaga aattgcacat taatagatgc aatgctagga gacccccact
 301 gtgatgtttt tcngtatgag aattgggacc tcttcataga aagaagcagc gctttcagca
 361 attgctaccc atatgacatc cctgactatg catcgctccg gtctattgtg gcatcctcag
 421 gaacattaga attcacagca gagggattca catggacagg tgtcactcaa aacggaagaa
 481 gtggagcctg caaaagggga tcagccgata gtttctttag ccgactgaat tggctaacaa
 541 aatctggaaa ttcttacccc acattgaatg tgacaatgcc taacaataac aatttcgata
 601 aactatacat ctgggggatt catcacccga gctcaaacaa tgagcagaca aaattgtata
 661 tccaagaatc aggacgagta acagtctcaa caaaaagaag tcaacaaaca ataatcccca
 721 acatcggatc tagaccgtgg gtcaggggtc aatcaggcag gataagcata tattggacca
 781 ttgtgaaacc tggagatatc ctaatgataa acagtaatgg caacttagtt gcaccgcggg
 841 gatatttcaa attgagaaca gggaaaagct ctgtaatgag atcagatgca cccatagaca
 901 cttgtgtgtc tgaatgtatt acaccaaatg gaagcatccc caacgacaaa ccattccaaa
 961 atgtgaacaa agttacatat ggaaaatgcc ccaagtatat caggcaaaac actttgaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaattcgga aggaacagga caagctggag atctaaagag cactcaagca gccatcgacc
1201 agatcaatgg aaaattaaac agagtgattg aaaggaccaa tgagaaattc catcaaatag
1261 agaaggaatt ctcagaagta gaagggagaa tccaggactt ggagaagtat gtagaagaca
1321 ccaaaataga cctatggtcc tacaatgcag aattgctggt ggctctagaa aatcaacata
1381 caattgactt aacagatgca gaaatgaata aattattcga gaagactagg cgccagttaa
1441 gagaaaacgc ggaagacatg ggaggtggat gtttcaggat ttaccacaaa tgtgataatg
1501 catgcattgg atcaataaga aatgggacat atgaccatta catatacaga gatgaagcat
1561 taaacaaccg atttcaaatt aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct
```

DEFINITION Influenza A/equine/Kentucky/2/86 (H3N8) hemagglutinin (HA) RNA (seg. 4), complete cds.

ACCESSION M24727 J04336; SEQ ID NO: 36

```
   1 agcaaaagca ggggatattt ctgtcaatca tgaagacaac cattattttg atactactga
  61 cccattgggt ctacagtcaa aacccaacca gtggcaacaa cacagccaca ttatgtctgg
 121 gacaccatgc agtagcaaat ggaacattgg taaaaacaat aactgatgac caaattgagg
 181 tgacaaatgc tactgaatta gttcagagca tttcaatagg gaaaatatgc aacaactcat
 241 atagggttct agatggaaga aattgcacat taatagatgc aatgctagga gacccccact
 301 gtgatgtttt tcngtatgag aattgggacc tcttcataga aagaagcagc gcttccagca
 361 attgctaccc atatgacatc cctgactatg catcgctccg gtctattgtg gcatcctcag
 421 gaacattaga attcacagca gagggattca catggacagg tgtcactcaa aacggaagaa
 481 gtggagcctg caaaagggga tcagccgata gtttctttag ccgactgaat tggctaacaa
 541 aatctggaaa ttcttacccc acattgaatg tgacaatgcc taacaataac aatttcgata
 601 agctatacat ctggggggatc catcacccga gctcaaacaa tgagcagaca aaattgtata
 661 tccaagaatc aggacgagta acagtctcaa caaaaagaag tcaacaaaca ataatcccca
 721 acatcggatc tagaccgtgg gtcaggggtc aatcaggcag gataagcata tattggacca
 781 ttgtgaaacc tggagatatc ctaataataa acagtaatgg caacttagtt gcaccgcggg
```

```
 841 gatatttcaa attgcgaaca gggaaaagct ctgtaatgag atcagatgca cccatagaca
 901 cttgtgtgtc tgaatgtatt acaccaaatg gaagcatccc caacgacaaa ccatttcaaa
 961 atgtgaacaa agttacatat ggaaaatgcc ccaagtatat caggcaaaac actttgaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctggag atctaaagag cactcaagca gccatcgacc
1201 agatcaatgg aaaattgaac agagtgattg aaaggaccaa tgagaaattc catcaaatag
1261 agaaggaatt ctcagaagta gaagggagaa tccaggactt ggagaagtat gtagaagaca
1321 ccaaaataga cctatggtcc tacaatgcag agttgctggt ggctctagaa aatcaacata
1381 caattgactt aacagatgca gaaatgaata aactattcga gaagactagg cgccagttaa
1441 gagaaaacgc ggaagacatg ggaggtggat gtttcaagat ttatcacaaa tgtgataatg
1501 catgcattgg atcaataaga aatgggacat atgaccatta catatacaga gatgaagcat
1561 taaacaaccg atttcaaatt aaaggtgtag agctgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct
```

<u>EXAMPLE 5:</u> Newmarket Equine Influenza Nucleotide Sequences

DEFINITION Influenza A virus (A/equine 2/Suffolk/89(H3N8)) NS1 gene.

ACCESSION X80060; SEQ ID NO: 37

```
  1 atggattcca acactgtgtc aagctttcag gtagactgtt ttctttggca tgtccgcaaa
 61 cgatttgcag accaagaact gggtgatgcc ccattccttg accggcttcg ccgagaccag
121 aagtccctaa aaggaagagg tagcactctt ggtctggaca tcgaaacagc cactcgtgca
181 ggaaagcaga tagtggagca gattctggaa gaggaatcag atgaggcatt taaaatgacc
241 attgcctctg ttcctgcttc acgctactta actgacatga ctcttgatga gatgtcaaga
301 gactggttca tgctcatgcc caagcagaaa gtaacaggct ccctatgtat aagaatggac
361 caggcaatca tggataagaa catcatactt aaagcaaact ttagtgtgat tttcgaaagg
421 ctggagacac taatactact cagggccttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataat acggttagag tctctgaaac tctacagaga
601 ttcgcttgga gaagcagtca tgagaatggg agaccttcat tccctccaaa gcagaaacga
661 aaaatggaga gaacaattga gtcagaagtt tga
```

DEFINITION Influenza A virus (A/eq/Newmarket/93/(H3N8)) HAI gene for HAY subunit of haemagglutinin, genomic RNA.

ACCESSION X85089; SEQ ID NO: 38

```
   1 atgaagacaa ccattatttt gatactactg acccattggg tctacagtca aaacccaacc
  61 agtggcaaca acacagccac attatgtctg ggacaccatg cagtagcaaa tggaacattg
 121 gtaaaaacaa taactgatga ccaaattgag gtgacaaatg ctactgaatt agtccagagc
 181 atttcaatag ggaaaatatg caacaactca tatagggttc tagatggaag aaattgcaca
 241 ttaatagatg caatgctagg agacccccat tgtgatgatt ttcagtatga gaattgggac
 301 ctcttcatag aaagaagcag cgctttcagc aattgctacc catatgacat ccctgactat
 361 gcatcgctcc ggtccattgt agcatcctca ggaacattag aattcacagc agaggggttc
 421 acatggacag gtgtcactca aaacggagga agtggagcct gcaaaagggg atcagccgat
 481 agtttcttta gccgactgaa ttggctaaca aaatctggaa attcttaccc catattgaat
 541 gtgacaatgc ctaacaataa aaatttcgat aaactataca tctgggggat tcatcacccg
 601 agctcaaaca aagagcagac aaaattatat atccaagaat caggacgagt aacagtctca
 661 acagaaagaa gtcaacaaac agtaatccct aacatcggat ctaggccgtg ggtcaggggt
 721 caatcaggca ggataagcat atactggacc attgtaaaac ctggagatat tctaatgata
 781 aacagtaatg gcaacttagt tgcaccgcgg ggatatttta aattgagaac agggaaaagc
 841 tctgtaatga gatcagatgc actcatagac acttgtgtgt ctgaatgtat tacaccaaat
 901 ggaagcatcc ccaacgacaa accatttcaa aatgtgaaca aaattacata tggaaaatgc
 961 cccaagtata tcaggcaaaa cactttaaag ctggccactg ggatgaggaa tgtaccagaa
1021 aagcaaatca ga
```

DEFINITION Influenza A virus (A/eq/Newmarket/93/(H3N8)) HAI gene for HAY subunit of haemagglutinin, genomic RNA.

ACCESSION X85088; SEQ ID NO: 39

```
   1 atgaagacaa ccattatttt gatactactg acccattggg tctacagtca aaacccaacc
  61 agtggcaaca acacagccac attatgtctg ggacaccatg cagtagcaaa tggaacattg
 121 gtaaaaacaa taactgatga ccaaattgag gtgacaaatg ctactgaatt agttcagagc
 181 atttcaatag ggaaaatatg caacaactca tatagagttc tagatggaag aaattgcaca
 241 ttaatagatg caatgctagg agacccccac tgtgatgtct ttcagtatga gaattgggac
 301 ctcttcatag aaagaagcag cgctttcagc aattgctacc catatgacat ccctgactat
 361 gcatcgctcc ggtccattgt agcatcctca ggaacattgg aattcacagc agagggattc
 421 acatggacag gtgtcactca aaacggaaga agtggagcct gcaaaagggg atcagccgat
 481 agtttcttta gccgactgaa ttggctaaca aaatctggaa actcttaccc cacattgaat
 541 gtgacaatgc ctaacaataa aaatttcgac aaactataca tctgggggat tcatcacccg
 601 agctcaaacc aacagcagac agaattgtac atccaagaat caggacgagt aacagtctca
 661 acaaaaagaa gtcaacaaac gataatccct aatatcggat ctagaccatg ggtcaggggt
 721 caatcaggca ggataagcat atactggacc attgtaaaac ctggagatat cctaatgata
 781 aacagtaatg gcaacttagt tgcaccgcgg ggatatttta aattgaaaac agggaaaagc
 841 tctgtaatga gatcagatgc acccatagac atttgtgtgt ctgaatgtat tacaccaaat
 901 ggaagcatcc ccaacgacaa accatttcaa aatgtgaaca aagttacata tggaaaatgc
 961 cccaagtata tcaggcaaaa cactttaaag ctggccactg ggatgaggaa tgtaccagaa
1021 aagcaaatca ga
```

DEFINITION Influenza A virus (A/eq/Sussex/89/(H3N8)) HAI gene for HAY subunit of haemagglutinin, genomic RNA.

ACCESSION X85090; SEQ ID NO: 40

```
   1 atgaagacaa ccattatttt gatactactg acccattggg tctacagtca aaacccaacc
  61 agtggcaaca acacagccac attatgtctg ggacaccatg cagtagcaaa tggaacattg
 121 gtaaaaacaa taactgatga ccaaattgag gtgacaaatg ctactgaatt agttcagagc
 181 atttcaatag ggaaaatatg caacaactca tatagggttc tagatggaag aaattgcaca
 241 ttaatagatg caatgctagg agacccccac tgtgatgttt ttcagtatga gaattgggac
 301 ctcttcatag aaagaagcag cgctttcagc aattgctacc catatgacat ccctgactat
 361 gcatcgctcc ggtccattgt agcatcctca ggaacattag aattcacagc agagggattc
 421 acatggacag gtgtcactca aaacggaaga agtggagcct gcaaaagggg atcagccgat
 481 agtttcttta gccgactgaa ttggctaaca aaatctggaa attcttaccc catattgaat
 541 gtgacaatgc ctaacaataa aaatttcgat aaactataca tctgggggat tcatcacccg
 601 agctcaaaca aagagcagac aaaattgtat atccaagaat caggacgagt aacagtctca
 661 acagaaagaa gtcaacaaac agtaatccct aacatcggat ctagaccgtg ggtcaggggt
 721 caatcaggca ggataagcat atactggacc attgtaaaac ctggagatat tctaatgata
 781 aacagtaatg gcaacttagt tgcaccgcgg ggatatttta aattgagaac agggaaaagc
 841 tctgtaatga gatcagatgc actcataggc acttgtgtgt ctgaatgtat tacaccaaat
 901 ggaagcatcc ccaacgacaa accatttcaa aatgtgaaca aagttacata tggaaaatgc
 961 cccaagtata tcaggcaaaa cactttaaag ctggccactg ggatgaggaa tgtaccagaa
1021 aagcaaatca ga
```

DEFINITION Influenza A virus (A/eq/Lambourn/92/(H3N8)) HAI gene for HAY subunit of haemagglutinin, genomic RNA.

ACCESSION X85087; SEQ ID NO: 41

```
   1 atgaagacaa ccattatttt gatactactg acccattggg tctacagtca aaacccaacc
  61 agtggcaaca acacagccac attatgtctg ggacaccatg cagtagcaaa tggaacattg
 121 gtaaaaacaa taactgatga ccaaattgag gtgacaaatg ctactgaatt agttcagagc
 181 atttcaatag ggaaaatatg caacaactca tatagggttc tagatggaag aaattgcaca
 241 ttaatagatg caatgctagg agacccccat tgtgatgatt ttcagtatga gaattgggac
 301 ctcttcatag aaagaagcag tgctttcagc aattgctacc catatggcat ccctgactat
 361 gcatcgctcc ggtccattgt agcatcctca ggaacattag aattcacagc agaggggttc

 421 acatggacag gtgtcactca aaacggaaga agtggagcct gcaaaagggg atcagccgat
 481 agtttcttta gccgactcaa ttggctaaca aaatctggaa attcttaccc catattgaat
 541 gtgacaatgc ctaacaataa aaatttcgat aaactataca tctgggggat tcatcacccg
 601 agctcaaaca aagagcagac aaaattatat atccaagaat caggacgagt aacagtctca
 661 acagaaagaa gtcaacaaac agtaatccct aacatcggat ctaggccgtg ggtcaggggt
 721 caatcaggca ggataagcat atactggacc attgtaaaac ctggagatat tctaatgata
 781 aacaataatg gcaacttagt tgcaccgcgg ggatatttta aattgagaac agggaaaagc
 841 tctgtaatga gatcagatgc actcatagac acttgtgtgt ctgaatgtat tacaccaaat
 901 ggaagcatcc ccaacgacaa accatttcaa aatgtgaaca aagttacata tggaaaatgc
 961 cccaagtata tcaggcaaaa cactttaaag ctggccactg ggatgaggaa tataccagaa
1021 aagcaaatca ga
```

DEFINITION Influenza A virus (A/eq/Ella/89/(H3N8)) HAI gene for HAY subunit of haemagglutinin, genomic RNA.

ACCESSION X85086; SEQ ID NO: 42

```
   1 atgaagacaa ccattatttt gatactactg acccattggg tctacagtca aaacccaacc
  61 agtggcaaca acacagccac attatgtctg ggacaccatg cagtagcaaa tggaacattg
 121 gtaaaaacaa taactgatga ccaaattgag gtgacaaatg ctactgaatt agttcagagc
 181 atttcaatag ggaaaatatg caacaactca tatagggttc tagatggaag aaattgcaca
 241 ttaatagatg caatgctagg agacccccac tgtgatgttt ttcagtatga gaattgggac
 301 ctcttcatag aaagaagcag cgctttcagc aattgctacc catatgacat ctctgactat
 361 gcatcgctcc ggtccattgt agcatcctca ggaacattag aattcacagc agagggattc
 421 acatggacag gtgtcactca aaacggaaga agtggagcct gcaaaagggg atcagccgat
 481 agtttcttta gccgactgaa ttggctaaca aaatctggaa attcttaccc catattgaat
 541 gtgacaatgc ctaacaataa aaatttcgat aaactataca tctgggggat tcatcacccg
 601 agctcaaaca aagagcagac aaaattgtat atccaagaat caggacgagt aacagtctca
 661 acagaaagaa gtcaacaaac agtaatccct aacatcggat ctagaccgtg ggtcaggggt
 721 caatcaggca ggataagcat atactggacc attgtaaaac ctggagatat tctaatgata
 781 aacagtaatg gcaacttagt tgcaccgcgg ggatatttta aattgagaac agggaaaagc
 841 tctgtaatga gatcagatgc acccataggc acttgtgtgt ctgaatgtat tacaccaaat
 901 ggaagcatcc ccaacgacaa accatttcaa aatgtgaaca aagttacata tggaaaatgc
 961 cccaagtata tcaggcaaaa cactttaaag ctggccactg ggatgaggaa tgtaccagaa
1021 aagcaaatca ga
```

DEFINITION Influenza A virus (A/eq/Arundel/91/(H3N8)) HAI gene for HAY subunit of haemagglutinin, genomic RNA.

ACCESSION X85085; SEQ ID NO: 43

```
   1 atgaagacaa ccattatttt gatactactg acccattggg tctacagtca aaacccaacc
  61 agtggcaaca acacagccac attatgtctg ggacaccatg cagtagcaaa tggaacattg
 121 gtaaaaacaa taactgatga ccaaattgag gtgacaaatg ctactgaatt agttcagagc
 181 atttcaatag ggaaaatatg caacaactca tatagagttc tagatggaag aaattgcaca
 241 ttaatagatg caatgctagg agacccccac tgtgatgtct ttcagtatga gaattgggac
 301 ctcttcatag aaagaagcag cgctttcagc aattgctacc catatgacat ccctgactat
 361 gcatcgctcc ggtccattgt agcatcctca ggaacattag aattcacagc agagggattc
 421 acatggacag gtgtcactca aaacggaaga agtggagcct gcaaaagggg atcagccgat
 481 agtttcttta gccgactgaa ttggctaaca aaatctggaa attcttaccc catattgaat
 541 gtgacaatgc ctaacaataa aaatttcgat aaactataca tctgggggat tcatcacccg
 601 agctcaaaca aagagcagac aaaattgtac atccaagaat caggacgagt aacagtctca
 661 acaaaaagaa gtcaacaaac aataatccct aacatcggat ctagaccgtg ggtcaggggt
 721 caatcaggca ggataagcat atactggacc attgtaaaac ctggagatat cctaatgata
 781 aacagtaatg gcaacttagt tgcaccgcgg ggatatttta aattgagaac agggaaaagc
 841 tctgtaatga gatcagatgc acccatagac atttgtgtgt ctgaatgtat tacaccaaat
 901 ggaagcatcc ccagcgacaa accatttcaa aatgtaaaca aagttacata tggaaaatgc
 961 cccaagtata tcaggcaaaa cactttaaag ctggccactg ggatgaggaa tgtaccagaa
1021 aagcaaatca ga
```

DEFINITION Influenza A virus (A/equine/Suffolk/89(H3N8)) HA gene for haemagglutinin.

ACCESSION X68437; SEQ ID NO: 44

```
   1 ctgtcaatca tgaagacaac cattattttg atactactga cccattgggt ctacagtcaa
  61 aacccaacca gtggcaacaa cacagccaca ttatgtctgg gacaccatgc agtagcaaat
 121 ggaacattgg taaaaacaat aactgatgac caaattgagg tgacaaatgc tactgaatta
 181 gttcagagca tttcaatagg gaaaatatgc aacaactcat atagggttct agatggaaga
 241 aattgcacat taatagatgc aatgctagga gacccccact gtgatgtttt tcagtatgag
 301 aattgggacc tcttcataga aagaagcagc gctttcagca attgctaccc atatgacatc
 361 cctgactatg catcgctccg gtccattgta gcatcctcag gaacattaga attcacagca
 421 gagggattca catggacagg tgtcactcaa aacggaagaa gtggagcctg caaaagggga
 481 tcagccgata gtttctttag ccgactgaat tggctaacaa aatctggaaa ttcttacccc
 541 atattgaatg tgacaatgcc taacaataaa aatttcgata aactatacat ctgggggatt
 601 catcacccga gctcaaacaa agagcagaca aaattgtata tccaagaatc aggacgagta
 661 acagtctcaa cagaaagaag tcaacaaaca gtaatcccta acatcggatc tagaccgtgg
 721 gtcaggggtc aatcaggcag gataagcata tactggacca ttgtaaaacc tggagatatt
 781 ctaacgataa acagtaatgg caacttagtt gcaccgcggg gatattttaa attgagaaca
 841 gggaaaagct ctgtaatgag atcagatgca cccatagaca cttgtgtgtc tgaatgtatt
 901 acaccaaatg gaagcatccc caacgacaaa ccatttcaaa atgtgaacaa agttacatat
 961 ggaaaatgcc ccaagtatat caggcaaaac actttaaagc tggccaccgg gatgaggaat
1021 gtaccagaaa agcaaatcag aggaatcttt ggagcaatag cgggattcat agaaaacggc
1081 tgggaaggaa tggttgatgg gtggtatgga ttccgatatc aaaactcgga aggaacagga
1141 caagctgcag atctaaagag cactcaagca gccatcgacc agatcaatgg aaaattaaac
1201 agagtgattg aaaggaccaa tgagaaattc catcaaatag agaaggaatt ctcagaagta
1261 gaagggagaa tccaggattt ggagaagtat gtagaagaca ccaaaataga cctatggtcc
1321 tacaatgcag aattgctggt ggctctagaa aatcaacata caattgactt aacagatgca
1381 gaaatgaata aattattcga gaagactagg cgccagttaa gagaaaacgc ggaagacatg
1441 ggaggtggat gtttcaagat ttaccacaaa tgtgataatg catgcattgg atcaataaga
1501 aatgggacat atgaccatta catatacaga gatgaagcat taaacaaccg atttcaaatc
1561 aaaggtgttg agttgaaatc aggctacaaa gattggatac tgtggatttc attcgccata
1621 tcatgcttct taatttgcgt tgttctattg ggtttcatta tgtgggcttg ccaaaaaggc
1681 aacatcagat gcaacatttg catttgagta aactgatagt taaaaacacc cttgtttcta
1741 ct
```

DEFINITION Influenza A virus (A/equine/Newmarket/1/77(H7N7)) gene for haemagglutinin.

ACCESSION X62554; SEQ ID NO: 45

```
   1 nnnnnnnnnn nnnnnnnnna aatgaacact cagattctaa tattagccat ttcggcattc
  61 ctctgtgtac gtgcagataa aatctgccta ggacatcatg ctgtgtctaa tggaaccaaa
 121 gtagacaccc ttactgaaaa gggaatagaa gtcgtcaatg caacagaaac agttgaacaa
 181 aaaaacatcc ccaagatctg ctcaaaaggg aaacagacta ttgaccttgg tcaatgtgga
 241 ttactaggga ccactattgg tccccccaa tgcgaccaat ttcttgaatt ctctgctaat
 301 ttaataattg agagaagaga aggtgatgat atttgttatc caggcaaatt tgacaatgaa
 361 gaaacattga gacaaatact cagaaaatcc ggaggaatta aaaaggagaa tatgggattc
 421 acatataccg gagtgagaac caatggagag actagcgcct gtagaaggtc aagatcttcc
 481 ttttatgcag aaatgaaatg gctcctatct aacacagaca atggggtatt cccacaaatg
 541 acaaaatcct acaagaacac taagaaggag ccagctctga taatctgggg aatccaccac
 601 tcaggatcaa ctgctgaaca gactagattg tatggaagtg gaaacaagtt gataacagtt
 661 tggagttcca aataccaaca atcttttgcc ccaaaccctg gaccaaggcc gcaaatgaat
 721 ggccaatcag gaagaattga cttttactgg ctgatgttag atcccaatga tactgttaat
 781 ttcagtttta atggggcctt tatagcacct gaccgcgcca gttttctaag aggtaaatct
 841 ctaggaattc agagtgacgc acaacttgac aacaattgtg aaggtgaatg ttatcatatt
 901 ggaggtacca taattagcaa cttgcccttt caaaacatta atagcagagc aattgggaaa
 961 tgccccagat acgtaaagca aaaaagctta atgctagcaa ccggaatgaa aaatgttcct
1021 gaaaattcta cacacaaaca gttaactcat cacatgcgca aaaaaagagg tttatttggt
1081 gcaatagcag gatttattga aaatggatgg gaaggattaa tagatggatg gtatggatac
1141 agacatcaga atgcacaagg agaaggaact gctgcagact acaaaagtac acaatctgct
1201 gtcaatcaaa taaccgggaa attaaacaga ctaatagaaa aaaccaacca gcaatttgaa
```

```
1261 ctaatagata atgaattcaa tgaaatagaa aagcaaattg gcaatgttat taactggact
1321 agagattcta tcatcgaaat atggtcatat aatgcagaat tcctcgtggc agtggagaat
1381 caacacacta ttgatttaac tgattcagag atgaacaaat tatatgaaaa ggtaagaaga
1441 cagctgagag aaaatgctga ggaagatggt aatggctgtt ttgaaatatt tcaccaatgt
1501 gacaatgatt gcatggccag cattagaaac aatacatatg atcataaaaa atacagaaag
1561 gaggcaatac aaaacagaat tcagattgat gcagtaaagt tgagcagcgg ttacaaagaa
1621 ataatacttt ggtttagctt cggggcatca tgtttcttat ttcttgccat tgcaatggtt
1681 cttgctttca tatgcataaa aaatggaaac atgcggtgca ctatttgtat ataagtttga
1741 aaaaacaccc ttgtttctan n
```

DEFINITION Influenza A virus HA partial gene for haemagglutinin, genomic RNA, strain A/equine/Newmarket-Bob Champion/89(H3N8).

ACCESSION AJ223193; SEQ ID NO: 46

```
   1 agtcaaaacc caaccagtgg caacaacaca gccacattat gtctgggaca ccatgcagta
  61 gcaaatggaa cattggtaaa aacaataact gatgaccaaa ttgaggtgac aaatgctact
 121 gaattagttc agagcatttc aatagggaaa atatgcaaca actcatatag ggttctagat
 181 ggaagaaatt gcacattaat agatgcaatg ctaggagacc cccactgtga tgtttttcag
 241 tatgagaatt gggacctctt catagaaaga agcagcgctt tcagcaattg ctacccatat
 301 gacatccctg actatgcatc gctccggtcc attgtagcat cctcaggaac attagaattc
 361 acagcagagg gattcacatg gacaggtgtc actcaaaacg gaagaagtgg agcctgcaaa
 421 aggggatcag ccgatagttt ctttagccga ctgaattggc taacaaaatc tggaaattct
 481 taccccatat tgaatgtgac aatgcctaac aataaaaatt tcgataaact atacatctgg
 541 gggattcatc acccgagctc aaacaaagag cagacaaaat tgtatatcca agaatcagga
 601 cgagtaacag tctcaacaga aagaagtcaa caaacagtaa tccctaacat cggatctaga
 661 ccgtgggtca ggggtcaatc aggcaggata agcatatact ggaccattgt aaaacctgga
 721 gatattctaa tgataaacag taatggcaac ttagttgctc cgcgggggata ttttaaattg
 781 agaacaggga aaagctctgt aatgagatca gatgcaccca tagacacttg tgtgtctgaa
 841 tgtattacac caaatggaag catccccaac gacaaaccat ttcaaaatgt gaacaaagtt
 901 acatatggaa aatgccccaa gtatatcagg caaaacactt taaagctggc cactgggatg
 961 aggaatgtac cagaaaagca aatcagagga atctttggag caatagaggg attcatagaa
1021 aacggctggg aaggaatggt tgatgggtgg tatggattcc gatatcaaaa ctcggaagga
1081 acaggacaag ctgcag
```

DEFINITION Influenza A virus (A/Equine/NewMarket/D64/79(H3N8)) gene for hemagglutinin precursor, partial cds.

ACCESSION D30677; SEQ ID NO: 47

```
   1 ctgtcaatca tgaagacaac cattattttg atactactga cccattgggt ctacagtcaa
  61 aacccaacca gtggcaacaa cacagccaca ctatgtctgg gacaccatgc agtagcaaat
 121 ggaacattgg taaaaacaat aactgatgac caaattgagg tgacaaatgc cactgaatta
 181 gttcagagca cttcaatagg gaaaatatgc aacaacccat atagggttct agatggaaga
 241 aactgcacat taatagatgc aatgctagga gatcccccact gtgatgtttt tcagtatgag
 301 aattgggacc tcttcataga aagaagcagc gctttcagca attgctaccc atatgacatc
 361 cctgactatg catcgctccg gtctattgtg gcatcttcag gaacattaga attcacagca
 421 gagggattca catggacagg tgtcactcaa aacggaagaa gtggcgcctg cagaagggga
 481 tcagccgata gtttctttag ccgactgaat tggctaacaa aatctggaga ttcttacccc
 541 acattgaatg tgacaatgcc taacaataac aatttcgata aactatacat ctgggggatc
 601 catcacccga gcacaaacaa tgagcagaca aaattgtatg tccaagaatc agggcgagta
 661 acagtctcaa caaaaagaag tcaacaaaca ataatcccca acatcggatc tagaccgtgg
 721 gtcaggggtc aaccaggcag gataagcata tattggacca ttgtgaaacc tggagatatc
 781 ctaatgataa acagtaatgg caacttagtt gcaccgcggg gatatttcaa aatgcgaaca
 841 ggaaaaagct ctataatgag atcagatgca cccatagaca cttgtgtgtc cgagtgtatt
 901 acaccaaatg gaagcatccc caacgacaaa ccatttcaaa atgtgaacaa agttacatat
 961 gggaaatgcc ccaagtatat caagcagaat actttgaagc tggccactgg gatgaggaat
1021 gtaccagaaa agcaaatcag aggaatcttt ggagcaatag cgggattcat agaaaatggc
1081 tgggag
```

DEFINITION Influenza A virus (A/eq/Newmarket/1/77(H7N7)) matrix proteins M1 and M2 (M) gene, complete cds.

ACCESSION AF001686; SEQ ID NO: 48

```
  1 atgagtcttc tgaccgaggt cgaaacgtac gttctctcta tcgtaccatc aggcccccctc
 61 aaagccgaga tcgcgcagag acttgaagat gtctttgcag gaaagaacac cgatcttgag
121 gcactcatgg aatggctaaa gacaagacca atcctgtcac ctctgactaa ggggatttta
181 ggatttgtgt tcacgctcac cgtgcccagt gagcgaggac tgcagcgtag acgctttgtc
241 caaaatgccc ttaatgggaa cggagatcca aacaacatgg acagagcagt aaaactgtac
301 aggaagctta aaagggaaat aacattccat ggggcaaaag aggtggcact cagctattcc
361 actggtgcac tagccagctg catgggactc atatacaaca gaatggggac tgtgacaacc
421 gaagtggcat ttggcctggt atgcgccaca tgtgaacaga ttgctgattc ccagcaccga
481 tctcacagac agatggtgac aacaaccaac ccactaatca gacacgagaa cagaatggta
541 ctagccagta ccacagctaa agccatggag cagatggcag ggtcgagtga gcaggcagca
601 gaggccatgg aggttgctag tcaggccagg cagatggtgc aggcaatgag aaccattggg
661 acccacccta gctccagtgc cggtttgaaa aatgatcttc ttgaaaattt gcaggcctac
721 cagaaacgga tgggagtgca aatgcagcga ttcaagtgat cctctcgtta ttgcagcaag
781 tatcattggg atcttgcact tgatattgtg gattcttgat cgtcttttct tcaaatgcat
841 ttatcgtcgt cttaaatacg gtttgaaaag agggccttct acggaaggag tacctgagtc
901 tatgagggaa gaatatcggc aggaacagca gagtgctgtg gatgttgacg atggtcattt
961 tgtcaacata gagctggagt aa
```

DEFINITION Influenza A virus (A/eq/Newmarket/79(H3N8)) matrix proteins M1 and M2 (M) gene, complete cds.

ACCESSION AF001675; SEQ ID NO: 49

```
  1 atgagtcttc taaccgaggt cgaaacgtac gttctctcta tcgtaccatc aggcccccctc
 61 aaagccgaga tcgcgcagag acttgaagat gtctttgcag ggaagaacac cgatcttgag
121 gcactcatgg aatggctaaa gacaagacca atcctgtcac ctctgactaa agggatttta
181 ggatttgtgt tcacgctcac cgtgcccagt gagcgaggac tgcagcgtag acgctttgtc
241 caaaatgccc ttagtggaaa cggagatcca aacaacatgg acagagcagt aaaactgtac
301 aggaagctta aaagagaaat aacattccat ggggcaaaag aggtggcact cagctattcc
361 actggtgcac tagccagctg catgggactc atatacaaca gaatggggac tgtgacaacc
421 gaagtggcat ttggcctggt atgcgccaca tgtgaacaga ttgctgattc ccagcatcga
481 tctcacaggc agatggtgac aacaaccaac ccactaatca gacatgaaaa cagaatggta
541 ctagccagta ccacagctaa agccatggag cagatggcag ggtcgagtga gcaggcagca
601 gaggccatgg aggttgctag taaggccagg cagatggtac aggcaatgag gaccattggg
661 acccacccta gctccagtgc cggtttgaaa gatgatcttc ttgaaaattt gcaggcctac
721 cagaaacgga tgggagtgca aatgcagcga ttcaagtgat cctctcgtta ttgcagcaag
781 tatcattggg atcttgcact tgatattgtg gattcttgat cgccttttct tcaaattcat
841 ttatcgtcgc cttaaatacg gtttgaaaag agggccttct acggaaggag tacctgagtc
901 tatgagggaa gaatatcggc aggaacagca gaatgctgtg gatgttgacg atggtcattt
961 tgtcaacata gagctggagt aa
```

DEFINITION Influenza A virus (A/eq/Newmarket/1/77(H7N7)) nonstructural proteins NS1 and NS2 (NS) gene, complete cds.

ACCESSION AF001663; SEQ ID NO: 50

```
  1 atggattcca acactgtgtc aagctttcag gtagactgtt ttctttggca tgtccgcaaa
 61 cgatttgcag accaagaact gggtgatgcc ccattccttg accggcttcg ccgagaccag
121 aagtccctaa aaggaagagg cagcactctt ggtctggaca tcgaaacagc cactcatgca
181 ggaaagcaga tagtggagcg aattctggaa gaggaatcag atgaggcact taaaatgacc
241 atagcctctg ttcctacttc acgctactta actgacatga ctcttgatga gatgtcaaga
301 gactggttca tgctcatgcc caagcagaaa gtaacaggct ccctatgtat aaggatggac
361 caagcaatca tggataagaa catcatacta aaagcaaact ttagtgtgat tttcgaaagg
421 ctggagacac taatactact tagagctttc accgaagaag gagcagtcgt tggcgaaatt
481 tcaccattgc cttctcttcc aggacatact aatgaggatg tcaaaaatgc aattggggtc

541 ctcatcggag gacttaaatg gaatgataac acagttagag tctctgaaac tctacagaga
601 ttcgcttgga gaagcagtca tgagaatggg agaccttcat tccctccaaa gcagaaacga
661 aaaatggcga gaacaattga gtcagaagtt tgaagaaata aggtggttga ttgaagaagt
721 gagacataga ttgaaaaata cagaaaatag ttttgaacaa ataacattta tgcaagcctt
781 acaactattg cttgaagtag aacaagagat aagaactttc tcgtttcagc ttatttaa
```

DEFINITION Influenza A virus (A/eq/Newmarket/D63/79(H3N8)) nonstructural proteins NS1 and NS2 (NS) gene, complete cds.

ACCESSION AF001662; SEQ ID NO: 51

```
  1 atggattcca acactgtgtc aagctttcag gtagactgtt ttctttggca tgtccgcaaa
 61 cgatttgcag accaagaact gggtgatgcc ccattccttg accggcttcg ccgagaccag
121 aagtccctaa aaggaagagg cagcactctt ggtctggaca tcgaaacagc cactcgtgca
181 ggaaagcaga tagtggagcg gattctggaa gaggagtcag atgaggcact taaaatgacc
241 attgcctctg ttcctgcttc acgctactta actgacatga ctcttgatga gatgtcaaga
301 gactggttca tgctcatgcc caagcagaaa gtaacaggct ccctatgtat aaggatggac
361 caggcaatca tggataagaa catcatacta aaagcaaact ttagtgtgat tttcgaaagg
421 ctggagacac taatactact tagagccttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataat acagttagag tctctgaaac tctacagaga
601 ttcgcttgga gaagcagtca tgagaatggg agaccttcat tccctccaaa gcagaaacga
661 aaaatggcga gaacaattga gccagaagtt tgaagaaata agatggttga ttgaagaagt
721 gcgacataga ttgaaaaata cagaaaatag ttttgaacaa ataacattta tgcaagcctt
781 acaactattg cttgaagtag aacaagagat aagaactttc tcgtttcagc ttatttaa
```

EXAMPLE 6: Efficacy of inactivated influenza virus vaccine in canines.

Vaccine active ingredient (AI) is produced either in 9-11 day-old embryonated chicken eggs or in continuous cell cultures such as MDCK cells.

Egg Production, Harvest and Purification. Vaccine AI production in embryonating chicken eggs is prepared by inoculation 0.1 - 0.25 ml of virus suspension containing 100-1000 EID50 (egg infectious dose) into the allantoic cavity. Allantoic fluid is harvested after incubation at 33-37 °C for 2-6 days and pooled. The virus pool is clarified by centrifugation at 1500 - 4000g for 5-15 minutes and filtered through a 20 μm filter (1). The virus maybe concentrated by ultra-filtration using a 300 KDa membrane with a maximun concentration factor of 20 (2).

Further the concentrated virus is optionally purified by zonal ultra-centrifugation on a sucrose gradient followed by filtration of the virus containing fraction through a 0.5 μm membrane. This step can be repeated. The virus containing fraction is diluted in a suitable buffer such as a phosphate buffer (3).

Further purification consists of treatment with Tween/ether at 0.1 % final concentration (w/v)) and 50% final concentration (w/w), respectively. The resulting solution is centrifuged and the aqueous phase is collected. This step can be repeated. The aqueous phase is treated with nitrogen to remove residual ether (4).

Cell Culture Production, Harvest and Purification. Active ingredient is prepared in cells (e.g. MDCK, BHK, PerC6 cells) by inoculation at a rate of MOI of 0.001-1 TCID50. Culture is harvested after incubation at 35-37C for 48-96 hrs when cytopathic effect (CPE) is approximately 50-90%. Culture is sonicated and centrifuged at 1500-4000g for 5-15 minutes (1). The virus is concentrated by ultra-filtration using a 300 KD membrane with a max. concentration factor of 20 (2).

The virus is optionally purified by exclusion chromatography (Sepharose 6 fast flow)(3).

Inactivation. Virus (fraction 1, 2, 3 or 4) are inactivated by addition of formalin or beta-propiolactone to a final concentration of 0.05 % (w/v) or 0.01 % (w/v), respectively and the suspension is held respectively at 5 °C for 16-18 hours or at 20°C for 25-36 hours with continuous agitation.

Active ingredient inactivation is confirmed by inoculation into embryonated chicken eggs or onto MDCK cells.

Preparation of Final Vaccine. Vaccine consists of the inactivated influenza virus and an adjuvant such as aluminium hydroxide, aluminum phosphate, Carbomer or oil-in-water emulsion, which may contain other immunostimulating components such as CpG (1-100 ug/dose). Vaccine typically contains 15-50 μg of hemagglutinin measured by single radial-immunodiffusion (SRD) or >400 haemagglutination Units

The vaccine is administered either by subcutaneous or intramuscular route. Two doses are administered at an interval of 2-5 weeks to pups from 4 weeks of age. Duration of immunity: 6-12 months after a primary vaccination course.

Vaccination/Challenge Protocol. Dogs (less than 4 weeks old) are vaccinated with 2 doses of inactivated vaccine at 3-4 weeks interval via the subcutaneous route. Blood samples are collected on the day of the first and second vaccination and 2-4 weeks after second vaccination to determine the levels of anti-influenza virus specific antibodies using virus neutralization, hemagglutination inhibition, ELISA or Single Radial Heamolysis (SRH) tests.

Two to 4 weeks after $2^{nd}$ immunization dogs are challenged with a virulent strain of influenza H3N8 to determine efficacy of the vaccines.

Vaccinated and control dogs are challenged by the following route:

By spray: animals are placed into an enclosed container or a mask is placed on the nose of the dogs and the virus suspension is aerosolized to generate 1-100 μM droplets. Animals are kept in the aerosolization chambers for 30 minutes to allow inhalation of the aerosol.

Alternativelly thy can be challenged by one the following routes:

Intra-nasally: 0.5 ml containing $10^{5-8}$ EID50 of challenge virus is dropped to each nostril by the aid of dropper.

Intra-tracheally: 1-2 ml containing $10^{5-8}$ $EID_{50}$ virus is directly instilled into the trachea.

Orally: 5- 10 ml containing $10^{5-8}$ EID50 of challenge virus is orally administered.

Animals are observed daily for 14 days following challenge for clinical signs including fever, cough, nasal, ocular discharge, respiratory distress, anorexia and lethargy. In addition, groups of animals are euthanized and evaluated for pathological findings of pulmonary and pleural hemorrhage, tracheitis, bronchitis, broncholilitis, and bronchopneumonia.

Tracheal swabs are collected from all animals post challenge days 1-14 for virus isolation.

Presence or absence of viral antigens in respiratory tissues is evaluated by immunohistochemistry on days 3, 7 and 10 post-challenge.

Blood samples are collected on days 7 and 14 post-challenge and are analyzed for the presence of anti-influenza H3N8 virus specific antibody.

EXAMPLE 7: Vaccination of Dogs with Equine Influenza Vaccines to Induce a Humoral Immune Response Against CIV

A study was conducted in which 35 dogs were randomly allocated into four groups of 9, 9, 9 and 8 dogs. None of the dogs had detectable antibodies to CIV, as determined by HI tests (below) at the commencement of the study. One group was immunized with vCP1529 (canarypox recombinant expressing HA gene from Kentucky equine influenza, A/eq/Kentucky/94) $10^{5.7}$ $TCID_{50}$/ 1 ml dose, which was administered subcutaneously, and two groups were immunized with vCP2242 (canarypox recombinant expressing HA gene from A/eq/Ohio/03), which was administered to one group subcutaneously ($10^{5.7}$ $TCID_{50}$/ 1 ml dose) and to one group transdermally ($10^{5.7}$ $TCID_{50}$/ 0.25 ml dose). Vaccinates received 2 doses of vaccine on Day 0 and Day 28. The fourth group was unvaccinated and served as a control. All dogs were examined for local reactions at the site of inoculation and for general clinical signs.

Blood samples were collected for serology on days -1, 7, 13 , 21, 28, 35, and 42 for all groups. A hemagglutination inhibition (HI) test was performed as described previously (Karaca et al., 2007). Briefly, sera were separated and were heat inactivated (56°C for 30 minutes). Sera were then treated with potassium periodate followed by incubation with turkey RBCs to remove nonspecific HAs and inhibitors. Four hemagglutination units of antigen were added to serial 2-fold dilutions of treated sera and incubated at 18° to 22°C for 30 minutes. Turkey RBCs were added, and plates were incubated for an additional 30 minutes. Wells were then examined for evidence of hemagglutination. The lowest dilution of serum tested was 1:8, and antibody titers corresponding to the reciprocal of the highest dilution that inhibited hemagglutination were presented as geometric mean titers (GMTs).

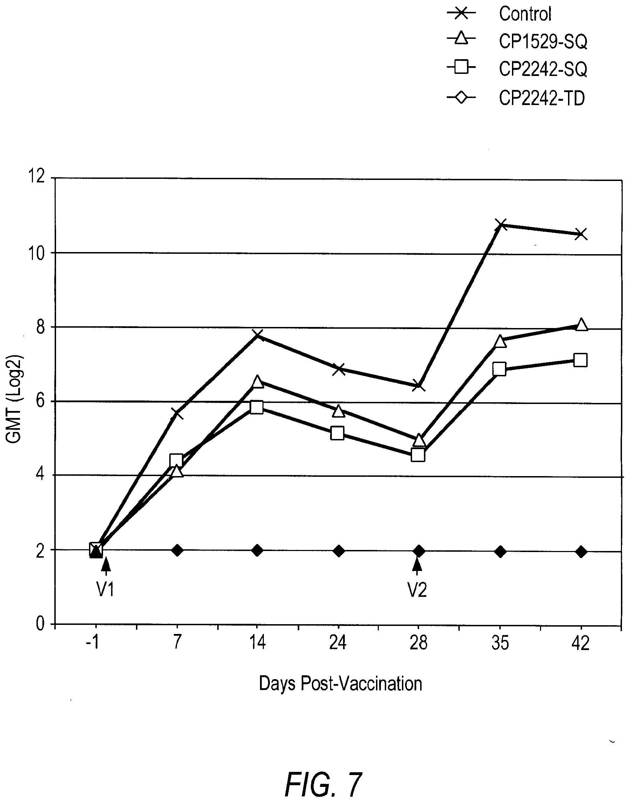

All dogs tolerated the inoculations well. None of the dogs had a systemic or local adverse reaction, regardless of the inoculum or route of administration. Antibody responses of dogs as measured by HI tests are shown in FIG. 7. On day -1, all dogs were seronegative (antibody titres <8). Control dogs remained seronegative throughout the duration of the study. All inoculated dogs had detectable antibodies against CIV on days 13, 21, and 28 after the initial inoculation. Although there was no significant difference in antibody titers between subcutaneously inoculated groups, antibody titres of the transdermally inoculated group were significantly higher than the others after the first and second inoculations. (repeated measures ANOVA, $p<0.01$).

EXAMPLE 8: Vaccination of Dogs with Equine Influenza Vaccines and challenge with a virulent Canine Influenza Isolate.

A study was conducted in which 33 dogs were randomly allocated to one of two groups. Dogs of group 1 (n=16) were not inoculated and served as a control. Dogs of group 2 (n=17) were inoculated subcutaneously on day 0 and day 28 with $10^{5.7}$ $TCID_{50}$ of CP2242 (canarypox recombinant expressing HA gene from A/eq/Ohio/03). All dogs were challenged on day 43 with $10^{7.0}$ $TCID_{50}$ of CIV-NY05 first by syringe 0.5ml directly into the nares followed with challenge by aerosolization into a closed chamber for 5-15 minutes, with oxygen supplied using a commercially available nebulizer. All dogs were clinically observed pre and post challenge according to the timeline in Table 2.

**Table 2: Timeline**

| (VI = virus isolation; Bb = *Bordetella bronchiseptica* isolation) | | |
|---|---|---|
| **Study Day** | **DPC*** | **ACTIVITY** |
| D-4 | N/A | Baseline injection site observations<br>Nasal swab for VI and *Bb*<br>Baseline Blood for serology |
| D0 | | Vaccination #1 |
| D1-4 | | Injection site observations |
| D7 | | Injection site observations<br>Weigh |
| D16-18 | | Baseline Injection site observations |
| D21 | | Baseline Injection site observations<br>Weigh<br>Nasal swabs for VI and *Bb*<br>Baseline Blood Collection for serology<br>Vaccination #2 |
| D22-25 | | Injection site observations |

(continued)

| (VI = virus isolation; Bb = *Bordetella bronchiseptica* isolation) | | |
|---|---|---|
| **Study Day** | **DPC*** | **ACTIVITY** |
| D28 | | Injection site observations<br>Baseline Clinical observations<br>Nasal swabs for VI and *Bb*<br>Baseline Blood Collection for serology |
| D42 | | Baseline Clinical Observations (Weigh)<br>Nasal swabs for VI and *Bb*<br>Baseline Blood collection for serology |
| D43 | | Challenge |
| D44-48 | 1-5 | Clinical observations<br>Nasal swabs for VI and*Bb* |
| D49 | 6 | Clinical observations (Weigh)<br>Nasal swabs for VI and*Bb* |
| D50 | 7 | Clinical observations<br>Nasal swabs for VI and Bb<br>Blood for serology |
| D52-53 | 9-10 | Clinical observations<br>Nasal swabs for VI and Bb |
| D57 | 14 | Clinical observations (Weigh)<br>Nasal swabs for VI and Bb<br>Blood for serology<br>Euthanasia- End of Study |
| * Days Post-Challenge | | |

The clinical signs that were recorded as absent or present include cough (spontaneous or inducible by mild tracheal palpation), ocular/nasal discharge (serous or purulent), rectal temperature (°F), depression/lethargy, dyspnea, and weight loss.

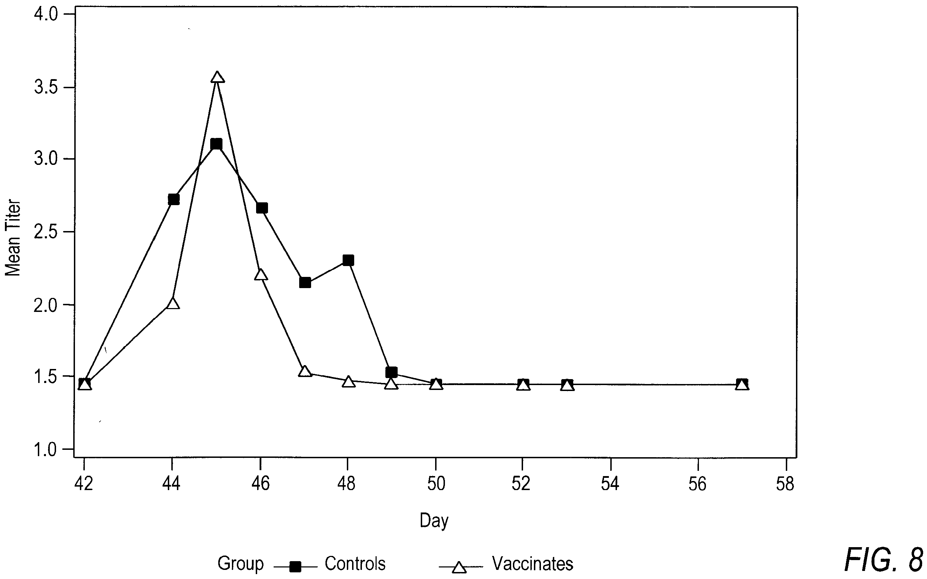

All dogs were evaluated for CIV viral load, body temperature, HI antibody responses to CIV HA antigen, and virus shedding. To determine CIV viral loads, nasal swabs were collected from all animals on days -4, 21, 28, and 42 pre-challenge samples and on days 44-50, 52, 53 and 57 (1-7, 9, 10 and 14 days post-challenge). Levels of virus were determined using a virus titration assay on Madine Darby canine kidney (MDCK) cells and the titer results were expressed as $TCID_{50}$. FIG. 8 illustrates describes the mean viral titers from swab extracts in controls and vaccinates. In this example, a $TCID_{50}$ of 1.45 is the minimum detectable titer, therefore a titer of 1.45 = no virus detected. The vaccinated animals stop shedding at Day 47 whereas control animals shed until Day 49. Controls had a significantly higher titer than vaccinates on Day 44 ($p<0.0001$), Day 46 ($p=0.0016$), Day 47 ($p=0.0027$) and Day 48 ($p<0.0001$).

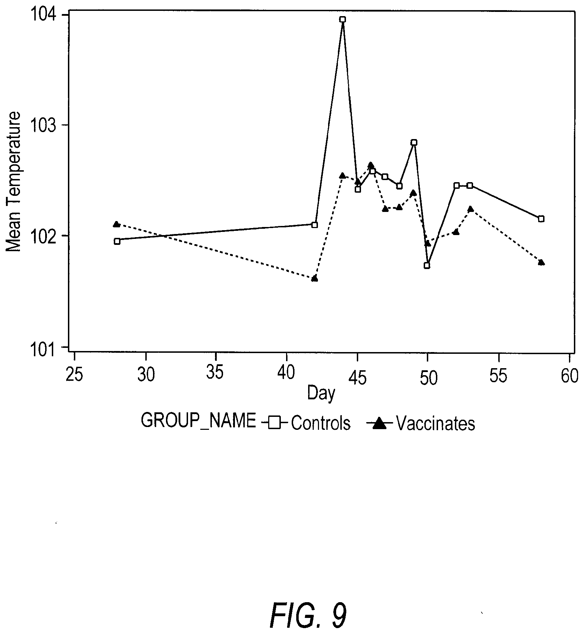

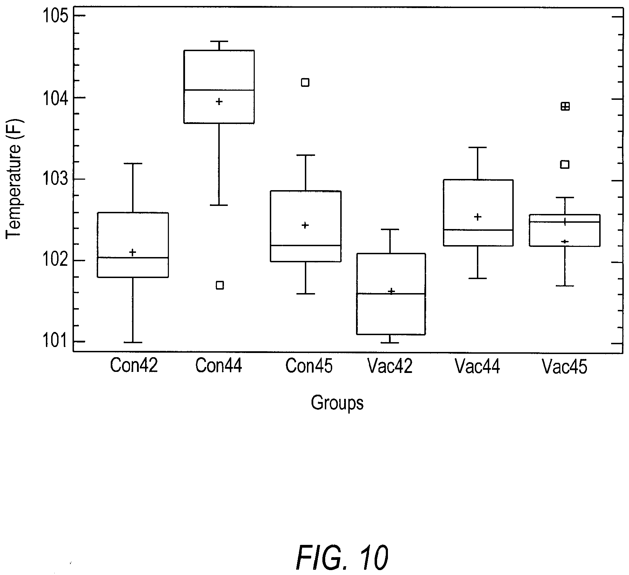

Body temperatures of dogs were evaluated throughout the study. The mean body temperatures of dogs vaccinated with vCP2242 were lower than the mean body temperatures of control dogs (FIGS. 9 and 10).

FIG. 9 shows the mean temperature over time from Day 28 to Day 57. Controls show an increase from a ~102oF baseline to ~104oF, 24 hours after challenge whereas vaccinate temperature increase from 102oF to ~102.5oF (statistically significant difference on that day with $p<0.0001$). Controls also have a significantly higher mean body temperature on day 49 ($p=0.048$).

FIG. 10 is a boxplot for Days 42 (1 day pre-challenge), 44 and 45 (1 and 2 days post-challenge).

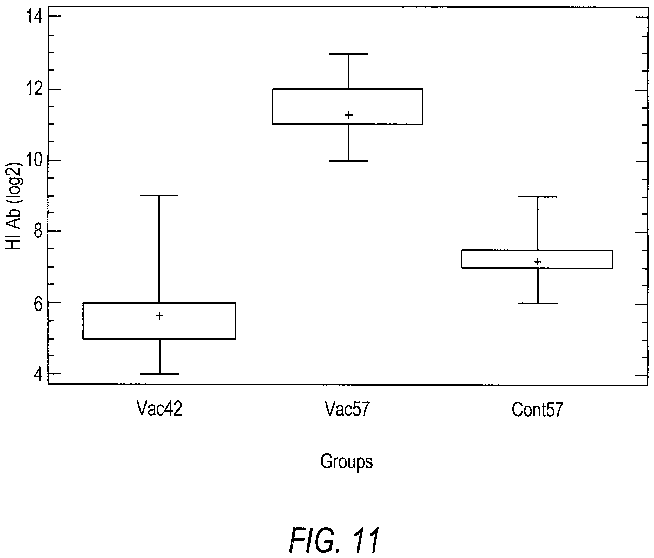

FIG. 11 shows the HI titers on Day 42 (1 day pre-challenge) and Day 57 (14 days post-challenge). A virulent CIV-NY05 challenge boosts the HI titers of dogs vaccinated with vCP2242. Although unvaccinated animals seroconverted after challenge, their mean Day 57 HI titer was significantly lower than vaccinates. This is an evidence of the efficient priming provided by the vaccine.

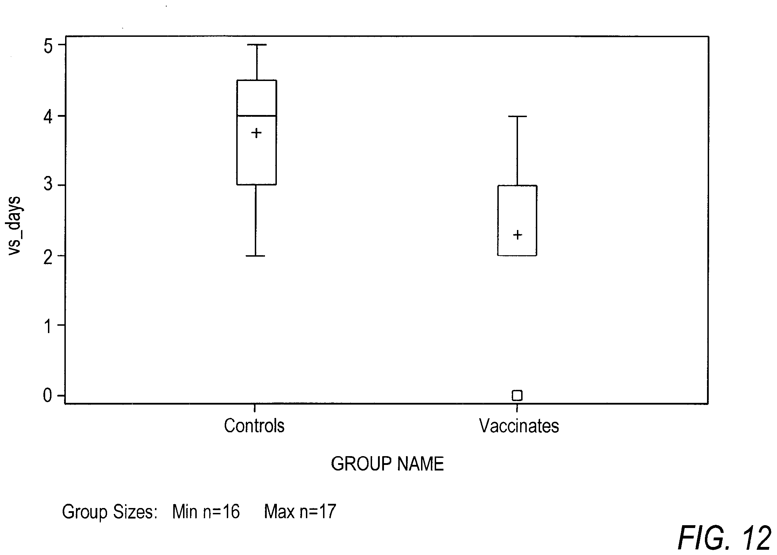

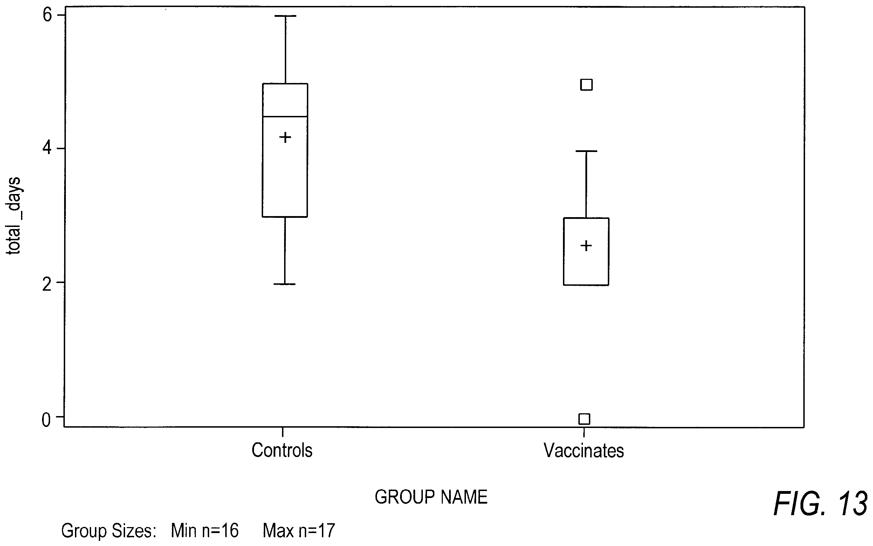

On average, dogs vaccinated with with HA antigen of A2/equine/Ohio/03 (vCP2242) followed by challenge at day 43 with CIV-NY05 isolate demonstrated a decreased number of shedding days as compared to control dogs (FIG. 12) as well as a decreased duration of virus shedding in the nasal cavity (FIG. 13).

FIG. 12 show the number of shedding days illustrating the decrease in number of shedding days in the vaccinates vs. controls.

FIG.13 shows the decrease in the duration of virus shedding in the vaccinates.

The invention is defined in the claims and the accompanying description.
Other aspects of the description are! described by the following numbered paragraphs:

1. A method of eliciting an immune response against influenza in a canine, comprising administering a formulation comprising an avipox expression vector comprising a polynucleotide encoding an influenza antigen, epitope or immunogen and a pharmaceutically or veterinarily acceptable carrier, excipient or vehicle in an effective amount for eliciting an immune response.
2. A method of inducing an immune response against influenza in a canine, comprising administering a formulation comprising an avipox expression vector comprising a polynucleotide encoding an influenza antigen, epitope or immunogen and a pharmaceutically or veterinarily acceptable carrier, excipient or vehicle in an effective amount for inducing an immune response.
3. The method of paragraph 1 or 2, wherein the formulation further comprises an adjuvant.
4. The method of any one of paragraphs 1 to 3, wherein the influenza antigen, epitope or immunogen is a hemagglutinin, matrix protein, membrane protein, neuraminidase, nonstructural protein, nucleoprotein, polymerase or any fragment thereof.
5. The method of any one of paragraphs 1 to 4, wherein the influenza antigen, epitope or immunogen is isolated from a canine infected with influenza.
6. The method of paragraph 5 wherein the influenza antigen, epitope or immunogen is isolated from the broncho alveolar lavage and/or lung tissues of the canine.
7. The method of any one of paragraphs 1 to 4, wherein the influenza antigen, epitope or immunogen is isolated from an equine influenza.
8. The method of paragraph 7, wherein the equine influenza is an Ohio equine influenza, a Kentucky equine influenza or a Newmarket equine influenza.
9. The method of any one of paragraphs 1 to 8, wherein the avipox expression vector is an attenuated avipox expression vector.
10. The method of paragraph 9, wherein the avipox expression vector is a canarypox vector.
11. The method of paragraph 10, wherein the canarypox vector is ALVAC.
12. The method of paragraph 10 or 11, wherein the influenza antigen, epitope or immunogen is a hemagglutinin.
13. The method of paragraph 12, wherein the hemagglutinin is H3.
14. The method of paragraph 12 or 13, wherein the canarypox vector is CP1529 or CP1533.
15. A method of eliciting an immune response against influenza in a canine, comprising administering a formulation comprising an inactivated influenza vaccine and a pharmaceutically or veterinarily acceptable carrier, excipient or vehicle in an effective amount for eliciting an immune response.
16. A method of inducing an immune response against influenza in a canine, comprising administering a formulation comprising an inactivated influenza vaccine and a pharmaceutically or veterinarily acceptable carrier, excipient or vehicle in an effective amount for inducing an immune response.
17. The method of paragraph 15 or 16, wherein the formulation further comprises an adjuvant.
18. The method of any one of paragraph 15 to 17, wherein the inactivated influenza vaccine is an inactivated canine influenza.
19. The method of any one of paragraphs 15 to 17, wherein the inactivated influenza vaccine is an inactivated equine influenza.
20. The method of paragraph 19, wherein the equine influenza is an Ohio equine influenza, a Kentucky equine influenza or a Newmarket equine influenza.
21. The method of any one of paragraphs 15 to 20, wherein the inactivated influenza vaccine is inactivated with formalin or beta-propiolactone.
22. The method of any one of paragraphs 17 to 21, wherein the adjuvant is aluminum hydroxide, alumimum phosphate, a carbomer or an oil-water-emulsion.
23. The method of any one of paragraphs 17 to 22, wherein the adjuvant further comprises CpG.
24. The method of any one of paragraphs 15 to 23, wherein the administration is subcutaneous or intramuscular.
25. A kit for performing any one of the methods of paragraphs 1 to 24 comprising the vaccine of any one of paragraphs 1 to * and instructions for performing the method of any one of paragraphs 1 to 24.

## Claims

1. A *Canidae* vaccine for use in reducing the duration of influenza viral shedding in the nasal cavity in a canine, wherein said vaccine comprises an effective amount of an expression vector that contains and expresses *in vivo* in a canine a polynucleotide encoding equine influenza antigen comprising equine influenza H3 operatively linked to a promoter and optionally to an enhancer, and a pharmaceutically or veterinarily acceptable carrier, excipient, or vehicle.

2. The vaccine for the use of claim 1, wherein the vaccine further comprises an adjuvant.

3. The vaccine for the use of claim 2, wherein the adjuvant is a cationic lipid containing a quaternary ammonium salt.

4. The vaccine for the use of any one of claims 1 to 3, wherein the vaccine is formulated for subcutaneous or intramuscular administration.

5. The vaccine for the use of claim 1, wherein the polynucleotide comprises H3N8 codon optimized HA.

6. The vaccine for the use according to claim 1 or 5, wherein the polynucleotide is isolated from a canine infected with equine influenza.

7. The vaccine for the use according to claim 1 or 5, wherein the polynucleotide is isolated from an equine influenza virus isolate.

8. The vaccine for the use according to claim 7, wherein the equine influenza isolate is selected from the group consisting of an Ohio equine influenza virus isolate, a Kentucky equine influenza virus isolate, a Newmarket equine influenza virus isolate or mixtures thereof.

9. The vaccine for the use according to any one of claims 1 to 8, wherein the expression vector is a poxvirus.

10. The vaccine for the use according to claim 9, wherein the poxvirus is an avipox virus.

11. The vaccine for the use according to claim 10, wherein the avipox virus is a canarypox virus.

12. The vaccine for the use according to claim 11, wherein the canarypox virus is ALVAC.

13. The vaccine for the use according to claim 11, wherein the canarypox virus is selected from CP1529, CP1533, or CP2242.

## Patentansprüche

1. Canidae-Impfstoff zur Verwendung beim Reduzieren der Dauer der Influenza-Virenfreisetzung in der Nasenhöhle eines Hundes, wobei der Impfstoff eine wirksame Menge eines Expressionsvektors umfasst, der *in vivo* in einem Hund ein Polynucleotid enthält und exprimiert, das ein Pferde-Influenza-Antigen codiert, das Pferde-Influenza H3 umfasst, das mit einem Promotor und gegebenenfalls mit einem Enhancer funktionell verknüpft ist, und einen pharmazeutisch oder veterinärmedizinisch verträglichen Träger, Exzipienten oder ein pharmazeutisch oder veterinärmedizinisch verträgliches Vehikel umfasst.

2. Impfstoff zur Verwendung nach Anspruch 1, wobei der Impfstoff des Weiteren ein Adjuvans umfasst.

3. Impfstoff zur Verwendung nach Anspruch 2, wobei das Adjuvans ein kationisches Lipid ist, das quartäres Ammoniumsalz enthält.

4. Impfstoff zur Verwendung nach einem der Ansprüche 1 bis 3, wobei der Impfstoff zur subkutanen oder intramuskulären Verabreichung formuliert ist.

5. Impfstoff zur Verwendung nach Anspruch 1, wobei das Polynucleotid H3N8-Codon-optimiertes HA umfasst.

6. Impfstoff zur Verwendung nach Anspruch 1 oder 5, wobei das Polynucleotid aus einem Hund isoliert ist, der mit Pferde-Influenza infiziert ist.

7. Impfstoff zur Verwendung nach Anspruch 1 oder 5, wobei das Polynucleotid aus einem Pferde-Influenza-Virus-Isolat isoliert ist.

8. Impfstoff zur Verwendung nach Anspruch 7, wobei das Pferde-Influenza-Isolat ausgewählt ist aus der Gruppe bestehend aus einem Ohio-Pferde-Influenza-Virus-Isolat, einem Kentucky-Pferde-Influenza-Virus-Isolat, einem Newmarket-Pferde-Influenza-Virus-Isolat oder Gemischen davon.

9. Impfstoff zur Verwendung nach einem der Ansprüche 1 bis 8, wobei der Expressionsvektor ein Pockenvirus ist.

10. Impfstoff zur Verwendung nach Anspruch 9, wobei das Pockenvirus ein Avipoxvirus ist.

11. Impfstoff zur Verwendung nach Anspruch 10, wobei das Avipoxvirus ein Kanarienpockenvirus ist

12. Impfstoff zur Verwendung nach Anspruch 11, wobei das Kanarienpockenvirus ALVAC ist.

13. Impfstoff zur Verwendung nach Anspruch 11, wobei das Kanarienpockenvirus ausgewählt ist aus CP1529, CP1533 oder CP2242 ist.

## Revendications

1. Vaccin pour *Canidae* destiné à être utilisé dans la réduction de la durée d'excrétion virale de la grippe dans la cavité nasale chez un canin, où ledit vaccin comprend une quantité efficace d'un vecteur d'expression qui contient et exprime *in vivo* chez un canin un polynucléotide codant un antigène de la grippe équine comprenant H3 de la grippe équine lié de manière fonctionnelle à un promoteur et éventuellement à un activateur, et un vecteur, excipient ou véhicule acceptable du point de vue pharmaceutique ou vétérinaire.

2. Vaccin destiné à être utilisé selon la revendication 1 où le vaccin comprend en outre un adjuvant.

3. Vaccin destiné à être utilisé selon la revendication 2 où l'adjuvant est un lipide cationique contenant un sel d'ammonium quaternaire.

4. Vaccin destiné à être utilisé selon l'une quelconque des revendications 1 à 3 où le vaccin est formulé pour l'administration sous-cutanée ou intramusculaire.

5. Vaccin destiné à être utilisé selon la revendication 1 où le polynucléotide comprend HA à codons optimisés selon H3N8.

6. Vaccin destiné à être utilisé selon la revendication 1 ou 5 où le polynucléotide est isolé à partir d'un canin infecté avec la grippe équine.

7. Vaccin destiné à être utilisé selon la revendication 1 ou 5 où le polynucléotide est isolé à partir d'un isolat de virus de la grippe équine.

8. Vaccin destiné à être utilisé selon la revendication 7 où l'isolat de la grippe équine est choisi dans le groupe consistant en un isolat de virus de la grippe équine Ohio, un isolat de virus de la grippe équine Kentucky, un isolat de virus de la grippe équine Newmarket et les mélanges de ceux-ci.

9. Vaccin destiné à être utilisé selon l'une quelconque des revendications 1 à 8 où le vecteur d'expression est un poxvirus.

**10.** Vaccin destiné à être utilisé selon la revendication 9 où le poxvirus est un avipoxvirus.

**11.** Vaccin destiné à être utilisé selon la revendication 10 où l'avipoxvirus est un virus de la variole du canari.

**12.** Vaccin destiné à être utilisé selon la revendication 11 où le virus de la variole du canari est ALVAC.

**13.** Vaccin destiné à être utilisé selon la revendication 11 où le virus de la variole du canari est choisi parmi CP1529, CP1533 et CP2242.

# FIG. 1

GTATTCTAAACTAGGAATAGATGAAATTATGTGCAAAGGAGATACCTTTAGATATGGATCTGATTTAT
TTGGTTTTTCATAATCATAATCTAACAACATTTTCACTATACTATACCTTCTTGCACAAGTCGCCATTA
GTAGTATAGACTTATACTTTGTAACCATAGTATACTTTAGCGCGTCATCTTCTTCATCTAAAACAGATT
TACAACAATAATCATCGTCGTCATCTTCATCTTCATTAAAGTTTTCATATTCAATAACTTTCTTTTCTAA
AACATCATCTGAATCAATAAACATAGAACGGTATAGAGCGTTAATCTCCATTGTAAAATATACTAACG
CGTTGCTCATGATGTACTTTTTTTTCATTATTTAGAAATTATGCATTTTAGATCTTTATAAGCGGCCGT
GATTAACTAGTCATAAAAACCCGGGATCGATTCTAGACTCGAGCGGCCGCCAGTGTGATGGATATCT
GCAGAATTCGGCTTTGGTCCTTACTCAAATGCAAATGTTGCACCTGATGTTGCCTTTTTGGCAAGCCC
ACATAATGAAACCCAATAGAACAACGCAAATTAAGAAGCATGATATGGCGAATGAAATCCACAGTAT
CCAATCTTTGTAGCCTGATTTCAACTCAACACTTTTGATTTGAAATCGGTTGTTTAATGCTTCATCTCT
GTATATGTAATGGTCATATGTCCCATTTCTTATTGATCCAATGCATGCATTATCACATTTGTGGTAAAT
CTTGAAACATCCACCTCCCATGTCTTCCGCGTTTTCTCTTAACTGGCGCCTAGTCTTCTCGAATAATTT
ATTCATTTCTGCATCTGTTAAGTCAATTGTATGTTGATTTTCTAGAGCCACCAGCAATTCTGCATTGTA
GGACCATAGGTCTATTTTGGTGTCTTCTACATACTTCTCCAAATCCTGGATTCTCCCTTCTACTTCTGA
GAATTCCTTTTCTATTTGATGGAATTTCTCATTGGTCCTTTCAATCACTCTGTTTAATTTTCCATTGATC
TGGTCTATGGCTGCTTGAGTGCTCTTTAGATCTGCAGCTTGTCCTGTTCCTTCCGAGTTTTGATATCG
GAATCCATACCACCCATCAACCATTCCTTCCCAGCCGTTTTCTATGAATCCCGCTATTGCTCCAAAGA
TTCCTCTGATTTGCTTTTCTGGTACATTCCTCATCCCAGTGGCCAGCTTTAAAGTGTTTTGCCTGATAT
ACTTGGGGCATTTTCCATATGTAATTTTGTTCACATTTTGAAATGGTTTGTCGTTGGGGATGCTTCCAT
TTGGTGTAATACATTCAGACACACAAGTGTCTATGAGTGCATCTGATCTCATTACAGAGCTTTTCCCT
GTTCTCAATTTAAAATATCCCCGCGGTGCAACTAAGTTGCCATTACTGTTTATCATTAGAATATCTCCA
GGTTTTACAATGGTCCAGTATATGCTTATCCTGCCTGATTGACCCCTGACCCACGGCCTAGATCCGAT
GTTAGGGATTACTGTTTGTTGACTTCTTTCTGTTGAGACTGTTACTCGTCCTGATTCTTGGATATATAA
TTTTGTCTGCTCTTTGTTTGAGCTCGGGTGATGAATCCCCCAGATGTATAGTTTATCGAAATTTTTATT
GTTAGGCATTGTCACATTCAATATGGGGTAAGAATTTCCAGATTTTGTTAGCCAATTCAGTCGGCTAA
AGAAACTATCGGCTGATCCCCTTTTGCAGGCTCCACTTCTTCCGTTTTGAGTGACACCTGTCCATGTG
AACCCCTCTGCTGTGAATTCTAATGTTCCTGAGGATGCTACAATGGACCGGAGCGATGCATAGTCAG
GGATGTCATATGGGTAGCAATTGCTGAAAGCGCTGCTTCTTTCTATGAAGAGGTCCCAATTCTCATAC
TGAAAATCATCACAATGGGGGTCTCCTAGCATTGCATCTATTAATGTGCAATTTCTTCCATCTAGAAC
CCTATATGAGTTGTTGCATATTTTCCCTATTGAAATGCTCTGGACTAATTCAGTAGCATTTGTCACCTC
AATTTGGTCATCAGTTATTGTTTTTACCAATGTTCCATTTGCTACTGCATGGTGTCCCAGACATAATGT
GGCTGTGTTGTTGCCACTGGTTGGGTTTTGACTGTAGACCCAATGGGTCAGTAGTATCAAAATAATG
GTTGTCTTCATTACGATACAAACTTAACGGATATCGCGATAATGAAATAATTTATGATTATTTCTCGCT
TTCAATTTAACACAACCCTCAAGAACCTTTGTATTTATTTTCACTTTTTAAGTATAGAATAAAGAAGCT
CTAATTAATTAACGAGCAGATAGTCTCGTTCTCGCCCTGCCTGATGACTAATTAATTAACCCGGATCC
TTTTTATAGCTAATTAGTCACGTACCTTTGAGAGTACCACTTCAGCTACCTCTTTTGTGTCTCAGAGTA
ACTTTCTTTAATCAATTCCAAAACAGTATATGATTTTCCATTTCTTTCAAAGATGTAGTTTACATCTGCT
CCTTTGTTGAAAAGTAGCCTGAGCACTTCTTTTCTACCATGAATTACAGCTGGCAAGATCAATTTTTCC
CAGTTCTGGACATTTTATTTTTTTTAAGTAGTGTGCTACATATTTCAATATTTCCAGATTGTACAGCGA
TCATTAAAGGAGTACGTCCCATGTTATCCAGCAAGTCAGTATCAGCACCTTTGTTCAATAGAAGTTTA
ACCATTGTTAAATTTTTATTTGATACGGCTATATGTAGAGGAGTTAACCGATCCGTGTTTGAAATATCT
ACATCCGCCGAATGAGCCAATAGAAGTTTAACCAAATTAACTTTGTTAAGGTAAGCTGCCAAACACAA
AGGAGTAAAGCCTCCGCTGTAAAGAACATTGTTTACATAGTTATTCTTCAACAGATCTTTCACTATTTT
GTAGTCGTCTCTCAACACCGCATCATGCAGACAAGAAGTTGTGCATTCAGTAACTACAGGTTTAGCT
CCATACCTCATCAAGATTTTTATAGCCTCGGTATTCTTGAACATTACAGCCATTTCAAGAGGAGATTG
TAGAGTACCATATTCCGTGTTAGGGTCGAATCCATTGTCCAAAAACCTATTTAGAGATGCATTGTCAT
TATCCATGATAGCCTCACAGACGTATATGTAAGCCATCTTGAATGTATAATTTTGTTGTTTTCAACAAC
CGCTCGTGAACAGCTTCTATACTTTTTCATTTTCTTCATGATTAATATAGTTTACGGAATATAAGTATA
CAAAAAGTTTATAGTAATCTCATAATATCTGAAACACATACATAAAACATGGAAGAATTACACGATGT
CGTTGAGATAAATGGCTTTTTATTGTCATAGTTTACAAATTCGCAGTAATCTTCATCTTTTACGAATAT

TO FIG. 1B

*FIG. 1B*

FROM FIG. 1A

TGCAGAATCTGTTTTATCCAACCAGTGATTTTTGTATAATATAACTGGTATCCTATCTTCCGATAGAAT
GCTGTTATTTAACATTTTTGCACCTATTAAGTTACATCTGTCAAATCCATCTTTCCAACTGACTTTATGT
AACGATGCGAAATAGCATTTATCACTATGTCGTACCCAATTATCATGACAAGATTCTCTTAAATACGTA
ATCTTATTATCTCTTGCATATTCGTAATAGTAATTGTAAAGAGTATAC GATAACAGTATAGATATACAC
GTGATATAAATATTTAACCCCATTCCTGAGTAAAATAATTACGATATTACATTTCCTTTTATTATTTTTA
TGTTTTAGTTATTTGTTAGGTTATACAAAAATTATGTTTATTTGTGTATATTTAAAGCGTCGTTAAGAAT
AAGCTTAGTTAACATATTATCGCTTAGGTTTTGTAGTATTTGAATCCTTTCTTTAAATGGATTATTTTTC
CAATGCATATTTATAGCTTCATCCAAAGTATAACATTTAACATTCA (SEQ ID NO: 1)

GTATTCTAAACTAGGAATAGATGAAATTATGTGCAAAGGAGATACCTTTAGATATGGATCTGATTTATTTGGTTTTT
CATAATCATAATCTAACAACATTTTCACTATACTATACCTTCTTGCACAAGTCGCCATTAGTAGTATAGACTTATAC
TTTGTAACCATAGTATACTTTAGCGCGTCATCTTCTTCATCTAAAACAGATTTACAACAATAATCATCGTCGTCATC
TTCATCTTCATTAAAGTTTTCATATTCAATAACTTTCTTTTCTAAAACATCATCTGAATCAATAAACATAGAACGGT
ATAGAGCGTTAATCTCCATTGTAAAATATACTAACGCGTTGCTCATGATGTACTTTTTTTTCATTATTTAGAAATTA
TGCATTTTAGATCTTTATAAGCGGCCGTGATTAACTAGTCATAAAAACCCGGGATCGATTCTAGACTCGAGGGTACC
TCAAATGCAAATGTTGCATCTGATGTTGCCTTTTTGGCAAGCCCACATAATGAAACCCAATAGAACAACGCAAATTA
AGAAGCATGATATGGCGAATGAAATCCACAGTATCCAATCTTTGTAGCCTGATTTCAACTCAACACCTTTGATTTGA
AATCGGTTGTTTAATGCTTCATCTCTGTATATGTAATGGTCATATGTCCCATTTCTTATTGATCCAATGCATGCATT
ATCACATTTGTGGTAAATCTTGAAACATCCACCTCCCATGTCTTCCGCGTTTTCTCTTAACTGGCGTCTAGTCTTCT
CGAATAATTTATTCATTTCTGCATCTGTTAAGTCAATTGTATGTTGATTTTCTAGAGCCACCAGCAATTCTGCATTG
TAGGACCATAGGTCTATTTTGGTGTCTTCTACATACTTCTCCAAGTCCTGGATTCTCCCTTCTACTTCTGAGAATTC
CTTCTCTATTTGATGGAATTTCTCATTGGTCCTTTCAATCACTCTGTTTAATTTTCCATTAATCTGGTCGATGGCTG
CTTGAGTGCTCTTTAGATCTGCAGCTTGTCCTGTTCCTTCCGAGTTTTGATATCGGAATCCATACCACCCATCAACC
ATTCCTTCCCAGCCGTTTTCTATGAATCCCGCTATTGCTCCAAAGATTCCTCTGATTTGCTTTTCTGGTACATTCCT
CATCCCAGTGGCCAGCTTTAAAGTGTTTTGCCTGATATACTTGGGGCATTTTCCATATGTAACTTTGTTCACATTTT
GAAATGGTTTGTCGTTGGGGATGCTTCCATTTGGTGTAATACATTCAGACACACAAATGTCTATGGGTGCATCTGAT
CTCATTACAGAGCTTTTCCCTGTTTTCAATTTAAAATATCCCCGCGGTGCAACTAAGTTGCCATTACTGTTTATCAT
TAGGATATCTCCAGGTTTTACAATGGTCCAGTATATGCTTATCCTGCCTGATTGACCCCTGACCCACGGTCTAGATC
CGATATTAGGGATTATCGTTTGTTGACTTCTTTTTGTTGAGACTGTTACTCGTCCTGATTCTTGGATGTACAATTCT
GTTTGCTGTTGGTTTGAGCTCGGGTGATGAATCCCCCAGATGTATAGTTTGTCGAAATTTTTATTGTTAGGCATTGT
CACATTCAATGTGGGGTAAGAGTTTCCAGATTTTGTTAGCCAATTCAGTCGGCTAAAGAAACTATCGGCTGATCCCC
TTTTGCAGGCTCCACTTCTTCCGTTTTGAGTGACACCTGTCCATGTGAATCCCTCTGCTGTGAATTCCAATGTTCCT
GAGGATGCTACAATGGACCGGAGCGATGCATAGTCAGGGATGTCATATGGGTAGCAATTGCTGAAAGCGCTGCTTCT
TTCTATGAAGAGGTCCCAATTCTCATACTGAAAGACATCACAGTGGGGGTCTCCTAGCATTGCATCTATTAATGTGC
AATTTCTTCCATCTAGAACTCTATATGAGTTGTTGCATATTTTCCCTATTGAAATGCTCTGAACTAATTCAGTAGCA
TTTGTCACCTCAATTTGGTCATCAGTTATTGTTTTTACCAATGTTCCATTTGCTACTGCATGGTGTCCCAGACATAA
TGTGGCTGTGTTGTTGCCACTGGTTGGGTTTTGACTGTAGACCCAATGGGTCAGTAGTATCAAAATAATGGTTGTCT
TCATTACGATACAAACTTAACGGATATCGCGATAATGAAATAATTTATGATTATTTCTCGCTTTCAATTTAACACAA
CCCTCAAGAACCTTTGTATTTATTTTCACTTTTTAAGTATAGAATAAAGAAGCTCTAATTAATTAACGAGCAGATAG
TCTCGTTCTCGCCCTGCCTGATGACTAATTAATTAACCCGGATCCTTTTTATAGCTAATTAGTCACGTACCTTTGAG
AGTACCACTTCAGCTACCTCTTTTGTGTCTCAGAGTAACTTTCTTTAATCAATTCCAAAACAGTATATGATTTTCCA
TTTCTTTCAAAGATGTAGTTTACATCTGCTCCTTTGTTGAAAAGTAGCCTGAGCACTTCTTTTCTACCATGAATTAC
AGCTGGCAAGATCAATTTTTCCCAGTTCTGGACATTTTATTTTTTTTAAGTAGTGTGCTACATATTTCAATATTTCC
AGATTGTACAGCGATCATTAAAGGAGTACGTCCCATGTTATCCAGCAAGTCAGTATCAGCACCTTTGTTCAATAGAA
GTTTAACCATTGTTAAATTTTTATTTGATACGGCTATATGTAGAGGAGTTAACCGATCCGTGTTTGAAATATCTACA
TCCGCCGAATGAGCCAATAGAAGTTTAACCAAATTAACTTTGTTAAGGTAAGCTGCCAAACACAAAGGAGTAAAGCC
TCCGCTGTAAAGAACATTGTTTACATAGTTATTCTTCAACAGATCTTTCACTATTTTGTAGTCGTCTCTCAACACCG
CATCATGCAGACAAGAAGTTGTGCATTCAGTAACTACAGGTTTAGCTCCATACCTCATCAAGATTTTTATAGCCTCG
GTATTCTTGAACATTACAGCCATTTCAAGAGGAGATTGTAGAGTACCATATTCCGTGTTAGGGTCGAATCCATTGTC
CAAAAACCTATTTAGAGATGCATTGTCATTATCCATGATAGCCTCACAGACGTATATGTAAGCCATCTTGAATGTAT
AATTTTGTTGTTTTCAACAACCGCTCGTGAACAGCTTCTATACTTTTTCATTTTCTTCATGATTAATATAGTTTACG
GAATATAAGTATACAAAAAGTTTATAGTAATCTCATAATATCTGAAACACATACATAAAACATGGAAGAATTACACG
ATGTCGTTGAGATAAATGGCTTTTTATTGTCATAGTTTACAAATTCGCAGTAATCTTCATCTTTTACGAATATTGCA
GAATCTGTTTTATCCAACCAGTGATTTTTGTATAATATAACTGGTATCCTATCTTCCGATAGAATGCTGTTATTTAA
CATTTTTGCACCTATTAAGTTACATCTGTCAAATCCATCTTTCCAACTGACTTTATGTAACGATGCGAAATAGCATT
TATCACTATGTCGTACCCAATTATCATGACAAGATTCTCTTAAATACGTAATCTTATTATCTCTTGCATATTCGTAA
TAGTAATTGTAAAGAGTATACGATAACAGTATAGATATACACGTGATATAAATATTTAACCCCATTCCTGAGTAAAA
TAATTACGATATTACATTTCCTTTTATTATTTTTATGTTTTAGTTATTTGTTAGGTTATACAAAAATTATGTTTATT
TGTGTATATTTAAAGCGTCGTTAAGAATAAGCTTAGTTAACATATTATCGCTTAGGTTTTGTAGTATTTGAATCCTT
TCTTTAAATGGATTATTTTTCCAATGCATATTTATAGCTTCATCCAAAGTATAACATTTAACATTCA **(SEQ ID NO:2)**

*FIG. 2*

# FIG. 3

```
                      1                                                50
HA Newmarket    (1)   MKTTIILILLTHWVYSQNPTSGNNTATLCLGHHAVANGTLVKTITDDQIE
HA Ohio         (1)   MKTTIILILLTHWAYSQNPISGNNTATLCLGHHAVANGTLVKTISDDQIE

                      51                                              100
HA Newmarket   (51)   VTNATELVQSISIGKICNNSYRVLDGRNCTLIDAMLGDPHCDDFQYENWD
HA Ohio        (51)   VTNATELVQSISMGKICNNSYRILDGRNCTLIDAMLGDPHCDAFQYENWD

                      101                                             150
HA Newmarket  (101)   LFIERSSAFSNCYPYDIPDYASLRSIVASSGTLEFTAEGFTWTGVTQNGR
HA Ohio       (101)   LFIERSSAFSNCYPYDIPDYASLRSIVASSGTLEFTAEGFTWTGVTQNGR

                      151                                             200
HA Newmarket  (151)   SGACKRGSADSFFSRLNWLTKSGNSYPILNVTMPNNKNFDKLYIWGIHHP
HA Ohio       (151)   SGACKRGSADSFFSRLNWLTKSGSSYPTLNVTMPNNKNFDKLYIWGIHHP

                      201                                             250
HA Newmarket  (201)   SSNKEQTKLYIQESGRVTVSTERSQQTVIPNIGSRPWVRGQSGRISIYWT
HA Ohio       (201)   SSNQEQTKLYIQESGRVTVSTKRSQQTIIPNIGSRPWVRGQSGRISIYWT

                      251                                             300
HA Newmarket  (251)   IVKPGDILMINSNGNLVAPRGYFKLRTGKSSVMRSDALIDTCVSECITPN
HA Ohio       (251)   IVKPGDILMINSNGNLVAPRGYFKLKTGKSSVMRSDVPIDICVSECITPN

                      301                                             350
HA Newmarket  (301)   GSIPNDKPFQNVNKITYGKCPKYIRQNTLKLATGMRNVPEKQIRGIFGAI
HA Ohio       (301)   GSISNDKPFQNVNKVTYGKCPKYIRQNTLKLATGMRNVPEKQIRGIFGAI

                      351                                             400
HA Newmarket  (351)   AGFIENGWEGMVDGWYGFRYQNSEGTGQAADLKSTQAAIDQINGKLNRVI
HA Ohio       (351)   AGFIENGWEGMVDGWYGFRYQNSEGTGQAADLKSTQAAIDQINGKLNRVI

                      401                                             450
HA Newmarket  (401)   ERTNEKFHQIEKEFSEVEGRIQDLEKYVEDTKIDLWSYNAELLVALENQH
HA Ohio       (401)   ERTNEKFHQIEKEFSEVEGRIQDLEKYVEDTKIDLWSYNAELLVALENQH

                      451                                             500
HA Newmarket  (451)   TIDLTDAEMNKLFEKTRRQLRENAEDMGGGCFKIYHKCDNACIGSIRNGT
HA Ohio       (451)   TIDLTDAEMNKLFEKTRRQLKENAEDMGGGCFKIYHKCDNACIGSIRNGT

                      501                                             550
HA Newmarket  (501)   YDHYIYRDEALNNRFQIKSVELKSGYKDWILWISFAISCFLICVVLLGFI
HA Ohio       (501)   YDHYIYRDEALNNRFQIKGVELKSGYKDWILWISFAISCFLICVVLLGFI

                      551            566
HA Newmarket  (551)   MWACQKGNIRCNICI- (SEQ ID NO: 3)
HA Ohio       (551)   MWACQKGNIRCNICI- (SEQ ID NO: 4)
```

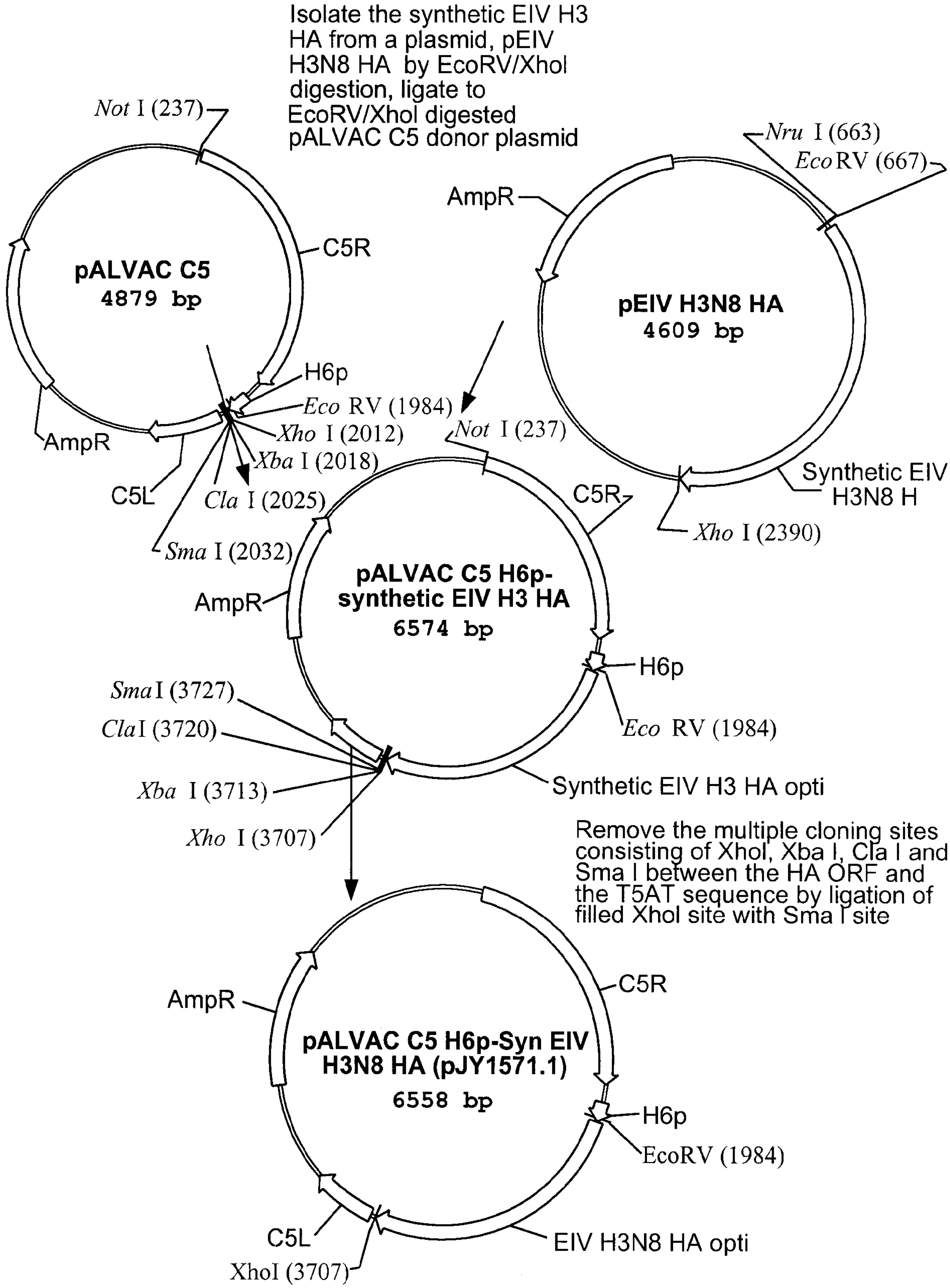

Isolate the synthetic EIV H3 HA from a plasmid, pEIV H3N8 HA by EcoRV/XhoI digestion, ligate to EcoRV/XhoI digested pALVAC C5 donor plasmid
Not I (237)
pALVAC C5
4879 bp
C5R
H6p
Eco RV (1984)
Xho I (2012)
Xba I (2018)
Cla I (2025)
Sma I (2032)
AmpR
C5L
Nru I (663)
EcoRV (667)
AmpR
pEIV H3N8 HA
4609 bp
Synthetic EIV H3N8 H
Xho I (2390)
Not I (237)
C5R
pALVAC C5 H6p-synthetic EIV H3 HA
6574 bp
AmpR
H6p
Eco RV (1984)
SmaI (3727)
ClaI (3720)
Xba I (3713)
Xho I (3707)
Synthetic EIV H3 HA opti
Remove the multiple cloning sites consisting of XhoI, Xba I, Cla I and Sma I between the HA ORF and the T5AT sequence by ligation of filled XhoI site with Sma I site
AmpR
C5R
pALVAC C5 H6p-Syn EIV H3N8 HA (pJY1571.1)
6558 bp
H6p
EcoRV (1984)
C5L
XhoI (3707)
EIV H3N8 HA opti


*FIG. 4A*

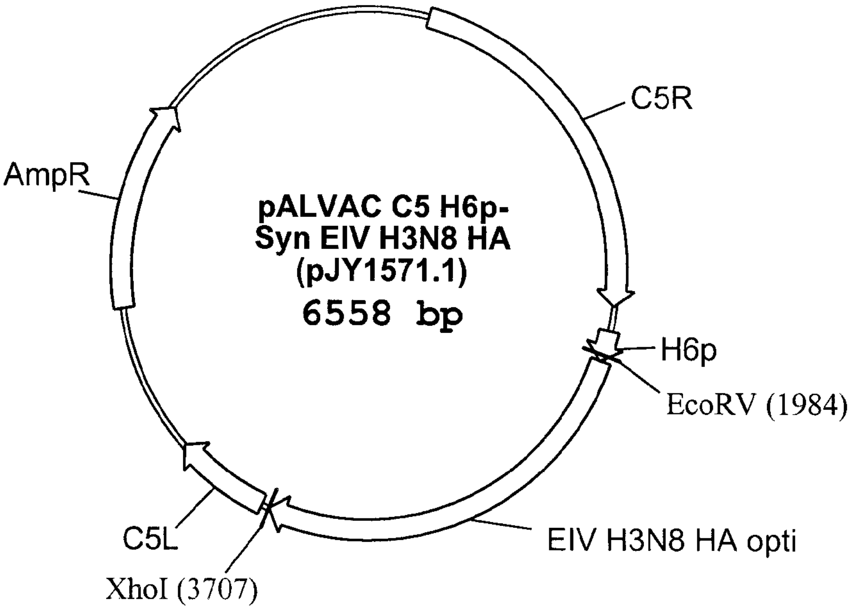
pALVAC C5 H6p-
Syn EIV H3N8 HA
(pJY1571.1)
6558 bp
C5R
AmpR
H6p
EcoRV (1984)
EIV H3N8 HA opti
C5L
XhoI (3707)

*FIG. 4B*

FIG. 5A

```
  1  MKTTIILILL THWAYSQNPI SGNNTATLCL GHHAVANGTL VKTISDDQIE
 51  VTNATELVQS ISMGKICNNS YRILDGRNCT LIDAMLGDPH CDAFQYENWD
101  LFIERSSAFS NCYPYDIPDY ASLRSIVASS GTLEFTAEGF TWTGVTQNGR
151  SGACKRGSAD SFFSRLNWLT KSGSSYPTLN VTMPNNKNFD KLYIWGIHHP
201  SSNQEQTKLY IQESGRVTVS TKRSQQTIIP NIGSRPWVRG QSGRISIYWT
251  IVKPGDILMI NSNGNLVAPR GYFKLKTGKS SVMRSDVPID ICVSECITPN
301  GSISNDKPFQ NVNKVTYGKC PKYIRQNTLK LATGMRNVPE KQIRGIFGAI
351  AGFIENGWEG MVDGWYGFRY QNSEGTGQAA DLKSTQAAID QINGKLNRVI
401  ERTNEKFHQI EKEFSEVEGR IQDLEKYVED TKIDLWSYNA ELLVALENQH
451  TIDLTDAEMN KLFEKTRRQL KENAEDMGGG CFKIYHKCDN ACIGSIRNGT
501  YDHYIYRDEA LNNRFQIKGV ELKSGYKDWI LWISFAISCF LICVVLLGFI
551  MWACQKGNIR CNICI* (SEQ ID NO: 5)
```

# FIG. 5B

```
            M13R                                             C5R
  1   GGAAACAGCT ATGACCATGA TTACGAATTG CGGCCGCAAT TCTGAATGTT
      CCTTTGTCGA TACTGGTACT AATGCTTAAC GCCGGCGTTA AGACTTACAA

 51   AAATGTTATA CTTTGGATGA AGCTATAAAT ATGCATTGGA AAAATAATCC
      TTTACAATAT GAAACCTACT TCGATATTTA TACGTAACCT TTTTATTAGG

101   ATTTAAAGAA AGGATTCAAA TACTACAAAA CCTAAGCGAT AATATGTTAA
      TAAATTTCTT TCCTAAGTTT ATGATGTTTT GGATTCGCTA TTATACAATT

151   CTAAGCTTAT TCTTAACGAC GCTTTAAATA TACACAAATA AACATAATTT
      GATTCGAATA AGAATTGCTG CGAAATTTAT ATGTGTTTAT TTGTATTAAA

201   TTGTATAACC TAACAAATAA CTAAAACATA AAAATAATAA AAGGAAATGT
      AACATATTGG ATTGTTTATT GATTTTGTAT TTTTATTATT TTCCTTTACA

251   AATATCGTAA TTATTTTACT CAGGAATGGG GTTAAATATT TATATCACGT
      TTATAGCATT AATAAAATGA GTCCTTACCC CAATTTATAA ATATAGTGCA

301   GTATATCTAT ACTGTTATCG TATACTCTTT ACAATTACTA TTACGAATAT
      CATATAGATA TGACAATAGC ATATGAGAAA TGTTAATGAT AATGCTTATA
                                                  7927.DC
351   GCAAGAGATA ATAAGATTAC GTATTTAAGA GAATCTTGTC ATGATAATTG
      CGTTCTCTAT TATTCTAATG CATAAATTCT CTTAGAACAG TACTATTAAC
      7927.DC
401   GGTACGACAT AGTGATAAAT GCTATTTCGC ATCGTTACAT AAAGTCAGTT
      CCATGCTGTA TCACTATTTA CGATAAAGCG TAGCAATGTA TTTCAGTCAA

451   GGAAAGATGG ATTTGACAGA TGTAACTTAA TAGGTGCAAA AATGTTAAAT
      CCTTTCTACC TAAACTGTCT ACATTGAATT ATCCACGTTT TTACAATTTA
                         7696.CXL
501   AACAGCATTC TATCGGAAGA TAGGATACCA GTTATATTAT ACAAAAATCA
      TTGTCGTAAG ATAGCCTTCT ATCCTATGGT CAATATAATA TGTTTTTAGT

551   CTGGTTGGAT AAAACAGATT CTGCAATATT CGTAAAAGAT GAAGATTACT
      GACCAACCTA TTTTGTCTAA GACGTTATAA GCATTTTCTA CTTCTAATGA

601   GCGAATTTGT AAACTATGAC AATAAAAAGC CATTTATCTC AACGACATCG
      CGCTTAAACA TTTGATACTG TTATTTTTCG GTAAATAGAG TTGCTGTAGC

651   TGTAATTCTT CCATGTTTTA TGTATGTGTT TCAGATATTA TGAGATTACT
      ACATTAAGAA GGTACAAAAT ACATACACAA AGTCTATAAT ACTCTAATGA

701   ATAAACTTTT TGTATACTTA TATTCCGTAA ACTATATTAA TCATGAAGAA
      TATTTGAAAA ACATATGAAT ATAAGGCATT TGATATAATT AGTACTTCTT
```

TO FIG. 5B-1

FROM FIG. 5B

```
 751  AATGAAAAAG TATAGAAGCT GTTCACGAGC GGTTGTTGAA AACAACAAAA
      TTACTTTTTC ATATCTTCGA CAAGTGCTCG CCAACAACTT TTGTTGTTTT
                          7926.DC
 801  TTATACATTC AAGATGGCTT ACATATACGT CTGTGAGGCT ATCATGGATA
      AATATGTAAG TTCTACCGAA TGTATATGCA GACACTCCGA TAGTACCTAT

 851  ATGACAATGC ATCTCTAAAT AGGTTTTTGG ACAATGGATT CGACCCTAAC
      TACTGTTACG TAGAGATTTA TCCAAAAACC TGTTACCTAA GCTGGGATTG

 901  ACGGAATATG GTACTCTACA ATCTCCTCTT GAAATGGCTG TAATGTTCAA
      TGCCTTATAC CATGAGATGT TAGAGGAGAA CTTTACCGAC ATTACAAGTT

 951  GAATACCGAG GCTATAAAAA TCTTGATGAG GTATGGAGCT AAACCTGTAG
      CTTATGGCTC CGATATTTTT AGAACTACTC CATACCTCGA TTTGGACATC
                     7697.CXL
1001  TTACTGAATG CACAACTTCT TGTCTGCATG ATGCGGTGTT GAGAGACGAC
      AATGACTTAC GTGTTGAAGA ACAGACGTAC TACGCCACAA CTCTCTGCTG

1051  TACAAAATAG TGAAAGATCT GTTGAAGAAT AACTATGTAA ACAATGTTCT
      ATGTTTTATC ACTTTCTAGA CAACTTCTTA TTGATACATT TGTTACAAGA

1101  TTACAGCGGA GGCTTTACTC CTTTGTGTTT GGCAGCTTAC CTTAACAAAG
      AATGTCGCCT CCGAAATGAG GAAACACAAA CCGTCGAATG GAATTGTTTC

1151  TTAATTTGGT TAAACTTCTA TTGGCTCATT CGGCGGATGT AGATATTTCA
      AATTAAACCA ATTTGAAGAT AACCGAGTAA GCCGCCTACA TCTATAAAGT

1201  AACACGGATC GGTTAACTCC TCTACATATA GCCGTATCAA ATAAAAATTT
      TTGTGCCTAG CCAATTGAGG AGATGTATAT CGGCATAGTT TATTTTTAAA
                         7925.DC
1251  AACAATGGTT AAACTTCTAT TGAACAAAGG TGCTGATACT GACTTGCTGG
      TTGTTACCAA TTTGAAGATA ACTTGTTTCC ACGACTATGA CTGAACGACC

1301  ATAACATGGG ACGTACTCCT TTAATGATCG CTGTACAATC TGGAAATATT
      TATTGTACCC TGCATGAGGA AATTACTAGC GACATGTTAG ACCTTTATAA

1351  GAAATATGTA GCACACTACT TAAAAAAAAT AAAATGTCCA GAACTGGGAA
      CTTTATACAT CGTGTGATGA ATTTTTTTTA TTTTACAGGT CTTGACCCTT

1401  AAATTGATCT TGCCAGCTGT AATTCATGGT AGAAAAGAAG TGCTCAGGCT
      TTTAACTAGA ACGGTCGACA TTAAGTACCA TCTTTTCTTC ACGAGTCCGA

1451  ACTTTTCAAC AAAGGAGCAG ATGTAAACTA CATCTTTGAA AGAAATGGAA
      TGAAAAGTTG TTTCCTCGTC TACATTTGAT GTAGAAACTT TCTTTACCTT
                                                 7792.SL
1501  AATCATATAC TGTTTTGGAA TTGATTAAAG AAAGTTACTC TGAGACACAA
      TTAGTATATG ACAAAACCTT AACTAATTTC TTTCAATGAG ACTCTGTGTT
        7792.SL
1551  AAGAGGTAGC TGAAGTGGTA CTCTCAAAGG TACGTGACTA ATTAGCTATA
      TTCTCCATCG ACTTCACCAT GAGAGTTTCC ATGCACTGAT TAATCGATAT

1601  AAAAGGATCC GGGTTAATTA ATTAGTCATC AGGCAGGGCG AGAACGAGAC
```

TO FIG. 5B-2

FIG. 5B-1

FROM FIG. 5B-1

```
      TTTTCCTAGG CCCAATTAAT TAATCAGTAG TCCGTCCCGC TCTTGCTCTG
                                  H6
1651  TATCTGCTCG TTAATTAATT AGAGCTTCTT TATTCTATAC TTAAAAAGTG
      ATAGACGAGC AATTAATTAA TCTCGAAGAA ATAAGATATG AATTTTTCAC

1701  AAAATAAATA CAAAGGTTCT TGAGGGTTGT GTTAAATTGA AAGCGAGAAA
      TTTTATTTAT GTTTCCAAGA ACTCCCAACA CAATTTAACT TTCGCTCTTT
                                                H3 HA  M ·
1751  TAATCATAAA TTATTTCATT ATCGCGATAT CCGTTAAGTT TGTATCGTAA
      ATTAGTATTT AATAAAGTAA TAGCGCTATA GGCAATTCAA ACATAGCATT
         11670JY
      ..K  T  T   I  I  L   I  L  L  T   H  W  A   Y  S  Q
1801  TGAAAACCAC CATCATCCTG ATCCTGCTGA CCCACTGGGC CTACAGCCAG
      ACTTTTGGTG GTAGTAGGAC TAGGACGACT GGGTGACCCG GATGTCGGTC
         11671JY
       N  P  I  S   G  N  N   T  A  T   L  C  L  G   H  H  A ·
1851  AACCCTATCA GCGGCAACAA CACCGCCACC CTGTGCCTGG GCCACCACGC
      TTGGGATAGT CGCCGTTGTT GTGGCGGTGG GACACGGACC CGGTGGTGCG

      .V  A  N   G  T  L  V   K  T  I   S  D  D   Q  I  E  V ·
1901  CGTGGCCAAC GGCACCCTGG TCAAGACCAT CAGCGACGAC CAGATCGAAG
      GCACCGGTTG CCGTGGGACC AGTTCTGGTA GTCGCTGCTG GTCTAGCTTC

      ..T  N  A   T  E  L   V  Q  S  I   S  M  G   K  I  C
1951  TGACCAACGC CACCGAGCTG GTGCAGAGCA TCAGCATGGG CAAGATCTGC
      ACTGGTTGCG GTGGCTCGAC CACGTCTCGT AGTCGTACCC GTTCTAGACG

       N  N  S  Y   R  I  L   D  G  R   N  C  T  L   I  D  A ·
2001  AACAACAGCT ACCGCATCCT GGACGGCAGA AACTGCACCC TGATCGACGC
      TTGTTGTCGA TGGCGTAGGA CCTGCCGTCT TTGACGTGGG ACTAGCTGCG

      .M  L  G   D  P  H  C   D  A  F   Q  Y  E   N  W  D  L ·
2051  CATGCTGGGC GACCCCCACT GCGACGCCTT CCAGTACGAG AACTGGGACC
      GTACGACCCG CTGGGGGTGA CGCTGCGGAA GGTCATGCTC TTGACCCTGG

      ..F  I  E   R  S  S   A  F  S  N   C  Y  P   Y  D  I
2101  TGTTCATCGA GAGGAGCAGC GCCTTCAGCA ACTGCTACCC CTACGACATC
      ACAAGTAGCT CTCCTCGTCG CGGAAGTCGT TGACGATGGG GATGCTGTAG

       P  D  Y  A   S  L  R   S  I  V   A  S  S  G   T  L  E ·
2151  CCTGACTACG CCAGCCTGAG AAGCATCGTG GCCAGCAGCG GCACCCTGGA
      GGACTGATGC GGTCGGACTC TTCGTAGCAC CGGTCGTCGC CGTGGGACCT
        11672JY
      .F  T  A   E  G  F  T   W  T  G   V  T  Q   N  G  R  S ·
2201  GTTCACCGCC GAGGGCTTCA CCTGGACCGG CGTGACCCAG AACGGCAGAA
      CAAGTGGCGG CTCCCGAAGT GGACCTGGCC GCACTGGGTC TTGCCGTCTT
        11673JY
      ..G  A  C   K  R  G   S  A  D  S   F  F  S   R  L  N
2251  GCGGCGCCTG CAAGAGAGGC AGCGCCGACA GCTTCTTCAG CCGCCTGAAC
      CGCCGCGGAC GTTCTCTCCG TCGCGGCTGT CGAAGAAGTC GGCGGACTTG

       W  L  T  K   S  G  S   S  Y  P   T  L  N  V   T  M  P ·
```

TO FIG. 5B-3

*FIG. 5B-2*

FROM FIG. 5B-2

```
2301  TGGCTGACCA AGAGCGGCAG CAGCTACCCC ACCCTGAACG TGACCATGCC
      ACCGACTGGT TCTCGCCGTC GTCGATGGGG TGGGACTTGC ACTGGTACGG

      . N  N  K   N  F  D  K   L  Y  I   W  G  I   H  H  P  S ·
2351  CAACAACAAG AACTTCGACA AGCTGTACAT CTGGGGCATC CACCACCCCA
      GTTGTTGTTC TTGAAGCTGT TCGACATGTA GACCCCGTAG GTGGTGGGGT

      .. S  N  Q   E  Q  T   K  L  Y  I   Q  E  S   G  R  V
2401  GCAGCAACCA GGAGCAGACC AAGCTGTACA TCCAGGAGAG CGGCAGAGTG
      CGTCGTTGGT CCTCGTCTGG TTCGACATGT AGGTCCTCTC GCCGTCTCAC

       T  V  S  T   K  R  S   Q  Q  T   I  I  P  N   I  G  S ·
2451  ACCGTGTCCA CCAAGAGAAG CCAGCAGACC ATCATCCCCA ACATCGGCAG
      TGGCACAGGT GGTTCTCTTC GGTCGTCTGG TAGTAGGGGT TGTAGCCGTC

      . R  P  W   V  R  G  Q   S  G  R   I  S  I   Y  W  T  I ·
2501  CAGACCTTGG GTGCGCGGCC AGTCCGGCAG GATCAGCATC TACTGGACCA
      GTCTGGAACC CACGCGCCGG TCAGGCCGTC CTAGTCGTAG ATGACCTGGT

      .. V  K  P   G  D  I   L  M  I  N   S  N  G   N  L  V
2551  TCGTGAAGCC TGGCGACATC CTGATGATCA ACAGCAACGG CAACCTGGTG
      AGCACTTCGG ACCGCTGTAG GACTACTAGT TGTCGTTGCC GTTGGACCAC
          11674JY
       A  P  R  G   Y  F  K   L  K  T   G  K  S  S   V  M  R ·
2601  GCCCCCAGAG GCTACTTCAA GCTGAAAACC GGCAAGAGCA GCGTGATGAG
      CGGGGGTCTC CGATGAAGTT CGACTTTTGG CCGTTCTCGT CGCACTACTC
          11675JY
      . S  D  V   P  I  D  I   C  V  S   E  C  I   T  P  N  G ·
2651  AAGCGACGTG CCCATCGACA TCTGCGTGTC CGAGTGCATC ACCCCTAACG
      TTCGCTGCAC GGGTAGCTGT AGACGCACAG GCTCACGTAG TGGGGATTGC

      .. S  I  S   N  D  K   P  F  Q  N   V  N  K   V  T  Y
2701  GCAGCATCAG CAACGACAAG CCCTTCCAGA ACGTGAACAA AGTGACCTAC
      CGTCGTAGTC GTTGCTGTTC GGGAAGGTCT TGCACTTGTT TCACTGGATG

       G  K  C  P   K  Y  I   R  Q  N   T  L  K  L   A  T  G ·
2751  GGCAAGTGCC CCAAGTACAT CCGCCAGAAC ACCCTGAAGC TGGCCACCGG
      CCGTTCACGG GGTTCATGTA GGCGGTCTTG TGGGACTTCG ACCGGTGGCC

      . M  R  N   V  P  E  K   Q  I  R   G  I  F   G  A  I  A ·
2801  CATGAGAAAC GTGCCCGAGA AGCAGATCAG AGGCATCTTC GGCGCCATCG
      GTACTCTTTG CACGGGCTCT TCGTCTAGTC TCCGTAGAAG CCGCGGTAGC

      .. G  F  I   E  N  G   W  E  G  M   V  D  G   W  Y  G
2851  CCGGCTTCAT CGAGAACGGC TGGGAGGGCA TGGTGGACGG CTGGTACGGC
      GGCCGAAGTA GCTCTTGCCG ACCCTCCCGT ACCACCTGCC GACCATGCCG

       F  R  Y  Q   N  S  E   G  T  G   Q  A  A  D   L  K  S ·
2901  TTCAGATACC AGAACAGCGA GGGCACCGGC CAGGCCGCCG ACCTGAAGAG
      AAGTCTATGG TCTTGTCGCT CCCGTGGCCG GTCCGGCGGC TGGACTTCTC

      . T  Q  A   A  I  D  Q   I  N  G   K  L  N   R  V  I  E ·
```

TO FIG. 5B-4

*FIG. 5B-3*

FROM FIG. 5B-3

```
2951  CACCCAGGCC GCCATCGACC AGATCAACGG CAAGCTGAAC CGCGTGATCG
      GTGGGTCCGG CGGTAGCTGG TCTAGTTGCC GTTCGACTTG GCGCACTAGC
          11676JY
      .. R  T  N   E  K  F   H  Q  I  E   K  E  F   S  E  V
3001  AGCGCACCAA CGAGAAGTTC CACCAGATCG AGAAGGAGTT CAGCGAAGTG
      TCGCGTGGTT GCTCTTCAAG GTGGTCTAGC TCTTCCTCAA GTCGCTTCAC
          11677JY
       E  G  R  I   Q  D  L   E  K  Y   V  E  D  T   K  I  D ·
3051  GAGGGCAGAA TCCAGGACCT GGAGAAGTAC GTGGAGGACA CCAAGATCGA
      CTCCCGTCTT AGGTCCTGGA CCTCTTCATG CACCTCCTGT GGTTCTAGCT

      . L  W  S   Y  N  A  E   L  L  V   A  L  E   N  Q  H  T ·
3101  CCTGTGGAGC TACAACGCCG AGCTGCTGGT CGCCCTGGAG AACCAGCACA
      GGACACCTCG ATGTTGCGGC TCGACGACCA GCGGGACCTC TTGGTCGTGT

      .. I  D  L   T  D  A   E  M  N  K   L  F  E   K  T  R
3151  CCATCGACCT GACCGACGCC GAGATGAACA AGCTGTTCGA AAAGACCAGG
      GGTAGCTGGA CTGGCTGCGG CTCTACTTGT TCGACAAGCT TTTCTGGTCC

       R  Q  L  K   E  N  A   E  D  M   G  G  G  C   F  K  I ·
3201  CGCCAGCTGA AGGAAAACGC CGAGGACATG GGCGGCGGCT GCTTCAAGAT
      GCGGTCGACT TCCTTTTGCG GCTCCTGTAC CCGCCGCCGA CGAAGTTCTA

      . Y  H  K   C  D  N  A   C  I  G   S  I  R   N  G  T  Y ·
3251  CTACCACAAG TGCGACAACG CCTGCATCGG CTCCATCAGG AACGGCACCT
      GATGGTGTTC ACGCTGTTGC GGACGTAGCC GAGGTAGTCC TTGCCGTGGA

      .. D  H  Y   I  Y  R   D  E  A  L   N  N  R   F  Q  I
3301  ACGACCACTA CATCTACAGG GACGAGGCCC TGAACAACCG CTTCCAGATC
      TGCTGGTGAT GTAGATGTCC CTGCTCCGGG ACTTGTTGGC GAAGGTCTAG

       K  G  V  E   L  K  S   G  Y  K   D  W  I  L   W  I  S ·
3351  AAGGGCGTGG AGCTGAAGAG CGGCTACAAG GACTGGATCC TGTGGATCAG
      TTCCCGCACC TCGACTTCTC GCCGATGTTC CTGACCTAGG ACACCTAGTC

      . F  A  I   S  C  F  L   I  C  V   V  L  L   G  F  I  M ·
3401  CTTCGCCATC AGCTGCTTCC TGATCTGCGT GGTGCTGCTG GGCTTCATCA
      GAAGCGGTAG TCGACGAAGG ACTAGACGCA CCACGACGAC CCGAAGTAGT
                                         11678JY
      .. W  A  C   Q  K  G   N  I  R  C   N  I  C   I(SEQ ID NO:5)

3451  TGTGGGCCTG CCAGAAGGGC AACATCCGCT GCAACATCTG CATCTGATGA
      ACACCCGGAC GGTCTTCCCG TTGTAGGCGA CGTTGTAGAC GTAGACTACT
                                         11679JY   C5L
3501  CTCGAGGGTT TTTATGACTA GTTAATCACG GCCGCTTATA AAGATCTAAA
      GAGCTCCCAA AAATACTGAT CAATTAGTGC CGGCGAATAT TTCTAGATTT
                                                 7928.DC
3551  ATGCATAATT TCTAAATAAT GAAAAAAAGT ACATCATGAG CAACGCGTTA
      TACGTATTAA AGATTTATTA CTTTTTTTCA TGTAGTACTC GTTGCGCAAT

3601  GTATATTTTA CAATGGAGAT TAACGCTCTA TACCGTTCTA TGTTTATTGA
      CATATAAAAT GTTACCTCTA ATTGCGAGAT ATGGCAAGAT ACAAATAACT
```

TO FIG. 5B-5

FIG. 5B-4

FROM FIG. 5B-4

```
3651  TTCAGATGAT GTTTTAGAAA AGAAAGTTAT TGAATATGAA AACTTTAATG
      AAGTCTACTA CAAAATCTTT TCTTTCAATA ACTTATACTT TTGAAATTAC

3701  AAGATGAAGA TGACGACGAT GATTATTGTT GTAAATCTGT TTTAGATGAA
      TTCTACTTCT ACTGCTGCTA CTAATAACAA CATTTAGACA AAATCTACTT

3751  GAAGATGACG CGCTAAAGTA TACTATGGTT ACAAAGTATA AGTCTATACT
      CTTCTACTGC GCGATTTCAT ATGATACCAA TGTTTCATAT TCAGATATGA

3801  ACTAATGGCG ACTTGTGCAA GAAGGTATAG TATAGTGAAA ATGTTGTTAG
      TGATTACCGC TGAACACGTT CTTCCATATC ATATCACTTT TACAACAATC

3851  ATTATGATTA TGAAAAACCA AATAAATCAG ATCCATATCT AAAGGTATCT
      TAATACTAAT ACTTTTTGGT TTATTTAGTC TAGGTATAGA TTTCCATAGA

3901  CCTTTGCACA TAATTTCATC TATTCCTAGT TTAGAATACC TGCAGCCAAG
      GGAAACGTGT ATTAAAGTAG ATAAGGATCA AATCTTATGG ACGTCGGTTC

3951  CTTGGCACTG GCCGTCGTTT TAC (SEQ ID NO: 6)
      GAACCGTGAC CGGCAGCAAA ATG (SEQ ID NO: 7)
                M13F
```

*FIG. 5B-5*

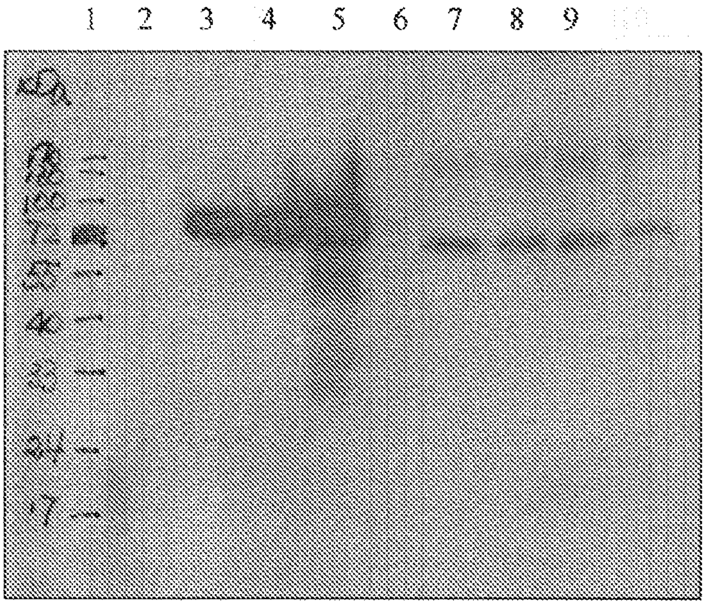


FIG. 6

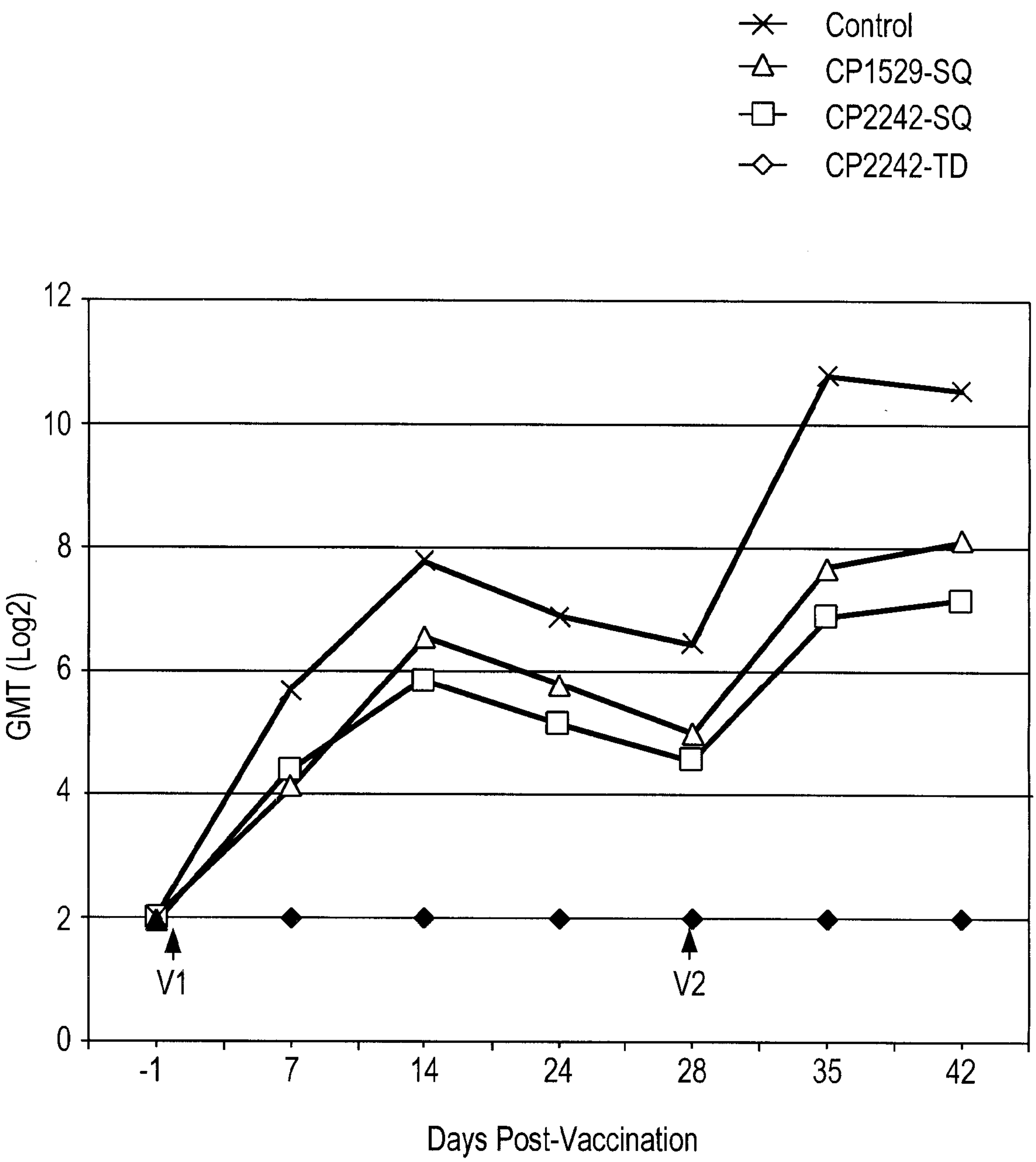
Control
CP1529-SQ
CP2242-SQ
CP2242-TD
12
10
8
6
4
2
0
GMT (Log2)
V1
V2
-1
7
14
24
28
35
42
Days Post-Vaccination

*FIG. 7*

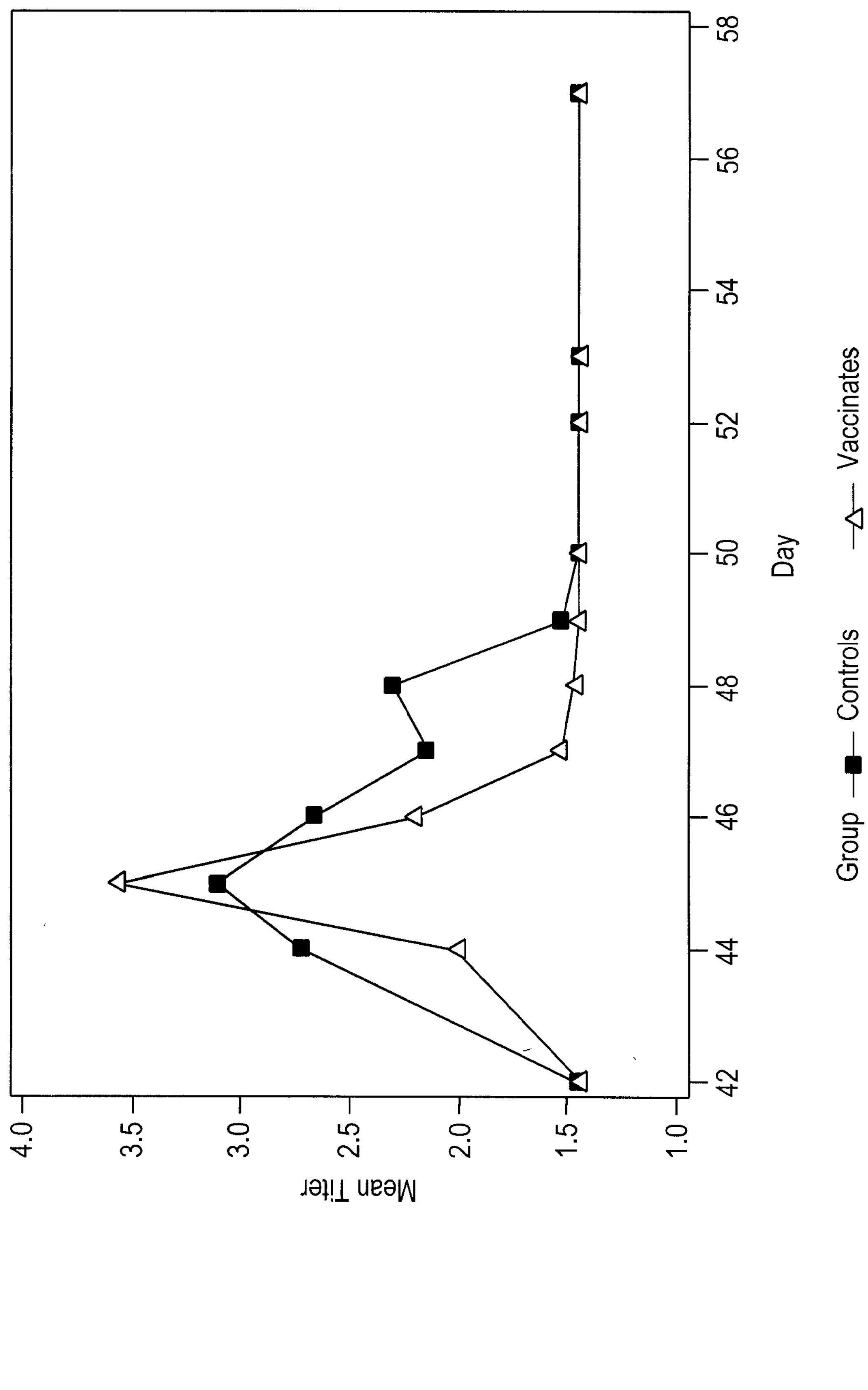
Mean Titer
4.0
3.5
3.0
2.5
2.0
1.5
1.0
42
44
46
48
50
52
54
56
58
Day
Group
Controls
Vaccinates

FIG. 8

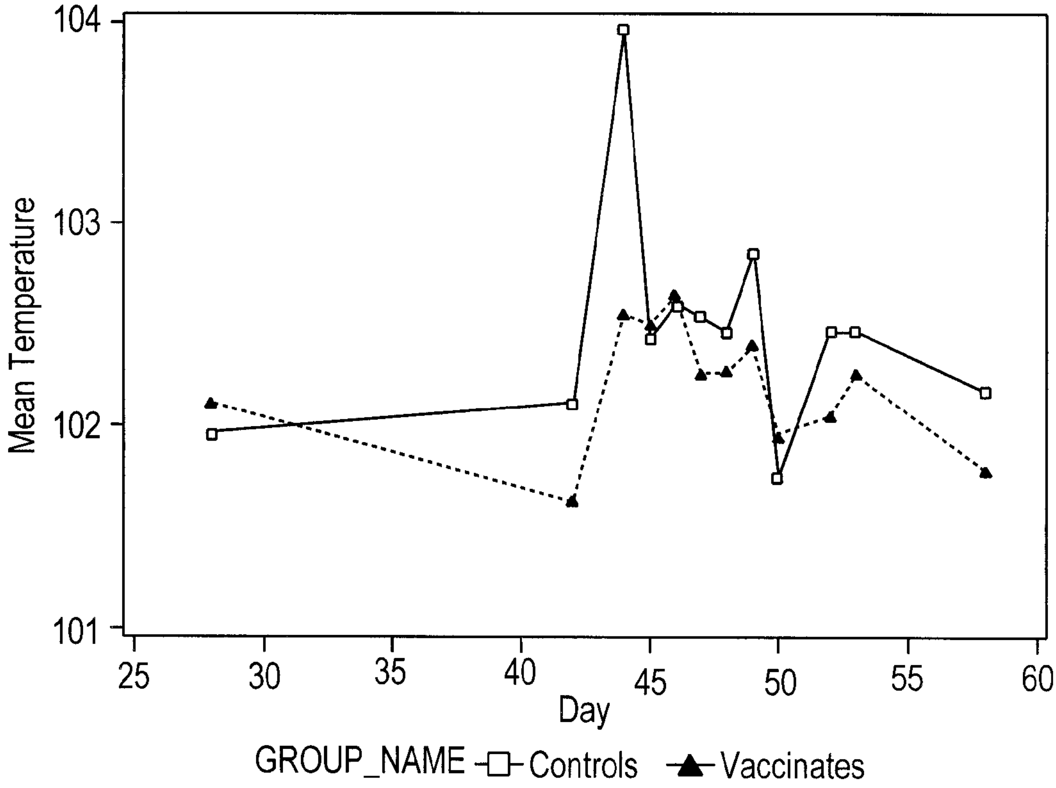
104
103
102
101
Mean Temperature
25
30
35
40
45
50
55
60
Day
GROUP_NAME Controls Vaccinates

*FIG. 9*

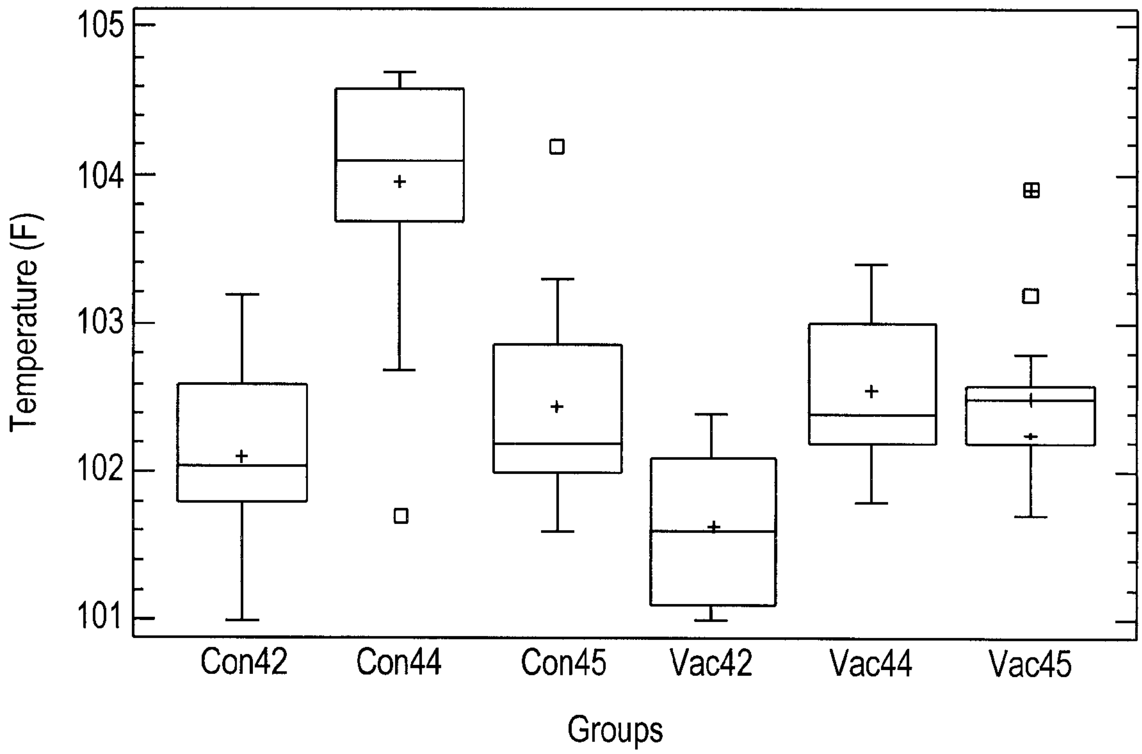
105
104
103
102
101
Temperature (F)
Con42
Con44
Con45
Vac42
Vac44
Vac45
Groups

*FIG. 10*

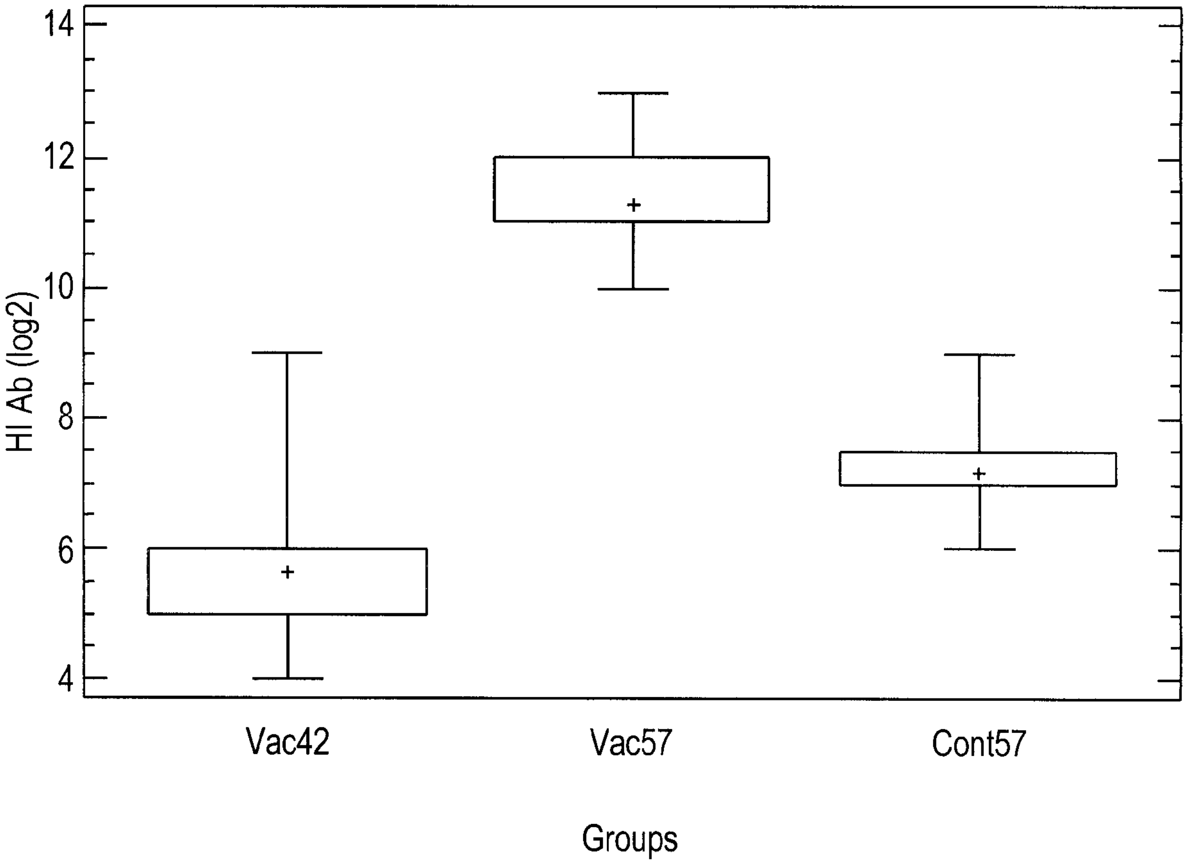


*FIG. 11*

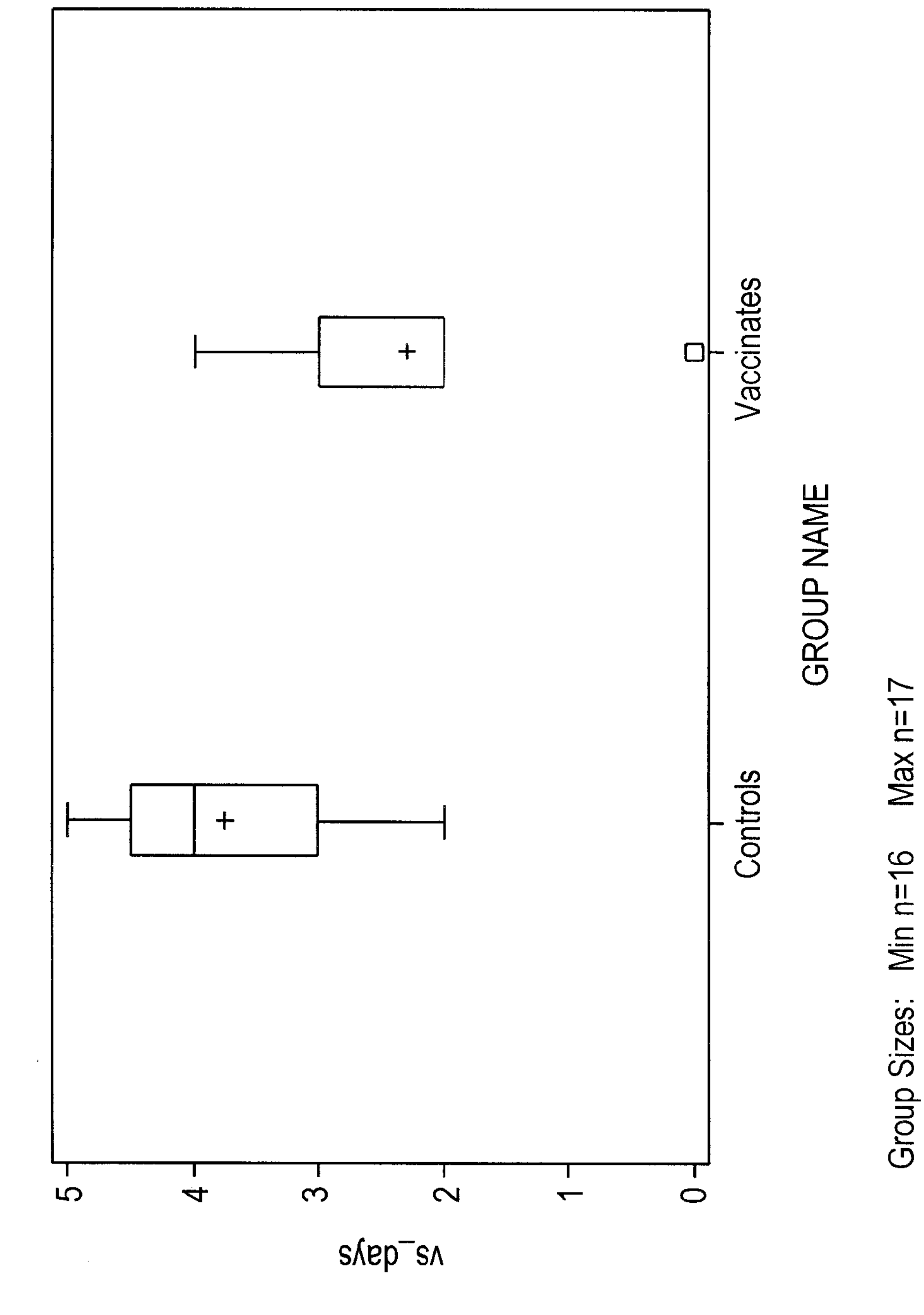
vs_days
0
1
2
3
4
5
Controls
Vaccinates
GROUP NAME
Group Sizes: Min n=16 Max n=17

*FIG. 12*

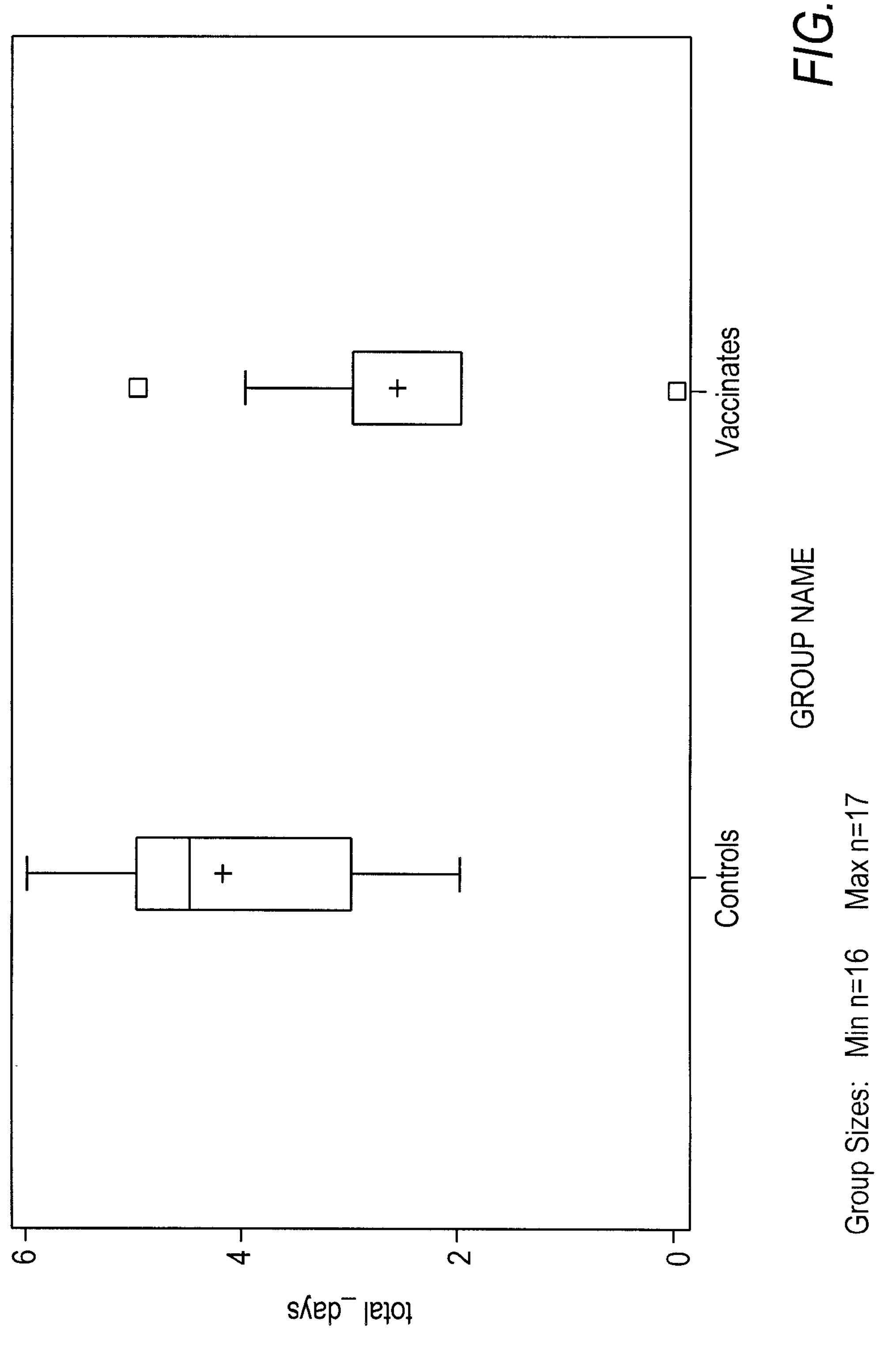

total_days
0
2
4
6
Controls
Vaccinates
GROUP NAME
Group Sizes: Min n=16 Max n=17


FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007024947 A
- US 4708871 A
- US 2004022605 W
- US 5505941 A
- US 5756103 A
- US 4603112 A
- US 4769330 A
- US 4394448 A
- US 4722848 A
- US 4745051 A
- US 4769331 A
- US 4945050 A
- US 5494807 A
- US 5514375 A
- US 5744140 A
- US 5744141 A
- US 5762938 A
- US 5766599 A
- US 5990091 A
- US 5174993 A
- US 5338683 A
- US 5591639 A
- US 5589466 A
- US 5677178 A
- US 5591439 A
- US 5552143 A
- US 5580859 A
- US 6130066 A
- US 6004777 A
- US 6497883 B
- US 6464984 B
- US 6451770 B
- US 6391314 B
- US 6387376 B
- US 6376473 B
- US 6368603 B
- US 6348196 B
- US 6306400 B
- US 6228846 B
- US 6221362 B
- US 6217883 B
- US 6207166 B
- US 6207165 B
- US 6159477 A
- US 6153199 A
- US 6090393 A
- US 6074649 A
- US 6045803 A
- US 6033670 A
- US 6485729 B
- US 6103526 A
- US 6224882 B
- US 6312682 B
- US 6348450 B
- US 6312683 B
- US 920197 A
- WO 9001543 A
- WO 9111525 A
- WO 9416716 A
- WO 9639491 A
- WO 9833510 A
- EP 265785 A
- EP 0370573 A
- WO 9640241 A
- WO 0105934 A
- WO 9012882 A
- US 5110587 A
- US 5382425 A
- WO 0003030 A
- US 6133028 A
- US 6692956 B
- US 5529780 A
- US 5688920 A
- WO 9514102 A
- US 6156567 A
- US 5846946 A
- US 6451769 B
- US 6852705 B
- US 6818628 B
- US 6586412 B
- US 6576243 B
- US 6558674 B
- EP 260148 A
- EP 323597 A
- US 5168062 A
- US 5385839 A
- US 4968615 A
- WO 8703905 A
- WO 9800166 A
- WO 8901036 A
- US 5122458 A
- WO 9634109 A
- US 6048537 A, J. Violay
- WO 9816247 A
- US 2909462 A
- WO 9901158 A
- WO 9944633 A
- WO 11264622 A

**Non-patent literature cited in the description**


- **CRAWFORD et al.** *Science,* 21 October 2005, vol. 310 (5747), 482-5
- **KARACA K et al.** *American Journal of Veterinary Research,* 2007, vol. 68, 208-212
- **CROUCH et al.** *Vaccine,* 02 December 2004, vol. 23 (3), 418-25
- **SHORT et al.** *J Vet Pharmacol Ther.,* December 1986, vol. 9 (4), 426-32
- **HORNER ; LEDGARD, N Z.** *Vet J.,* December 1988, vol. 36 (4), 205-6
- **BREATHNACH et al.** *Vet Immunol Immunopathol.,* April 2004, vol. 98 (3-4), 127-36
- **OLSEN et al.** *Vaccine,* July 1997, vol. 15 (10), 1149-56
- **MORLEY et al.** *Vet Microbiol.,* June 1995, vol. 45 (1), 81-92
- **OZAKI et al.** *Vet Microbiol.,* 20 September 2001, vol. 82 (2), 111-9
- **SUGIURA et al.** *J Virol Methods,* October 2001, vol. 98 (1), 1-8
- **GOTO et al.** *J Vet Med Sci.,* February 1993, vol. 55 (1), 33-7
- **YOUNGNER et al.** *Am J Vet Res.,* August 2001, vol. 62 (8), 1290-4
- **WEBSTER ; THOMAS.** *Vaccine,* 1993, vol. 11 (10), 987-93
- **DONOFRIO et al.** *J Vet Diagn Invest.,* January 1994, vol. 6 (1), 39-43
- **GROSS et al.** *Equine Vet J.,* November 1998, vol. 30 (6), 489-97
- **HELDENS et al.** *Vet J.,* March 2004, vol. 167 (2), 150-7
- **CHAMBERS et al.** *Equine Vet J.,* November 2001, vol. 33 (7), 630-6
- **LINDSTROM et al.** *Arch Virol.,* 1998, vol. 143 (8), 1585-98
- **EDLUND TOULEMONDE et al.** *Vet Rec.,* 19 March 2005, vol. 156 (12), 367-71
- **MOHLER et al.** *Biotechnol Bioeng.,* 05 April 2005, vol. 90 (1), 46-58
- **NAYAK et al.** *J Chromatogr B Analyt Technol Biomed Life Sci.,* 08 July 2005
- **HELDENS et al.** *J Immunol Methods,* 01 June 2002, vol. 264 (1-2), 11-7
- **WATTRANG et al.** *Viral Immunol.,* 2003, vol. 16 (1), 57-67
- **DUHAUT ; DIMMOCK.** *Virology,* 30 September 2000, vol. 275 (2), 278-85
- **NOBLE ; DIMMOCK.** *J Gen Virol.,* December 1994, vol. 75, 3485-91
- **DUHAUT ; DIMMOCK.** *Virology,* 01 September 1998, vol. 248 (2), 241-53
- **HANNANT ; MUMFORD.** *Vet Immunol Immunopathol.,* July 1989, vol. 21 (3-4), 327-37
- **HANNANT et al.** *Vet Microbiol.,* April 1989, vol. 19 (4), 293-303
- **HANNANT et al.** *Vet Rec.,* 06 February 1988, vol. 122 (6), 125-8
- **RICHARDS et al.** *Vet Immunol Immunopathol.,* June 1992, vol. 33 (1-2), 129-43
- **VAN MAANEN et al.** *Vet Microbiol.,* 10 June 2003, vol. 93 (4), 291-306
- **HOMER ; LEDGARD, N Z.** *Vet J.,* December 1988, vol. 36 (4), 205-6
- **MUMFORD et al.** *Vet Rec.,* 12 February 1994, vol. 134 (7), 158-62
- **THOMSON et al.** *Vet Rec.,* 28 May 1977, vol. 100 (22), 465-8
- **MUMFORD et al.** *Epidemiol Infect.,* June 1988, vol. 100 (3), 501-10
- **MUMFORD et al.** *J Hyg (Lond),* June 1983, vol. 90 (3), 385-95
- **LENEVA et al.** *Antimicrob Agents Chemother.,* April 2001, vol. 45 (4), 1216-24
- **FONI et al.** *New Microbiol.,* January 2005, vol. 28 (1), 31-5
- **AUDSLEY ; TANNOCK.** *Expert Opin Biol Ther.,* May 2004, vol. 4 (5), 709-17
- **YOUIL et al.** *Virus Res.,* 15 June 2004, vol. 102 (2), 165-76
- *Virology,* 15 August 2001, vol. 287 (1), 202-13
- **ABED et al.** *J Infect Dis.,* 15 October 2002, vol. 186 (8), 1074-80
- **SUZUKI et al.** *Biochem J.,* 01 September 1996, vol. 318, 389-93
- **SCHOLTISSEK et al.** *J Virol.,* February 2002, vol. 76 (4), 1781-6
- **CHARE et al.** *J Gen Virol.,* October 2003, vol. 84, 2691-703
- **MANTANI et al.** *Planta Med.,* April 2001, vol. 67 (3), 240-3
- **MIYAMOTO et al.** *Antiviral Res.,* August 1998, vol. 39 (2), 89-100
- **TREANOR et al.** *J Virol.,* December 1994, vol. 68 (12), 7684-8
- **ZAKAY-RONES et al.** J Altern Complement Med. *Winter,* 1995, vol. 1 (4), 361-9
- **GUBAREVA et al.** *J Gen Virol.,* November 2002, vol. 83, 2683-92
- **STASCHKE et al.** *Virology,* 01 September 1998, vol. 248 (2), 264-74
- **EGOROV et al.** *J Virol.,* August 1998, vol. 72 (8), 6437-41
- **SIDWELL et al.** *Antiviral Res.,* February 1998, vol. 37 (2), 107-20
- **NUNES-CORREIA et al.** *Biochemistry,* 19 January 1999, vol. 38 (3), 1095-101
- **TREE et al.** *Vaccine,* 14 May 2001, vol. 19 (25-26), 3444-50
- **MAZANEC et al.** *J Virol.,* February 1995, vol. 69 (2), 1339-43

- **BANTIA et al.** *Antimicrob Agents Chemother.,* April 1998, vol. 42 (4), 801-7
- **GUBAREVA et al.** *J Infect Dis.,* 15 February 2001, vol. 183 (4), 523-31
- **HALPERIN et al.** *Vaccine,* August 1998, vol. 16 (13), 1331-5
- **HAYDEN et al.** *Antiviral Res.,* October 1994, vol. 25 (2), 123-31
- **SHIMIZU et al.** *Virology,* 15 February 1999, vol. 254 (2), 213-9
- **LU et al.** *Arch Virol.,* 2002, vol. 147 (2), 273-84
- **GUJULUVA et al.** *Virology,* 01 November 1994, vol. 204 (2), 491-505
- **NAKAGAWA et al.** *J Virol Methods,* April 1999, vol. 79 (1), 13-20
- **NAKAGAWA et al.** *J Virol Methods,* April 1999, vol. 79 (1), 113-20
- **HOFFMANN et al.** *Proc Natl Acad Sci USA.,* 20 August 2002, vol. 99 (17), 11411-6
- **QUINLIVAN et al.** *J Clin Microbiol.,* February 2004, vol. 42 (2), 759-63
- **PALKER et al.** *Virus Res.,* October 2004, vol. 105 (2), 183-94
- **HALPERIN et al.** *Vaccine,* 15 January 2002, vol. 20 (7-8), 1240-7
- **NAGAI et al.** *Biol Pharm Bull.,* February 1995, vol. 18 (2), 295-9
- **NAGAI et al.** *Antiviral Res.,* January 1995, vol. 26 (1), 11-25
- **AUDSLEY ; TANNOCK.** *J Virol Methods,* February 2005, vol. 123 (2), 187-93
- **GAMBARYAN et al.** *Virus Res.,* 01 July 2005
- **LANDOLT et al.** *Am J Vet Res.,* January 2005, vol. 66 (1), 119-24
- **CLAVIJO et al.** *Can J Vet Res.,* April 2002, vol. 66 (2), 117-21
- **GUBAREVA et al.** *J Virol.,* May 1997, vol. 71 (5), 3385-90
- Epitope Mapping Protocols. Methods in Molecular Biology. Humana Press, 1996, vol. 66
- **GEYSEN et al.** *Proc. Natl. Acad. Sci. USA,* 1984, vol. 81, 3998-4002
- **GEYSEN et al.** *Molec. Immunol.,* 1986, vol. 23, 709-715
- **BERGMANN et al.** *Eur. J. Immunol.,* 1993, vol. 23, 2777-2781
- **BERGMANN et al.** *J. Immunol.,* 1996, vol. 157, 3242-3249
- **SUHRBIER, A.** *Immunol. and Cell Biol.,* 1997, vol. 75, 402-408
- **GARDNER et al.** *12th World AIDS Conference,* 28 June 1998
- **HEMMER B. et al.** *Immunology Today,* 1998, vol. 19 (4), 163-168
- **GEYSEN et al.** *Proc. Nat. Acad. Sci. USA,* 1984, vol. 81, 3998-4002
- **GEYSEN et al.** *Proc. Nat. Acad. Sci. USA,* 1985, vol. 82, 178-182
- **VAN DER ZEE R. et al.** *Eur. J. Immunol.,* 1989, vol. 19, 43-47
- **GEYSEN H.M.** *Southeast Asian J. Trop. Med. Public Health,* 1990, vol. 21, 523-533
- **DE GROOT A. et al.** *Nature Biotechnology,* 1999, vol. 17, 533-561
- **SAMBROOK et al.** Molecular Cloning: A Laboratory Manual. 1989
- **KARLIN ; ALTSCHUL.** *Proc. Natl. Acad. Sci. USA,* 1990, vol. 87, 2264-2268
- **KARLIN ; ALTSCHUL.** *Proc. Natl. Acad. Sci. USA,* 1993, vol. 90, 5873-5877
- **MYERS ; MILLER.** *CABIOS,* 1988, vol. 4, 11-17
- **PEARSON ; LIPMAN.** *Proc. Natl. Acad. Sci. USA,* 1988, vol. 85, 2444-2448
- Local alignment statistics. **ALTSCHUL ; GISH.** Methods in Enzymology. 1996, vol. 266, 460-480
- **ALTSCHUL et al.** *Journal of Molecular Biology,* 1990, vol. 215, 403-410
- **GISH ; STATES.** *Nature Genetics,* 1993, vol. 3, 266-272
- **KARLIN ; ALTSCHUL.** *Proc. Natl. Acad. Sci. USA,* 1993, vol. 90, 5873-5877
- **WILBUR ; LIPMAN.** *Proc Natl Acad Sci USA,* 1983, vol. 80, 726
- **EDLUND ; TOULEMONDE et al.** *Vet Rec.,* 19 March 2005, vol. 156 (12), 367-71
- **ANDREANSKY et al.** *Proc. Natl. Acad. Sci. USA,* 1996, vol. 93, 11313-11318
- **BALLAY et al.** *EMBO J.,* 1993, vol. 4, 3861-65
- **FELGNER et al.** *J. Biol. Chem.,* 1994, vol. 269, 2550-2561
- **FROLOV et al.** *Proc. Natl. Acad. Sci. USA,* 1996, vol. 93, 11371-11377
- **GRAHAM.** *Tibtech,* 1990, vol. 8, 85-87
- **GRUNHAUS et al.** *Sem. Virol.,* 1992, vol. 3, 237-52
- **JU et al.** *Diabetologia,* 1998, vol. 41, 736-739
- **KITSON et al.** *J. Virol.,* 1991, vol. 65, 3068-3075
- **MCCLEMENTS et al.** *Proc. Natl. Acad. Sci. USA,* 1996, vol. 93, 11414-11420
- **MOSS.** *Proc. Natl. Acad. Sci. USA,* 1996, vol. 93, 11341-11348
- **PAOLETTI.** *Proc. Natl. Acad. Sci. USA,* 1996, vol. 93, 11349-11353
- **PENNOCK et al.** *Mol. Cell. Biol.,* 1984, vol. 4, 399-406
- Methods in Molecular Biology. Baculovirus Expression Protocols. Humana Press Inc, 1995, 39
- **SMITH et al.** *Mol. Cell. Biol.,* 1983, vol. 3, 2156-2165
- **ROBERTSON et al.** *Proc. Natl. Acad. Sci. USA,* 1996, vol. 93, 11334-11340
- **ROBINSON et al.** *Sem. Immunol.,* 1997, vol. 9, 271

- **ROIZMAN.** *Proc. Natl. Acad. Sci. USA,* 1996, vol. 93, 11307-11312
- **STICKL ; HOCHSTEIN-MINTZEL.** *Munch. Med. Wschr.,* 1971, vol. 113, 1149-1153
- **SUTTER et al.** *Proc. Natl. Acad. Sci. U.S.A.,* 1992, vol. 89, 10847-10851
- **CARROLL M. W. et al.** *Vaccine,* 1997, vol. 15 (4), 387-394
- **STITTELAAR K. J. et al.** *J. Virol.,* 2000, vol. 74 (9), 4236-4243
- **SUTTER G. et al.** *Vaccine,* 1994, vol. 12 (11), 1032-1040
- **ANTOINE G.** *Virology,* 1998, vol. 244, 365-396
- **COCHRAN et al.** *J. Virology,* 1985, vol. 54, 30-35
- **RIVIERE et al.** *J. Virology,* 1992, vol. 66, 3424-3434
- **SHIDA.** *Virology,* 1986, vol. 150, 451-457
- **FUNAHASHI et al.** *J. Gen. Virol.,* 1988, vol. 69, 35-47
- **TAYLOR J. et al.** *Vaccine,* 1988, vol. 6, 504-508
- **GUO P. et al.** *J. Virol.,* 1989, vol. 63, 4189-4198
- **PERKUS M. et al.** *J. Virol.,* 1989, vol. 63, 3829-3836
- **J. CHROBOCZEK et al.** *Virol.,* 1992, vol. 186, 280-285
- **F. GRAHAM et al.** *J. Gen. Virol.,* 1977, vol. 36, 59-72
- **F. FALLOUX et al.** *Human Gene Therapy,* 1998, vol. 9, 1909-1917
- **J. SHRIVER et al.** *Nature,* 2002, vol. 415, 331-335
- Methods in Molecular Biology. **F. GRAHAM et al.** Gene Transfer and Expression Protocols. The Human Press Inc, 1991, vol. 7, 109-128
- **Y. ILAN et al.** *Proc. Natl. Acad. Sci.,* 1997, vol. 94, 2587-2592
- **S. TRIPATHY et al.** *Proc. Natl. Acad. Sci.,* 1994, vol. 91, 11557-11561
- **B. TAPNELL.** *Adv. Drug Deliv. Rev.,* 1993, vol. 12, 185-199
- **X. DANTHINNE et al.** *Gene Thrapy,* 2000, vol. 7, 1707-1714
- **K. BERKNER.** *Bio Techniques,* 1988, vol. 6, 616-629
- **K. BERKNER et al.** *Nucl. Acid Res.,* 1983, vol. 11, 6003-6020
- **C. CHAVIER et al.** *J. Virol.,* 1996, vol. 70, 4805-4810
- **M. BOSHART et al.** *Cell,* 1985, vol. 41, 521-530
- **X. LI et al.** *Nat. Biotechnol.,* 1999, vol. 17, 241-245
- **R. STENBERG et al.** *J. Virol.,* 1984, vol. 49, 190
- **L. FISCHER et al.** *Vaccine,* 2002, vol. 20, 3485-3497
- **LUKE C. et al.** *Journal of Infectious Diseases,* 1997, vol. 175, 91-97
- **HARTIKKA J. et al.** *Human Gene Therapy,* 1996, vol. 7, 1205-1217
- **BOSHART M. et al.** *Cell,* 1985, vol. 41, 521-530
- **KWISSA M. et al.** *Vaccine,* 2000, vol. 18, 2337-2344
- **MIYAZAKI J. et al.** *Gene,* 1989, vol. 79, 269-277
- **VAN OOYEN et al.** *Science,* 1979, vol. 206, 337-344
- **BEHR J. P.** *Bioconjugate Chemistry,* 1994, vol. 5, 382-389
- **C. ROSS et al.** *Archiv. Für die gesamte Virusforschung,* 1970, vol. 30, 82-88
- **T. TOLSTOVA et al.** *Acta Virol.,* 1966, vol. 10, 315-321
- **HO. MERTEN et al.** *Adv. Exp. Med. Biol.,* 1996, vol. 397, 141-151
- **J. TREE et al.** *Vaccine,* 2001, vol. 19, 3444-3450
- **Y. GHENDON et al.** *Vaccine,* 2005, vol. 23, 4678-4684
- **R. BRANDS et al.** *Dev. Biol. Stand.,* 1999, vol. 98, 93-100
- **R. YOUIL et al.** *J Virol. Methods,* 2004, vol. 120, 23-31
- **O. KISTNER et al.** *Vaccine,* 1998, vol. 16, 960-968
- **E. GOVORKOVA et al.** *J. Virol.,* 1996, vol. 70, 5519-5524
- **D. NAYAK et al.** J. Chromatogr. B Analyt. Technol. *Biomed. Life Sci.,* 2005, vol. 823, 75-81
- **S. TOMITA et al.** *Kitasato Arch. Exp. Med.,* 1971, vol. 44, 185-196
- **A. GARCIA et al.** *Avian Diseases,* 1998, vol. 42, 248-256
- **B. BDOWSKY et al.** *Vaccine,* 1991, vol. 9, 398-402
- *Vaccine,* 1993, vol. 11, 343-348
- **N. KEVERIN et al.** *Arch. Virol.,* 2000, vol. 145, 1059-1066
- **D. SWAYNE et al.** *Avian Diseases,* 2001, vol. 45, 355-365
- **KLINMAN et al.** *Proc. Natl. Acad. Sci.,* 1996, vol. 93, 2879-2883
- Vaccine Design, The Subunit and Adjuvant Approach. M. Powell, M. Newman, Plenum Press, 1995, 147
- **SJOLANDER et al.** *J. Leukocyte Biol.,* 1998, vol. 64 (6), 713-723
- **MOREIN B et al.** *Clin. Immunother.,* 1995, vol. 3, 461-475
- **BARR IG ; MITCHELL GF.** *Immunol. and Cell Biol.,* 1996, vol. 74, 8-25

- *Pharmeuropa,* 02 June 1996, vol. 8
- **J. FIELDS et al.** *Nature,* 04 June 1960, vol. 186, 778-780
- **S. TOLLEFSEN et al.** *Vaccine,* 2002, vol. 20, 3370-3378
- **S. TOLLEFSEN et al.** *Scand. J. Immunol.,* 2003, vol. 57, 229-238
- **S. BABIUK et al.** *Vaccine,* 2002, vol. 20, 3399-3408

SEQUENCE LISTING

<110> MINKE, JULES

<120> CANINE INFLUENZA VACCINES

<130> MER-05-051.CIP

<140> 11/264,622
<141> 2005-11-01

<160> 51

<170> PatentIn Ver. 3.3

<210> 1
<211> 3959
<212> DNA
<213> Influenza A virus

<400> 1

gtattctaaa ctaggaatag atgaaattat gtgcaaagga gataccttta gatatggatc 60
tgatttattt ggtttttcat aatcataatc taacaacatt ttcactatac tataccttct 120
tgcacaagtc gccattagta gtatagactt atactttgta accatagtat actttagcgc 180
gtcatcttct tcatctaaaa cagatttaca acaataatca tcgtcgtcat cttcatcttc 240
attaaagttt tcatattcaa taactttctt ttctaaaaca tcatctgaat caataaacat 300
agaacggtat agagcgttaa tctccattgt aaaatatact aacgcgttgc tcatgatgta 360
cttttttttc attatttaga aattatgcat tttagatctt tataagcggc cgtgattaac 420
tagtcataaa aacccgggat cgattctaga ctcgagcggc cgccagtgtg atggatatct 480
gcagaattcg gctttggtcc ttactcaaat gcaaatgttg cacctgatgt tgcctttttg 540
gcaagcccac ataatgaaac ccaatagaac aacgcaaatt aagaagcatg atatggcgaa 600
tgaaatccac agtatccaat ctttgtagcc tgatttcaac tcaacacttt tgatttgaaa 660
tcggttgttt aatgcttcat ctctgtatat gtaatggtca tatgtcccat ttcttattga 720
tccaatgcat gcattatcac atttgtggta aatcttgaaa catccacctc ccatgtcttc 780
cgcgttttct cttaactggc gcctagtctt ctcgaataat ttattcattt ctgcatctgt 840
taagtcaatt gtatgttgat tttctagagc caccagcaat tctgcattgt aggaccatag 900
gtctattttg gtgtcttcta catacttctc caaatcctgg attctccctt ctacttctga 960
gaattccttt tctatttgat ggaatttctc attggtcctt tcaatcactc tgtttaattt 1020
tccattgatc tggtctatgg ctgcttgagt gctctttaga tctgcagctt gtcctgttcc 1080
ttccgagttt tgatatcgga atccatacca cccatcaacc attccttccc agccgttttc 1140
tatgaatccc gctattgctc caaagattcc tctgatttgc ttttctggta cattcctcat 1200
cccagtggcc agctttaaag tgttttgcct gatatacttg gggcattttc catatgtaat 1260
tttgttcaca ttttgaaatg gtttgtcgtt ggggatgctt ccatttggtg taatacattc 1320
agacacacaa gtgtctatga gtgcatctga tctcattaca gagcttttcc ctgttctcaa 1380
tttaaaatat ccccgcggtg caactaagtt gccattactg tttatcatta gaatatctcc 1440
aggttttaca atggtccagt atatgcttat cctgcctgat tgacccctga cccacggcct 1500
agatccgatg ttagggatta ctgtttgttg acttctttct gttgagactg ttactcgtcc 1560
tgattcttgg atatataatt ttgtctgctc tttgtttgag ctcgggtgat gaatccccca 1620
gatgtatagt ttatcgaaat ttttattgtt aggcattgtc acattcaata tggggtaaga 1680
atttccagat tttgttagcc aattcagtcg gctaaagaaa ctatcggctg atcccctttt 1740
gcaggctcca cttcttccgt tttgagtgac acctgtccat gtgaacccct ctgctgtgaa 1800
ttctaatgtt cctgaggatg ctacaatgga ccggagcgat gcatagtcag ggatgtcata 1860
tgggtagcaa ttgctgaaag cgctgcttct ttctatgaag aggtcccaat tctcatactg 1920
aaaatcatca caatgggggt ctcctagcat tgcatctatt aatgtgcaat ttcttccatc 1980
tagaacccta tatgagttgt tgcatatttt ccctattgaa atgctctgga ctaattcagt 2040
agcatttgtc acctcaattt ggtcatcagt tattgttttt accaatgttc catttgctac 2100

tgcatggtgt cccagacata atgtggctgt gttgttgcca ctggttgggt tttgactgta 2160
gacccaatgg gtcagtagta tcaaaataat ggttgtcttc attacgatac aaacttaacg 2220
gatatcgcga taatgaaata atttatgatt atttctcgct ttcaatttaa cacaaccctc 2280
aagaaccttt gtatttattt tcacttttta agtatagaat aaagaagctc taattaatta 2340
acgagcagat agtctcgttc tcgccctgcc tgatgactaa ttaattaacc cggatccttt 2400
ttatagctaa ttagtcacgt acctttgaga gtaccacttc agctacctct tttgtgtctc 2460
agagtaactt tctttaatca attccaaaac agtatatgat tttccatttc tttcaaagat 2520
gtagtttaca tctgctcctt tgttgaaaag tagcctgagc acttcttttc taccatgaat 2580
tacagctggc aagatcaatt tttcccagtt ctggacattt tattttttt aagtagtgtg 2640
ctacatattt caatatttcc agattgtaca gcgatcatta aaggagtacg tcccatgtta 2700
tccagcaagt cagtatcagc acctttgttc aatagaagtt taaccattgt taaattttta 2760
tttgatacgg ctatatgtag aggagttaac cgatccgtgt ttgaaatatc tacatccgcc 2820
gaatgagcca atagaagttt aaccaaatta actttgttaa ggtaagctgc caaacacaaa 2880
ggagtaaagc ctccgctgta aagaacattg tttacatagt tattcttcaa cagatctttc 2940
actattttgt agtcgtctct caacaccgca tcatgcagac aagaagttgt gcattcagta 3000
actacaggtt tagctccata cctcatcaag atttttatag cctcggtatt cttgaacatt 3060
acagccattt caagaggaga ttgtagagta ccatattccg tgttagggtc gaatccattg 3120
tccaaaaacc tatttagaga tgcattgtca ttatccatga tagcctcaca gacgtatatg 3180
taagccatct tgaatgtata attttgttgt tttcaacaac cgctcgtgaa cagcttctat 3240
actttttcat tttcttcatg attaatatag tttacggaat ataagtatac aaaaagttta 3300
tagtaatctc ataatatctg aaacacatac ataaaacatg gaagaattac acgatgtcgt 3360
tgagataaat ggctttttat tgtcatagtt tacaaattcg cagtaatctt catcttttac 3420
gaatattgca gaatctgttt tatccaacca gtgatttttg tataatataa ctggtatcct 3480
atcttccgat agaatgctgt tatttaacat ttttgcacct attaagttac atctgtcaaa 3540
tccatctttc caactgactt tatgtaacga tgcgaaatag catttatcac tatgtcgtac 3600
ccaattatca tgacaagatt ctcttaaata cgtaatctta ttatctcttg catattcgta 3660
atagtaattg taaagagtat acgataacag tatagatata cacgtgatat aaatatttaa 3720
ccccattcct gagtaaaata attacgatat tacatttcct tttattattt ttatgtttta 3780
gttatttgtt aggttataca aaaattatgt ttatttgtgt atatttaaag cgtcgttaag 3840
aataagctta gttaacatat tatcgcttag gttttgtagt atttgaatcc tttctttaaa 3900
tggattattt ttccaatgca tatttatagc ttcatccaaa gtataacatt taacattca 3959

<210> 2
<211> 3917
<212> DNA
<213> Influenza A virus

<400> 2

gtattctaaa ctaggaatag atgaaattat gtgcaaagga gataccttta gatatggatc 60
tgatttattt ggtttttcat aatcataatc taacaacatt ttcactatac tataccttct 120
tgcacaagtc gccattagta gtatagactt atactttgta accatagtat actttagcgc 180
gtcatcttct tcatctaaaa cagatttaca acaataatca tcgtcgtcat cttcatcttc 240
attaaagttt tcatattcaa taactttctt ttctaaaaca tcatctgaat caataaacat 300
agaacggtat agagcgttaa tctccattgt aaaatatact aacgcgttgc tcatgatgta 360
ctttttttc attatttaga aattatgcat tttagatctt tataagcggc cgtgattaac 420
tagtcataaa aacccgggat cgattctaga ctcgagggta cctcaaatgc aaatgttgca 480
tctgatgttg ccttttttggc aagcccacat aatgaaaccc aatagaacaa cgcaaattaa 540
gaagcatgat atggcgaatg aaatccacag tatccaatct ttgtagcctg atttcaactc 600
aacacctttg atttgaaatc ggttgtttaa tgcttcatct ctgtatatgt aatggtcata 660
tgtcccattt cttattgatc caatgcatgc attatcacat ttgtggtaaa tcttgaaaca 720
tccacctccc atgtcttccg cgttttctct taactggcgt ctagtcttct cgaataattt 780
attcatttct gcatctgtta agtcaattgt atgttgattt tctagagcca ccagcaattc 840
tgcattgtag gaccataggt ctattttggt gtcttctaca tacttctcca agtcctggat 900
tctcccttct acttctgaga attccttctc tatttgatgg aatttctcat tggtcctttc 960
aatcactctg tttaatttc cattaatctg gtcgatggct gcttgagtgc tctttagatc 1020

tgcagcttgt cctgttcctt ccgagttttg atatcggaat ccataccacc catcaaccat 1080
tccttcccag ccgttttcta tgaatcccgc tattgctcca aagattcctc tgatttgctt 1140
ttctggtaca ttcctcatcc cagtggccag ctttaaagtg ttttgcctga tatacttggg 1200
gcattttcca tatgtaactt tgttcacatt ttgaaatggt ttgtcgttgg ggatgcttcc 1260
atttggtgta atacattcag acacacaaat gtctatgggt gcatctgatc tcattacaga 1320
gcttttccct gttttcaatt taaaatatcc ccgcggtgca actaagttgc cattactgtt 1380
tatcattagg atatctccag gttttacaat ggtccagtat atgcttatcc tgcctgattg 1440
acccctgacc cacggtctag atccgatatt agggattatc gtttgttgac ttctttttgt 1500
tgagactgtt actcgtcctg attcttggat gtacaattct gtttgctgtt ggtttgagct 1560
cgggtgatga atcccccaga tgtatagttt gtcgaaattt ttattgttag gcattgtcac 1620
attcaatgtg gggtaagagt ttccagattt tgttagccaa ttcagtcggc taaagaaact 1680
atcggctgat cccctttttgc aggctccact tcttccgttt tgagtgacac ctgtccatgt 1740
gaatccctct gctgtgaatt ccaatgttcc tgaggatgct acaatggacc ggagcgatgc 1800
atagtcaggg atgtcatatg ggtagcaatt gctgaaagcg ctgcttcttt ctatgaagag 1860
gtcccaattc tcatactgaa agacatcaca gtgggggtct cctagcattg catctattaa 1920
tgtgcaattt cttccatcta gaactctata tgagttgttg catattttcc ctattgaaat 1980
gctctgaact aattcagtag catttgtcac ctcaatttgg tcatcagtta ttgtttttac 2040
caatgttcca tttgctactg catggtgtcc cagacataat gtggctgtgt tgttgccact 2100
ggttgggttt tgactgtaga cccaatgggt cagtagtatc aaaataatgg ttgtcttcat 2160
tacgatacaa acttaacgga tatcgcgata atgaaataat ttatgattat ttctcgcttt 2220
caatttaaca caaccctcaa gaacctttgt atttattttc actttttaag tatagaataa 2280
agaagctcta attaattaac gagcagatag tctcgttctc gccctgcctg atgactaatt 2340
aattaacccg gatccttttt atagctaatt agtcacgtac ctttgagagt accacttcag 2400
ctacctcttt tgtgtctcag agtaactttc tttaatcaat tccaaaacag tatatgattt 2460
tccatttctt tcaaagatgt agtttacatc tgctcctttg ttgaaaagta gcctgagcac 2520
ttcttttcta ccatgaatta cagctggcaa gatcaatttt tcccagttct ggacatttta 2580
ttttttttaa gtagtgtgct acatatttca atatttccag attgtacagc gatcattaaa 2640
ggagtacgtc ccatgttatc cagcaagtca gtatcagcac ctttgttcaa tagaagttta 2700
accattgtta aatttttatt tgatacggct atatgtagag gagttaaccg atccgtgttt 2760
gaaatatcta catccgccga atgagccaat agaagtttaa ccaaattaac tttgttaagg 2820
taagctgcca aacacaaagg agtaaagcct ccgctgtaaa gaacattgtt tacatagtta 2880
ttcttcaaca gatctttcac tattttgtag tcgtctctca acaccgcatc atgcagacaa 2940
gaagttgtgc attcagtaac tacaggttta gctccatacc tcatcaagat ttttatagcc 3000
tcggtattct tgaacattac agccatttca agaggagatt gtagagtacc atattccgtg 3060
ttagggtcga atccattgtc caaaaaccta tttagagatg cattgtcatt atccatgata 3120
gcctcacaga cgtatatgta agccatcttg aatgtataat tttgttgttt tcaacaaccg 3180
ctcgtgaaca gcttctatac tttttcattt tcttcatgat taatatagtt tacggaatat 3240
aagtatacaa aaagtttata gtaatctcat aatatctgaa acacatacat aaaacatgga 3300
agaattacac gatgtcgttg agataaatgg ctttttattg tcatagttta caaattcgca 3360
gtaatcttca tcttttacga atattgcaga atctgtttta tccaaccagt gatttttgta 3420
taatataact ggtatcctat cttccgatag aatgctgtta tttaacattt ttgcacctat 3480
taagttacat ctgtcaaatc catctttcca actgacttta tgtaacgatg cgaaatagca 3540
tttatcacta tgtcgtaccc aattatcatg acaagattct cttaaatacg taatcttatt 3600
atctcttgca tattcgtaat agtaattgta aagagtatac gataacagta tagatataca 3660
cgtgatataa atatttaacc ccattcctga gtaaaataat tacgatatta catttccttt 3720
tattattttt atgttttagt tatttgttag gttatacaaa aattatgttt atttgtgtat 3780
atttaaagcg tcgttaagaa taagcttagt taacatatta tcgcttaggt tttgtagtat 3840
ttgaatcctt tctttaaatg gattattttt ccaatgcata tttatagctt catccaaagt 3900
ataacattta acattca 3917

<210> 3
<211 > 565
<212> PRT
<213> Influenza A virus

<400> 3

Met Lys Thr Thr Ile Ile Leu Ile Leu Leu Thr His Trp Val Tyr Ser
1 5 10 15

Gln Asn Pro Thr Ser Gly Asn Asn Thr Ala Thr Leu Cys Leu Gly His
20 25 30

His Ala Val Ala Asn Gly Thr Leu Val Lys Thr Ile Thr Asp Asp Gln
35 40 45

Ile Glu Val Thr Asn Ala Thr Glu Leu Val Gln Ser Ile Ser Ile Gly
50 55 60

Lys Ile Cys Asn Asn Ser Tyr Arg Val Leu Asp Gly Arg Asn Cys Thr
65 70 75 80

Leu Ile Asp Ala Met Leu Gly Asp Pro His Cys Asp Asp Phe Gln Tyr
85 90 95

Glu Asn Trp Asp Leu Phe Ile Glu Arg Ser Ser Ala Phe Ser Asn Cys
100 105 110

Tyr Pro Tyr Asp Ile Pro Asp Tyr Ala Ser Leu Arg Ser Ile Val Ala
115 120 125

Ser Ser Gly Thr Leu Glu Phe Thr Ala Glu Gly Phe Thr Trp Thr Gly
130 135 140

Val Thr Gln Asn Gly Arg Ser Gly Ala Cys Lys Arg Gly Ser Ala Asp
145 150 155 160

Ser Phe Phe Ser Arg Leu Asn Trp Leu Thr Lys Ser Gly Asn Ser Tyr
165 170 175

Pro Ile Leu Asn Val Thr Met Pro Asn Asn Lys Asn Phe Asp Lys Leu
180 185 190

Tyr Ile Trp Gly Ile His His Pro Ser Ser Asn Lys Glu Gln Thr Lys
195 200 205

Leu Tyr Ile Gln Glu Ser Gly Arg Val Thr Val Ser Thr Glu Arg Ser
210 215 220

Gln Gln Thr Val Ile Pro Asn Ile Gly Ser Arg Pro Trp Val Arg Gly
225 230 235 240

Gln Ser Gly Arg Ile Ser Ile Tyr Trp Thr Ile Val Lys Pro Gly Asp
245 250 255

Ile Leu Met Ile Asn Ser Asn Gly Asn Leu Val Ala Pro Arg Gly Tyr
260 265 270

Phe Lys Leu Arg Thr Gly Lys Ser Ser Val Met Arg Ser Asp Ala Leu
275 280 285

Ile Asp Thr Cys Val Ser Glu Cys Ile Thr Pro Asn Gly Ser Ile Pro

290 295 300

Asn Asp Lys Pro Phe Gln Asn Val Asn Lys Ile Thr Tyr Gly Lys Cys
305 310 315 320

Pro Lys Tyr Ile Arg Gln Asn Thr Leu Lys Leu Ala Thr Gly Met Arg
325 330 335

Asn Val Pro Glu Lys Gln Ile Arg Gly Ile Phe Gly Ala Ile Ala Gly
340 345 350

Phe Ile Glu Asn Gly Trp Glu Gly Met Val Asp Gly Trp Tyr Gly Phe
355 360 365

Arg Tyr Gln Asn Ser Glu Gly Thr Gly Gln Ala Ala Asp Leu Lys Ser
370 375 380

Thr Gln Ala Ala Ile Asp Gln Ile Asn Gly Lys Leu Asn Arg Val Ile
385 390 395 400

Glu Arg Thr Asn Glu Lys Phe His Gln Ile Glu Lys Glu Phe Ser Glu
405 410 415

Val Glu Gly Arg Ile Gln Asp Leu Glu Lys Tyr Val Glu Asp Thr Lys
420 425 430

Ile Asp Leu Trp Ser Tyr Asn Ala Glu Leu Leu Val Ala Leu Glu Asn
435 440 445

Gln His Thr Ile Asp Leu Thr Asp Ala Glu Met Asn Lys Leu Phe Glu
450 455 460

Lys Thr Arg Arg Gln Leu Arg Glu Asn Ala Glu Asp Met Gly Gly Gly
465 470 475 480

Cys Phe Lys Ile Tyr His Lys Cys Asp Asn Ala Cys Ile Gly Ser Ile
485 490 495

Arg Asn Gly Thr Tyr Asp His Tyr Ile Tyr Arg Asp Glu Ala Leu Asn
500 505 510

Asn Arg Phe Gln Ile Lys Ser Val Glu Leu Lys Ser Gly Tyr Lys Asp
515 520 525

Trp Ile Leu Trp Ile Ser Phe Ala Ile Ser Cys Phe Leu Ile Cys Val
530 535 540

Val Leu Leu Gly Phe Ile Met Trp Ala Cys Gln Lys Gly Asn Ile Arg
545 550 555 560

Cys Asn Ile Cys Ile
565

<210> 4
<211> 565
<212> PRT
<213> Influenza A virus

<400> 4

Met Lys Thr Thr Ile Ile Leu Ile Leu Leu Thr His Trp Ala Tyr Ser
1 5 10 15

Gln Asn Pro Ile Ser Gly Asn Asn Thr Ala Thr Leu Cys Leu Gly His
20 25 30

His Ala Val Ala Asn Gly Thr Leu Val Lys Thr Ile Ser Asp Asp Gln
35 40 45

Ile Glu Val Thr Asn Ala Thr Glu Leu Val Gln Ser Ile Ser Met Gly
50 55 60

Lys Ile Cys Asn Asn Ser Tyr Arg Ile Leu Asp Gly Arg Asn Cys Thr
65 70 75 80

Leu Ile Asp Ala Met Leu Gly Asp Pro His Cys Asp Ala Phe Gln Tyr
85 90 95

Glu Asn Trp Asp Leu Phe Ile Glu Arg Ser Ser Ala Phe Ser Asn Cys
100 105 110

Tyr Pro Tyr Asp Ile Pro Asp Tyr Ala Ser Leu Arg Ser Ile Val Ala
115 120 125

Ser Ser Gly Thr Leu Glu Phe Thr Ala Glu Gly Phe Thr Trp Thr Gly
130 135 140

Val Thr Gln Asn Gly Arg Ser Gly Ala Cys Lys Arg Gly Ser Ala Asp
145 150 155 160

Ser Phe Phe Ser Arg Leu Asn Trp Leu Thr Lys Ser Gly Ser Ser Tyr
165 170 175

Pro Thr Leu Asn Val Thr Met Pro Asn Asn Lys Asn Phe Asp Lys Leu
180 185 190

Tyr Ile Trp Gly Ile His His Pro Ser Ser Asn Gln Glu Gln Thr Lys
195 200 205

Leu Tyr Ile Gln Glu Ser Gly Arg Val Thr Val Ser Thr Lys Arg Ser
210 215 220

Gln Gln Thr Ile Ile Pro Asn Ile Gly Ser Arg Pro Trp Val Arg Gly
225 230 235 240

Gln Ser Gly Arg Ile Ser Ile Tyr Trp Thr Ile Val Lys Pro Gly Asp
245 250 255

Ile Leu Met Ile Asn Ser Asn Gly Asn Leu Val Ala Pro Arg Gly Tyr
260 265 270

Phe Lys Leu Lys Thr Gly Lys Ser Ser Val Met Arg Ser Asp Val Pro

275 280 285

Ile Asp Ile Cys Val Ser Glu Cys Ile Thr Pro Asn Gly Ser Ile Ser
290 295 300

Asn Asp Lys Pro Phe Gln Asn Val Asn Lys Val Thr Tyr Gly Lys Cys
305 310 315 320

Pro Lys Tyr Ile Arg Gln Asn Thr Leu Lys Leu Ala Thr Gly Met Arg
325 330 335

Asn Val Pro Glu Lys Gln Ile Arg Gly Ile Phe Gly Ala Ile Ala Gly
340 345 350

Phe Ile Glu Asn Gly Trp Glu Gly Met Val Asp Gly Trp Tyr Gly Phe
355 360 365

Arg Tyr Gln Asn Ser Glu Gly Thr Gly Gln Ala Ala Asp Leu Lys Ser
370 375 380

Thr Gln Ala Ala Ile Asp Gln Ile Asn Gly Lys Leu Asn Arg Val Ile
385 390 395 400

Glu Arg Thr Asn Glu Lys Phe His Gln Ile Glu Lys Glu Phe Ser Glu
405 410 415

Val Glu Gly Arg Ile Gln Asp Leu Glu Lys Tyr Val Glu Asp Thr Lys
420 425 430

Ile Asp Leu Trp Ser Tyr Asn Ala Glu Leu Leu Val Ala Leu Glu Asn
435 440 445

Gln His Thr Ile Asp Leu Thr Asp Ala Glu Met Asn Lys Leu Phe Glu
450 455 460

Lys Thr Arg Arg Gln Leu Lys Glu Asn Ala Glu Asp Met Gly Gly Gly
465 470 475 480

Cys Phe Lys Ile Tyr His Lys Cys Asp Asn Ala Cys Ile Gly Ser Ile
485 490 495

Arg Asn Gly Thr Tyr Asp His Tyr Ile Tyr Arg Asp Glu Ala Leu Asn
500 505 510

Asn Arg Phe Gln Ile Lys Gly Val Glu Leu Lys Ser Gly Tyr Lys Asp
515 520 525

Trp Ile Leu Trp Ile Ser Phe Ala Ile Ser Cys Phe Leu Ile Cys Val
530 535 540

Val Leu Leu Gly Phe Ile Met Trp Ala Cys Gln Lys Gly Asn Ile Arg
545 550 555 560

Cys Asn Ile Cys Ile
565

<210> 5
<211> 565
<212> PRT
<213> Influenza A virus

<400> 5

Met Lys Thr Thr Ile Ile Leu Ile Leu Leu Thr His Trp Ala Tyr Ser
1 5 10 15

Gln Asn Pro Ile Ser Gly Asn Asn Thr Ala Thr Leu Cys Leu Gly His
20 25 30

His Ala Val Ala Asn Gly Thr Leu Val Lys Thr Ile Ser Asp Asp Gln
35 40 45

Ile Glu Val Thr Asn Ala Thr Glu Leu Val Gln Ser Ile Ser Met Gly
50 55 60

Lys Ile Cys Asn Asn Ser Tyr Arg Ile Leu Asp Gly Arg Asn Cys Thr
65 70 75 80

Leu Ile Asp Ala Met Leu Gly Asp Pro His Cys Asp Ala Phe Gln Tyr
85 90 95

Glu Asn Trp Asp Leu Phe Ile Glu Arg Ser Ser Ala Phe Ser Asn Cys
100 105 110

Tyr Pro Tyr Asp Ile Pro Asp Tyr Ala Ser Leu Arg Ser Ile Val Ala
115 120 125

Ser Ser Gly Thr Leu Glu Phe Thr Ala Glu Gly Phe Thr Trp Thr Gly
130 135 140

Val Thr Gln Asn Gly Arg Ser Gly Ala Cys Lys Arg Gly Ser Ala Asp
145 150 155 160

Ser Phe Phe Ser Arg Leu Asn Trp Leu Thr Lys Ser Gly Ser Ser Tyr
165 170 175

Pro Thr Leu Asn Val Thr Met Pro Asn Asn Lys Asn Phe Asp Lys Leu
180 185 190

Tyr Ile Trp Gly Ile His His Pro Ser Ser Asn Gln Glu Gln Thr Lys
195 200 205

Leu Tyr Ile Gln Glu Ser Gly Arg Val Thr Val Ser Thr Lys Arg Ser
210 215 220

Gln Gln Thr Ile Ile Pro Asn Ile Gly Ser Arg Pro Trp Val Arg Gly
225 230 235 240

Gln Ser Gly Arg Ile Ser Ile Tyr Trp Thr Ile Val Lys Pro Gly Asp
245 250 255

Ile Leu Met Ile Asn Ser Asn Gly Asn Leu Val Ala Pro Arg Gly Tyr

260 265 270

Phe Lys Leu Lys Thr Gly Lys Ser Ser Val Met Arg Ser Asp Val Pro
275 280 285

Ile Asp Ile Cys Val Ser Glu Cys Ile Thr Pro Asn Gly Ser Ile Ser
290 295 300

Asn Asp Lys Pro Phe Gln Asn Val Asn Lys Val Thr Tyr Gly Lys Cys
305 310 315 320

Pro Lys Tyr Ile Arg Gln Asn Thr Leu Lys Leu Ala Thr Gly Met Arg
325 330 335

Asn Val Pro Glu Lys Gln Ile Arg Gly Ile Phe Gly Ala Ile Ala Gly
340 345 350

Phe Ile Glu Asn Gly Trp Glu Gly Met Val Asp Gly Trp Tyr Gly Phe
355 360 365

Arg Tyr Gln Asn Ser Glu Gly Thr Gly Gln Ala Ala Asp Leu Lys Ser
370 375 380

Thr Gln Ala Ala Ile Asp Gln Ile Asn Gly Lys Leu Asn Arg Val Ile
385 390 395 400

Glu Arg Thr Asn Glu Lys Phe His Gln Ile Glu Lys Glu Phe Ser Glu
405 410 415

Val Glu Gly Arg Ile Gln Asp Leu Glu Lys Tyr Val Glu Asp Thr Lys
420 425 430

Ile Asp Leu Trp Ser Tyr Asn Ala Glu Leu Leu Val Ala Leu Glu Asn
435 440 445

Gln His Thr Ile Asp Leu Thr Asp Ala Glu Met Asn Lys Leu Phe Glu
450 455 460

Lys Thr Arg Arg Gln Leu Lys Glu Asn Ala Glu Asp Met Gly Gly Gly
465 470 475 480

Cys Phe Lys Ile Tyr His Lys Cys Asp Asn Ala Cys Ile Gly Ser Ile
485 490 495

Arg Asn Gly Thr Tyr Asp His Tyr Ile Tyr Arg Asp Glu Ala Leu Asn
500 505 510

Asn Arg Phe Gln Ile Lys Gly Val Glu Leu Lys Ser Gly Tyr Lys Asp
515 520 525

Trp Ile Leu Trp Ile Ser Phe Ala Ile Ser Cys Phe Leu Ile Cys Val
530 535 540

Val Leu Leu Gly Phe Ile Met Trp Ala Cys Gln Lys Gly Asn Ile Arg
545 550 555 560

Cys Asn Ile Cys Ile
565

<210> 6

<211> 3973
<212> DNA
<213> Influenza A virus

<220>
<221> CDS
<222> (1800)..(3494)

<400> 6

ggaaacagct atgaccatga ttacgaattg cggccgcaat tctgaatgtt aaatgttata 60

ctttggatga agctataaat atgcattgga aaaataatcc atttaaagaa aggattcaaa 120

tactacaaaa cctaagcgat aatatgttaa ctaagcttat tcttaacgac gctttaaata 180

tacacaaata aacataattt ttgtataacc taacaaataa ctaaaacata aaaataataa 240

aaggaaatgt aatatcgtaa ttattttact caggaatggg gttaaatatt tatatcacgt 300

gtatatctat actgttatcg tatactcttt acaattacta ttacgaatat gcaagagata 360

ataagattac gtatttaaga gaatcttgtc atgataattg ggtacgacat agtgataaat 420

gctatttcgc atcgttacat aaagtcagtt ggaaagatgg atttgacaga tgtaacttaa 480

taggtgcaaa aatgttaaat aacagcattc tatcggaaga taggatacca gttatattat 540

acaaaaatca ctggttggat aaaacagatt ctgcaatatt cgtaaaagat gaagattact 600

gcgaatttgt aaactatgac aataaaaagc catttatctc aacgacatcg tgtaattctt 660

ccatgtttta tgtatgtgtt tcagatatta tgagattact ataaactttt tgtatactta 720

tattccgtaa actatattaa tcatgaagaa aatgaaaaag tatagaagct gttcacgagc 780

ggttgttgaa aacaacaaaa ttatacattc aagatggctt acatatacgt ctgtgaggct 840

atcatggata atgacaatgc atctctaaat aggtttttgg acaatggatt cgaccctaac 900

acggaatatg gtactctaca atctcctctt gaaatggctg taatgttcaa gaataccgag 960

gctataaaaa tcttgatgag gtatggagct aaacctgtag ttactgaatg cacaacttct 1020

tgtctgcatg atgcggtgtt gagagacgac tacaaaatag tgaaagatct gttgaagaat 1080

aactatgtaa acaatgttct ttacagcgga ggctttactc ctttgtgttt ggcagcttac 1140

cttaacaaag ttaatttggt taaacttcta ttggctcatt cggcggatgt agatatttca 1200

aacacggatc ggttaactcc tctacatata gccgtatcaa ataaaaattt aacaatggtt 1260

aaacttctat tgaacaaagg tgctgatact gacttgctgg ataacatggg acgtactcct 1320

ttaatgatcg ctgtacaatc tggaaatatt gaaatatgta gcacactact taaaaaaaat 1380

aaaatgtcca gaactgggaa aaattgatct tgccagctgt aattcatggt agaaaagaag 1440

tgctcaggct acttttcaac aaaggagcag atgtaaacta catctttgaa agaaatggaa 1500

aatcatatac tgttttggaa ttgattaaag aaagttactc tgagacacaa aagaggtagc 1560

tgaagtggta ctctcaaagg tacgtgacta attagctata aaaaggatcc gggttaatta 1620

attagtcatc aggcagggcg agaacgagac tatctgctcg ttaattaatt agagcttctt 1680

tattctatac ttaaaaagtg aaaataaata caaaggttct tgagggttgt gttaaattga 1740

aagcgagaaa taatcataaa ttatttcatt atcgcgatat ccgttaagtt tgtatcgta 1799

atg aaa acc acc atc atc ctg atc ctg ctg acc cac tgg gcc tac agc 1847
Met Lys Thr Thr Ile Ile Leu Ile Leu Leu Thr His Trp Ala Tyr Ser
1 5 10 15

cag aac cct atc agc ggc aac aac acc gcc acc ctg tgc ctg ggc cac 1895
Gln Asn Pro Ile Ser Gly Asn Asn Thr Ala Thr Leu Cys Leu Gly His
20 25 30

cac gcc gtg gcc aac ggc acc ctg gtc aag acc atc agc gac gac cag 1943
His Ala Val Ala Asn Gly Thr Leu Val Lys Thr Ile Ser Asp Asp Gln
35 40 45

atc gaa gtg acc aac gcc acc gag ctg gtg cag agc atc agc atg ggc 1991
Ile Glu Val Thr Asn Ala Thr Glu Leu Val Gln Ser Ile Ser Met Gly
50 55 60

aag atc tgc aac aac agc tac cgc atc ctg gac ggc aga aac tgc acc 2039
Lys Ile Cys Asn Asn Ser Tyr Arg Ile Leu Asp Gly Arg Asn Cys Thr
65 70 75 80

ctg atc gac gcc atg ctg ggc gac ccc cac tgc gac gcc ttc cag tac 2087
Leu Ile Asp Ala Met Leu Gly Asp Pro His Cys Asp Ala Phe Gln Tyr
85 90 95

gag aac tgg gac ctg ttc atc gag agg agc agc gcc ttc agc aac tgc 2135
Glu Asn Trp Asp Leu Phe Ile Glu Arg Ser Ser Ala Phe Ser Asn Cys
100 105 110

tac ccc tac gac atc cct gac tac gcc agc ctg aga agc atc gtg gcc 2183
Tyr Pro Tyr Asp Ile Pro Asp Tyr Ala Ser Leu Arg Ser Ile Val Ala
115 120 125

agc agc ggc acc ctg gag ttc acc gcc gag ggc ttc acc tgg acc ggc 2231
Ser Ser Gly Thr Leu Glu Phe Thr Ala Glu Gly Phe Thr Trp Thr Gly
130 135 140

gtg acc cag aac ggc aga agc ggc gcc tgc aag aga ggc agc gcc gac 2279
Val Thr Gln Asn Gly Arg Ser Gly Ala Cys Lys Arg Gly Ser Ala Asp

145 150 155 160

agc ttc ttc agc cgc ctg aac tgg ctg acc aag agc ggc agc agc tac 2327
Ser Phe Phe Ser Arg Leu Asn Trp Leu Thr Lys Ser Gly Ser Ser Tyr
165 170 175

ccc acc ctg aac gtg acc atg ccc aac aac aag aac ttc gac aag ctg 2375
Pro Thr Leu Asn Val Thr Met Pro Asn Asn Lys Asn Phe Asp Lys Leu
180 185 190

tac atc tgg ggc atc cac cac ccc agc agc aac cag gag cag acc aag 2423
Tyr Ile Trp Gly Ile His His Pro Ser Ser Asn Gln Glu Gln Thr Lys
195 200 205

ctg tac atc cag gag agc ggc aga gtg acc gtg tcc acc aag aga agc 2471
Leu Tyr Ile Gln Glu Ser Gly Arg Val Thr Val Ser Thr Lys Arg Ser
210 215 220

cag cag acc atc atc ccc aac atc ggc agc aga cct tgg gtg cgc ggc 2519
Gln Gln Thr Ile Ile Pro Asn Ile Gly Ser Arg Pro Trp Val Arg Gly
225 230 235 240

cag tcc ggc agg atc agc atc tac tgg acc atc gtg aag cct ggc gac 2567
Gln Ser Gly Arg Ile Ser Ile Tyr Trp Thr Ile Val Lys Pro Gly Asp
245 250 255

atc ctg atg atc aac agc aac ggc aac ctg gtg gcc ccc aga ggc tac 2615
Ile Leu Met Ile Asn Ser Asn Gly Asn Leu Val Ala Pro Arg Gly Tyr
260 265 270

ttc aag ctg aaa acc ggc aag agc agc gtg atg aga agc gac gtg ccc 2663
Phe Lys Leu Lys Thr Gly Lys Ser Ser Val Met Arg Ser Asp Val Pro
275 280 285

atc gac atc tgc gtg tcc gag tgc atc acc cct aac ggc agc atc agc 2711
Ile Asp Ile Cys Val Ser Glu Cys Ile Thr Pro Asn Gly Ser Ile Ser
290 295 300

aac gac aag ccc ttc cag aac gtg aac aaa gtg acc tac ggc aag tgc 2759
Asn Asp Lys Pro Phe Gln Asn Val Asn Lys Val Thr Tyr Gly Lys Cys
305 310 315 320

ccc aag tac atc cgc cag aac acc ctg aag ctg gcc acc ggc atg aga 2807
Pro Lys Tyr Ile Arg Gln Asn Thr Leu Lys Leu Ala Thr Gly Met Arg
325 330 335

aac gtg ccc gag aag cag atc aga ggc atc ttc ggc gcc atc gcc ggc 2855
Asn Val Pro Glu Lys Gln Ile Arg Gly Ile Phe Gly Ala Ile Ala Gly
340 345 350

ttc atc gag aac ggc tgg gag ggc atg gtg gac ggc tgg tac ggc ttc 2903
Phe Ile Glu Asn Gly Trp Glu Gly Met Val Asp Gly Trp Tyr Gly Phe
355 360 365

aga tac cag aac agc gag ggc acc ggc cag gcc gcc gac ctg aag agc 2951
Arg Tyr Gln Asn Ser Glu Gly Thr Gly Gln Ala Ala Asp Leu Lys Ser

370 375 380

acc cag gcc gcc atc gac cag atc aac ggc aag ctg aac cgc gtg atc 2999
Thr Gln Ala Ala Ile Asp Gln Ile Asn Gly Lys Leu Asn Arg Val Ile
385 390 395 400

gag cgc acc aac gag aag ttc cac cag atc gag aag gag ttc agc gaa 3047
Glu Arg Thr Asn Glu Lys Phe His Gln Ile Glu Lys Glu Phe Ser Glu
405 410 415

gtg gag ggc aga atc cag gac ctg gag aag tac gtg gag gac acc aag 3095
Val Glu Gly Arg Ile Gln Asp Leu Glu Lys Tyr Val Glu Asp Thr Lys
420 425 430

atc gac ctg tgg agc tac aac gcc gag ctg ctg gtc gcc ctg gag aac 3143
Ile Asp Leu Trp Ser Tyr Asn Ala Glu Leu Leu Val Ala Leu Glu Asn
435 440 445

cag cac acc atc gac ctg acc gac gcc gag atg aac aag ctg ttc gaa 3191
Gln His Thr Ile Asp Leu Thr Asp Ala Glu Met Asn Lys Leu Phe Glu
450 455 460

aag acc agg cgc cag ctg aag gaa aac gcc gag gac atg ggc ggc ggc 3239
Lys Thr Arg Arg Gln Leu Lys Glu Asn Ala Glu Asp Met Gly Gly Gly
465 470 475 480

tgc ttc aag atc tac cac aag tgc gac aac gcc tgc atc ggc tcc atc 3287
Cys Phe Lys Ile Tyr His Lys Cys Asp Asn Ala Cys Ile Gly Ser Ile
485 490 495

agg aac ggc acc tac gac cac tac atc tac agg gac gag gcc ctg aac 3335
Arg Asn Gly Thr Tyr Asp His Tyr Ile Tyr Arg Asp Glu Ala Leu Asn
500 505 510

aac cgc ttc cag atc aag ggc gtg gag ctg aag agc ggc tac aag gac 3383
Asn Arg Phe Gln Ile Lys Gly Val Glu Leu Lys Ser Gly Tyr Lys Asp
515 520 525

tgg atc ctg tgg atc agc ttc gcc atc agc tgc ttc ctg atc tgc gtg 3431
Trp Ile Leu Trp Ile Ser Phe Ala Ile Ser Cys Phe Leu Ile Cys Val
530 535 540

gtg ctg ctg ggc ttc atc atg tgg gcc tgc cag aag ggc aac atc cgc 3479
Val Leu Leu Gly Phe Ile Met Trp Ala Cys Gln Lys Gly Asn Ile Arg
545 550 555 560

tgc aac atc tgc atc tgatgactcg agggttttta tgactagtta atcacggccg 3534
Cys Asn Ile Cys Ile
565

cttataaaga tctaaaatgc ataatttcta aataatgaaa aaaagtacat catgagcaac 3594

gcgttagtat attttacaat ggagattaac gctctatacc gttctatgtt tattgattca 3654

gatgatgttt tagaaaagaa agttattgaa tatgaaaact ttaatgaaga tgaagatgac 3714

gacgatgatt attgttgtaa atctgtttta gatgaagaag atgacgcgct aaagtatact 3774

atggttacaa agtataagtc tatactacta atggcgactt gtgcaagaag gtatagtata 3834

gtgaaaatgt tgttagatta tgattatgaa aaaccaaata aatcagatcc atatctaaag 3894

gtatctcctt tgcacataat ttcatctatt cctagtttag aatacctgca gccaagcttg 3954

gcactggccg tcgttttac 3973

<210> 7
<211> 3973
<212> DNA
<213> Influenza A virus

<400> 7

gtaaaacgac ggccagtgcc aagcttggct gcaggtattc taaactagga atagatgaaa 60
ttatgtgcaa aggagatacc tttagatatg gatctgattt atttggtttt tcataatcat 120
aatctaacaa cattttcact atactatacc ttcttgcaca agtcgccatt agtagtatag 180
acttatactt tgtaaccata gtatacttta gcgcgtcatc ttcttcatct aaaacagatt 240
tacaacaata atcatcgtcg tcatcttcat cttcattaaa gttttcatat tcaataactt 300
tcttttctaa aacatcatct gaatcaataa acatagaacg gtatagagcg ttaatctcca 360
ttgtaaaata tactaacgcg ttgctcatga tgtacttttt ttcattattt agaaattatg 420
catttttagat ctttataagc ggccgtgatt aactagtcat aaaaaccctc gagtcatcag 480
atgcagatgt tgcagcggat gttgcccttc tggcaggccc acatgatgaa gcccagcagc 540
accacgcaga tcaggaagca gctgatggcg aagctgatcc acaggatcca gtccttgtag 600
ccgctcttca gctccacgcc cttgatctgg aagcggttgt tcagggcctc gtccctgtag 660
atgtagtggt cgtaggtgcc gttcctgatg gagccgatgc aggcgttgtc gcacttgtgg 720
tagatcttga agcagccgcc gcccatgtcc tcggcgtttt ccttcagctg gcgcctggtc 780
ttttcgaaca gcttgttcat ctcggcgtcg gtcaggtcga tggtgtgctg gttctccagg 840
gcgaccagca gctcggcgtt gtagctccac aggtcgatct tggtgtcctc cacgtacttc 900
tccaggtcct ggattctgcc ctccacttcg ctgaactcct tctcgatctg gtggaacttc 960
tcgttggtgc gctcgatcac gcggttcagc ttgccgttga tctggtcgat ggcggcctgg 1020
gtgctcttca ggtcggcggc ctggccggtg ccctcgctgt tctggtatct gaagccgtac 1080
cagccgtcca ccatgccctc ccagccgttc tcgatgaagc cggcgatggc gccgaagatg 1140
cctctgatct gcttctcggg cacgtttctc atgccggtgg ccagcttcag ggtgttctgg 1200
cggatgtact tggggcactt gccgtaggtc actttgttca cgttctggaa gggcttgtcg 1260
ttgctgatgc tgccgttagg ggtgatgcac tcggacacgc agatgtcgat gggcacgtcg 1320
cttctcatca cgctgctctt gccggttttc agcttgaagt agcctctggg ggccaccagg 1380
ttgccgttgc tgttgatcat caggatgtcg ccaggcttca cgatggtcca gtagatgctg 1440
atcctgccgg actggccgcg cacccaaggt ctgctgccga tgttggggat gatggtctgc 1500
tggcttctct tggtggacac ggtcactctg ccgctctcct ggatgtacag cttggtctgc 1560
tcctggttgc tgctggggtg gtggatgccc cagatgtaca gcttgtcgaa gttcttgttg 1620
ttgggcatgg tcacgttcag ggtggggtag ctgctgccgc tcttggtcag ccagttcagg 1680
cggctgaaga agctgtcggc gctgcctctc ttgcaggcgc cgcttctgcc gttctgggtc 1740
acgccggtcc aggtgaagcc ctcggcggtg aactccaggg tgccgctgct ggccacgatg 1800
cttctcaggc tggcgtagtc agggatgtcg taggggtagc agttgctgaa ggcgctgctc 1860
ctctcgatga acaggtccca gttctcgtac tggaaggcgt cgcagtgggg gtcgcccagc 1920
atggcgtcga tcagggtgca gtttctgccg tccaggatgc ggtagctgtt gttgcagatc 1980
ttgcccatgc tgatgctctg caccagctcg gtggcgttgg tcacttcgat ctggtcgtcg 2040
ctgatggtct tgaccagggt gccgttggcc acggcgtggt ggcccaggca cagggtggcg 2100
gtgttgttgc cgctgatagg gttctggctg taggcccagt gggtcagcag gatcaggatg 2160
atggtggttt tcattacgat acaaacttaa cggatatcgc gataatgaaa taatttatga 2220
ttatttctcg ctttcaattt aacacaaccc tcaagaacct ttgtatttat tttcactttt 2280
taagtataga ataaagaagc tctaattaat taacgagcag atagtctcgt tctcgccctg 2340

cctgatgact aattaattaa cccggatcct ttttatagct aattagtcac gtacctttga 2400
gagtaccact tcagctacct cttttgtgtc tcagagtaac tttctttaat caattccaaa 2460
acagtatatg attttccatt tctttcaaag atgtagttta catctgctcc tttgttgaaa 2520
agtagcctga gcacttcttt tctaccatga attacagctg gcaagatcaa tttttcccag 2580
ttctggacat tttatttttt ttaagtagtg tgctacatat ttcaatattt ccagattgta 2640
cagcgatcat taaaggagta cgtcccatgt tatccagcaa gtcagtatca gcacctttgt 2700
tcaatagaag tttaaccatt gttaaatttt tatttgatac ggctatatgt agaggagtta 2760
accgatccgt gtttgaaata tctacatccg ccgaatgagc caatagaagt ttaaccaaat 2820
taactttgtt aaggtaagct gccaaacaca aaggagtaaa gcctccgctg taaagaacat 2880
tgtttacata gttattcttc aacagatctt tcactatttt gtagtcgtct ctcaacaccg 2940
catcatgcag acaagaagtt gtgcattcag taactacagg tttagctcca tacctcatca 3000
agatttttat agcctcggta ttcttgaaca ttacagccat ttcaagagga gattgtagag 3060
taccatattc cgtgttaggg tcgaatccat tgtccaaaaa cctatttaga gatgcattgt 3120
cattatccat gatagcctca cagacgtata tgtaagccat cttgaatgta taattttgtt 3180
gttttcaaca accgctcgtg aacagcttct atactttttc attttcttca tgattaatat 3240
agtttacgga atataagtat acaaaaagtt tatagtaatc tcataatatc tgaaacacat 3300
acataaaaca tggaagaatt acacgatgtc gttgagataa atggcttttt attgtcatag 3360
tttacaaatt cgcagtaatc ttcatctttt acgaatattg cagaatctgt tttatccaac 3420
cagtgatttt tgtataatat aactggtatc ctatcttccg atagaatgct gttatttaac 3480
atttttgcac ctattaagtt acatctgtca aatccatctt tccaactgac tttatgtaac 3540
gatgcgaaat agcatttatc actatgtcgt acccaattat catgacaaga ttctcttaaa 3600
tacgtaatct tattatctct tgcatattcg taatagtaat tgtaaagagt atacgataac 3660
agtatagata tacacgtgat ataaatattt aaccccattc ctgagtaaaa taattacgat 3720
attacatttc cttttattat ttttatgttt tagttatttg ttaggttata caaaaattat 3780
gtttatttgt gtatatttaa agcgtcgtta agaataagct tagttaacat attatcgctt 3840
aggttttgta gtatttgaat cctttcttta aatggattat ttttccaatg catatttata 3900
gcttcatcca aagtataaca tttaacattc agaattgcgg ccgcaattcg taatcatggt 3960
catagctgtt tcc 3973

<210> 8
<211> 890
<212> DNA
<213> Influenza A virus

<400> 8

agcaaaagca gggtgacaaa aacataatgg attccaacac tgtgtcaagc tttcaggtag 60
actgttttct ttggcatgtc cgcaaacgat tcgcagacca agaactgggt gatgccccat 120
tccttgaccg gcttcgccga gaccagaagt ccctaagggg aagaggtatc actcttggtc 180
tggacatcga aacagccact catgcaggaa agcagatagt ggagcagatt ctggaaaagg 240
aatcagatga ggcacttaaa atgaccattg cctctgttcc tacttcacgc tacttaactg 300
acatgactct tgatgagatg tcaagagact ggttcatgct catgcccaag caaaaagtaa 360
caggctccct atgtataaga atggaccagg caatcatgga taagaacatc atacttaaag 420
caaactttag tgtgattttc gaaaggctgg aaacactaat actacttaga gccttcaccg 480
aagaaggagc agtcgttggc gaaatttcac cattaccttc tcttccagga catactaatg 540
aggatgtcaa aaatgcaatt ggggtcctca tcggaggact taaatggaat gataatacgg 600
ttagaatctc tgaaactcta cagagattcg cttggagaag cagtcatgag aatgggagac 660
cttcattccc ttcaaagcag aaatgaaaaa tggagagaac aattaagcca gaaatttgaa 720
gaaataagat ggttgattga agaagtgcga catagattga aaaatacaga aaatagtttt 780
gaacaaataa catttatgca agccttacaa ctattgcttg aagtagaaca agagataaga 840
actttctcgt ttcagcttat ttaatgataa aaaacaccct tgtttctact 890

<210> 9
<211> 1027
<212> DNA
<213> Influenza A virus

<400> 9

agcaaaagca ggtagatatt taaagatgag tcttctgacc gaggtcgaaa cgtacgttct 60
ctctatcgta ccatcaggcc ccctcaaagc cgagatcgcg cagagacttg aagatgtctt 120
tgcagggaag aacaccgatc ttgaggcact catggaatgg ctaaagacaa gaccaatcct 180
gtcacctttg actaaaggga ttttaggatt tgtattcacg ctcaccgtgc ccagtgagcg 240
aggactgcag cgtagacgct ttgtccaaaa tgcccttagt ggaaacggag atccaaacaa 300
catggacaga gcagtaaaac tgtacaggaa gcttaaaaga gaaataacat tccatggggc 360
aaaagaggtg gcactaagct attccactgg tgcactagcc agctgcatgg gactcatata 420
caacagaatg ggaactgtga caaccgaagt ggcatttggc ctggtatgcg ccacatgtga 480
acagattgct gattcccagc atcgatctca caggcagatg gtgacaacaa ccaacccatt 540
aatcagacat gaaaacagaa tggtattagc cagtaccacg gctaaagcca tggaacagat 600
ggcaggatca agtgagcagg cagcagaggc catggaggtt gctagtaagg ctaggcagat 660
ggtacaggca atgagaacca ttgggaccca ccctagctcc agtgccggtt tgaaagatga 720
tctccttgaa aatttacagg cctaccagaa acggatggga gtgcaaatgc agcgattcaa 780
gtgatcctct cgttattgca gcaagtatca ttgggatctt gcacttgata ttgtggattc 840
ttgatcgtct tttcttcaaa ttcatttatc gtcgccttaa atacgggttg aaaagagggc 900
cttctacgga aggagtacct gagtctatga gggaagaata tcggcaggaa cagcagaatg 960
ctgtggatgt tgacgatggt cattttgtca acatagagct ggagtaaaaa actaccttgt 1020
ttctact 1027

<210> 10
<211> 1460
<212> DNA
<213> Influenza A virus

<400> 10

agcaaaagca ggagtttaaa atgaatccaa atcaaaagat aatagcaatt ggatttgcat 60
cattggggat attaatcatt aacgtcattc tccatgtagt cagcattata gtaacagtac 120
tggtcctcaa taacaatgga acaggtctga actgcaaagg gacgatcata agagagtaca 180
atgaaacagt aagagtagaa aaaattactc aatggtataa taccagtgca attaagtaca 240
tagagagacc tccaaatgaa tactacatga acaacaccga accactttgt gaggcccaag 300
gctttgcacc attttccaaa gataatggaa tacgaattgg gtcgagaggc catgtttttg 360
tgataagaga accttttgta tcatgttcgc cctcagaatg tagaaccttt ttcctcacac 420
agggctcatt actcaatgac aaacattcta acggcacagt aaaggaccga agtccatata 480
ggactttgat gagtgtcaaa atagggcaat cacctaatgt gtatcaagct aggtttgaat 540
cggtggcatg gtcagcaaca gcatgccatg atggaaaaaa atggatgaca gttggagtca 600
cagggcccga caatcaagca attgcagtag tgaactatgg aggtgttccg gttgatatta 660
ttaattcatg ggcaggggat atcttaagaa cccaagaatc atcatgcacc tgcattaaag 720
gagactgtta ttgggtaatg actgatggac cggcaaatag gcaagctaaa tataggatat 780
tcaaagcaaa agatggaaga gtaattggac agactgatat aagtttcaat gggggacaca 840
tagaggagtg ttcttgttac cccaatgaag ggaaggtgga atgcatatgc agggacaatt 900
ggactggaac aaatagacca attctggtaa tatcttctga tctatcgtac acagttggat 960
atttgtgtgc tggcattccc actgacactc ctaggggaga ggatagtcaa ttcacaggct 1020
catgtacaag acctttggga aataaaggat acggtgtaaa aggtttcggg tttcgacaag 1080
gaactgacgt atgggccgga aggacaatta gtaggacttc aagatcagga ttcgaaataa 1140
taaaaatcag gaatggttgg acacagaaca gtaaagacca aatcaggagg caagtgatta 1200
tcgatgaccc aaattggtca ggatatagcg gttctttcac attgccggtt gaactaacaa 1260
aaaagggatg tttggtcccc tgtttctggg ttgaaatgat tagaggtaaa cctgaagaaa 1320
caacaatatg gacctctagc agctccattg tgatgtgtgg agtagatcat aaaattgcca 1380
gttggtcatg gcacgatgga gctattcttc cctttgacat cgataagatg taatttacga 1440
aaaaactcct tgtttctact 1460

<210> 11
<211> 1565
<212> DNA
<213> Influenza A virus

<400> 11

agcaaaagca gggtagataa tcactcactg agtgacatca aagtcatggc gtctcaaggc 60
accaaacgat cctatgaaca gatggaaact gatggggaac gccagaatgc aactgaaatc 120
agagcatctg tcggaaggat ggtgggagga atcggccggt tttatgttca gatgtgtact 180
gagcttaaac taaacgacca tgaagggcgg ctgattcaga acagcataac aatagaaagg 240
atggtacttt cggcattcga cgaaagaaga aacaagtatc tcgaggagca tcccagtgct 300
ggaaaagacc ctaagaaaac gggaggcccg atatacagaa ggaaagatgg gaaatggatg 360
agggaactca tcctccatga taaagaagaa atcatgagaa tctggcgtca ggccaacaat 420
ggtgaagacg ctactgctgg tcttactcat atgatgatct ggcactccaa tctcaatgac 480
accacatacc aaagaacaag ggctcttgtt cggactggga tggatcccag aatgtgctct 540
ctgatgcaag gctcaaccct cccacggaga tctggagccg ctggtgctgc agtaaaaggt 600
gttggaacaa tggtaatgga actcatcaga atgatcaaac gcggaataaa tgatcggaat 660
ttctggagag gtgaaaatgg tcgaaggacc agaattgctt atgaaagaat gtgcaatatc 720
ctcaaaggga aatttcagac agcagcacaa cgggctatga tggaccaggt gagggaaggc 780
cgcaatcctg gaaacgctga gattgaggat ctcattttct tagcacgatc agcacttatt 840
ttgagaggat cagtagccca taaatcatgc ctacctgcct gtgtttatgg ccttgcagta 900
accagtgggt atgactttga gaaggaagga tactctctgg ttggaattga tcctttcaaa 960
ctactccaga acagtcaaat tttcagtcta atcagaccaa aagaaaaccc agcacacaag 1020
agccagttgg tgtggatggc atgccattct gcagcatttg aggacctgag agttttgaat 1080
ttcattagag gaaccaaagt aatcccaaga ggacagttaa caaccagagg agttcaaatt 1140
gcttcaaatg aaaacatgga gacaatagat tctagcacac ttgaactgag aagcaaatat 1200
tgggcaataa ggaccagaag tggaggaaac accagtcaac agagagcatc tgcaggacag 1260
ataagtgtgc aacctacttt ctcagtacag agaaatcttc cctttgagag agcaaccatt 1320
atggctgcat tcactggtaa cactgaaggg aggacttccg acatgagaac ggaaatcata 1380
aggatgatgg aaagtgccaa atcagaagat gtgtctttcc aggggcgggg agtcttcgag 1440
ctctcggacg aaaaggcaac gaacccgatc gtgccttcct ttgacatgag caatgaaggg 1500
tcttatttct tcggagacaa tgctgaggaa tttgacagtt aaagaaaaat acccttgttt 1560
ctact 1565

<210> 12
<211> 1762
<212> DNA
<213> Influenza A virus

<400> 12

agcaaaagca ggggatattt ctgtcaatca tgaagacaac cattattttg atactactga 60
cccattgggc ctacagtcaa aacccaatca gtggcaacaa cacagccaca ttgtgtctgg 120
gacaccatgc agtagcaaat ggaacattgg taaaaacaat aagtgatgat caaattgagg 180
tgacaaatgc tacagaatta gttcagagca tttcaatggg gaaaatatgc aacaactcat 240
atagaattct agatggaaga aattgcacat taatagatgc aatgctagga gacccccact 300
gtgacgtctt tcagtatgag aattgggacc tctttataga aagaagcagc gctttcagca 360
attgctaccc atatgacatc cctgactatg catcgctccg atccattgta gcatcctcag 420
gaacattgga attcacagca gagggattca catggacagg tgtcactcaa aacggaagaa 480
gtggagcctg caaaagggga tcagccgata gtttctttag ccgactgaat tggctaacaa 540
aatctggaaa ctcttacccc acattgaatg tgacaatgcc taacaataaa aatttcgaca 600
agctatacat ctgggggatt catcacccga gctcaaatca agagcagaca aaattgtaca 660
tccaagaatc aggacgagta acagtctcaa caaaaagaag tcaacaaaca ataatcccta 720
acatcggatc tagaccgtgg gtcagaggtc aatcaggcag gataagcata tactggacca 780
ttgtaaaacc tggagatatc ctaatgataa acagtaatgg caacttagtt gcaccgcggg 840
gatattttaa attgaaaaca gggaaaagct ctgtaatgag atcagatgca cccatagaca 900

tttgtgtgtc tgaatgtatt acaccaaatg gaagcatctc caacgacaag ccattccaaa 960
atgtgaacaa agttacatat ggaaaatgcc ccaagtatat caggcaaaac actttaaagt 1020
tggccactgg gatgaggaat gtaccagaaa agcaaatcag aggaatcttt ggagcaatag 1080
cgggattcat cgaaaacggc tgggaaggaa tggttgatgg gtggtatggg ttccgatatc 1140
aaaactctga aggaacaggg caagctgcag atctaaagag cactcaagca gccatcgacc 1200
agattaatgg aaagttaaac agagtgattg aaagaaccaa tgagaaattc catcaaatag 1260
agaaggaatt ctcagaagta gaaggaagaa ttcaggactt ggagaaatat gtagaagaca 1320
ccaaaataga cctatggtcc tacaatgcag aattgctggt ggctctagaa aatcaacata 1380
caattgactt aacagatgca gaaatgaata aattattcga gaagactaga cgccagttaa 1440
gagaaaacgc agaagacatg ggaggtggat gtttcaagat ttaccacaaa tgtgataatg 1500
catgcattgg atcaataaga aatgggacat atgaccatta catatacaga gatgaagcat 1560
taaacaaccg atttcaaatc aaaggtgttg agttgaaatc aggctacaaa gattggatac 1620
tgtggatttc attcgccata tcatgcttct taatttgcgt tgttctattg ggtttcatta 1680
tgtgggcttg ccaaaaaggc aacatcagat gcaacatttg catttgagta aactgatagt 1740
taaaaacacc cttgtttcta ct 1762

<210> 13
<211> 2233
<212> DNA
<213> Influenza A virus

<400> 13

agcgaaagca ggtactgatc caaaatggaa gactttgtgc gacagtgctt caatccaatg 60
atcgtcgagc ttgcggaaaa ggcaatgaaa gaatatggag aggacccgaa aatcgaaaca 120
aacaaatttg cagcaatatg cactcacttg gaagtctgct tcatgtactc ggatttccac 180
tttattaatg aactgggtga gtcagtggtc atagagtctg gtgacccaaa tgctcttttg 240
aaacacagat ttgaaatcat tgaggggaga gatcgaacaa tggcatggac agtggtaaac 300
agcatctgca acaccacaag agctgaaaaa cctaaatttc ttccagattt atacgactat 360
aaggagaaca gatttgttga aattggtgtg acaaggagag aagttcacat atactacctg 420
gagaaggcca acaaaataaa gtctgagaaa acacatatcc acattttctc atttacagga 480
gaggaaatgg ctacaaaagc ggactatact cttgatgaag agagtagagc caggatcaag 540
accagactat tcactataag acaagaaatg gccagtagag gcctctggga ttcctttcgt 600
cagtccgaga gaggcgaaga gacaattgaa gaaagatttg aaatcacagg gacgatgcgc 660
aagcttgcca attacagtct cccaccgaac ttctccagcc ttgaaaattt tagagtctat 720
gtggatggat tcgaaccgaa cggctgcatt gagagtaagc tttctcaaat gtccaaagaa 780
gtaaatgcca gaatcgaacc attttcaaag acaacacccc gaccactcaa aatgccaggt 840
ggtccaccct gccatcagcg atctaaattc ttgctaatgg atgctctgaa actgagcatt 900
gaggacccaa gtcacgaggg agagggaata ccactatatg atgcaatcaa atgcatgaaa 960
actttctttg gatggaaaga gcccagtatt gttaaaccac atgaaaaggg tataaacccg 1020
aactatctcc aaacttggaa gcaagtatta gaagaaatac aagaccttga gaacgaagaa 1080
aggacccccca agaccaagaa tatgaaaaaa acaagccaat tgaaatgggc actaggtgaa 1140
aatatggcac cagagaaagt ggattttgag gattgtaaag acatcagtga tttaaaacag 1200
tatgacagcg atgagccaga aacaaggtct cttgcaagtt ggattcaaag tgagttcaac 1260
aaagcttgtg agctgacaga ttcaagctgg atagagctcg atgaaattgg ggaggatgtc 1320
gccccaatag aatacattgc gagcatgagg agaaattatt ttactgctga gatttcccat 1380
tgtagagcaa cagaatatat aatgaaagga gtgtacatca acactgctct actcaatgca 1440
tcctgtgctg cgatggatga atttcaatta attccgatga taagtaaatg caggaccaaa 1500
gaagggagaa ggaaaacaaa tttatatgga ttcataataa agggaaggtc ccatttaaga 1560
aatgatactg acgtggtgaa ctttgtaagt atggaatttt ctctcactga tccaagattt 1620
gagccacaca aatgggaaaa atactgcgtt ctagaaattg gagacatgct tctaaggact 1680
gctgtaggtc aagtgtcaag acccatgttt ttgtatgtaa ggacaaatgg aacctctaaa 1740
attaaaatga aatggggaat ggaaatgagg cgctgcctcc ttcagtctct gcaacagatt 1800
gaaagcatga tcgaagctga gtcctcagtc aaagaaaagg acatgaccaa agaatttttt 1860
gagaacaaat cagagacatg gcctatagga gagtccccca aaggagtgga agagggctca 1920
atcgggaagg tttgcaggac cttattagca aaatctgtgt ttaacagttt atatgcatct 1980

ccacaactgg aagggttttc agctgaatct aggaaattac ttctcattgt tcaggctctt 2040
agggataacc tggaacctgg aacctttgat attggggggt tatatgaatc aattgaggag 2100
tgcctgatta atgatccctg ggttttgctt aatgcatctt ggttcaactc cttccttaca 2160
catgcactga agtagttgtg gcaatgctac tatttgctat ccatactgtc caaaaaagta 2220
ccttgtttct act 2233

<210> 14
<211> 2341
<212> DNA
<213> Influenza A virus

<400> 14

agcgaaagca ggcaaaccat ttgaatggat gtcaatccga ctctactttt cttaaaggtg 60
ccagcgcaaa atgctataag cacaacattc ccttatactg gagatcctcc ctacagtcat 120
ggaacaggga caggatacac catggatact gtcaacagaa cacaccaata ttcagaaaag 180
gggaaatgga caacaaacac tgagattgga gcaccacaac ttaatccaat cgatggacca 240
cttcctgaag acaatgaacc aagtgggtac gcccaaacag attgtgtatt ggaagcaatg 300
gctttccttg aagaatccca tcccggaatc tttgaaaatt cgtgtcttga aacgatggag 360
gtgattcagc agacaagagt ggacaaacta acacaaggcc gacaaactta tgattggacc 420
ttgaatagga atcaacctgc cgcaacagca cttgctaata caattgaagt gttcagatca 480
aatggtctga cttccaatga atcagggagg ttgatggact tcctcaaaga tgtcatggag 540
tccatgaaca aggaagaaat ggaaataaca acacacttcc aacgaaagag aagagtaaga 600
gacaacatga caaagagaat ggtaacacag agaaccatag ggaagaaaaa acaacgatta 660
aacagaaaga gttatctaat cagaacatta accctaaaca caatgaccaa ggacgctgag 720
agagggaaat tgaaacgacg agcaatcgca accccaggga tgcagataag aggatttgta 780
tattttgttg aaacactagc ccgaagaata tgtgaaaagc ttgaacaatc aggattgcca 840
gttggcggta atgagaaaaa ggccaaactg gctaatgtcg tcagaaaaat gatgactaat 900
tcccaagaca ctgaactctc cttcaccatc actggggaca ataccaaatg gaatgaaaat 960
cagaacccac gcatattcct ggcaatgatc acatacataa ctagaaacca gccagaatgg 1020
ttcagaaatg ttctaagcat tgcaccgatt atgttctcaa ataaaatggc aagactgggg 1080
aaaggatata tgtttgaaag caaaagtatg aaactgagag ctcaaatacc agcagaaatg 1140
ctagcaagca ttgacctgaa atatttcaat gattcaacaa aaaagaaaat taaaaagata 1200
cgaccacttc tggttgacgg gactgcttca ctgagtcctg gcatgatgat gggaatgttc 1260
aacatgttga gcactgtgct gggtgtatcc atattaaacc tgggccagag gaaatacaca 1320
aagaccacat actggtggga tggtctgcaa tcatccgatg actttgcttt gatagtgaat 1380
gcgcctaatc atgaaggaat acaagctgga gtagacagat tctatagaac ttgcaaactg 1440
gtcgggatca acatgagcaa aaagaagtcc tacataaata gaaccggaac attcgaattc 1500
acaagctttt tctaccggta tggttttgta gccaatttca gcatggaact acccagtttt 1560
ggggtttccg gaataaatga atctgcagac atgagcattg gagtgacagt catcaaaaac 1620
aacatgataa ataatgatct cggtcctgcc acggcacaaa tggcactcca actcttcatt 1680
aaggattacc ggtacacata ccggtgccat agaggtgata cccagataca aaccagaaga 1740
tcttttgagt tgaagaaact gtgggaacag actcgatcaa agactggtct actggtatca 1800
gatgggggtc caaacctata taacatcaga aacctacaca tcccggaagt ttgtttaaaa 1860
tgggagctaa tggatgaaga ttataagggg aggctatgca atccattaaa tcctttcgtt 1920
agtcacaaag aaattgaatc agtcaacagt gcagtagtaa tgcctgcgca tggccctgcc 1980
aaaagcatgg agtatgatgc tgttgcaaca acacattctt ggatccccaa gaggaaccgg 2040
tccatattga acacaagcca aaggggaata ctcgaagatg agcagatgta tcagaaatgc 2100
tgcaacctgt ttgaaaaatt cttccccagc agctcataca gaagaccagt cggaatttct 2160
agtatggttg aggccatggt gtccagggcc cgcattgatg cacgaattga cttcgaatct 2220
ggacggataa agaaggatga gttcgctgag atcatgaaga tctgttccac cattgaagag 2280
ctcagacggc aaaaatagtg aatttagctt gatcttcatg aaaaaatgcc ttgtttctac 2340
t 2341

<210> 15
<211> 2341
<212> DNA
<213> Influenza A virus

<400> 15

agcgaaagca ggtcaaatat attcaatatg gagagaataa aagaactgag agatctgatg 60
ttacaatccc gcacccgcga gatactaaca aaaactactg tggaccacat ggccataatc 120
aagaaataca catcaggaag acaagagaag aaccctgcac ttaggatgaa atggatgatg 180
gcaatgaaat acccaattac agcagataag aggataatgg agatgattcc tgagagaaat 240
gaacagggac aaacccttg gagcaaaacg aacgatgctg gctcagaccg cgtaatggta 300
tcacctctgg cagtgacatg gtggaatagg aatggaccaa caacgagcac aattcattat 360
ccaaaagtct acaaaactta ttttgaaaag gttgaaagat tgaaacacgg aacctttggc 420
cccgttcatt ttaggaatca agtcaagata agacgaagag ttgatgtaaa ccctggtcac 480
gcggacctca gtgccaaaga agcacaagat gtgatcatgg aagttgtttt cccaaatgaa 540
gtgggagcca gaattctaac atcggaatca caactaacaa taaccaaaga gaaaaaggaa 600
gaacttcagg actgcaaaat tgctcccttg atggtagcat acatgctaga aagagagttg 660
gtccgaaaaa caaggttcct cccagtggca ggcggaacaa gcagtgtata cattgaagtg 720
ttgcatctga ctcagggaac atgctgggag caaatgtaca ccccaggagg agaagttaga 780
aacgatgata ttgatcaaag tttaattatt gcagcccgga acatagtgag aagagcgaca 840
gtatcagcag atccactagc atccctactg gaaatgtgcc acagtacaca gattggtgga 900
ataaggatgg tagacatcct taagcagaat ccaacagagg aacaagctgt ggatatatgc 960
aaagcagcaa tgggattgag aattagctca tcattcagct ttggtggatt caccttcaaa 1020
agaacaagtg gatcatcagt caagagagaa gaagaaatgc ttacgggcaa ccttcaaaca 1080
ttgaaaataa gagtgcatga gggctatgaa gaattcacaa tggtcggaag aagagcaaca 1140
gccattctca gaaaggcaac cagaagattg attcaattga tagtaagtgg gagagatgaa 1200
caatcaattg ctgaagcaat aattgtagcc atggtgtttt cgcaagaaga ttgcatgata 1260
aaagcagttc gaggcgattt gaactttgtt aatagagcaa atcagcgttt gaaccccatg 1320
catcaactct tgaggcattt ccaaaaagat gcaaaagtgc ttttccaaaa ttggggaatt 1380
gaacccatcg acaatgtaat gggaatgatt ggaatattgc ctgacatgac cccaagcacc 1440
gagatgtcat tgagaggagt gagagtcagc aaaatgggag tggatgagta ctccagcact 1500
gagagagtgg tggtgagcat tgaccgtttt ttaagagttc gggatcaaag gggaaacata 1560
ctactgtccc ctgaagaagt cagtgaaaca caaggaacgg aaaagctgac aataatttat 1620
tcgtcatcaa tgatgtggga gattaatggt cccgaatcag tgttggtcaa tacttatcaa 1680
tggatcatca ggaactggga aattgtaaaa attcagtggt cacaggaccc cacaatgtta 1740
tacaataaga tagaatttga gccattccag tccctggtcc ctagggccac cagaagccaa 1800
tacagcggtt tcgtaagaac cctgtttcag caaatgcgag atgtacttgg aacatttgat 1860
actgctcaaa taataaaact cctccctttt gccgctgctc ctccggaaca gagtaggatg 1920
cagttctctt ctttgactgt taatgtaaga ggatcgggaa tgaggatact tgtaagaggc 1980
aattccccag tgttcaacta caataaagcc actaaaaggc tcacagtcct cggaaaggat 2040
gcaggtgcgc ttactgagga cccagatgaa ggtacggctg gagtagaatc tgctgttcta 2100
agagggtttc tcattttagg taaagaaaac aagagatatg gcccagcact aagcatcaat 2160
gaactaagca aacttgcaaa aggggagaaa gccaatgtac taattgggca aggggacgta 2220
gtgttggtaa tgaaacggaa acgtgactct agcatactta ctgacagcca gacagcgacc 2280
aaaaggattc ggatggccat caattagtgt tgaattgttt aaaaacgacc ttgtttctac 2340
t 2341

<210> 16
<211> 1762
<212> DNA
<213> Influenza A virus

<400> 16

agcaaaagca ggggatattt ctgtcaatca tgaagacaac cattattttg atactactga 60
cccattgggt ctacagtcaa aacccaacca gtggcaacaa cacagccaca ttatgtctgg 120
gacaccatgc agtagcaaat ggaacattgg taaaaacaat aactgatgac caaattgagg 180

tgacaaatgc tactgaatta gttcagagca tttcaatagg gaaaatatgc aacaactcat 240
atagagttct agatggaaga aattgcacat taatagatgc aatgctagga gacccccact 300
gtgatgtctt tcagtatgag aattgggacc tcttcataga aagaagcagc gctttcagca 360
gttgctaccc atatgacatc cctgactatg catcgctccg gtccattgta gcatcctcag 420
gaacattgga attcacagca gagggattca catggacagg tgtcactcaa aacggaagaa 480
gtggatcctg caaaagggga tcagccgata gtttctttag ccgactgaat tggctaacaa 540
aatctggaaa ctcttacccc acattgaatg tgacaatgcc taacaataaa aatttcgaca 600
aactatacat ctggggggatt catcacccga gctcaaacga agagcagaca aaattgtaca 660
tccaagaatc aggacgagta acagtctcaa caaaaagaag tcaacaaaca ataatcccta 720
acatcggatc tagaccgtgg gtcaggggtc aatcaggcag gataagcata tactggacca 780
ttgtaaaacc tggagatatc ctaatgataa acagtaatgg caacttagtt gcaccgcggg 840
gatattttaa attgaaaaca gggaaaagct ctgtaatgag atcagatgca cccatagaca 900
tttgtgtgtc tgaatgtatt acaccaaatg gaagcatccc caacgacaaa ccatttcaaa 960
atgtgaacaa agttacatat ggaaaatgcc ccaagtatat caggcaaaac actttaaagc 1020
tggccactgg gatgaggaat gtaccagaaa agcaaatcag aggaatcttt ggagcaatag 1080
cgggattcat agaaaacggc tgggaaggaa tggttgatgg gtggtatgga ttccgatatc 1140
aaaactcgga aggaacagga caagctgcag atctaaagag cactcaagca gccatcgacc 1200
agatcaatgg aaaattaaac agagtgattg aaaggaccaa tgagaaattc catcaaatag 1260
agaaggaatt ctcagaagta gaagggagaa tccaggattt ggagaagtat gtagaagaca 1320
ccaaaataga cctatggtcc tacaatgcag aattgctggt ggctctagaa aatcaacata 1380
caattgactt aacagatgca gaaatgaata aattattcga gaagactaga cgccagttaa 1440
gagaaaacgc ggaagacatg ggaggtggat gtttcaagat ataccacaaa tgtgataatg 1500
catgcattgg atcaataaga aatgggacat atgaccatta catatacaga gatgaagcat 1560
taaacaaccg gtttcaaatc aaaggtgttg agttgaaatc aggctacaaa gattggatac 1620
tgtggatttc attcgccata tcatgcttct taatttgcgt tgttctattg ggtttcatta 1680
tgtgggcttg ccaaaaaggc aacatcagat gcaacatttg catttgagta aactgatagt 1740
taaaaacacc cttgtttcta ct 1762

<210> 17
<211> 1762
<212> DNA
<213> Influenza A virus

<400> 17

agcaaaagca ggggatattt ctgtcaatca tgaagacaac cattattttg atactactga 60
cccattgggt ctacagtcaa aacccaacca gtggcaacaa cacagccaca ttatgtctgg 120
gacaccatgc agtagcaaat ggaacattgg taaaaacaat aactgatgac caaattgagg 180
tgacaaatgc tactgaatta gttcagagca tttcaatagg gaaaatatgc aacaactcat 240
atagggttct agatggaaga aattgcacat taatagatgc aatgctagga gacccccact 300
gtgatgtctt tcagtatgag aattgggacc tcttcataga aagaagcagc gctttcagca 360
attgctaccc atatgacatc cctgactatg catcgctccg gtccattgta gcatcctcag 420
gaacattaga attcacagca gagggattca catggacagg tgtcactcaa aacggaagaa 480
gtggagcctg caaaagggga tcagccgata gtttctttag ccgactgaat tggctaacaa 540
aatctggaaa ctcttacccc acattgaatg tgacaatgcc taacaataaa aatttcgaca 600
aactatacat ctggggggatt catcacccga gctcaaacaa tgagcagaca aaattgtata 660
tccaagaaac aggacgagta acagtctcaa caaaaagaag tcaacaaaca ataatcccta 720
acatcggatc tagaccgtgg gtcaggggtc aatcaggcag gataagcata tactggacca 780
ttgtaaaacc tggagatatc ctaatgataa acagcaatgg caacttagtt gcaccgcggg 840
gatattttaa attgagaaca gggagaagct ctgtaatgag atcagatgca cccatagaca 900
tttgtgtgtc tgaatgtatt acaccaaatg gaagcatccc caacgacaaa ccatttcaaa 960
atgtgaacaa agttacatat ggaaaatgcc ccaaatatat caggcaaaac actttaaagc 1020
tggccactgg gatgaggaat gtaccagaaa agcaaatcag aggaatcttt ggagcaatag 1080
cgggattcat agaaaacggc tgggaaggaa tggttgatgg gtggtatgga ttccgatatc 1140
aaaactcgga aggaacagga caagctgcag atctaaagag cactcaagca gccatcgacc 1200
agatcaatgg aaaattaaac agagtgattg aaaggaccaa tgagaaattc catcaaatag 1260

agaaggaatt ctcagaagta gaagggagaa tccaggattt ggagaagtat gtagaagaca 1320
ccaaaataga cctatggtcc tacaatgcag aattgctagt ggctctagaa aatcaacata 1380
caattgactt aacagacgca gaaatgaata aattattcga gaagactaga cgccagttaa 1440
gagaaaacgc ggaagacatg ggaggtggat gtttcaagat ttaccacaaa tgtgataatg 1500
catgcattgg atcaataaga aatgggacat atgaccatta catatacaga gatgaagcat 1560
taaacaaccg atttcaaatc aaaggtgttg aattaaaatc aggctacaaa gattggatac 1620
tgtggatttc attcgccata tcatgcttct taatttgtgt tgttctattg ggtttcatta 1680
tgtgggcttg ccaaaaaggc aacatcagat gcaacatttg catttgagta aactgatagt 1740
taaaaacacc cttgtttcta ct 1762

<210> 18
<211> 1762
<212> DNA
<213> Influenza A virus

<400> 18

agcaaaagca ggggatattt ctgtcaatca tgaaaacaac cattattttg atactactga 60
cccattgggt ctacagtcaa aacccaacca gtggcaacaa cacagccaca ttatgtctgg 120
gacaccatgc agtagcaaat ggaacattgg taaaaacaat aactgatgac caaattgagg 180
tgacaaatgc tactgaatta gttcagagca tttcaatagg aaaaatatgc aacaactcat 240
atagggttct agatggaaga aattgcacat taatagatgc aatgctagga gaccctcact 300
gtgatgtctt tcagtatgag aattgggacc tcttcataga aagaagcagc gctttcagca 360
attgctaccc atatgacatc cctgactatg catcgctccg gtccattgta gcatcctcag 420
gaacattaga attcacagca gagggattca catggacagg tgtcactcaa aacggaggaa 480
gtggagcctg caaaagagga tcagccgata gtttctttag ccgactgaat tggctaacaa 540
aatctggaaa ctcttacccc acattgaatg tgacaatgcc taacaataaa aatttcgaca 600
aactatacat ctgggggatt catcacccga gctcaaacaa tgagcagaca aaattgtata 660
tccaagaatc aggacgagta acagtctcaa caaaaagaag tcaacaaaca ataatcccta 720
acatcggatc tagaccgtgg gtcaggggtc aatcaggcag gataagcata tactggacca 780
ttgtaaaacc tggagatatc ctaatgataa acagtaatgg caacttagtt gcaccgcggg 840
gatattttaa attgagaaca gggaaaagct ctgtaatgag atcagatgca cccatagaca 900
cttgtgtgtc tgaatgtatt acaccaaatg gaagcatccc caacgacaaa ccatttcaaa 960
atgtgaacaa agttacatat ggaaaatgcc ccaagtatat caggcaaaac actttaaagc 1020
tggccactgg gatgaggaat gtaccagaaa agcaaatcag aggaatcttt ggagcaatag 1080
cgggattcat agaaaacggc tgggaaggaa tggttgatgg gtggtatgga ttccgatatc 1140
aaaactcgga aggaacagga caagctgcag atctaaagag cactcaagca gccatcgacc 1200
agatcaatgg aaaattaaac agagtgattg aaaggaccaa tgagaaattc catcaaatag 1260
agaaggaatt ctcagaagta gaagggagaa tcaaggactt ggagaagtat gtagaagaca 1320
ccaaaataga cctatggtcc tacaatgcag aattgctggt ggctctagaa aatcaacata 1380
caattgactt aacagatgca gaaatgaata aattattcga gaagactaga cgccagttaa 1440
gagaaaacgc ggaagacatg ggaggtggat gtttcaagat ctaccacaaa tgtgataatg 1500
catgcattgg atcaataaga aatgggacat atgaccatta catatacaaa gatgaagcat 1560
taaacaaccg atttcaaatc aaaggtgttg agttgaaatc aggctacaaa gattggatac 1620
tgtggatttc attcgccata tcatgcttct taatttgcgt tgttctattg ggtttcatta 1680
tgtgggcttg ccaaaaaggc aacatcagat gcaacatttg catttgagta aactgatagt 1740
taaaaacacc cttgtttcta ct 1762

<210> 19
<211> 1762
<212> DNA
<213> Influenza A virus

<400> 19

agcaaaagca ggggatattt ctgtcaatca tgaagacaac cattattttg atactactga 60

cccattgggt ctacagtcaa aacccaacca gtggcaacaa cacagccaca ttatgtctgg 120
gacaccatgc agtagcaaat ggaacattgg taaaaacaat aactgatgac caaattgagg 180
tgacaaatgc tactgaatta gttcagagca tttcaatagg gaaaatatgc aacaactcat 240
atagagttct agatggaaga aattgcacat taatagatgc aatgctagga gacccccact 300
gtgatgtctt tcagtatgag aattgggacc tcttcataga aagaagcagc gctttcagca 360
attgctaccc atatgacatc cctgactatg catcgctccg gtccattgta gcatcctcag 420
gaacattgga attcacagca gagggattca catggacagg tgtcactcaa aacggaagaa 480
gtggagcctg caaaagggga tcagccgata gtttctttag ccgactgaat tggctaacaa 540
aatctggaaa ctcttacccc acattgaatg tgacaatgcc taacaataaa aatttcgaca 600
aactatacat ctggggggatt catcacccga gctcaaacca acagcaaaca gaattgtaca 660
tccaagaatc aggacgagta acagtctcaa caaaaagaag tcaacaaacg ataatcccta 720
atatcggatc tagaccgtgg gtcaggggtc aatcaggcag gataagcata tactggacca 780
ttgtaaaacc tggagatatc ctaatgataa acagtaatgg caacttagtt gcaccgcggg 840
gatattttaa attgaaaaca gggaaaagct ctgtaatgag atcagatgca cccatagaca 900
tttgtgtgtc tgaatgtatt acaccaaatg gaagcatccc caacgacaaa ccatttcaaa 960
atgtgaacaa agttacatat ggaaaatgcc ccaagtatat caggcaaaac actttaaagc 1020
tggccactgg gatgaggaat gtaccagaaa agcaaatcag aggaatcttt ggagcaatag 1080
cgggattcat agaaaacggc tgggaaggaa tggttgatgg gtggtatgga ttccgatatc 1140
aaaactcgga aggaacagga caagctgcag atctaaagag cactcaagca gccatcgacc 1200
agattaatgg aaaattaaac agagtgattg aaaggaccaa tgagaaattc catcaaatag 1260
agaaggaatt ctcagaagta gaagggagaa tccaggactt ggagaagtat gtagaagaca 1320
ccaaaataga cctatggtcc tacaatgcag aattgctggt ggctctagaa aatcaacata 1380
caattgactt aacagatgca gaaatgaata aattattcga gaagactaga cgccagttaa 1440
gagaaaacgc ggaagacatg ggaggtggat gtttcaagat ttaccacaaa tgtgataatg 1500
catgcattgg atcaataaga aatgggacat atgaccatta catatacaga gatgaagcat 1560
taaacaaccg atttcaaatc aaaggtgttg agttgaaatc aggctacaaa gattggatac 1620
tgtggatttc attcgccata tcatgcttct taatttgcgt tgttctattg ggtttcatta 1680
tgtgggcttg ccaaaaaggc aacatcagat gcaacatttg catttgagta aactgatagt 1740
taaaaacacc cttgtttcta ct 1762

<210> 20
<211> 1565
<212> DNA
<213> Influenza A virus

<400> 20

agcaaaagca gggtagataa tcactcactg agtgacatca aagtcatggc gtctcaaggc 60
accaaacgat cttatgagca gatggaaact ggtggggaac gccagaatgc aactgaaatc 120
agagcatctg ttggaaggat ggtgggagga atcggccggt tctatgttca aatgtgtact 180
gagcttaaac tcaacgacca tgaagggcgg ctgattcaga acagcataac aatagaaagg 240
atggtacttt cggcattcga cgaaagaaga aacaagtacc tcgaggagca tcccagtgct 300
gggaaagacc ccaagaaaac gggaggcccg atatacagaa ggagagatgg gaaatggatg 360
agagaactca tcctccatga taaagaagaa atcatgagga tctggcgtca ggccaacaat 420
ggtgaagacg ctactgctgg tcttactcat atgatgatct ggcactccaa tctcaatgac 480
accacctacc aaagaacaag ggctcttgtt cggctgggga tggatcccag aatgtgctct 540
ctgatgcaag gatcaactct cccacggaga tctggagctg ccggtgctgc agtgaagggt 600
gttggaacaa tggtaatgga actcatcagg atgatcaaac gcgggataaa tgatcgaaac 660
ttctggagag gtgaaaatgg tcgaagaacc agaattgctt atgaaagaat gtgcaacatc 720
ctcaagggga aattccaaac agcagcacaa cgagcaatga tggaccaagt gagggagggc 780
cgcaatcctg gaaatgctga gattgaggat ctcattttct tggcacgatc agcactcatt 840
ctgagaggat cagtagccca taaatcatgc ctacctgcct gtgtttatgg ccttgcagta 900
gccagtgggt atgactttga gaaagaggga tactctctgg ttggaattga tcctttcaaa 960
ctactccaga acagccaaat tttcagtcta atcagaccga aagaaaatcc agcacacaag 1020
agccagctgg tgtggatggc atgccattct gcagcatttg aggacctgag agtttcgaat 1080
ttcattagag gaaccaaagt aatcccaaga ggacagttag caaccagggg agtgcaaatt 1140

gcttcaaatg aaaacatgga gacaatagat tctagcacac tcgaactgag gagcagatat 1200
tgggcaataa ggaccaggag tggggggaac accagtcaac agagagcatc tgcaggacag 1260
ataagtgtgc aacccacttt ctcagtgcag agaaatcttc cctttgaaag agcaaccatt 1320
atggctgcat tcactggaaa cactgagggg aggacttccg acatgagaac ggaaatcata 1380
aggatgatgg aaaatgccag atcagaagat gtgtctttcc aggggcgggg agtcttcgag 1440
ctctcggacg aaaaggcaac gaacccgatc gtgccttcct ttgacatgag caatgaaggg 1500
tcttatttct tcggagacaa tgctgaggag tttgacagtt aaagaaaaat acccttgttt 1560
ctact 1565

<210> 21
<211> 1096
<212> DNA
<213> Influenza A virus

<400> 21

gtcaatcatg aagacaacca ttattttgat actactgacc cattgggtct acagtcaaaa 60
cccaaccagt ggcaacaaca cagccacatt atgtctggga caccatgcag tagcaaatgg 120
aacattggta aaaacaataa ctgatgacca aattgaggtg acaaatgcta ctgaattagt 180
tcagagcatt tcaataggga aaatatgcaa caactcatat agggttctag atggaagaaa 240
ttgcacatta atagatgcaa tgctaggaga cccccactgt gatgtctttc agtatgagaa 300
ttgggacctc ttcatagaaa gaagcagcgc tttcagcaat tgctacccat atgacatccc 360
tgactatgca tcgctccggt ccattgtagc atcctcagga acattagaat tcacagcaga 420
gggattcaca tggacaggtg tcactcaaaa cggaggaagt ggagcctgca aaagggggatc 480
agccgatagt ttctttagcc gactgaattg gctaacaaaa tctggaaact cttaccccac 540
attgaatgtg acaatgccta acaataaaaa tttcgacaaa ctatacatct gggggattca 600
tcacccgagc tcaaacaatg agcagacaaa attgtatatc caagaaacag gacgagtaac 660
agtctcaaca aaaagaagtc aacaaacaat aatccctaac atcggatcta gaccgtgggt 720
caggggtcaa tcaggcagga taagcatata ctggaccatt gtaaaacctg gagatatcct 780
aatgataaac agcaatggca acttagttgc accgcgggga tattttaaat tgagaacagg 840
gagaagctct gtaatgagat cagatgcacc catagacatt tgtgtgtctg aatgtattac 900
accaaatgga agcatcccca acgacaaacc atttcaaaat gtgaacaaag ttacatatgg 960
aaaatgcccc aaatatatca ggcaaaacac tttaaagctg gccactggga tgaggaatgt 1020
accagaaaag caaatcagag gaatctttgg agcaatagcg ggattcatag aaaacggctg 1080
ggaaggaatg gttgat 1096

<210> 22
<211> 1061
<212> DNA
<213> Influenza A virus

<400> 22

agcaaaagca ggggatattt ctgtcaatca tgaagacaac cattattttg atactactga 60
cccattgggc ctacagtcaa aacccaatca gtggcaacaa cacagccaca ttgtgtctgg 120
gacaccatgc agtagcaaat ggaacattgg taaaaacaat aactgatgat caaattgagg 180
tgacaaatgc tactgaatta gttcagagca tttcaatggg gaaaatatgc aacaactcat 240
atagagttct agatggaaga aattgcacat taatagatgc aatgctagga gaccccccact 300
gtgatgtctt tcagtatgag aattgggacc tctttataga aagaagcagc gctttcagca 360
attgctaccc atatgacatc cctgactatg catcgctccg gtccattgta gcatcctcag 420
gaacattgga attcacagca gagggattca catggacagg tgtcactcaa aacggaagaa 480
gtggagcctg caaaagggga tcagccgata gtttctttag ccgactgaat tggctaacaa 540
aatctggaaa ctcttacccc acattgaatg tgacaatgcc taacaataaa aatttcgaca 600
aactatacat ctgggggatt catcacccga gctcaaacca agagcagaca aaattgtaca 660
tccaagaatc aggacgagta acagtctcaa caaaaagaag tcaacaaaca ataatcccta 720
acatcggatc tagaccgtgg gtcaggggtc aatcaggcag gataagcata tactggacca 780

ttgtaaaacc tggagatatc ctaatgataa acagtaatgg caacttagtt gcaccgcggg 840
gatattttaa attgaaaaca gggaaaagct ctgtaatgag atcagatgca cccatagaca 900
tttgtgtgtc tgaatgtatt acaccaaatg gaagcatctc caacgacaaa ccattccaaa 960
atgtgaacaa agttacatat ggaaaatgcc ccaagtatat caggcaaaac actttaaagc 1020
tggccactgg gatgaggaat gaaccagaaa agcaaatcag a 1061

<210> 23
<211> 1061
<212> DNA
<213> Influenza A virus

<400> 23

agcaaaagca ggggatattt ctgtcaatca tgaagacaac cattattttg atactactga 60
cccattgggt ctacagtcaa aacccaacca gtggcaacaa cacagccaca ttatgtctgg 120
gacaccatgc agtagcaaat ggaacattgg taaaaacaat aactgatgac caaattgagg 180
tgacaaatgc tactgaatta gttcagagca tttcaatagg gaaaatatgc aacaactcat 240
atagagttct agatggaaga aattgcacat taatagatgc aatgctagga gacccccact 300
gtgatgtctt ccagtatgag aattgggacc tcttcataga aagaagcagc gctttcagca 360
attgctaccc atatgacatc cctgactatg catcgctccg gtccattgta gcatcctcag 420
gaacattaga attcacagca gagggattca catggacagg tgtcactcaa aacggaagaa 480
gtggagcctg caaaagggga tcagccgata gtttctttag ccgactgaat tggctaacaa 540
aatctggaaa ctcttacccc acattgaatg tgacaatgcc taacaataaa aatttcgaca 600
aactatacat ctggggggatt catcacccga gctcaaacca aaagcagaca gaattgtaca 660
tccaagaatc aggacgagta acagtctcaa caaaaagaag tcaacaaacg ataatcccta 720
atatcggatc tagaccgtgg gttaggggtc aatcaggcag gataagcata tactggacca 780
ttgtaaaacc tggagatatc ctaatgataa acagtaatgg caacttagtt gcaccgcggg 840
gatattttaa attgaaaaca gggaaaagct ctgtaatgag atcagatgca cccatagaca 900
tttgtgtgtc tgaatgtatt acaccaaatg gaagcatccc caacgacaaa ccatttcaaa 960
atgtgaacaa agttacatat ggaaaatgcc ccaagtatat caggcaaaac actttaaagc 1020
tggccactgg gatgaggaat gtaccagaaa agcaaatcag a 1061

<210> 24
<211> 1061
<212> DNA
<213> Influenza A virus

<400> 24

agcaaaagca ggggatattt ctgtcaatca tgaagacaac cattattttg atactactga 60
cccattgggt ctacagtcaa aacccaacca gtggaaacaa cacagccaca ttatgtctgg 120
gacaccatgc agtagcaaat ggaacattgg taaaaacaat aactgatgac caaattgagg 180
tgacaaatgc tactgaatta gttcagagca tttcaatagg gaaaatatgc aacaactcat 240
atagagttct agatggaaga aattgcacat taatagatgc aatgctagga gacccccact 300
gtgatgtctt ccagtatgag aattgggacc tcttcataga aagaagcagc gctttcagca 360
attgctaccc atatgacatc cctgactatg catcgctccg gtccattgta gcatcctcag 420
gaacattgga attcacagca gagggattca catggacagg tgtcactcaa aacggaagaa 480
gtggagcctg caaaagggga tcagccgata gtttctttag ccgactgaat tggctaacaa 540
aatctggaaa ctcttacccc acattgaatg tgacaatgcc taacaataaa aatttcgaca 600
aactatacat ctggggggatt catcacccga gctcaaacca aaagcagaca gaattgtaca 660
tccaagaatc aggacgagta acagtctcaa caaaaagaag tcaacaaacg ataatcccta 720
atatcggatc tagaccgtgg gtcaggggtc aatcaggcag gataagcata tactggacca 780
ttgtaaaacc tggagatatc ctaatgataa acagtaatgg caacttagtt gcaccgcggg 840
gatattttaa attgaaaaca gggaaaagct ctgtaatgag atcagatgca cccatagaca 900
tttgtgtgtc tgaatgtatt acaccaaatg gaagcatccc caacgacaaa ccatttcaaa 960
atgtgaacaa agttacatat ggaaaatgcc ccaagtatat caggcaaaac actttaaagc 1020

tggccactgg gatgaggaat gtaccagaaa agcaaatcag a 1061

<210> 25
<211> 1061
<212> DNA
<213> Influenza A virus

<400> 25

agcaaaagca ggggatattt ctgtcaatca tgaagacaac cattattttg atactactga 60
cccattgggt ctacagtcaa aacccaacca gtggaaacaa cacagccaca ttatgtctgg 120
gacaccatgc agtagcaaat ggaacattgg taaaaacaat aactgatgac caaattgagg 180
tgacaaatgc tactgaatta gttcagagca tttcaatagg gaaaatatgc aacaactcat 240
ataaagttct agatggaaga aattgcacat taatagatgc aatgctagga gaccccccact 300
gtgatgtctt ccagtatgag aattgggacc tcttcataga aagaagcagc gctttcagca 360
attgctaccc atatgacatc cctgactatg catcgctccg gtccattgta gcatcctcag 420
gaacattaga attcacagca gagggattca catggacagg tgtcactcaa aacggaagaa 480
gtggagcctg caaaagggga tcagccgata gtttctttag ccgactgaat tggctaacaa 540
aatctggaaa ctcttacccc acattgaatg tgacaatgcc taacaataaa aatttcgaca 600
aactatacat ctgggggatt catcacccga gctcaaacca acagcagaca gaattgtaca 660
tccaagaatc aggacgagta acagtctcaa caaaaagaag tcaacaaacg atagtcccta 720
atatcggatc tagaccgtgg gttaggggtc aatcaggcag gataagcata tactggacca 780
ttgtaaaacc tggagatatc ctaatgataa acagtaatgg caacttagtt gcaccgcggg 840
gatattttaa attgaaaaca gggaaaagct ctgtaatgag atcagatgca cccatagaca 900
tttgtgtgtc tgaatgtatt acaccaaatg gaagcatccc caacgacaaa ccatttcaaa 960
atgtgaacaa agttacatat ggaaaatgcc ccaagtatat caggcaaaac actttaaagc 1020
tggccactgg gatgaggaat ataccagaaa agcaaatcag a 1061

<210> 26
<211> 1763
<212> DNA
<213> Influenza A virus

<400> 26

agcaaaagca ggggatactt tctgtcaatc atgaagacaa ccattatttt gatactactg 60
acccattggg tctacagtca aaacccaacc agtggcaaca acacagccac actatgtctg 120
ggacaccatg cagtagcaaa tggaacattg gtaaaaacaa taactgatga ccaaattgag 180
gtgacaaatg ctactgaatt agttcagagc acttcaatag ggaaaatatg caacaaccca 240
tatagggttc tagatggaag aaactgcaca ttaatagatg caatgctagg agatccccac 300
tgtgatgttt ttcagtatga gaattgggac ctcttcatag aaagaagcag cgctttcagc 360
aattgctacc catatgacat ccctgactat gcatcgctcc ggtctattgt ggcatcttca 420
ggaacattag aattcacagc agagggattc acatggacag gtgtcactca aaacggagga 480
agtggagcct gcagaagggg gtcagccgat agtttcttta gccgactgaa ttggctaaca 540
aaatctggaa attcttaccc cacattgaat gtaacaatgc ctaacaataa caatttcgat 600
aaactataca tctgggggat ccatcacccg agcacaaaca atgagcagac aaaattgtat 660
atccaagaat cagggcgagt aacagtctca acaaaaagaa gtcaacaaac aataatcccc 720
aacatcggat ctagaccgtg ggtcaggggt caatcaggca ggataagcat atattggacc 780
attgtgaaac ctggagatat cctaatgata aacagtaatg gcaacttagt tgcaccgcgg 840
ggatatttta aaatgcgaac agggaaaagc tctgtaatga gatcagatgc acccatagac 900
acttgtgtgt ccgagtgtat tacaccaaat ggaagcatcc ccaacgacaa accatttcaa 960
aatgtgaaca aagttacata tggaaaatgc cccaagtata tcaagcagaa tactttgaag 1020
ctggccactg ggatgaggaa tgtaccagaa aagcaaatca gaggaatctt tggagcaata 1080
gcgggattca tagaaaacgg ctgggaagga atggttgatg ggtggtatgg attccgatat 1140
cagaattcgg aaggaacagg acaagctgca gatctaaaga gcactcaagc agccatcgac 1200
cagatcaatg gaaaattgaa cagagtgatt gaaaggacca atgagaaatt ccatcaaata 1260

gagaaggaat tctcagaagt agaagggaga atccaggact tggagaagta tgtagaagac 1320
accaaaatag acctatggtc ctacaatgca gagttactgg tggctctaga aaatcaacat 1380
acgattgact taacagatgc agaaatgaat aaattattcg agaagactag gcgccagtta 1440
agagaaaacg cggaagacat ggggggtgga tgtttcaaga tttatcacaa atgtgataat 1500
gcatgcattg gatcaataag aaatgggaca tatgaccatt acatatacag agatgaagca 1560
ttaaacaacc gatttcaaat taaaggtgtt gaattgaaat caggctacaa agattggata 1620
ctgtggattt cattcgccat atcatgcttc ttaatttgcg ttgttctatt gggtttcatc 1680
atgtgggctt gccaaaaagg caacatcaga tgcaacattt gcatttgagt aaactgataa 1740
ttaaaaacac ccttgtttct act 1763

<210> 27
<211> 1027
<212> DNA
<213> Influenza A virus

<400> 27

agcaaaagca ggtagatatt taaagatgag tcttctaacc gaggtcgaaa cgtacgttct 60
ctctattgta ccatcaggcc ccctcaaagc cgagatcgcg cagagacttg aagatgtctt 120
tgcagggaag aacaccgatc ttgaggcact catggaatgg ctaaagacaa gaccaatcct 180
gtcacctctg actaaaggga ttttaggatt tgtgttcacg ctcaccgtgc ccagtgagcg 240
aggactgcaa cgtagacgct ttgtccaaaa tgcccttagt ggaaacggag atccaaataa 300
catggacaga gcagtaaaac tgtacaagaa gcttaaaaga gaaataacat tccatggggc 360
aaaagaggtg gcactcagct attccactgg tgcactagcc agctgcatgg gactcatata 420
caacagaatg ggactgtga caaccgaagt ggcatttggc ctggtatgcg ccacatgtga 480
acagattgct gattcccagc atcgatctca caggcagatg gtgacaacaa ccaacccact 540
aatcagacat gaaaacagaa tggtactagc cagtaccaca gctaaaacca tggagcaggt 600
ggcagggtcg agtgagcagg cagcagaggc catggaggtt gctagtaagg ccaggcagat 660
ggtgcaggca atgaggacca ttgggaccca ccctagctcc agtgccggtt tgaaagatga 720
tcttcttgaa aatttgcagg cctaccagaa acggatggga gtgcaaatgc agcggttcaa 780
gtgatcctct cgttattgca gcaagtatca ttgggatctt gcacttgata ttgtggattc 840
ttgatcgcct tttcttcaaa ttcatttatc gtctccttaa atacggtttg aaaagagggc 900
cttctacgga aggagtacct gagtctatga gggaagaata tcggcaggaa cagcagaatg 960
ctgtggatgt tgacgatggt cattttgtca acatagagct ggagtaaaaa actaccttgt 1020
ttctact 1027

<210> 28
<211> 890
<212> DNA
<213> Influenza A virus

<400> 28

agcaaaagca gggtgacaaa aacataatgg attccaacac tgtgtcaagc tttcaggtag 60
actgttttct ttggcatgtc cgcaaacgat ttgcagacca agaactgggt gatgccccat 120
tccttgaccg gcttcgccga gaccagaagt ccctaaaagg aagaggcagc actcttggtc 180
tggacatcga aacagccact cgtgcaggaa agcagatagt ggagcggatt ctggaagagg 240
agtcagatga ggcacttaaa atgaccattg cctctgttcc tgcttcacgc tacttaactg 300
acatgactct tgatgagatg tcaagagact ggttcatgct catgcccaag cagaaagtaa 360
caggctccct atgtataagg atggaccagg caatcatgga taagaacatc atactaaaag 420
caaactttag tgtgattttc gaaaggctgg agacactaat actacttaga gctttcaccg 480
aagaaggagc agtcgttggc gaaatttcac cattgccttc tcttccagga catactaatg 540
aggatgtcaa aaatgcaatt ggggtcctca tcggaggact taaatggaat gataacacag 600
ttagaatctc tgaaactcta cagagattcg cttggagaag cagtcatgag aatgggagac 660
cttcattccc tccaaagcag aaacgaaaaa tggcgagaac aattgagtca gaagtttgaa 720
gaaataaggt ggttgattga agaagtgcga catagattga aaaatacaga aaatagtttt 780

gaacaaataa catttatgca agccttacaa ctattgcttg aagtagaaca agagataaga 840
actttctcgt ttcagcttat ttaatgataa aaaacaccct tgtttctact 890

<210> 29
<211> 982
<212> DNA
<213> Influenza A virus

<400> 29

atgagtcttc tgaccgaggt cgaaacgtac gttctctcta tcgtaccatc aggccccctc 60
aaagccgaga tcgcgcagag acttgaagat gtctttgcag ggaagaacac cgatcttgag 120
gcactcatgg aatggctaaa gacaagacca atcctgtcac ctctgactaa agggatttta 180
ggattcgtat tcacgctcac cgtgcccagt gagcgaggac tgcagcgtag acgctttgtc 240
caaaatgccc ttagtggaaa cggagatcca aacaacatgg acagagcagt aaaactgtac 300
aggaaactta aaagagaaat aacattccat ggggcaaaag aggtggcact cagctattcc 360
actggtgcac tagccagctg catgggactc atatacaaca gaatgggaac tgtgacaacc 420
gaagtggcat ttggcctagt atgcgccaca tgtgaacaga ttgctgattc ccagcatcga 480
tctcacaggc agatggtgac aacaaccaac ccattaatca gacatgaaaa cagaatggta 540
ttagccagta ccacggctaa agccatggag cagatggcag ggtcgagtga gcaggcagca 600
gaggccatgg aggttgctag taaggctagg cagatggtac aggcaatgag gaccattggg 660
acccacccta gctccagtgc cggtttgaaa aatgatctcc ttgaaaattt gcaggcctac 720
cagaaacgga tgggagtgca aatgcagcga ttcaagtgat cctctcgtta ttgcagcaag 780
tatcattggg atcttgcact tgatattgtg gattcttgat cgccttttct tcaaattcat 840
ttatcgtcgc cttaaatacg ggttgaaaag agggccttct acggaaggag tacctgagtc 900
tatgagggaa gaatatcggc aggaacagca gaatgctgtg gatgttgacg atggtcattt 960
tgtcaacata gagctggagt aa 982

<210> 30
<211> 982
<212> DNA
<213> Influenza A virus

<400> 30

atgagtcttc taaccgaggt cgaaacgtac gttctctcta tcgtaccatc aggccccctc 60
aaagccgaga tcgcgcagag acttgaagat gtctttgcag ggaagaacac cgatcttgag 120
gcactcatgg aatggctaaa gacaagacca atcctgtcac ctctgactaa agggatttta 180
ggatttgtgt tcacgctcac cgtgcccagt gagcgaggac tgcagcgtag acgctttgtc 240
caaaatgccc ttagtggaaa cggagatcca aacaacatgg acagagcagt aaaactgtac 300
aggaagctta aaagagaaat aacattccat ggggcaaaag aggtggcact cagctattcc 360
actggtgcac tagccagctg catgggactc atatacaaca gaatggggac tgtgacaacc 420
gaagtggcat ttggcctggt atgcgccaca tgtgaacaga ttgctgattc ccagcatcga 480
tctcacaggc agatggtgac aacaaccaac ccactaatca gacatgaaaa cagaatggta 540
ctagccagta ccacagctaa agccatggaa cagatggcag ggtcgagtga gcaggcagca 600
gaggccatgg aggttgctag taaggccagg cagatggtac aggcaatgag gaccattggg 660
acccacccta gctccagtgc cggtttgaaa gatgatcttc ttgaaaattt gcaggcctac 720
cagaaacgga tgggagtgca aatgcagcga ttcaagtgac cctctcgtta ttgcagcaag 780
tatcattggg atcttgcact tgatattgtg gattcttgat cgccttttct tcaaattcat 840
ttatcgtcgc cttaaatacg gtttgaaaag agggccttct acggaaggag tacctgagtc 900
tatgagggaa gaatatcggc aggaacagca gaatgctgtg gatgttgacg atggtcattt 960
tgtcaacata gagctggagt aa 982

<210> 31
<211> 838
<212> DNA
<213> Influenza A virus

<400> 31

atggattcca acactgtgtc aagctttcag gtagactgtt ttctttggca tgtccgcaaa 60
cgattcgcag accaagaact gggtgatgcc ccattccttg accggcttcg ccgagaccag 120
aagtccctaa aaggaagagg tagcactctt ggtctggaca tcgaaacagc cactcgtgca 180
ggaaagcaga tagtggagca gattctggaa gaggaatcag atgaggcact taaaatgacc 240
attgcctctg ttcctgcttc acgctactta actgacatga ctcttgatga gatgtcaaga 300
gactggttca tgctcatgcc caagcagaaa gtaacaggct ccctatgtat aagaatggac 360
caggcaatca tggataagaa catcatactt aaagcaaact ttagtgtgat tttcgaaagg 420
ctggaaacac taatactact tagagccttc accgaagaag gagcagtcgt tggcgaaatt 480
tcaccattgc cttctcttcc aggacatact aatgaggatg tcaaaaatgc aattggggtc 540
ctcatcggag gacttaaatg gaatgataat acggttagaa tctctgaaac tctacagaga 600
ttcgcttgga gaagcagtca tgagaatggg agaccttcat tccctccaaa gcagaaacga 660
aaaatggaga gaacaattga gccagaagtt tgaagaaata agatggttga ttgaagaagt 720
gcgacataga ttgaaaaata cagaaaatag ttttgaacaa ataacattta tgcaagcctt 780
acaactattg cttgaagtag aacaagagat aagaactttc tcgtttcagc ttatttaa 838

<210> 32
<211> 838
<212> DNA
<213> Influenza A virus

<400> 32

atggattcca acactgtgtc aagctttcag gtagactgtt ttctttggca tgtccgcaaa 60
cgatttgcag accaagaact gggtgatgcc ccattccttg accggcttcg ccgagaccag 120
aagtccctaa aaggaagagg cagcactctt ggtctggaca tcgaaacagc cactcgtgca 180
ggaaagcaga tagtggagca gattctggaa gaggaatcag atgaggcact taaaatgacc 240
attgcctctg ttcctgcttc acgctactta actgacatga ctcttgatga gatgtcaaga 300
gactggttca tgctcatgcc caagcagaaa gtaacaggct ccctatgtat aaggatggac 360
caggcaatca tggataagaa catcatacta aaagcaaact ttagtgtgat tttcgaaagg 420
ctggagacac taatactact tagagccttc accgaagaag gagcagtcgt tggcgaaatt 480
tcaccattgc cttctcttcc aggacatact aatgaggatg tcaaaaatgc aattggggtc 540
ctcatcggag gacttaaatg gaatgataat acagttagag tctctgaaac tctacagaga 600
ttcgcttgga gaagcagtca tgagaatggg agaccttcat tccctccaaa gcagaaacga 660
aaaatggcga gaacaattga gccagaagtt tgaagaaata agatggttga ttgaagaagt 720
gcgacataga ttgaaaaata cagaaaatag ttttgaacaa ataacattta tgcaagcctt 780
acaactattg cttgaagtag aacaagagat aagaactttc tcgtttcagc ttatttaa 838

<210> 33
<211> 1565
<212> DNA
<213> Influenza A virus

<400> 33

agcaaaagca gggtagataa tcactcactg agtgacatca aagtcatggc gtctcaaggc 60
accaaacgat cttatgagca gatggaaact ggtggggaac gccagaatgc aactgaaatc 120
agagcatctg tcggaaggat ggtgggagga atcggccggt tctatgttca gatgtgtact 180
gagcttaaac tcaacgacca tgaagggcgg ctgattcaga acagcataac aatagaaagg 240
atggtacttt cggcattcga cgaaagaaga aacaagtacc tcgaggagca tcccagtgct 300
gggaaagacc ccaagaaaac gggaggcccg atatacagaa ggaaagatgg gaaatggatg 360
agagaactca tcctccatga taaagaagaa atcatgagga tctggcgtca ggccaacaat 420
ggtgaagacg ctactgctgg tcttactcat atgatgatct ggcactccaa tctcaatgac 480

accacatacc aaagaacaag ggctcttgtt cgggctggga tggatcccag aatgtgctct 540
ctgatgcaag gatcaaccct cccacggaga tctggagctg ccggtgctgc agtaaaaggt 600
gttggaacaa tggtaatgga actcatcagg atgatcaaac gcgggataaa tgatcgaaat 660
ttctggagag gtgaaaatgg tcgaagaacc agaattgctt atgaaagaat gtgcaatatc 720
ctcaaaggga aattccaaac agcagcacaa cgggcaatga tggaccaagt gagggagggc 780
cgcaatcctg gaaatgctga gattgaggat ctcattttct tggcacgatc agcactcatt 840
ttgagaggat cagtagccca taaatcatgc ctacctgcct gtgtttatgg ccttgcagta 900
gccagtgggt atgactttga gaaggaagga tactctctgg ttggaattga tcctttcaaa 960
ctactccaga acagccaaat tttcagtcta atcagaccga aagaaaatcc agcacacaag 1020
agccagttgg tgtggatggc atgccattct gcagcatttg aggacctgag agttttgaat 1080
ttcattagag gaaccaaagt aatcccaaga ggacagttag caaccagagg agtgcaaatt 1140
gcttcaaatg aaaacatgga gacaatagat tctagcacac tcgaactgag gagcagatat 1200
tgggcaataa ggaccaggag tggagggaac accagtcaac agagagcatc tgcaggacag 1260
ataagtgtgc aacccacttt ctcagtgcag agaaatcttc cctttgaaag agcaaccatt 1320
atggctgcat tcactgggaa cactgagcgg aggacttccg acatgagaac ggaaatcata 1380
aggatgatgg aaaatgccag atcagaagat gtgtctttcc aggggcgggg agtcttcgag 1440
ctctcggacg aaaaggcaac gaacccgatc gtgccttcct ttgacatgag caatgaaggg 1500
tcttatttct tcggagacaa tgctgaggag tttgacagtt aaagaaaaat acccttgttt 1560
ctact 1565

<210> 34
<211> 2341
<212> DNA
<213> Influenza A virus

<400> 34

agcaaaagca ggtcaaatat attcaatatg gagagaataa aagaactgag agatctaatg 60
tcacagtccc gcacccgcga gatactaaca aaaactactg tggaccatat ggccataatc 120
aagaaataca catcaggaag acaagagaag aaccccgcac ttaggatgaa gtggatgatg 180
gcaatgaaat acccaattac agcagataag aggataatgg aaatgattcc tgagagaaat 240
gaacaggggc aaaccctttg gagcaaaacg aacgatgctg gctcagaccg cgtaatggta 300
tcacctctgg cagtgacatg gtggaatagg aatggaccaa caacgagcac aattcattat 360
ccaaaagtct acaaaactta ttttgaaaaa gttgaaaggt taaaacacgg aacctttggc 420
cccgttcatt ttaggaatca agtcaagata agacggagag ttgacgtaaa ccctggtcac 480
gcggacctca gtgccaaaga agcacaagat gtgatcatgg aagttgtttt cccaaatgaa 540
gtgggagcca gaattctaac atcggaatca caactaacaa taaccaaaga gaaaaaagaa 600
gaacttcagg actgcaaaat tgcccccttg atggtagcat acatgctaga aagagagttg 660
gtccgaaaaa caaggttcct cccagtggct ggcggaacaa gcagtgtata cattgaggtg 720
ttgcatctga ctcagggaac gtgctgggaa caaatgtaca ccccaggagg agaagttaga 780
aacgatgaca ttgatcaaag tttaattatt gctgcccgga acatagtgaa aagagcgaca 840
gtatcagcag atccactagc atccctgctg gagatgtgcc acagtacaca gattggtgga 900
ataaggatgg tagacatcct taagcagaat ccaacagagg aacaagctgt ggatatatgc 960
aaagcagcaa tggggttaag aattagctca tcattcagct ttggtggatt cacctttaag 1020
agaacaagtg gatcatcagt caagagagaa gaagaaatgc ttacgggcaa ccttcaaaca 1080
ttgaaaataa gagtgcatga gggctatgaa gaattcacaa tggtcggaag aagagcaaca 1140
gccattctca gaaagacaac cagaagattg attcaattga tagtaagtgg gagagatgaa 1200
cagtcaattg ctgaagcaat aattgtagcc atggtgtttt cgcaagaaga ttgcatgata 1260
aaagcagttc gaggcgattt gaacttcgtt aatagagcaa atcagcgctt gaaccccatg 1320
catcaactct tgaggcattt ccaaaaggat gcaaaagtgc ttttccagaa ttgggggatt 1380
gaacccatcg acaatgtgat gggaatgatc ggaatattgc ccgacatgac ccaagcacc 1440
gagatgtcat tgagaggagt gagagtcagc aaaatgggag tggatgagta ctccagcact 1500
gagagagtgg tggtgagcat tgaccgtttt ttaagagttc gggatcaaag gggaaacata 1560
ctactgtccc ctgaagaggt cagtgaaaca caaggaacgg aaaagctgac aataatttat 1620
tcatcatcaa tgatgtggga gattaatggt cccgagtcag tgttggtcaa tacttatcaa 1680
tggatcatca gaaactggga aattgtgaaa attcaatggt cacaggatcc cacaatgtta 1740

tacaataaga tagaatttga gccattccag tccctggtcc ctagggccac cagaagccaa 1800
tacagcggtt tcgtaaggac cctgtttcag caaatgcgag atgtacttgg aacatttgac 1860
actgctcaaa taataaaact cctccctttt gccgctgctc ctccggaaca gagtagaatg 1920
cagttctctt ctttgactgt taatgtaaga ggatcgggaa tgaggatact tgtaagaggc 1980
aattccccag tgttcaacta caacaaagcc actaagaggc tcacagtcct cggaaaggat 2040
gcaggtgcgc ttactgaaga cccagatgaa ggtacggctg gagtagaatc tgctgttctg 2100
agagggtttc tcatcttagg taaagaaaac aagagatatg gcccagcact aagcatcaat 2160
gaactgagca aacttacaaa aggggagaaa gctaatgtgc taattgggca aggggacgtg 2220
gtgttggtaa tgaaacggaa acgtgactct agcatactta ctgacagtca gacagcgacc 2280
aaaaggattc ggatggccat caattagtgt tgaattgttt aaaaacgacc ttgtttctac 2340
t 2341

<210> 35
<211> 1762
<212> DNA
<213> Influenza A virus

<220>
<221> modified_base
<222> (313)
<223> a, c, g, t, unknown or other

<400> 35

agcaaaagca ggggatattt ctgtcaatca tgaagacaac cattgttttg atactactga 60
cccattgggt ctacagtcaa aacccaacca gtggcaacaa cacagccaca ttatgtctgg 120
gacaccatgc agtagcaaat ggaacactgg taaaaacaat aactgatgac cagattgagg 180
tgacaaatgc tactgaatta gttcagagca tttcaatagg gaaaatatgc aacaactcat 240
atagggttct agatggaaga aattgcacat taatagatgc aatgctagga gaccccccact 300
gtgatgtttt tcngtatgag aattgggacc tcttcataga aagaagcagc gctttcagca 360
attgctaccc atatgacatc cctgactatg catcgctccg gtctattgtg gcatcctcag 420
gaacattaga attcacagca gagggattca catggacagg tgtcactcaa aacggaagaa 480
gtggagcctg caaaagggga tcagccgata gtttctttag ccgactgaat tggctaacaa 540
aatctggaaa ttcttacccc acattgaatg tgacaatgcc taacaataac aatttcgata 600
aactatacat ctgggggatt catcacccga gctcaaacaa tgagcagaca aaattgtata 660
tccaagaatc aggacgagta acagtctcaa caaaaagaag tcaacaaaca ataatcccca 720
acatcggatc tagaccgtgg gtcaggggtc aatcaggcag gataagcata tattggacca 780
ttgtgaaacc tggagatatc ctaatgataa acagtaatgg caacttagtt gcaccgcggg 840
gatatttcaa attgagaaca gggaaaagct ctgtaatgag atcagatgca cccatagaca 900
cttgtgtgtc tgaatgtatt acaccaaatg gaagcatccc caacgacaaa ccattccaaa 960
atgtgaacaa agttacatat ggaaaatgcc ccaagtatat caggcaaaac actttgaagc 1020
tggccactgg gatgaggaat gtaccagaaa agcaaatcag aggaatcttt ggagcaatag 1080
cgggattcat agaaaacggc tgggaaggaa tggttgatgg gtggtatgga ttccgatatc 1140
aaaattcgga aggaacagga caagctggag atctaaagag cactcaagca gccatcgacc 1200
agatcaatgg aaaattaaac agagtgattg aaaggaccaa tgagaaattc catcaaatag 1260
agaaggaatt ctcagaagta gaagggagaa tccaggactt ggagaagtat gtagaagaca 1320
ccaaaataga cctatggtcc tacaatgcag aattgctggt ggctctagaa aatcaacata 1380
caattgactt aacagatgca gaaatgaata aattattcga gaagactagg cgccagttaa 1440
gagaaaacgc ggaagacatg ggaggtggat gtttcaggat ttaccacaaa tgtgataatg 1500
catgcattgg atcaataaga aatgggacat atgaccatta catatacaga gatgaagcat 1560
taaacaaccg atttcaaatt aaaggtgttg agttgaaatc aggctacaaa gattggatac 1620
tgtggatttc attcgccata tcatgcttct taatttgcgt tgttctattg ggtttcatta 1680
tgtgggcttg ccaaaaaggc aacatcagat gcaacatttg catttgagta aactgatagt 1740
taaaaacacc cttgtttcta ct 1762

<210> 36
<211> 1762
<212> DNA
<213> Influenza A virus

<220>
<221> modified_base
<222> (313)
<223> a, c, g, t, unknown or other

<400> 36

agcaaaagca ggggatattt ctgtcaatca tgaagacaac cattattttg atactactga 60
cccattgggt ctacagtcaa aacccaacca gtggcaacaa cacagccaca ttatgtctgg 120
gacaccatgc agtagcaaat ggaacattgg taaaaacaat aactgatgac caaattgagg 180
tgacaaatgc tactgaatta gttcagagca tttcaatagg gaaaatatgc aacaactcat 240
atagggttct agatggaaga aattgcacat taatagatgc aatgctagga gaccccccact 300
gtgatgtttt tcngtatgag aattgggacc tcttcataga aagaagcagc gcttccagca 360
attgctaccc atatgacatc cctgactatg catcgctccg gtctattgtg gcatcctcag 420
gaacattaga attcacagca gagggattca catggacagg tgtcactcaa aacggaagaa 480
gtggagcctg caaaagggga tcagccgata gtttctttag ccgactgaat tggctaacaa 540
aatctggaaa ttcttacccc acattgaatg tgacaatgcc taacaataac aatttcgata 600
agctatacat ctgggggatc catcacccga gctcaaacaa tgagcagaca aaattgtata 660
tccaagaatc aggacgagta acagtctcaa caaaaagaag tcaacaaaca ataatcccca 720
acatcggatc tagaccgtgg gtcaggggtc aatcaggcag gataagcata tattggacca 780
ttgtgaaacc tggagatatc ctaataataa acagtaatgg caacttagtt gcaccgcggg 840
gatatttcaa attgcgaaca gggaaaagct ctgtaatgag atcagatgca cccatagaca 900
cttgtgtgtc tgaatgtatt acaccaaatg gaagcatccc caacgacaaa ccatttcaaa 960
atgtgaacaa agttacatat ggaaaatgcc ccaagtatat caggcaaaac actttgaagc 1020
tggccactgg gatgaggaat gtaccagaaa agcaaatcag aggaatcttt ggagcaatag 1080
cgggattcat agaaaacggc tgggaaggaa tggttgatgg gtggtatgga ttccgatatc 1140
aaaactcgga aggaacagga caagctggag atctaaagag cactcaagca gccatcgacc 1200
agatcaatgg aaaattgaac agagtgattg aaaggaccaa tgagaaattc catcaaatag 1260
agaaggaatt ctcagaagta gaagggagaa tccaggactt ggagaagtat gtagaagaca 1320
ccaaaataga cctatggtcc tacaatgcag agttgctggt ggctctagaa aatcaacata 1380
caattgactt aacagatgca gaaatgaata aactattcga gaagactagg cgccagttaa 1440
gagaaaacgc ggaagacatg ggaggtggat gtttcaagat ttatcacaaa tgtgataatg 1500
catgcattgg atcaataaga aatgggacat atgaccatta catatacaga gatgaagcat 1560
taaacaaccg atttcaaatt aaaggtgtag agctgaaatc aggctacaaa gattggatac 1620
tgtggatttc attcgccata tcatgcttct taatttgcgt tgttctattg ggtttcatta 1680
tgtgggcttg ccaaaaaggc aacatcagat gcaacatttg catttgagta aactgatagt 1740
taaaaacacc cttgtttcta ct 1762

<210> 37
<211> 693
<212> DNA
<213> Influenza A virus

<400> 37

atggattcca acactgtgtc aagctttcag gtagactgtt ttctttggca tgtccgcaaa 60
cgatttgcag accaagaact gggtgatgcc ccattccttg accggcttcg ccgagaccag 120
aagtccctaa aaggaagagg tagcactctt ggtctggaca tcgaaacagc cactcgtgca 180
ggaaagcaga tagtggagca gattctggaa gaggaatcag atgaggcatt taaaatgacc 240
attgcctctg ttcctgcttc acgctactta actgacatga ctcttgatga gatgtcaaga 300
gactggttca tgctcatgcc caagcagaaa gtaacaggct ccctatgtat aagaatggac 360
caggcaatca tggataagaa catcatactt aaagcaaact ttagtgtgat tttcgaaagg 420

ctggagacac taatactact cagggccttc accgaagaag gagcagtcgt tggcgaaatt 480
tcaccattgc cttctcttcc aggacatact aatgaggatg tcaaaaatgc aattggggtc 540
ctcatcggag gacttaaatg gaatgataat acggttagag tctctgaaac tctacagaga 600
ttcgcttgga gaagcagtca tgagaatggg agaccttcat tccctccaaa gcagaaacga 660
aaaatggaga gaacaattga gtcagaagtt tga 693

<210> 38
<211> 1032
<212> DNA
<213> Influenza A virus

<400> 38

```
atgaagacaa ccattatttt gatactactg acccattggg tctacagtca aaacccaacc 60
agtggcaaca acacagccac attatgtctg ggacaccatg cagtagcaaa tggaacattg 120
gtaaaaacaa taactgatga ccaaattgag gtgacaaatg ctactgaatt agtccagagc 180
atttcaatag ggaaaatatg caacaactca tatagggttc tagatggaag aaattgcaca 240
ttaatagatg caatgctagg agaccccat tgtgatgatt ttcagtatga gaattgggac 300
ctcttcatag aaagaagcag cgctttcagc aattgctacc catatgacat ccctgactat 360
gcatcgctcc ggtccattgt agcatcctca ggaacattag aattcacagc agaggggttc 420
acatggacag gtgtcactca aaacggagga agtggagcct gcaaaagggg atcagccgat 480
agtttcttta gccgactgaa ttggctaaca aaatctggaa attcttaccc catattgaat 540
gtgacaatgc ctaacaataa aaatttcgat aaactataca tctgggggat tcatcacccg 600
agctcaaaca aagagcagac aaaattatat atccaagaat caggacgagt aacagtctca 660
acagaaagaa gtcaacaaac agtaatccct aacatcggat ctaggccgtg ggtcaggggt 720
caatcaggca ggataagcat atactggacc attgtaaaac ctggagatat tctaatgata 780
aacagtaatg gcaacttagt tgcaccgcgg ggatatttta aattgagaac agggaaaagc 840
tctgtaatga gatcagatgc actcatagac acttgtgtgt ctgaatgtat tacaccaaat 900
ggaagcatcc ccaacgacaa accatttcaa aatgtgaaca aaattacata tggaaaatgc 960
cccaagtata tcaggcaaaa cactttaaag ctggccactg ggatgaggaa tgtaccagaa 1020
aagcaaatca ga 1032
```

<210> 39
<211> 1032
<212> DNA
<213> Influenza A virus

<400> 39

```
atgaagacaa ccattatttt gatactactg acccattggg tctacagtca aaacccaacc 60
agtggcaaca acacagccac attatgtctg ggacaccatg cagtagcaaa tggaacattg 120
gtaaaaacaa taactgatga ccaaattgag gtgacaaatg ctactgaatt agttcagagc 180
atttcaatag ggaaaatatg caacaactca tatagagttc tagatggaag aaattgcaca 240
ttaatagatg caatgctagg agaccccac tgtgatgtct ttcagtatga gaattgggac 300
ctcttcatag aaagaagcag cgctttcagc aattgctacc catatgacat ccctgactat 360
gcatcgctcc ggtccattgt agcatcctca ggaacattgg aattcacagc agagggattc 420
acatggacag gtgtcactca aaacggaaga agtggagcct gcaaaagggg atcagccgat 480
agtttcttta gccgactgaa ttggctaaca aaatctggaa actcttaccc cacattgaat 540
gtgacaatgc ctaacaataa aaatttcgac aaactataca tctgggggat tcatcacccg 600
agctcaaacc aacagcagac agaattgtac atccaagaat caggacgagt aacagtctca 660
acaaaaagaa gtcaacaaac gataatccct aatatcggat ctagaccatg ggtcaggggt 720
caatcaggca ggataagcat atactggacc attgtaaaac ctggagatat cctaatgata 780
aacagtaatg gcaacttagt tgcaccgcgg ggatatttta aattgaaaac agggaaaagc 840
tctgtaatga gatcagatgc acccatagac atttgtgtgt ctgaatgtat tacaccaaat 900
ggaagcatcc ccaacgacaa accatttcaa aatgtgaaca aagttacata tggaaaatgc 960
cccaagtata tcaggcaaaa cactttaaag ctggccactg ggatgaggaa tgtaccagaa 1020

aagcaaatca ga 1032
```

<210> 40
<211> 1032
<212> DNA
<213> Influenza A virus

<400> 40

atgaagacaa ccattatttt gatactactg acccattggg tctacagtca aaacccaacc 60
agtggcaaca acacagccac attatgtctg ggacaccatg cagtagcaaa tggaacattg 120
gtaaaaacaa taactgatga ccaaattgag gtgacaaatg ctactgaatt agttcagagc 180
atttcaatag ggaaaatatg caacaactca tatagggttc tagatggaag aaattgcaca 240
ttaatagatg caatgctagg agacccccac tgtgatgttt ttcagtatga gaattgggac 300
ctcttcatag aaagaagcag cgctttcagc aattgctacc catatgacat ccctgactat 360
gcatcgctcc ggtccattgt agcatcctca ggaacattag aattcacagc agagggattc 420
acatggacag gtgtcactca aaacggaaga agtggagcct gcaaaagggg atcagccgat 480
agtttcttta gccgactgaa ttggctaaca aaatctggaa attcttaccc catattgaat 540
gtgacaatgc ctaacaataa aaatttcgat aaactataca tctgggggat tcatcacccg 600
agctcaaaca aagagcagac aaaattgtat atccaagaat caggacgagt aacagtctca 660
acagaaagaa gtcaacaaac agtaatccct aacatcggat ctagaccgtg ggtcaggggt 720
caatcaggca ggataagcat atactggacc attgtaaaac ctggagatat tctaatgata 780
aacagtaatg gcaacttagt tgcaccgcgg ggatatttta aattgagaac agggaaaagc 840
tctgtaatga gatcagatgc actcataggc acttgtgtgt ctgaatgtat tacaccaaat 900
ggaagcatcc ccaacgacaa accatttcaa aatgtgaaca aagttacata tggaaaatgc 960
cccaagtata tcaggcaaaa cactttaaag ctggccactg ggatgaggaa tgtaccagaa 1020
aagcaaatca ga 1032

<210> 41
<211> 1032
<212> DNA
<213> Influenza A virus

<400> 41

atgaagacaa ccattatttt gatactactg acccattggg tctacagtca aaacccaacc 60
agtggcaaca acacagccac attatgtctg ggacaccatg cagtagcaaa tggaacattg 120
gtaaaaacaa taactgatga ccaaattgag gtgacaaatg ctactgaatt agttcagagc 180
atttcaatag ggaaaatatg caacaactca tatagggttc tagatggaag aaattgcaca 240
ttaatagatg caatgctagg agacccccat tgtgatgatt ttcagtatga gaattgggac 300
ctcttcatag aaagaagcag tgctttcagc aattgctacc catatggcat ccctgactat 360
gcatcgctcc ggtccattgt agcatcctca ggaacattag aattcacagc agaggggttc 420
acatggacag gtgtcactca aaacggaaga agtggagcct gcaaaagggg atcagccgat 480
agtttcttta gccgactcaa ttggctaaca aaatctggaa attcttaccc catattgaat 540
gtgacaatgc ctaacaataa aaatttcgat aaactataca tctgggggat tcatcacccg 600
agctcaaaca aagagcagac aaaattatat atccaagaat caggacgagt aacagtctca 660
acagaaagaa gtcaacaaac agtaatccct aacatcggat ctaggccgtg ggtcaggggt 720
caatcaggca ggataagcat atactggacc attgtaaaac ctggagatat tctaatgata 780
aacaataatg gcaacttagt tgcaccgcgg ggatatttta aattgagaac agggaaaagc 840
tctgtaatga gatcagatgc actcatagac acttgtgtgt ctgaatgtat tacaccaaat 900
ggaagcatcc ccaacgacaa accatttcaa aatgtgaaca aagttacata tggaaaatgc 960
cccaagtata tcaggcaaaa cactttaaag ctggccactg ggatgaggaa tataccagaa 1020
aagcaaatca ga 1032

<210> 42
<211> 1032
<212> DNA
<213> Influenza A virus

<400> 42

atgaagacaa ccattatttt gatactactg acccattggg tctacagtca aaacccaacc 60
agtggcaaca acacagccac attatgtctg ggacaccatg cagtagcaaa tggaacattg 120
gtaaaaacaa taactgatga ccaaattgag gtgacaaatg ctactgaatt agttcagagc 180
atttcaatag ggaaaatatg caacaactca tatagggttc tagatggaag aaattgcaca 240
ttaatagatg caatgctagg agacccccac tgtgatgttt ttcagtatga gaattgggac 300
ctcttcatag aaagaagcag cgctttcagc aattgctacc catatgacat ctctgactat 360
gcatcgctcc ggtccattgt agcatcctca ggaacattag aattcacagc agagggattc 420
acatggacag gtgtcactca aaacggaaga agtggagcct gcaaaagggg atcagccgat 480
agtttcttta gccgactgaa ttggctaaca aaatctggaa attcttaccc catattgaat 540
gtgacaatgc ctaacaataa aaatttcgat aaactataca tctgggggat tcatcacccg 600
agctcaaaca aagagcagac aaaattgtat atccaagaat caggacgagt aacagtctca 660
acagaaagaa gtcaacaaac agtaatccct aacatcggat ctagaccgtg ggtcaggggt 720
caatcaggca ggataagcat atactggacc attgtaaaac ctggagatat tctaatgata 780
aacagtaatg gcaacttagt tgcaccgcgg ggatatttta aattgagaac agggaaaagc 840
tctgtaatga gatcagatgc acccataggc acttgtgtgt ctgaatgtat tacaccaaat 900
ggaagcatcc ccaacgacaa accatttcaa aatgtgaaca aagttacata tggaaaatgc 960
cccaagtata tcaggcaaaa cactttaaag ctggccactg ggatgaggaa tgtaccagaa 1020
aagcaaatca ga 1032

<210> 43
<211> 1032
<212> DNA
<213> Influenza A virus

<400> 43

atgaagacaa ccattatttt gatactactg acccattggg tctacagtca aaacccaacc 60
agtggcaaca acacagccac attatgtctg ggacaccatg cagtagcaaa tggaacattg 120
gtaaaaacaa taactgatga ccaaattgag gtgacaaatg ctactgaatt agttcagagc 180
atttcaatag ggaaaatatg caacaactca tatagagttc tagatggaag aaattgcaca 240
ttaatagatg caatgctagg agacccccac tgtgatgtct ttcagtatga gaattgggac 300
ctcttcatag aaagaagcag cgctttcagc aattgctacc catatgacat ccctgactat 360
gcatcgctcc ggtccattgt agcatcctca ggaacattag aattcacagc agagggattc 420
acatggacag gtgtcactca aaacggaaga agtggagcct gcaaaagggg atcagccgat 480
agtttcttta gccgactgaa ttggctaaca aaatctggaa attcttaccc catattgaat 540
gtgacaatgc ctaacaataa aaatttcgat aaactataca tctgggggat tcatcacccg 600
agctcaaaca aagagcagac aaaattgtac atccaagaat caggacgagt aacagtctca 660
acaaaaagaa gtcaacaaac aataatccct aacatcggat ctagaccgtg ggtcaggggt 720
caatcaggca ggataagcat atactggacc attgtaaaac ctggagatat cctaatgata 780
aacagtaatg gcaacttagt tgcaccgcgg ggatatttta aattgagaac agggaaaagc 840
tctgtaatga gatcagatgc acccatagac atttgtgtgt ctgaatgtat tacaccaaat 900
ggaagcatcc ccagcgacaa accatttcaa aatgtaaaca aagttacata tggaaaatgc 960
cccaagtata tcaggcaaaa cactttaaag ctggccactg ggatgaggaa tgtaccagaa 1020
aagcaaatca ga 1032

<210> 44
<211> 1742
<212> DNA
<213> Influenza A virus

<400> 44

ctgtcaatca tgaagacaac cattattttg atactactga cccattgggt ctacagtcaa 60
aacccaacca gtggcaacaa cacagccaca ttatgtctgg gacaccatgc agtagcaaat 120
ggaacattgg taaaaacaat aactgatgac caaattgagg tgacaaatgc tactgaatta 180
gttcagagca tttcaatagg gaaaatatgc aacaactcat atagggttct agatggaaga 240
aattgcacat taatagatgc aatgctagga gacccccact gtgatgtttt tcagtatgag 300
aattgggacc tcttcataga aagaagcagc gctttcagca attgctaccc atatgacatc 360
cctgactatg catcgctccg gtccattgta gcatcctcag gaacattaga attcacagca 420
gagggattca catggacagg tgtcactcaa aacggaagaa gtggagcctg caaaagggga 480
tcagccgata gtttctttag ccgactgaat tggctaacaa aatctggaaa ttcttacccc 540
atattgaatg tgacaatgcc taacaataaa aatttcgata aactatacat ctggggggatt 600
catcacccga gctcaaacaa agagcagaca aaattgtata tccaagaatc aggacgagta 660
acagtctcaa cagaaagaag tcaacaaaca gtaatcccta acatcggatc tagaccgtgg 720
gtcaggggtc aatcaggcag gataagcata tactggacca ttgtaaaacc tggagatatt 780
ctaacgataa acagtaatgg caacttagtt gcaccgcggg gatattttaa attgagaaca 840
gggaaaagct ctgtaatgag atcagatgca cccatagaca cttgtgtgtc tgaatgtatt 900
acaccaaatg gaagcatccc caacgacaaa ccatttcaaa atgtgaacaa agttacatat 960
ggaaaatgcc ccaagtatat caggcaaaac actttaaagc tggccaccgg gatgaggaat 1020
gtaccagaaa agcaaatcag aggaatcttt ggagcaatag cgggattcat agaaaacggc 1080
tgggaaggaa tggttgatgg gtggtatgga ttccgatatc aaaactcgga aggaacagga 1140
caagctgcag atctaaagag cactcaagca gccatcgacc agatcaatgg aaaattaaac 1200
agagtgattg aaaggaccaa tgagaaattc catcaaatag agaaggaatt ctcagaagta 1260
gaagggagaa tccaggattt ggagaagtat gtagaagaca ccaaaataga cctatggtcc 1320
tacaatgcag aattgctggt ggctctagaa aatcaacata caattgactt aacagatgca 1380
gaaatgaata aattattcga gaagactagg cgccagttaa gagaaaacgc ggaagacatg 1440
ggaggtggat gtttcaagat ttaccacaaa tgtgataatg catgcattgg atcaataaga 1500
aatgggacat atgaccatta catatacaga gatgaagcat taaacaaccg atttcaaatc 1560
aaaggtgttg agttgaaatc aggctacaaa gattggatac tgtggatttc attcgccata 1620
tcatgcttct taatttgcgt tgttctattg ggtttcatta tgtgggcttg ccaaaaaggc 1680
aacatcagat gcaacatttg catttgagta aactgatagt taaaaacacc cttgtttcta 1740
ct 1742

<210> 45
<211> 1761
<212> DNA
<213> Influenza A virus

<220>
<221> modified_base
<222> (1)..(19)
<223> a, c, g, t, unknown or other

<220>
<221> modified_base
<222> (1760)..(1761)
<223> a, c, g, t, unknown or other

<400> 45

nnnnnnnnnn nnnnnnnnna aatgaacact cagattctaa tattagccat ttcggcattc 60
ctctgtgtac gtgcagataa aatctgccta ggacatcatg ctgtgtctaa tggaaccaaa 120
gtagacaccc ttactgaaaa gggaatagaa gtcgtcaatg caacagaaac agttgaacaa 180
aaaaacatcc ccaagatctg ctcaaaaggg aaacagacta ttgaccttgg tcaatgtgga 240
ttactaggga ccactattgg tcccccccaa tgcgaccaat ttcttgaatt ctctgctaat 300
ttaataattg agagaagaga aggtgatgat atttgttatc caggcaaatt tgacaatgaa 360
gaaacattga gacaaatact cagaaaatcc ggaggaatta aaaaggagaa tatgggattc 420

acatataccg gagtgagaac caatggagag actagcgcct gtagaaggtc aagatcttcc 480
ttttatgcag aaatgaaatg gctcctatct aacacagaca atggggtatt cccacaaatg 540
acaaaatcct acaagaacac taagaaggag ccagctctga taatctgggg aatccaccac 600
tcaggatcaa ctgctgaaca gactagattg tatggaagtg gaaacaagtt gataacagtt 660
tggagttcca aataccaaca atcttttgcc ccaaaccctg gaccaaggcc gcaaatgaat 720
ggccaatcag gaagaattga cttttactgg ctgatgttag atcccaatga tactgttaat 780
ttcagtttta atggggcctt tatagcacct gaccgcgcca gttttctaag aggtaaatct 840
ctaggaattc agagtgacgc acaacttgac aacaattgtg aaggtgaatg ttatcatatt 900
ggaggtacca taattagcaa cttgcccttt caaaacatta atagcagagc aattgggaaa 960
tgccccagat acgtaaagca aaaaagctta atgctagcaa ccggaatgaa aaatgttcct 1020
gaaaattcta cacacaaaca gttaactcat cacatgcgca aaaaaagagg tttatttggt 1080
gcaatagcag gatttattga aaatggatgg gaaggattaa tagatggatg gtatggatac 1140
agacatcaga atgcacaagg agaaggaact gctgcagact acaaaagtac acaatctgct 1200
gtcaatcaaa taaccgggaa attaaacaga ctaatagaaa aaaccaacca gcaatttgaa 1260
ctaatagata atgaattcaa tgaaatagaa aagcaaattg gcaatgttat taactggact 1320
agagattcta tcatcgaaat atggtcatat aatgcagaat tcctcgtggc agtggagaat 1380
caacacacta ttgatttaac tgattcagag atgaacaaat tatatgaaaa ggtaagaaga 1440
cagctgagag aaaatgctga ggaagatggt aatggctgtt ttgaaatatt tcaccaatgt 1500
gacaatgatt gcatggccag cattagaaac aatacatatg atcataaaaa atacagaaag 1560
gaggcaatac aaaacagaat tcagattgat gcagtaaagt tgagcagcgg ttacaaagaa 1620
ataatacttt ggtttagctt cggggcatca tgtttcttat ttcttgccat tgcaatggtt 1680
cttgctttca tatgcataaa aaatggaaac atgcggtgca ctatttgtat ataagtttga 1740
aaaaacaccc ttgtttctan n 1761

<210> 46
<211> 1096
<212> DNA
<213> Influenza A virus

<400> 46

agtcaaaacc caaccagtgg caacaacaca gccacattat gtctgggaca ccatgcagta 60
gcaaatggaa cattggtaaa aacaataact gatgaccaaa ttgaggtgac aaatgctact 120
gaattagttc agagcatttc aatagggaaa atatgcaaca actcatatag ggttctagat 180
ggaagaaatt gcacattaat agatgcaatg ctaggagacc cccactgtga tgtttttcag 240
tatgagaatt gggacctctt catagaaaga agcagcgctt tcagcaattg ctacccatat 300
gacatccctg actatgcatc gctccggtcc attgtagcat cctcaggaac attagaattc 360
acagcagagg gattcacatg gacaggtgtc actcaaaacg gaagaagtgg agcctgcaaa 420
aggggatcag ccgatagttt ctttagccga ctgaattggc taacaaaatc tggaaattct 480
taccccatat tgaatgtgac aatgcctaac aataaaaatt tcgataaact atacatctgg 540
gggattcatc acccgagctc aaacaaagag cagacaaaat tgtatatcca agaatcagga 600
cgagtaacag tctcaacaga aagaagtcaa caaacagtaa tccctaacat cggatctaga 660
ccgtgggtca ggggtcaatc aggcaggata agcatatact ggaccattgt aaaacctgga 720
gatattctaa tgataaacag taatggcaac ttagttgctc cgcggggata ttttaaattg 780
agaacaggga aaagctctgt aatgagatca gatgcaccca tagacacttg tgtgtctgaa 840
tgtattacac caaatggaag catccccaac gacaaaccat ttcaaaatgt gaacaaagtt 900
acatatggaa aatgccccaa gtatatcagg caaaacactt taaagctggc cactgggatg 960
aggaatgtac cagaaaagca aatcagagga atctttggag caatagaggg attcatagaa 1020
aacggctggg aaggaatggt tgatgggtgg tatggattcc gatatcaaaa ctcggaagga 1080
acaggacaag ctgcag 1096

<210> 47
<211> 1086
<212> DNA
<213> Influenza A virus

<400> 47

ctgtcaatca tgaagacaac cattattttg atactactga cccattgggt ctacagtcaa 60
aacccaacca gtggcaacaa cacagccaca ctatgtctgg gacaccatgc agtagcaaat 120
ggaacattgg taaaaacaat aactgatgac caaattgagg tgacaaatgc cactgaatta 180
gttcagagca cttcaatagg gaaaatatgc aacaacccat atagggttct agatggaaga 240
aactgcacat taatagatgc aatgctagga gatccccact gtgatgtttt tcagtatgag 300
aattgggacc tcttcataga aagaagcagc gctttcagca attgctaccc atatgacatc 360
cctgactatg catcgctccg gtctattgtg gcatcttcag gaacattaga attcacagca 420
gagggattca catggacagg tgtcactcaa aacggaagaa gtggcgcctg cagaaggga 480
tcagccgata gtttctttag ccgactgaat tggctaacaa aatctggaga ttcttacccc 540
acattgaatg tgacaatgcc taacaataac aatttcgata aactatacat ctgggggatc 600
catcacccga gcacaaacaa tgagcagaca aaattgtatg tccaagaatc agggcgagta 660
acagtctcaa caaaaagaag tcaacaaaca ataatcccca acatcggatc tagaccgtgg 720
gtcaggggtc aaccaggcag gataagcata tattggacca ttgtgaaacc tggagatatc 780
ctaatgataa acagtaatgg caacttagtt gcaccgcggg gatatttcaa aatgcgaaca 840
ggaaaaagct ctataatgag atcagatgca cccatagaca cttgtgtgtc cgagtgtatt 900
acaccaaatg gaagcatccc caacgacaaa ccatttcaaa atgtgaacaa agttacatat 960
gggaaatgcc ccaagtatat caagcagaat actttgaagc tggccactgg gatgaggaat 1020
gtaccagaaa agcaaatcag aggaatcttt ggagcaatag cgggattcat agaaaatggc 1080
tgggag 1086

<210> 48
<211> 982
<212> DNA
<213> Influenza A virus

<400> 48

atgagtcttc tgaccgaggt cgaaacgtac gttctctcta tcgtaccatc aggccccctc 60
aaagccgaga tcgcgcagag acttgaagat gtctttgcag gaaagaacac cgatcttgag 120
gcactcatgg aatggctaaa gacaagacca atcctgtcac ctctgactaa ggggatttta 180
ggatttgtgt tcacgctcac cgtgcccagt gagcgaggac tgcagcgtag acgctttgtc 240
caaaatgccc ttaatgggaa cggagatcca aacaacatgg acagagcagt aaaactgtac 300
aggaagctta aaagggaaat aacattccat ggggcaaaag aggtggcact cagctattcc 360
actggtgcac tagccagctg catgggactc atatacaaca gaatggggac tgtgacaacc 420
gaagtggcat ttggcctggt atgcgccaca tgtgaacaga ttgctgattc ccagcaccga 480
tctcacagac agatggtgac aacaaccaac ccactaatca gacacgagaa cagaatggta 540
ctagccagta ccacagctaa agccatggag cagatggcag ggtcgagtga gcaggcagca 600
gaggccatgg aggttgctag tcaggccagg cagatggtgc aggcaatgag aaccattggg 660
acccacccta gctccagtgc cggtttgaaa aatgatcttc ttgaaaattt gcaggcctac 720
cagaaacgga tgggagtgca aatgcagcga ttcaagtgat cctctcgtta ttgcagcaag 780
tatcattggg atcttgcact tgatattgtg gattcttgat cgtcttttct tcaaatgcat 840
ttatcgtcgt cttaaatacg gtttgaaaag agggccttct acggaaggag tacctgagtc 900
tatgagggaa gaatatcggc aggaacagca gagtgctgtg gatgttgacg atggtcattt 960
tgtcaacata gagctggagt aa 982

<210> 49
<211> 982
<212> DNA
<213> Influenza A virus

<400> 49

atgagtcttc taaccgaggt cgaaacgtac gttctctcta tcgtaccatc aggccccctc 60
aaagccgaga tcgcgcagag acttgaagat gtctttgcag ggaagaacac cgatcttgag 120

gcactcatgg aatggctaaa gacaagacca atcctgtcac ctctgactaa agggatttta 180
ggatttgtgt tcacgctcac cgtgcccagt gagcgaggac tgcagcgtag acgctttgtc 240
caaaatgccc ttagtggaaa cggagatcca aacaacatgg acagagcagt aaaactgtac 300
aggaagctta aaagagaaat aacattccat ggggcaaaag aggtggcact cagctattcc 360
actggtgcac tagccagctg catgggactc atatacaaca gaatggggac tgtgacaacc 420
gaagtggcat ttggcctggt atgcgccaca tgtgaacaga ttgctgattc ccagcatcga 480
tctcacaggc agatggtgac aacaaccaac ccactaatca gacatgaaaa cagaatggta 540
ctagccagta ccacagctaa agccatggag cagatggcag ggtcgagtga gcaggcagca 600
gaggccatgg aggttgctag taaggccagg cagatggtac aggcaatgag gaccattggg 660
acccacccta gctccagtgc cggtttgaaa gatgatcttc ttgaaaattt gcaggcctac 720
cagaaacgga tgggagtgca aatgcagcga ttcaagtgat cctctcgtta ttgcagcaag 780
tatcattggg atcttgcact tgatattgtg gattcttgat cgcctttttct tcaaattcat 840
ttatcgtcgc cttaaatacg gtttgaaaag agggccttct acggaaggag tacctgagtc 900
tatgagggaa gaatatcggc aggaacagca gaatgctgtg gatgttgacg atggtcattt 960
tgtcaacata gagctggagt aa 982

<210> 50
<211> 838
<212> DNA
<213> Influenza A virus

<400> 50

atggattcca acactgtgtc aagctttcag gtagactgtt ttctttggca tgtccgcaaa 60
cgatttgcag accaagaact gggtgatgcc ccattccttg accggcttcg ccgagaccag 120
aagtccctaa aaggaagagg cagcactctt ggtctggaca tcgaaacagc cactcatgca 180
ggaaagcaga tagtggagcg aattctggaa gaggaatcag atgaggcact taaaatgacc 240
atagcctctg ttcctacttc acgctactta actgacatga ctcttgatga gatgtcaaga 300
gactggttca tgctcatgcc caagcagaaa gtaacaggct ccctatgtat aaggatggac 360
caagcaatca tggataagaa catcatacta aaagcaaact ttagtgtgat tttcgaaagg 420
ctggagacac taatactact tagagctttc accgaagaag gagcagtcgt tggcgaaatt 480
tcaccattgc cttctcttcc aggacatact aatgaggatg tcaaaaatgc aattggggtc 540
ctcatcggag gacttaaatg gaatgataac acagttagag tctctgaaac tctacagaga 600
ttcgcttgga gaagcagtca tgagaatggg agaccttcat tccctccaaa gcagaaacga 660
aaaatggcga gaacaattga gtcagaagtt tgaagaaata aggtggttga ttgaagaagt 720
gagacataga ttgaaaaata cagaaaatag ttttgaacaa ataacattta tgcaagcctt 780
acaactattg cttgaagtag aacaagagat aagaactttc tcgtttcagc ttatttaa 838

<210> 51
<211> 838
<212> DNA
<213> Influenza A virus

<400> 51

atggattcca acactgtgtc aagctttcag gtagactgtt ttctttggca tgtccgcaaa 60
cgatttgcag accaagaact gggtgatgcc ccattccttg accggcttcg ccgagaccag 120
aagtccctaa aaggaagagg cagcactctt ggtctggaca tcgaaacagc cactcgtgca 180
ggaaagcaga tagtggagcg gattctggaa gaggagtcag atgaggcact taaaatgacc 240
attgcctctg ttcctgcttc acgctactta actgacatga ctcttgatga gatgtcaaga 300
gactggttca tgctcatgcc caagcagaaa gtaacaggct ccctatgtat aaggatggac 360
caggcaatca tggataagaa catcatacta aaagcaaact ttagtgtgat tttcgaaagg 420
ctggagacac taatactact tagagccttc accgaagaag gagcagtcgt tggcgaaatt 480
tcaccattgc cttctcttcc aggacatact aatgaggatg tcaaaaatgc aattggggtc 540
ctcatcggag gacttaaatg gaatgataat acagttagag tctctgaaac tctacagaga 600
ttcgcttgga gaagcagtca tgagaatggg agaccttcat tccctccaaa gcagaaacga 660

aaaatggcga gaacaattga gccagaagtt tgaagaaata agatggttga ttgaagaagt 720
gcgacataga ttgaaaaata cagaaaatag ttttgaacaa ataacattta tgcaagcctt 780
acaactattg cttgaagtag aacaagagat aagaactttc tcgtttcagc ttatttaa 838